# EUROPEAN PATENT SPECIFICATION

(11) **EP 1 339 683 B1**
(45) Date of publication and mention of the grant of the patent: **14.06.2006**
(21) Application number: 01995915.4
(22) Date of filing: 21.11.2001
(51) Int. Cl.: C07D 209/56, C07D 235/04, C07D 249/18, A61K 31/404, A61P 5/30

(54) **ESTROGEN RECEPTOR MODULATORS**
ÖSTROGENREZEPTORMODULATOREN
MODULATEURS DE RECEPTEURS D'OESTROGENES

(30) Priority: 27.11.2000 US 253114 P
(43) Date of publication of application: 03.09.2003
(73) Proprietor: Merck & Co., Inc., Rahway, New Jersey 07065-0907 (US)
(72) Inventor: WILKENING, Robert, R., Rahway, NJ 07065-0907 (US); PARKER, Dann Leroy, Jr., Rahway, NJ 07065-0907 (US); WILDONGER, Kenneth, J., Rahway, NJ 07065-0907 (US); MENG, Dongfang, Rahway, NJ 07065-0907 (US); RATCLIFFE, Ronald, W., Rahway, NJ 07065-0907 (US)
(74) Representative: Horgan, James Michael Frederic
(86) International application number: PCT/US2001/044010
(87) International publication number: WO 2002/041835

(56) References cited:
- DATABASE CAPLUS [Online] MURATAKE ET AL.: 'Total synthesis of marine alkaloids-hapalindoles j and m', XP002953950 Retrieved from ACS Database accession no. 1990:21176 & TETRAHEDRON LETTERS vol. 30, no. 14, 1989,

## Description

### BACKGROUND OF THE INVENTION

Naturally occurring and synthetic estrogens have broad therapeutic utility, including: relief of menopausal symptoms, treatment of acne, treatment of dysmenorrhea and dysfunctional uterine bleeding, treatment of osteoporosis, treatment of hirsutism, treatment of prostatic cancer, treatment of hot flashes and prevention of cardiovascular disease. Because estrogen is very therapeutically valuable, there has been great interest in discovering compounds that mimic estrogen-like behavior in estrogen responsive tissues.

For example, estrogen-like compounds would be beneficial in the treatment and prevention of bone loss. Bone loss occurs in a wide range of subjects, including women that are post-menopausal or have had a hysterectomy, patients who were or are currently being treated with corticosteroids, and patient's having gonadal dysgenesis. The current major bone diseases of public concern are osteoporosis, hypercalcemia of malignancy, osteopenia due to bone metastases, periodontal disease, hyperparathyroidism, periarticular erosions in rheumatoid arthritis, Paget's disease, immobilization-induced osteopenia, and glucocorticoid-induced osteoporosis. All of these conditions are characterized by bone loss, resulting from an imbalance between bone resorption, i.e. breakdown, and bone formation, which continues throughout life at the rate of about 14% per year on the average. However, the rate of bone turnover differs from site to site, for example, it is higher in the trabecular bone of the vertebrae and the alveolar bone in the jaws than in the cortices of the long bones. The potential for bone loss is directly related to turnover and can amount to over 5% per year in vertebrae immediately following menopause, a condition which leads to increased fracture risk.

In the U.S., there are currently about 20 million people with detectable fractures of the vertebrae due to osteoporosis. In addition, there are about 250,000 hip fractures per year attributed to osteoporosis. This clinical situation is associated with a 12% mortality rate within the first two years, while 30% of the patients require nursing home care after the fracture.

Osteoporosis affects approximately 20 to 25 million post-menopausal women in the U.S. alone. It has been theorized that the rapid loss of bone mass in these women is due to the cessation of estrogen production of the ovaries. Since studies have shown that estrogen slows the reduction of bone mass due to osteoporosis, estrogen replacement therapy is a recognized treatment for post-menopausal osteoporosis.

In addition to bone mass, estrogen appears to have an effect on the biosynthesis of cholesterol and cardiovascular health. Statistically, the rate of occurrence of cardiovascular disease is roughly equal in postmenopausal women and men; however, premenopausal women have a much lower incidence of cardiovascular disease than men. Because postmenopausal women are estrogen deficient, it is believed that estrogen plays a beneficial role in preventing cardiovascular disease. The mechanism is not well understood, but evidence indicates that estrogen can upregulate the low density lipid (LDL) cholesterol receptors in the liver to remove excess cholesterol.

Postmenopausal women given estrogen replacement therapy experience a return of lipid levels to concentrations comparable to levels associated with the premenopausal state. Thus, estrogen replacement therapy could be an effective treatment for such disease. However, the side effects associated with long term estrogen use limit the use of this alternative.

Also, the estrogen receptor ligands of the present invention can have utility as an anti-depressant, especially when the depression results from an estrogen deficiency.

In models, estrogen has been shown to have beneficial effects on cognitive functioning, such as relieveing anxiety and depression and treating and/or preventing Alzheimer's disease. Estrogen affects the central nervous system by increasing cholinergic functioning, neurotrophin and neurotrophin receptor expression. Estrogen also increases glutamergic synaptic transmission, alters amyloid precursor protein processing and provides neuroprotection. Thus, the estrogen receptor modulators of the present invention could be beneficial for improving cognitive functioning.

Specifically, estrogen receptor beta (ERP) selective agonists would be useful in the treatment of anxiety and/or depressive illness, as either a single agent or in combination with other agents. Clinical studies have demonstrated the efficacy of the natural estrogen, 17β-estradiol for the treatment of various forms of depressive illness, See Schmidt PJ. Nieman L. Danaceau MA, Tobin MB, Roca CA, Murphy JH, Rubinow DR. Estrogen replacement in perimenopause-related depression: a preliminary report. Am *J Obstet Gynecol* **183**:414-20, 2000; and Soares CN, Almeida OP, Joffe H, Cohen LS.Efficacy of estradiol for the treatment of depressive disorders in perimenopausal women: a double-blind, randomized, placebo-controlled trial. *Arch Gen Psychiatry.* **58**:537-8, 2001; which are hereby incorporated by reference. Bethea et al (Lu NZ, Shlaes TA, Gundlah C, Dziennis SE, Lyle RE, Bethea CL. Ovarian steroid action on tryptophan hydroxylase protein and serotonin compared to localization of ovarian steroid receptors in midbrain of guinea pigs. *Endocrine* 11:257-67, 1999, which is hereby incorporated by reference) have suggested that the anti-depressant activity of estrogen may be mediated via regulation of serotonin synthesis in the serotonin containing cells concentrated in the dorsal raphe nucleus.

It is believed by some in the field that the physiological responses to estrogen are generally mediated via a series of biochemical events initiated by a selective, high affinity interaction between estrogen and an estrogen receptor. There are two estrogen receptors, ERα and ERβ, and there is co-localization of ERβ (and not ERα) in the serotonin containing cells of the rodent raphe nucleus. Using ERβ selective compounds, estrogen increases transcription of the tryptophan hydroxylase gene (TPH, the key enzyme in serotonin synthesis) via an ERβ mediated event. Potential ERβ selective agonists can be tested in a rodent model of depression by methods familiar to those skilled in the art, for example in a forced swim assay. Likewise, potential ERβ selective agonists can be tested in a rodent model of anxiety by methods familiar to those skilled in the art, for example a guinea pig pup vocalization assay and the resident intruder assay.

Other disease states that affect postmenopausal women include estrogen-dependent breast cancer and uterine cancer. Anti-estrogen compounds, such as tamoxifen, have commonly been used as chemotherapy to treat breast cancer patients. Tamoxifen, a dual antagonist and agonist of estrogen receptors, is beneficial in treating estrogen-dependent breast cancer. However, treatment with tamoxifen is less than ideal because tamoxifen's agonist behavior enhances its unwanted estrogenic side effects. For example, tamoxifen and other compounds that agonize estrogen receptors tend to increase cancer cell production in the uterus. A better therapy for such cancers would be an anti-estrogen compound that has negligible or nonexistent agonist properties.

Although estrogen can be beneficial for treating pathologies such as bone loss, increased lipid levels, and cancer, long-term estrogen therapy has been implicated in a variety of disorders, including an increase in the risk of uterine and endometrial cancers. These and other side effects of estrogen replacement therapy are not acceptable to many women, thus limiting its use.

Alternative regimens, such as a combined progestogen and estrogen dose, have been suggested in an attempt to lessen the risk of cancer. However, such regimens cause the patient to experience withdrawal bleeding, which is unacceptable to many older women. Furthermore, combining estrogen with progestogen reduces the beneficial cholesterol-lowering effect of estrogen therapy. In addition, the long term effects of progestogen treatment are unknown.

In addition to post-menopausal women, men suffering from prostatic cancer can also benefit from anti-estrogen compounds. Prostatic cancer is often endocrine-sensitive; androgen stimulation fosters tumor growth, while androgen suppression retards tumor growth. The administration of estrogen is helpful in the treatment and control of prostatic cancer because estrogen administration lowers the level of gonadotropin and, consequently, androgen levels.

The estrogen receptor has been found to have two forms: ERα and ERβ. Ligands bind differently to these two forms, and each form has a different tissue specificity to binding ligands. Thus, it is possible to have compounds that are selective for ERα or ERβ, and therefore confer a degree of tissue specificity to a particular ligand.

What is needed in the art are compounds that can produce the same positive responses as estrogen replacement therapy without the negative side effects. Also need are estrogen-like compounds that exert selective effects on different tissues of the body.

The compounds of the instant invention are ligands for estrogen receptors and as such may be useful for treatment or prevention of a variety of conditions related to estrogen functioning including: bone loss, bone fractures, osteoporosis, cartilage degeneration, endometriosis, uterine fibroid disease, hot flashes, increased levels of LDL cholesterol, cardiovascular disease, impairment of cognitive functioning, cerebral degenerative disorders, restinosis, gynacomastia, vascular smooth muscle cell proliferation, obesity, incontinence, depression resulting from an estrogen deficiency, and cancer, in particular of the breast, uterus and prostate.

### SUMMARY OF THE INVENTION

The present invention relates to compounds of the following chemical formula: 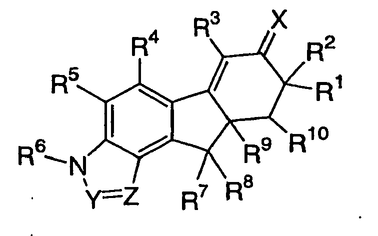 wherein
- X: is selected from the group consisting of: O, N-OR^{a}, N-NR^{a}R^{b} and C₁₋₆ alkylidene, wherein said alkylidene group is unsubstituted or substituted with a group selected from hydroxy, amino, O(C₁₋₄alkyl), NH(C₁₋₄alkyl), or N(C₁₋₄alkyl)₂;
- Y: is selected from the group consisting of N and CR^{e};
- Z: is selected from the group consisting of N and CR^{f};
- R¹: is selected from the group consisting of hydrogen, C₁₋₆alkyl, C₂₋₆alkenyl, and C₂₋₆alkynyl, wherein said alkyl, alkenyl and alkynyl groups are either unsubstituted or substituted with a group selected from OR^{c}, SR^{c}, NR^{b}R^{c}, C(=O)R^{c}, C(=O)CH₂OH, bromo, 1-3 chloro, 1-5 fluoro or phenyl, wherein said phenyl group can either be unsubstituted or substituted with a substituent selected from the group consisting of C₁₋₄alkyl, OH and O(C₁₋₄alkyl);
- R²: is selected from the group consisting of hydrogen, hydroxy, iodo, O(C=O)R^{c}, C(=O)R^{c}, CO₂R^{c}, C₁₋₆alkyl, C₂₋₆alkenyl, and C₂₋₆alkynyl, wherein said alkyl, alkenyl and alkynyl groups are either unsubstituted or substituted with a group selected from OR^{c}, SR^{c}, NR^{b}R^{c} ,C(=O)R^{c}, C(=O)CH₂OH, or phenyl, wherein said phenyl group can either be unsubstituted or substituted with a substituent selected from the group consisting of C₁₋₄alkyl, OH and O(C₁₋₄alkyl);
or R¹ and R², when taken together with the carbon atom to which they are attached, form a carbonyl group;
or R¹ and R², when taken together with the carbon atom to which they are attached, form a C₃₋₇ cycloalkyl or ₃₋₇ heterocycloalkyl ring,
wherein said ring is either unsubstituted or substituted with a group selected from C₁₋₄alkyl, OH, O(C₁₋₄ alkyl) and oxo;
or R¹ and R², when taken together, form a C₁₋₆ alkylidene group, wherein said alkylidene group is either unsubstituted or substituted with a group selected from the group consisting of hydroxy, O(C₁₋₄alkyl), N(C₁₋₄alkyl)₂, and phenyl, wherein said phenyl group can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)₂, halo,CN, NO₂, CO₂H, CO₂(C₁₋₄alkyl), C(O)H, and C(O)(C₁₋₄alkyl);
- R³: is selected from the group consisting of hydrogen, fluoro, chloro, bromo, iodo, cyano, nitro, NR^{a}R^{c}, OR^{a}, S(O)R^{a}, SO₂R^{a}, SR^{a}, C(=O)R^{a}, CO₂R^{c}, CONR^{a}R^{c}, C₁₋₁₀alkyl, C₂₋₁₀alkenyl, C₂₋₁₀alkynyl, C₃₋₇cycloalkyl, 4-7 membered heterocycloalkyl, cycloalkylalkyl, aryl, heteroaryl, arylalkyl, and heteroarylalkyl, wherein said alkyl, alkenyl, alkynyl, cycloalkyl, aryl and heteroaryl groups are either unsubstituted or independently substituted with 1,2 or 3 groups selected from fluoro, chloro, bromo, iodo, cyano, OR^{a}, NR^{a}R^{c}, O(C=O)R^{a}, O(C=O)NR^{a}R^{c}, NR^{a}(C=O)R^{c}, NR^{a}(C=O)OR^{c}, C(=O)R^{a}, CO₂R^{a}, CONR^{a}R^{c}, CSNR^{a}R^{c}, SR^{a}, S(O)R^{a}, SO₂R^{a}, SO₂NR^{a}R^{c}, LR^{d}, and MLR^{d} ;
- R⁴ and R⁵: are each independently selected from the group consisting of hydrogen, hydroxy, amino, methyl, CF₃, fluoro, chloro, and bromo;
- R⁶: is selected from the group consisting of hydrogen, (C=O)R^{a}, (C=O)OR^{a}, and SO₂R^{a};
- R⁷ and R⁸: are each independently selected from the group consisting of hydrogen, C₁₋₆alkyl, C₂₋₆alkenyl, C₂₋₆alkynyl, fluoro, chloro, bromo, cyano, hydroxy, O(C₁₋₆ alkyl), azido, amino, NH(C₁₋₄alkyl), and N(C₁₋₄alkyl)₂;
or R⁷ and R⁸, when taken together with the carbon atom to which they are attached, form a 3-5 membered cycloalkyl ring;
or R⁷ and R⁸, when taken together with the carbon atom to which they are attached, form a carbonyl group;
or R⁷ and R⁸, when taken together, form a C₁₋₆alkylidene group,
wherein said alkylidene group is either unsubstituted or substituted with a group selected from cyano, C(=O)H, C(=O)(C₁₋₄alkyl), or C(=O)OC₁₋₄alkyl,
- R⁹: is selected from the group consisting of hydrogen, C₁₋₁₀alkyl, C₂₋₁₀alkenyl, C₂₋₁₀alkynyl, C₃₋₆cycloalkyl, cycloalkylalkyl, aryl, heteroaryl, arylalkyl and heteroarylalkyl; wherein said alkyl, alkenyl, alkynyl, cycloalkyl, cycloalkylalkyl, aryl, heteroaryl, arylalkyl and heteroarylalkyl groups' can be optionally substituted with a group selected from bromo, iodo, OR^{b}, SR^{b}, C(=O)R^{b}, 1-3 chloro, or 1-5 fluoro;
or R⁹ and R¹, when taken together with the three intervening carbon atoms to which they are attached, form a 5-6 membered cycloalkyl or cycloalkenyl ring which can be optionally substituted with 1-3 groups independently selected from oxo, hydroxy, fluoro, C₁₋₆alkyl, C₂₋₆alkenyl, C₂₋₆alkynyl, C₁₋₆alkylidenyl, C₃₋₆cycloalkyl,
cycloalkylalkyl, phenyl, or phenylalkyl, wherein said alkyl, alkenyl, alkynyl, alkylidenyl, cycloalkyl, cycloalkylalkyl, phenyl, and phenylalkyl groups can be optionally substituted with a group selected from chloro, bromo, iodo, OR^{b}, SR^{b}, C₁₋₃alkyl, C(=O)R^{b}, or 1-5 fluoro;
or R⁹ and R⁸, when taken together with the two intervening carbon atoms to which they are attached, form a cyclopropyl ring which can be optionally substituted with 1-2 groups independently selected from C₁₋₆alkyl, C₂₋₆alkenyl, C₂₋₆alkynyl, C₃₋₆cycloalkyl, cycloalkylalkyl, phenyl, or phenylalkyl, wherein said alkyl, alkenyl, alkynyl; cycloalkyl, cycloalkylalkyl, phenyl and phenylalkyl groups can be optionally substituted with a group selected from chloro, bromo, iodo, OR^{b}, SR^{b}, C₁₋₃alkyl, C(=O)R^{b}, or 1-5 fluoro;
- R¹⁰: is selected from the group consisting of hydrogen; C₁₋₁₀alkyl, and C₂₋₁₀alkenyl;
- R^{a}: is selected from the group consisting of hydrogen, C₁₋₁₀alkyl, and phenyl,
wherein said alkyl group can be optionally substituted with a group selected from hydroxy, amino, O(C₁₋₄alkyl), NH(C₁₋₄alkyl), N(C₁₋₄alkyl)₂, phenyl, or 1-5 fluoro, and
wherein said phenyl groups can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄alkyl);
- R^{b}: is selected from the group consisting of hydrogen, C₁₋₁₀alkyl, benzyl and phenyl, wherein said phenyl group can either be unsubstituted or substituted with 1-3 substituents independently selected from the group consisting of C₁₋₄alkyl; OH, O(C₁₋₄alkyl), NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄alkyl);
- R^{c}: is selected from the group consisting of hydrogen, C₁₋₁₀alkyl and phenyl, wherein said phenyl group can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄ alkyl);
or R^{a} and R^{c}, whether or not on the same atom, can be taken together with any attached and intervening atoms to form a 4-7 membered ring;
- R^{d}: is selected from the group consisting of NR^{b}R^{c}, OR^{a}, CO₂R^{a}, O(C=O)R^{a}, CN, NR^{c}(C=O)R^{b}, CONR^{a}R^{c}, SO₂NR^{a}R^{c}, and a 4-7 membered N-heterocycloalkyl ring that can be optionally interrupted by O, S, NR^{c}, or C=O;
- R^{e}: is selected from the group consisting of hydrogen, C₁₋₆alkyl, C₂₋₆alkenyl, CF₃, halo, O(C₁₋₄alkyl), NH₂, NH(C₁₋₄alkyl), and N(C₁₋₄alkyl)₂;
- R^{f}: is selected from the group consisting of hydrogen, C₁₋₆alkyl, C₂₋₆alkenyl, CF₃, halo, O(C₁₋₄alkyl); NO₂, NH₂, NH(C₁₋₄alkyl), and N(C₁₋₄alkyl)₂;
- L: is selected from the group consisting of CR^{b}R^{c}, C₂₋₆alkylene and C₂₋₆ alkenylene, wherein said alkylene and alkenylerie groups can be optionally interrupted by O, S, or NR^{c};
- M is: selected from the group consisting of O, S, NR^{c}, C=O, O(C=O), (C=O)O, NR^{c}(C=O) or (C=O)NR^{c};
and the pharmaceutically acceptable salts thereof.

The present invention also relates to pharmaceutical compositions comprising the compounds of the present invention and a pharmaceutically acceptable carrier.

The present invention also relates to methods for making the pharmaceutical compositions of the present invention.

The present invention also relates to methods for eliciting an estrogen receptor modulating effect in a mammal in need thereof by administering the compounds and pharmaceutical compositions of the present invention.

The present invention also relates to methods for eliciting an estrogen receptor antagonizing effect in a mammal in need thereof by administering the compounds and pharmaceutical compositions of the present invention.

The present invention also relates to methods for eliciting an estrogen receptor agonizing effect in a mammal in need thereof by administering the compounds and pharmaceutical compositions of the present invention.

The present invention also relates to methods for treating or preventing disorders related to estrogen functioning, bone loss, bone fractures, osteoporosis, cartilage degeneration, endometriosis, uterine fibroid disease, cancer of the breast, uterus or prostate, hot flashes, cardiovascular disease, impairment of cognitive function, cerebral degenerative disorders, restenosis, gynacomastia, vascular smooth muscle cell proliferation, obesity and incontinence in a mammal in need thereof by administering the compounds and pharmaceutical compositions of the present invention.

The present invention also relates to methods for reducing bone loss, lowering LDL cholesterol levels and eliciting a vasodilatory effect, in a mammal in need thereof by administering the compounds and pharmaceutical compositions of the present invention.

### DETAILED DESCRIPTION OF THE INVENTION

The present invention relates to compounds useful as estrogen receptor modulators. Compounds of the present invention are described by the following chemical formula: 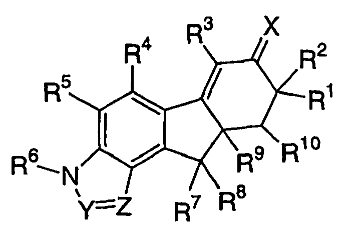 wherein
- X: is selected from the group consisting of: O, N-OR^{a}, N-NR^{a}R^{b} and C₁₋₆ alkylidene, wherein said alkylidene group is unsubstituted or substituted with a group selected from hydroxy, amino, O(C₁₋₄alkyl), NH(C₁₋₄alkyl), or N(C₁₋₄alkyl)₂;
- Y: is selected from the group consisting of N and CR^{e};
- Z: is selected from the group consisting of N and CR^{f};
- R¹: is selected from the group consisting of hydrogen, C₁₋₆alkyl, C₂₋₆alkenyl, and C₂₋₆alkynyl, wherein said alkyl, alkenyl and alkynyl groups are either unsubstituted or substituted with a group selected from OR^{c}, SR^{c}, NR^{b}R^{c}, C(=O)R^{c}, C(=O)CH₂OH, bromo, 1-3 chloro, 1-5 fluoro or phenyl, wherein said phenyl group can either be unsubstituted or substituted with a substituent selected from the group consisting of C₁₋₄alkyl, OH and O(C₁₋₄alkyl);
- R²: is selected from the group consisting of hydrogen, hydroxy, iodo, O(C=O)R^{c}, C(=O)R^{c}, CO₂R^{c}, C₁₋₆alkyl, C₂₋₆alkenyl, and C₂₋₆alkynyl, wherein said alkyl, alkenyl and alkynyl groups are either unsubstituted or substituted with a group selected from OR^{c}, SR^{c}, NR^{b}R^{c} ,C(=O)RC, C(=O)CH₂OH, or phenyl, wherein said phenyl group can either be unsubstituted or substituted with a substituent selected from the group consisting of C₁₋₄alkyl, OH and O(C₁₋₄alkyl);
or R¹ and R², when taken together with the carbon atom to which they are attached, form a carbonyl group;
or R¹ and R², when taken together with the carbon atom to which they are attached, form a C₃₋₇ cycloalkyl or ₃₋₇ heterocycloalkyl ring,
wherein said ring is either unsubstituted or substituted with a group selected from C₁₋₄ alkyl, OH, O(C₁₋₄ alkyl) and oxo;
or R¹ and R², when taken together, form a C₁₋₆ alkylidene group,
wherein said alkylidene group is either unsubstituted or substituted with a group selected-from the group consisting of hydroxy, O(C₁₋₄alkyl), N(C₁₋₄alkyl)₂; amd phenyl, wherein said phenyl group can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄alkyl);
- R³: is selected from the group consisting of hydrogen, fluoro, chloro, bromo, iodo, cyano, nitro, NR^{a}R^{c}, OR^{a}, (O)R^{a}; SO₂R^{a}, SR^{a}, C(=O)R^{a}, CO₂R^{c}, CONR^{a}R^{c}, C₁₋₁₀alkyl, C₂₋₁₀alkenyl, C₂₋₁₀alkynyl, C₃₋₇cycloalkyl, 4-7 membered heterocycloalkyl, cycloalkylalkyl, aryl, heteroaryl, arylalkyl, and heteroarylalkyl, wherein said alkyl, alkenyl, alkynyl, cycloalkyl, aryl and heteroaryl groups are either unsubstituted or independently substituted with 1, 2 or 3 groups selected from fluoro, chloro, bromo, iodo; cyano, OR^{a}, NR^{a}R^{c}, O(C=O)R^{a}, O(C=O)NR^{a}R^{c}, NR^{a}(C=O)R^{c}, NR^{a}(C=O)OR^{c}, C(=O)R^{a}, CO₂R^{a}, CONR^{a}R^{c}, CSNR^{a}R^{c}, SR^{a}, S(O)R^{a}, SO₂R^{a}, SO₂NR^{a}R^{c}, LR^{d}, and MLR^{d} ;
- R⁴: and R⁵ are each independently selected from the group consisting of hydrogen, hydroxy, amino, methyl, CF₃, fluoro, chloro, and bromo;
- R⁶: is selected from the group consisting of hydrogen, (C=O)R^{a}, (C=O)OR^{a}, and SO₂R^{a};
- R⁷: and R⁸ are each independently selected from the group consisting of hydrogen, C₁₋₆alkyl, C₂₋₆alkenyl, C₂₋₆alkynyl, fluoro, chloro, bromo, cyano, hydroxy, O(C₁₋₆ alkyl), azido, amino, NH(C₁₋₄alkyl), and N(C₁₋₄ alkyl)₂;
or R⁷ and R⁸, when taken together with the carbon atom to which they are attached, form a 3-5 membered cycloalkyl ring;
or R⁷ and R⁸, when taken together with the carbon atom to which they are attached, form a carbonyl group;
or R⁷ and R⁸, when taken together, form a C₁₋₆alkylidene group,
wherein said alkylidene group is either unsubstituted or substituted with a group selected from cyano, C(=O)H, C(=O)(C₁₋₄alkyl), or C(=O)OC₁₋₄alkyl;
- R⁹: is selected from the group consisting of hydrogen, C₁₋₁₀alkyl, C₂₋₁₀alkenyl, C₂₋₁₀ alkynyl, C₃₋₆cycloalkyl, cycloalkylalkyl, aryl, heteroaryl, arylalkyl and heteroarylalkyl, wherein said alkyl, alkenyl, alkynyl, cycloalkyl, cycloalkylalkyl, aryl, heteroaryl, arylalkyl and heteroarylalkyl groups can be optionally substituted with a group selected from bromo, iodo, OR^{b}, SR^{b}, C(=O)R^{b}, 1-3 chloro, or 1-5 fluoro;
or R9 and R¹, when taken together with the three intervening carbon atoms to which they are attached, form a 5-6 membered cycloalkyl or cycloalkenyl ring which can be optionally substituted with 1-3 groups independently selected from oxo, hydroxy, fluoro, C₁₋₆alkyl, C₂₋₆alkenyl, C₂-6alkynyl, C₁₋₆alkylidenyl, C₃₋₆cycloalkyl, cycloalkylalkyl, phenyl, or phenylalkyl, wherein said alkyl, alkenyl, alkynyl, alkylidenyl, cycloalkyl, cycloalkylalkyl, phenyl, and phenylalkyl groups can be optionally substituted with a group selected from chloro, bromo, iodo, OR^{b}, SR^{b}, C₁₋₃alkyl, C(=O)R^{b}, or 1-5 fluoro;
or R⁹ and R⁸, when taken together with the two intervening carbon atoms to which they are attached, form a cyclopropyl ring which can be optionally substituted with 1-2 groups independently selected from C₁₋₆alkyl, C₂₋₆alkenyl, C₂₋₆alkynyl, C₃₋₆cycloalkyl, cyaoalkcylalkyl, phenyl, or phenylalkyl, wherein said alkyl, alkenyl, alkynyl, cycloalkyl, cycloalkylalkyl, phenyl, and phenylalkyl groups can be optionally substituted with a group selected from chloro, bromo, iodo, OR^{b}, SR^{b}, C₁₋₃alkyl, C(=O)R^{b}, or 1-5 fluoro;
- R¹⁰: is selected from the group consisting of hydrogen, C₁₋₁₀alkyl, and C₂₋₁₀alkenyl;
- R^{a}: is selected from the group consisting of hydrogen, C₁₋₁₀alkyl, and phenyl,
wherein said alkyl group can be optionally substituted with a group selected from hydroxy, amino, O(C₁₋₄alkyl), NH(C₁₋₄alkyl), N(C₁₋₄ alkyl)₂, phenyl, or 1-5 fluoro, and
wherein said phenyl groups can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄alkyl);
- R^{b}: is selected from the group consisting of hydrogen, C₁₋₁₀alkyl, benzyl and phenyl,
wherein said phenyl group can either be unsubstituted or substituted with 1-3 substituents independently selected from the group consisting of C₁₋₄alkyl,OH,O(C₁₋₄alkyl),NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄alkyl);
- R^{c}: is selected from the group consisting of hydrogen, C₁₋₁₀alkyl and phenyl, wherein said phenyl group can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)2, halo, CN, NO2, CO₂H, CO₂(C₁₋₄alkyl), C(O)H, and C(O)(C₁₋₄alkyl);
or R^{a} and R^{c}, whether or not on the same atom, can be taken together with any attached and intervening atoms to form a 4-7 membered ring;
- R^{d}: is selected from the group consisting of NR^{b}R^{c}, OR^{a}, CO₂R^{a}, O(C=O)R^{a}, CN, NR^{c}(C=O)R^{b}, CONR^{a}R^{c}, SO₂NR^{a}R^{c}, and a 4-7 membered N-heterocycloalkyl ring that can be optionally interrupted by O, S, NR^{c}, or C=O;
- R^{e}: is selected from the group consisting of hydrogen, C₁₋₆alkyl, C₂₋₆alkenyl, CF₃, halo, O(C₁₋₄alkyl), NH₂, NH(C₁₋₄alkyl), and N(C₁₋₄alkyl)₂;
- R^{f}: is selected from the group consisting of hydrogen, C₁₋₆alkyl, C₂₋₆alkenyl, CF₃, halo, (C₁₋₄alkyl), NO₂, NH₂, NH(C₁₋₄alkyl), and N(C₁₋₄alkyl)₂;
- L: is selected from the group consisting of CR^{b}R^{c}, C₂₋₆ alkylene and C₂₋₆ alkenylene, wherein said alkylene and alkenylene groups can be Optionally interrupted by O, S, or NR^{c};
- M: is selected from the group consisting of O, S, NR^{c}, C=O, O(C=O), (C=O)O, NR^{c}(C=O) or (C=O)NR^{c};
and the pharmaceutically acceptable salts thereof.

In a class of the invention, X is selected from O and N-OR^{a}. In a subclass of the invention, X is selected from O, N-OH and N-OCH₃. In a further subclass of the invention, X is O.

In a class of the invention, Y is selected from N and CH.

In a class of the invention, Z is selected from N and CR^{f}. In a subclass of the invention, Z is selected from N, CH, CF and CCl. In a further subclass of the invention, Z is selected from N and CH.

In a class of the invention, R¹ is selected from hydrogen, C₁₋₆alkyl, C₂₋₆alkenyl, and C₂₋₆alkynyl, wherein said alkyl, alkenyl and alkynyl groups are either unsubstituted or substituted with a group selected from OR^{c} or C(=O)R^{c}. In a subclass of the invention, R¹ is selected from hydrogen and C₁₋₃alkyl.

In a class of the invention, R² is selected from hydrogen, hydroxy, iodo, C₁₋₆alkyl, C₂₋₆alkenyl, and C₂₋₆alkynyl, wherein said alkyl, alkenyl and alkynyl groups are either unsubstituted or substituted with a group selected from OR^{c} or C(=O)R^{c}. In a subclass of the invention, R² is selected from hydrogen, hydroxy, iodo and C₁₋₃alkyl.

In a class of the invention, R³ is selected from hydrogen, chloro, bromo, iodo, C₁₋₁₀alkyl, C₂₋₁₀alkenyl, C₃₋₇cycloalkyl, aryl and heteroaryl, wherein said alkyl, alkenyl, cycloalkyl, aryl and heteroaryl groups are either unsubstituted or independently substituted with 1, 2 or 3 groups selected from fluoro, chloro, bromo, cyano, OR^{a}, NR^{a}R^{c}, C(=O)R^{a}, CO₂R^{a}, CONR^{a}R^{c}, SR^{a}, NR^{a}(C=O)R^{c}, LR^{d}, and MLR^{d}. In a subclass of the invention, R³ is selected from hydrogen, chloro, bromo, iodo, C₁₋₁₀alkyl, C₂₋₁₀alkenyl, C₃₋₇cycloalkyl, and aryl, wherein said alkyl, alkenyl, cycloalkyl, and aryl groups are either unsubstituted or independently substituted with 1, 2 or 3 groups selected from fluoro, chloro, bromo, cyano, OR^{a}, NR^{a}R^{c}, LR^{d}, and MLR^{d}.

In a class of the invention, R⁴ is selected from hydrogen, hydroxy, methyl, fluoro, and chloro. In a subclass of the invention, R⁴ is selected from hydrogen, methyl and fluoro.

In a class of the invention, R⁵ is selected from hydrogen, hydroxy, fluoro and chloro. In a subclass of the invention, R⁵ is selected from hydrogen and fluoro.

In a class of the invention, R⁶ is selected from hydrogen, (C=O)R^{a} and C(=O)OR^{a}. In a subclass of the invention, R⁶ is selected from hydrogen and C(=O)OR^{a}.

In a class of the invention, R⁷ and R⁸ are each independently selected from hydrogen and C₁₋₆alkyl, or R⁷ and R⁸, when taken together with the carbon atom to which they are attached, form a carbonyl group. In a subclass of the invention, R⁷ and R⁸ are each independently selected from hydrogen and C₁₋₆alkyl.

In a class of the invention, R⁹ is selected from hydrogen, C₁₋₁₀alkyl, C₂₋₁₀alkenyl, C₃₋₆cycloalkyl and cycloalkylalkyl, wherein said alkyl, alkenyl, cycloalkyl and cycloalkylalkyl groups can be optionally substituted with a group selected from chloro, OR^{b}, SR^{b} or 1-5 fluoro. In a subclass of the invention, R⁹ is selected from C₁₋₁₀alkyl, C₂₋₁₀alkenyl, C₃₋₆cycloalkyl and cycloalkylalkyl.

In a class of the invention, R⁹ and R¹, when taken together with the three intervening carbon atoms to which they are attached, form a 5-6 membered cycloalkyl ring which can be optionally substituted with a group selected from C₁₋₆alkyl, C₂₋₆alkenyl, and C₃₋₆cycloalkylalkyl, wherein said alkyl, alkenyl, and cycloalkylalkyl groups can be optionally substituted with a group selected from chloro, OR^{b}, SR^{b} or 1-5 fluoro.

In a class of the invention, R¹⁰ is selected from hydrogen and C₁₋₁₀alkyl. In a subclass of the invention, R¹⁰ is hydrogen.

An embodiment of the invention is a method of eliciting an estrogen receptor modulating effect in a mammal in need thereof, comprising administering to the mammal a therapeutically effective amount of any of the compounds or any of the above pharmaceutical compositions described above.

A class of the embodiment is the method wherein the estrogen receptor modulating effect is an antagonizing effect.

A subclass of the embodiment is the method wherein the estrogen receptor is an ERα receptor.

A second subclass of the embodiment is the method wherein the estrogen receptor is an ERβ receptor.

A third subclass of the embodiment is the method wherein the estrogen receptor modulating effect is a mixed ERα and ERβ receptor antagonizing effect.

A second class of the embodiment is the method wherein the estrogen receptor modulating effect is an agonizing effect.

A subclass of the embodiment is the method wherein the estrogen receptor is an ERα receptor.

A second subclass of the embodiment is the method wherein the estrogen receptor is an ERβ receptor.

A third subclass of the embodiment is the method wherein the estrogen receptor modulating effect is a mixed ERα and ERβ receptor agonizing effect.

A third class of the embodiment is the method wherein the ERα receptor modulating effect is an agonizing and antagonizing effect.

A fourth class of the embodiment is the method wherein the ERβ receptor modulating effect is an agonizing and antagonizing effect.

A fifth class of the embodiment is the method wherein the estrogen receptor modulating effect is a mixed ERα and ERβ receptor agonizing and antagonizing effect.

Another embodiment of the invention is a method of treating or preventing hot flashes in a mammal in need thereof by administering to the mammal a therapeutically effective amount of any of the compounds or pharmaceutical compositions described above.

Another embodiment of the invention is a method of treating or preventing anxiety in a mammal in need thereof by administering to the mammal a therapeutically effective amount of any of the compounds or pharmaceutical compositions described above.

Another embodiment of the invention is a method of treating or preventing depression in a mammal in need thereof by administering to the mammal a therapeutically effective amount of any of the compounds or pharmaceutical compositions described above.

Another embodiment of the invention is a method of treating or preventing anxiety in a mammal in need thereof by administering to the mammal a therapeutically effective amount of an estrogen receptor beta selective agonist.

Another embodiment of the invention is a method of treating or preventing depression in a mammal in need thereof by administering to the mammal a therapeutically effective amount of an estrogen receptor beta selective agonist.

Exemplifying the invention is a pharmaceutical composition comprising any of the compounds described above and a pharmaceutically acceptable carrier. Also exemplifying the invention is a pharmaceutical composition made by combining any of the compounds described above and a pharmaceutically acceptable carrier. An illustration of the invention is a process for making a pharmaceutical composition comprising combining any of the compounds described above and a pharmaceutically acceptable carrier.

Also exemplifying the invention is a pharmaceutical composition comprising an estrogen receptor beta selective agonist and a pharmaceutically acceptable carrier. Also exemplifying the invention is a pharmaceutical composition made by combining an estrogen receptor beta selective agonist and a pharmaceutically acceptable carrier. An illustration of the invention is a process for making a pharmaceutical composition comprising combining an estrogen receptor beta selective agonist and a pharmaceutically acceptable carrier.

Further exemplifying the invention is the use of any of the compounds described above in the preparation of a medicament for the treatment and/or prevention of osteoporosis in a mammal in need thereof. Still further exemplifying the invention is the use of any of the compounds described above in the preparation of a medicament for the treatment and/or prevention of: bone loss, bone resorption, bone fractures, cartilage degeneration, endometriosis, uterine fibroid disease, breast cancer, uterine cancer, prostate cancer, hot flashes, cardiovascular disease, impairment of congnitive functioning, cerebral degenerative disorder, postmenopausal depression, postpartum depression, verbal memory loss in ovarectomized women, Alzheimer disease, anxiety, restenosis, vascular smooth muscle cell proliferation, incontinence, and/or disorders related to estrogen functioning.

The present invention is also directed to combinations of any of the compounds or any of the pharmaceutical compositions described above with one or more agents useful in the prevention or treatment of osteoporosis. For example, the compounds of the instant invention may be effectively administered in combination with effective amounts of other agents such as an organic bisphosphonate or a cathepsin K inhibitor. Nonlimiting examples of said organic bisphosphonates include alendronate, clodronate, etidronate, ibandronate, incadronate, minodronate, neridronate, risedronate, piridronate, pamidronate, tiludronate, zoledronate, pharmaceutically acceptable salts or esters thereof, and mixtures thereof. Preferred organic bisphosphonates include alendronate and pharmaceutically acceptable salts and mixtures thereof. Most preferred is alendronate monosodium trihydrate.

The precise dosage of the bisphosphonate will vary with the dosing schedule, the oral potency of the particular bisphosphonate chosen, the age, size, sex and condition of the mammal or human, the nature and severity of the disorder to be treated, and other relevant medical and physical factors. Thus, a precise pharmaceutically effective amount cannot be specified in advance and can be readily determined by the caregiver or clinician. Appropriate amounts can be determined by routine experimentation from animal models and human clinical studies. Generally, an appropriate amount of bisphosphonate is chosen to obtain a bone resorption inhibiting effect, i.e. a bone resorption inhibiting amount of the bisphosphonate is administered. For humans, an effective oral dose of bisphosphonate is typically from about 1.5 to about 6000 µg/kg body weight and preferably about 10 to about 2000 µg/kg of body weight.

For human oral compositions comprising alendronate, pharmaceutically acceptable salts thereof, or pharmaceutically acceptable derivatives thereof, a unit dosage typically comprises from about 8.75 mg to about 140 mg of the alendronate compound, on an alendronic acid active weight basis, i.e. on the basis of the corresponding acid.

For use in medicine, the salts of the compounds of this invention refer to non-toxic "pharmaceutically acceptable salts." Other salts may, however, be useful in the preparation of the compounds according to the invention or of their pharmaceutically acceptable salts. When the compounds of the present invention contain a basic group, salts encompassed within the term "pharmaceutically acceptable salts" refer to non-toxic salts which are generally prepared by reacting the free base with a suitable organic or inorganic acid. Representative salts include the following: acetate, benzenesulfonate, benzoate, bicarbonate, bisulfate, bitartrate, borate, bromide, calcium, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, oxalate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

The compounds of the present invention can have chiral centers and occur as racemates, racemic mixtures, diastereomeric mixtures, and as individual diastereomers, or enantiomers with all isomeric forms being included in the present invention. Therefore, where a compound is chiral, the separate enantiomers, substantially free of the other, are included within the scope of the invention; further included are all mixtures of the two enantiomers. Also included within the scope of the invention are polymorphs, hydrates and solvates of the compounds of the instant invention.

The present invention includes within its scope prodrugs of the compounds of this invention. In general, such prodrugs will be functional derivatives of the compounds of this invention which are readily convertible *in vivo* into the required compound. Thus, in the methods of treatment of the present invention, the term "administering" shall encompass the treatment of the various conditions described with the compound specifically disclosed or with a compound which may not be specifically disclosed, but which converts to the specified compound *in vivo* after administration to the patient. Conventional procedures for the selection and preparation of suitable prodrug derivatives are described, for example, in "Design of Prodrugs," ed. H. Bundgaard, Elsevier, 1985, which is incorporated by reference herein in its entirety. Metabolites of these compounds include active species produced upon introduction of compounds of this invention into the biological milieu.

The term "therapeutically effective amount" shall mean that amount of a drug or pharmaceutical agent that will elicit the biological or medical response of a tissue, system, animal or human that is being sought by a researcher or clinician.

The term "bone resorption," as used herein, refers to the process by which osteoclasts degrade bone.

The term "alkyl" shall mean a substituting univalent group derived by conceptual removal of one hydrogen atom from a straight or branched-chain acyclic saturated hydrocarbon (i.e., -CH₃, -CH₂CH₃, -CH₂CH₂CH₃, -CH(CH₃)₂, -CH₂CH₂CH₂CH₃, -CH₂CH(CH₃)₂, -C(CH₃)₃, etc).

The term "alkenyl" shall mean a substituting univalent group derived by conceptual removal of one hydrogen atom from a straight or branched-chain acyclic unsaturated hydrocarbon containing at least one double bond (i.e., -CH=CH₂, -CH₂CH=CH₂, -CH=CHCH₃, -CH₂CH=C(CH₃)₂, etc.).

The term "alkynyl" shall mean a substituting univalent group derived by conceptual removal of one hydrogen atom from a straight or branched-chain acyclic unsaturated hydrocarbon containing at least one triple bond (i.e., -C≡CH, -CH₂C≡CH, -C≡CCH₃, -CH₂C≡CCH₂(CH₃)₂, etc.).

The term "alkylene" shall mean a substituting bivalent group derived from a straight or branched-chain acyclic saturated hydrocarbon by conceptual removal of two hydrogen atoms from different carbon atoms (i.e., -CH₂CH₂-, -CH₂CH₂CH₂CH₂-, -CH₂C(CH₃)₂CH₂-, etc.).

The term "alkylidene" shall mean a substituting bivalent group derived from a straight or branched-chain acyclic saturated hydrocarbon by conceptual removal of two hydrogen atoms from the same carbon atom (i.e., =CH₂, =CHCH₃, =C(CH₃)₂, etc.).

The term "alkenylene" shall mean a substituting bivalent group derived from a straight or branched-chain acyclic unsaturated hydrocarbon by conceptual removal of two hydrogen atoms from different carbon atoms (i.e., -CH=CH-, -CH₂CH=CH-, CH₂CH=CHCH₂-, -C(CH₃)=C(CH₃)-, etc.).

The term "cycloalkyl" shall mean a substituting univalent group derived by conceptual removal of one hydrogen atom from a saturated monocyclic hydrocarbon (i.e., cyclopropyl, cyclobutyl, cyclopentyl, cyclohexyl, or cycloheptyl).

The term "cycloalkenyl" shall mean a substituting univalent group derived by conceptual removal of one hydrogen atom from an unsaturated monocyclic hydrocarbon containing a double bond (i.e., cyclopentenyl or cyclohexenyl).

The term "heterocycloalkyl" shall mean a substituting univalent group derived by conceptual removal of one hydrogen atom from a heterocycloalkane wherein said heterocycloalkane is derived from the corresponding saturated monocyclic hydrocarbon by replacing one or two carbon atoms with atoms selected from N, O or S. Examples of heterocycloalkyl groups include, but are not limited to, oxiranyl, azetidinyl, pyrrolidinyl, piperidinyl, piperazinyl, and morpholinyl. Heterocycloalkyl substituents can be attached at a carbon atom. If the substituent is a nitrogen containing heterocycloalkyl substituent, it can be attached at the nitrogen atom.

The term "aryl" as used herein refers to a substituting univalent group derived by conceptual removal of one hydrogen atom from a monocyclic or bicyclic aromatic hydrocarbon. Examples of aryl groups are phenyl, indenyl, and naphthyl.

The term "heteroaryl" as used herein refers to a substituting univalent group derived by the conceptual removal of one hydrogen atom from a monocyclic or bicyclic aromatic ring system containing 1, 2, 3, or 4 heteroatoms selected from N, O, or S. Examples of heteroaryl groups include, but are not limited to, pyrrolyl, furyl, thienyl, imidazolyl, pyrazolyl, oxazolyl, isoxazolyl, thiazolyl, pyridyl, pyrimidinyl, pyrazinyl, benzimidazolyl, indolyl, and purinyl. Heteraryl substituents can be attached at a carbon atom or through the heteroatom.

In the compounds of the present invention, alkyl, alkenyl, alkynyl, alkylidene, alkenylene, cycloalkyl, cycloalkenyl, heterocycloalkyl, aryl and heteroaryl groups can be further substituted by replacing one or more hydrogen atoms be alternative non-hydrogen groups. These include, but are not limited to, halo, hydroxy, mercapto, amino, carboxy, cyano and carbamoyl.

Whenever the term "alkyl" or "aryl" or either of their prefix roots appear in a name of a substituent (e.g., aryl C₁₋₈ alkyl) it shall be interpreted as including those limitations given above for "alkyl" and "aryl." Designated numbers of carbon atoms (e.g., C₁₋₁₀) shall refer independently to the number of carbon atoms in an alkyl or cyclic alkyl moiety or to the alkyl portion of a larger substituent in which alkyl appears as its prefix root.

The terms "arylalkyl" and "alkylaryl" include an alkyl portion where alkyl is as defined above and to include an aryl portion where aryl is as defined above. Examples of arylalkyl include, but are not limited to, benzyl, fluorobenzyl, chlorobenzyl, phenylethyl, phenylpropyl, fluorophenylethyl, and chlorophenylethyl. Examples of alkylaryl include, but are not limited to, toluyl, ethylphenyl, and propylphenyl.

The term "heteroarylalkyl," as used herein, shall refer to a system that includes a heteroaryl portion, where heteroaryl is as defined above, and contains an alkyl portion. Examples of heteroarylalkyl include, but are not limited to, thienylmethyl, thienylethyl, thienylpropyl, pyridylmethyl, pyridylethyl and imidazoylmethyl.

The term "cycloalkylalkyl," as used herein, shall refer to a system that includes a 3- to 8-membered fully saturated cyclic ring portion and also includes an alkyl portion, wherein cycloalkyl and alkyl are as defined above.

In the compounds of the present invention, R¹ and R² can be taken together with the carbon atom to which they are attached to form a 3-6 membered ring.

In the compounds of the present invention, R^{a} and R^{b} can be taken together with any of the atoms to which they may be attached or are between them to form a 4-6 membered ring system.

The term "halo" shall include iodo, bromo, chloro and fluoro.

The term "oxy" means an oxygen (O) atom. The term "thio" means a sulfur (S) atom. The term "oxo" means =O. The term "oximino" means the =N-O group.

The term "substituted" shall be deemed to include multiple degrees of substitution by a named substitutent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

Under standard nonmenclature used throughout this disclosure, the terminal portion of the designated side chain is described first, followed by the adjacent functionality toward the point of attachment. For example, a C₁₋₅ alkylcarbonylamino C₁₋₆ alkyl substituent is equivalent to

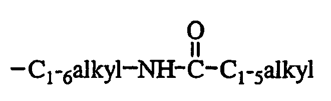

In choosing compounds of the present invention, one of ordinary skill in the art will recognize that the various substituents, i.e. R¹, R², R³, R⁴, R⁵, R⁶, R⁷, R⁸, R⁹, R¹⁰, R^{a}, R^{b}, R^{c}, R^{d}, R^{e}, R^{f}, LR^{d}, and MLYR^{d} are to be chosen in conformity with well-known principles of chemical structure connectivity.

Representative compounds of the present invention typically display submicromolar affinity for alpha and/or beta estrogen receptors. Compounds of this invention are therefore useful in treating mammals suffering from disorders related to estrogen functioning. Pharmacologically effective amounts of the compound, including the pharmaceutically effective salts thereof, are administered to the mammal, to treat disorders related to estrogen functioning, such as bone loss, hot flashes and cardiovascular disease.

The compounds of the present invention are available in racemic form or as individual enantiomers. For convenience, some structures are graphically represented as a single enantiomer but, unless otherwise indicated, are meant to include both racemic and enantiomeric forms.

It is generally preferable to administer compounds of the present invention as enantiomerically pure formulations since most of all of the desired bioactivity resides with a single enantiomer. Racemic mixtures can be separated into their individual enantiomers by any of a number of conventional methods. These include chiral chromatography, derivatization with a chiral auxillary followed by separation by chromatography or crystallization, and fractional crystallization of diastereomeric salts.

When the fused five-membered ring contains two or three nitrogen atoms, tautomeric (R⁶ is hydrogen) and positional (R⁶ is a non-hydrogen group) isomers are possible. These isomeric forms, as shown below, are contemplated to fall within the scope of the present invention:

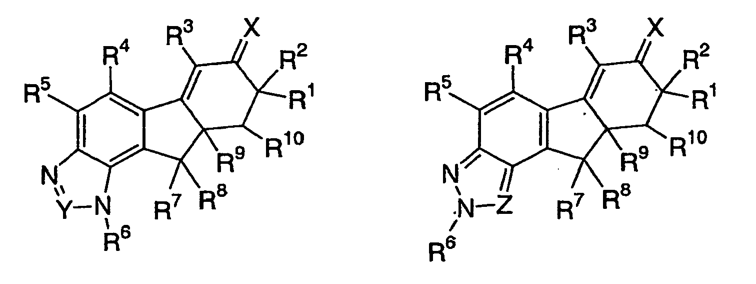

The compounds of the present invention can be used in combination with other agents useful for treating estrogen-mediated conditions. The individual components of such combinations can be administered separately at different times during the course of therapy or concurrently in divided or single combination forms. The instant invention is therefore to be understood as embracing all such regimes of simultaneous or alternating treatment and the term "administering" is to be interpreted accordingly. It will be understood that the scope of combinations of the compounds of this invention with other agents useful for treating estrogen-mediated conditions includes in principle any combination with any pharmaceutical composition useful for treating disorders related to estrogen functioning.

As used herein, the term "composition" is intended to encompass a product comprising the specified ingredients in the specified amounts, as well as any product which results, directly or indirectly, from combination of the specified ingredients in the specified amounts.

The compounds of the present invention can be administrered in such oral dosage forms as tablets, capsules (each of which includes sustained release or timed release formulations), pills, powders, granules, elixers, tinctures, suspensions, syrups and emulsions. Likewise, they may also be administered in intravenous (bolus or infusion), intraperitoneal, topical (e.g., ocular eyedrop), subcutaneous, intramuscular or transdermal (e.g., patch) form, all using forms well known to those of ordinary skill in the pharmaceutical arts.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 to 10 mg/kg/day, and most preferably 0.1 to 5.0 mg/kg/day. For oral administration, the compositions are preferably provided in the form of tablets containing 0.01, 0.05, 0.1, 0.5, 1.0, 2.5, 5.0, 10.0, 15.0, 25.0, 50.0, 100 and 500 milligrams of the active ingredient for the symptomatic adjustment of the dosage to the patient to be treated. A medicament typically contains from about 0.01 mg to about 500 mg of the active ingredient, preferably, from about 1 mg to about 100 mg of active ingredient. Intravenously, the most preferred doses will range from about 0.1 to about 10 mg/kg/minute during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittant throughout the dosage regimen.

In the methods of the present invention, the compounds herein described in detail can form the active ingredient, and are typically administered in admixture with suitable pharmaceutical diluents, excipients or carriers (collectively referred to herein as 'carrier' materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamide-phenol, polyhydroxy-ethylaspartamide-phenol, or polyethyleneoxide-polylysine substituted with palmitoyl residues. Furthermore, the compounds of the present invention may be coupled to a class of biodegradable polymers useful in achieving controlled release of a drug, for example, polylactic acid, polyglycolic acid, copolymers of polyactic and polyglycolic acid, polyepsilon caprolactone, polyhydroxy butyric acid, polyorthoesters, polyacetals, polydihydropyrans, polycyanoacrylates and crosslinked or amphipathic block copolymers of hydrogels.

The novel compounds of the present invention can be prepared according to the procedures of the following schemes and examples, using appropriate materials, and are further exemplified by the following specific examples. The compounds illustrated in the examples are not, however, to be construed as forming the only genus that is considered as the invention. The following examples further illustrate details for the preparation of the compounds of the present invention. Those skilled in the art will readily understand that known variations of the conditions and processes of the following preparative procedures can be used to prepare these compounds. All temperatures are degrees Celsius unless otherwise noted.

The compounds of the present invention are prepared according to the general methods outlined in Schemes I-XIII. In these schemes, R^{*I*} represents one or two of R⁴ and R⁵, or precursors thereof; CHR^{*II*}R^{*III*} and CR^{*III*}*=*CR^{*IIa*}R^{*IIb*} represent non-hydrogen values of R⁹, or precursors thereof; R^{*IV*} represents R³ or a precursor thereof; R^{*IVa*} and R^{*IVb*} represent non-hydrogen values of R³, or precursors thereof; R^{*Va*}*,* R^{*Vb*} and CH(OH)R^{*Vc*} represent non-hydrogen values of R¹ and R², or precursors thereof; R^{*VIa*}*,* R^{*VIb*} and R^{*VIc*} represents non-hydrogen values of R⁷ and R⁸, or precursors thereof; R^{*VII*} represents OR^{a} and NR^{a}R^{b}; R^{*VIII*} represents hydrogen or a C₁₋₅ alkyl group; R^{*O*} represents an acyl group such as acetyl or the like; and R^{*P*} represents a *N*-protecting group for an indole, indazole, benzimidazole, or benzotriazole group. Other R groups are defined in the schemes in which they appear.

The four classes of tetracyclic compounds described herein are prepared from 5-(acylamino)-1-indanone intermediates, which are either known compounds or are prepared by the methods outlined in Schemes I and II. An example of a particularly useful and known indanone of this type is 5-(acetylamino)-1-indanone.

In step 1 of Scheme I, an (acylamino)benzene (1) reacts with an acid halide-Lewis acid combination to afford the ketone (2). Condensation of (2) with an aldehyde or aldehyde surrogate (step 2) provides the enone (3) which is cyclized in the presence of acid (step 3) to provide the indanone (4). Alternatively, a 3-nitrobenzoic acid of type (5a) is converted in several steps to a 3-[3-(acylamino)phenyl]-propionic acid (6) which undergoes Lewis acid mediated cyclization (step 4) to the indanone (7). In yet another approach, a 4-(acylamino)benzoic acid (5b) or its corresponding methyl ketone is converted to the indanone (7) in a sequence of steps analogous to the preparation of (4) from (2). Indanones (7) react with aldehydes or ketones under basic conditions (step 5) to afford the 2-alkylidene-1-indanones (8). Reduction of the double bond (step 6) affords the indanones (4). Steps 5 and 6 of Scheme I are conveniently combined to provide (4) directly from (7). Alternatively, 2-substituted indanones (4) are obtained by reacting (7) with a suitable alkylating agent in the presence of a base (step 7). 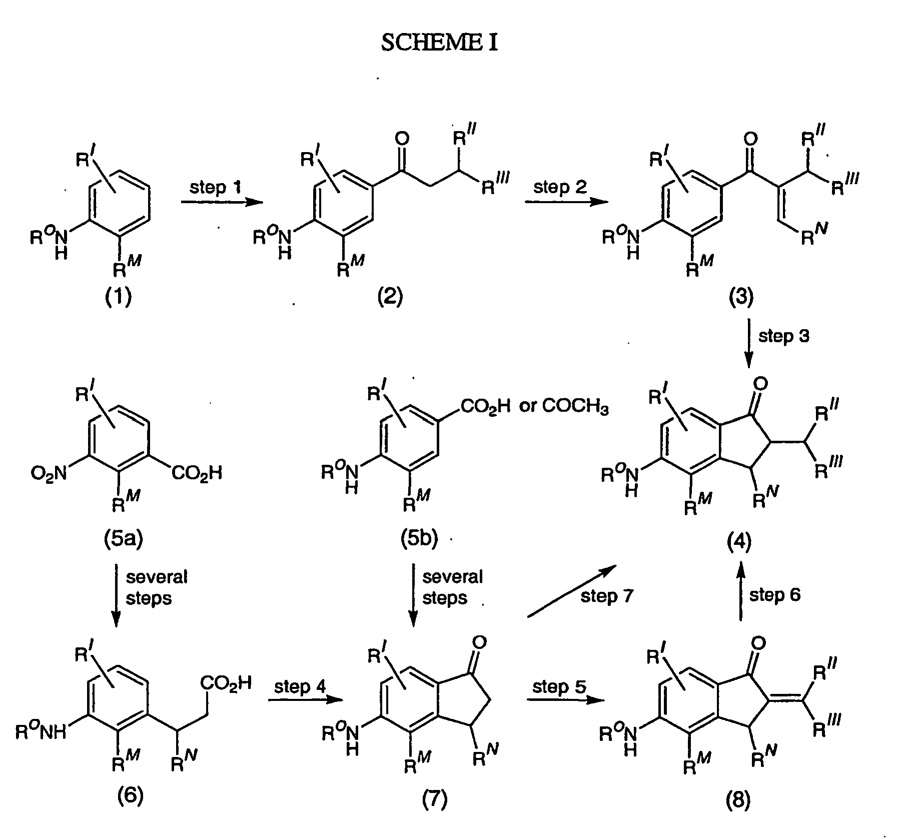 where
- R^{*M*}: is H, Cl, Br, or CH₃
- R^{*N*}: is H or C₁₋₆alkyl

Representative reagents and reaction conditions indicated in Scheme I as steps 1-7 are as follows:
- Step 1: R^{*II*}R^{*III*}CHCH₂COCl, AlCl₃, CH₂Cl₂, 0°C to rt
- Step 2: R^{*N*}CHO, K₂CO₃, MeOH, rt or
R^{*N*}CH(NMe₂)₂, Ac₂O, 95°C
- Step 3: H₂SO₄, 0°C to 50°C
- Step 4: PPA, 80°C
- Step 5: R^{*II*}C(O)R^{*III*}*,* KOH or NaOMe, EtOH, 0°C to rt or
LiN(iPr)₂, THF, -78°C then R^{*II*}C(O)R^{*III*}, -78°C to rt
when R^{*II*}C(O)R^{*III*} is an aldehyde
R^{*II*}C(O)R^{*III*}, LiN(iPr)₂, HMPA, -78°C to rt
when R^{*II*}C(O)R^{*III*} is a ketone
- Step 6: H₂, 10% Pd/C or 20% Pd(OH)₂/C, EtOH or EtOAc, rt
- Steps 5 and 6: can be combined
R^{*II*}C(O)R^{*III*}, KOH, H₂, 10% Pd/C, EtOH, rt or
R^{*II*}C(O)R^{*III*}, NaOMe, H₂, 20% Pd(OH)₂/C, MeOH or EtOH, 0 to 70°C
- Step 7: R^{*II*}R^{*III*}CHX, NaH, DMF, 0°C to rt or
R^{*II*}R^{*III*}CHX, LiN(iPr)₂, THF, -78°C to rt
where X is Br, I, or OSO₂CF₃

Scheme II illustrates several methods for converting 4-unsubstituted-5-(acylamino)-1-indanones (4a) and (7a) into 4-substituted derivatives suitable for subsequent construction of the fused heteroaromatic ring. Bromination (step 1) of (4a) and (7a) provides the 4-bromo compounds (4b) and (7b) which can be converted (step 2) into the 4-methyl derivatives (4c) and (7c) using Stille methodology. Indanone (4a) also undergoes nitration (step 4) to produce (4d). Hydrolysis of the acyl group (step 5) followed by nitro reduction (step 6) affords the 4,5-diamino-1-indanone (4f). The 2-unsubstituted intermediates (7b) and (7c) are converted (step 3) to the corresponding 2-substituted compounds (4b) and (4c) using the combined aldol-reduction sequence described in Scheme I. When compounds (4a) or (7a) possess an open 6-position, bromination and nitration can also occur at that site. These byproducts are conveniently separated from the desired products by chromatography or crystallization.

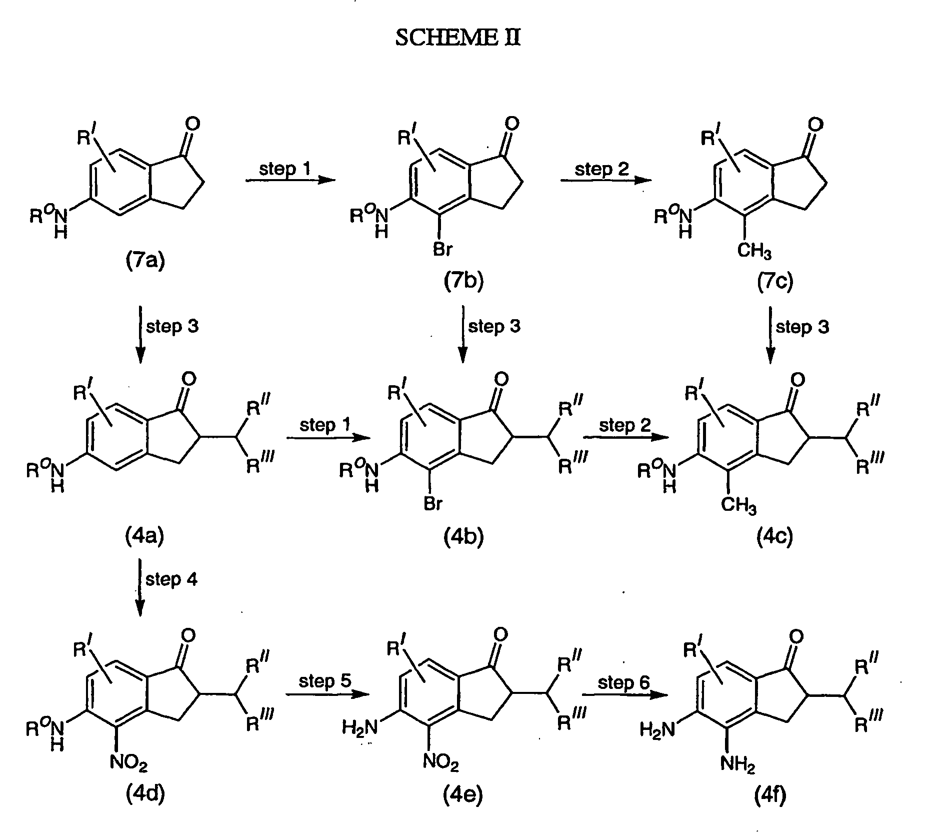

Representative reagents and reaction conditions indicated in Scheme II as steps 1-6 are as follows:
- Step 1: NBS, MeCN or DMF, 60°C
- Step 2: Me₄Sn, PdCl₂(PPh₃)₂, PPh₃, LiCl, DMF, 100°C
- Step 3: R^{*II*}C(O)R^{*III*}, KOH, H₂, 10% Pd/C, EtOH, rt or
R^{*II*}C(O)R^{*III*}, NaOMe, H₂, 20% Pd(OH)₂/C, MeOH or EtOH, 0 to 70°C
- Step 4: 90% HNO₃, -78°C to 0°C
- Step 5: HCl, H₂O-MeOH, 80°C
- Step 6: H₂, 10% Pd/C, EtOH and/or EtOAc, rt

Scheme III illustrates methods for constructing the tetracyclic 8,9,9a,10-tetrahydroindeno[2,1-*e*]indol-7(3*H*)-one compounds of the present invention. In step 1, the 4-bromo indanone (4b) reacts with a vinyl ketone in the presence of base to provide the diketone (9). Cyclization of the diketone under acidic conditions (step 2 i) followed by re-acylation of the amino group (step 2 ii) yields a 1,2,9,9a-tetrahydro-3*H*-fluoren-3-one intermediate (10) suitable for annulation of the pyrrole ring. This is accomplished in either of two ways. Intermediate (10) reacts with an allylating agent (step 3) and the *N*-allyl product (11) undergoes palladium catalyzed cyclization (step 4) to produce the tetracyclic product (12). Deacylation (step 5) of (12) affords a 1-substituted-8,9,9a,10-tetrahydroindeno[2,1-e]indol-7(3*H*)-one product (13). In the second approach, intermediate (10) undergoes Stille coupling (step 6) with a vinyl stannane or vinyl borinate to produce (14) which is deacylated (step 7) to (15). Intermediate (15) undergoes palladium catalyzed cyclization (step 8) to provide a 2-unsubstituted or 2-substituted-8,9,9a,10-tetrahydroindeno[2,1-e]indol-7(3*H*)-one product (16). Product (16) can be subjected to selective electrophilic aromatic substitution (step 9) at the 1-postion to provide products of type (17). 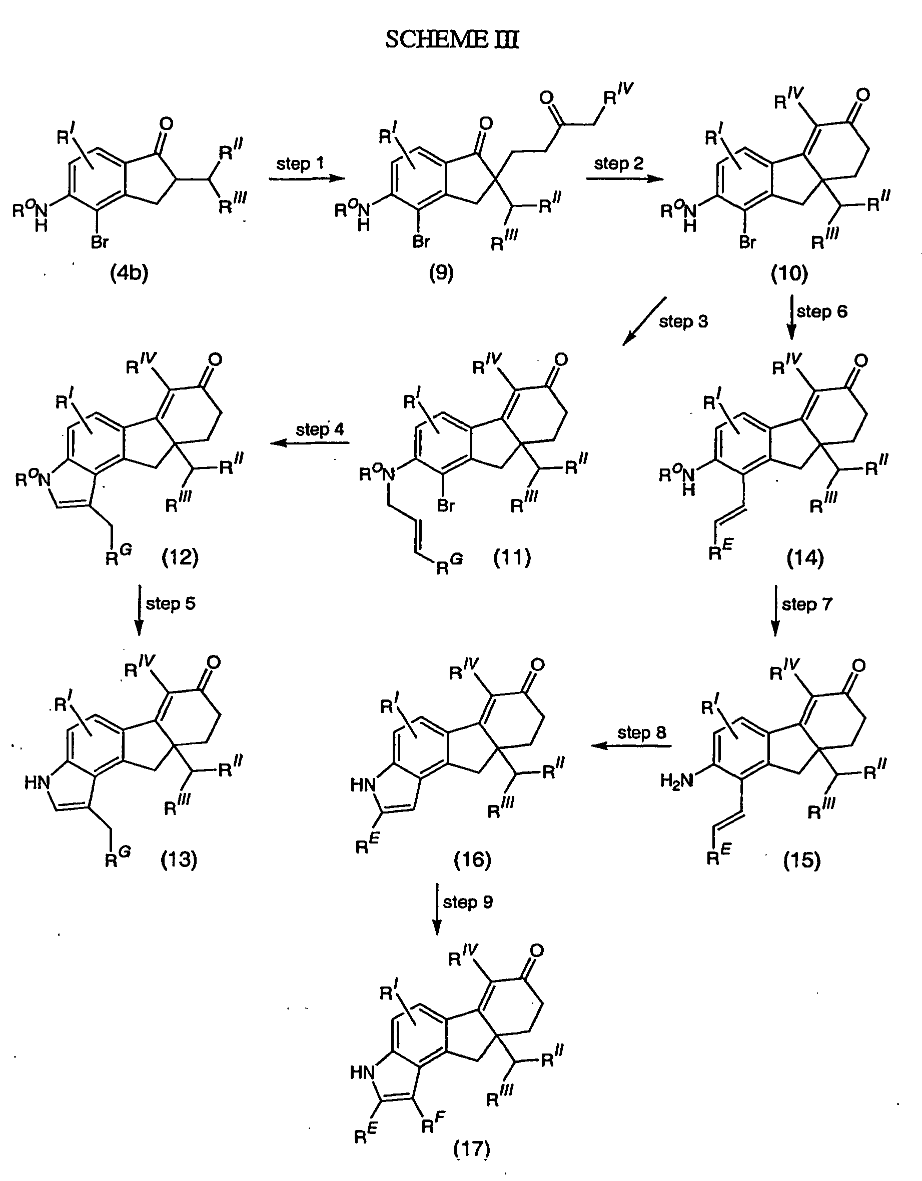 where
- R^{*E*}: is H, C₁₋₆alkyl, C₂₋₆alkenyl, CF₃, O(C₁₋₄alkyl), or N(C₁₋₄alkyl)₂
- R^{*F*}: is F, Cl, Br, I,or NO₂
- R^{*G*}: is H or C₁₋₅alkyl

Representative reagents and reaction conditions indicated in Scheme III as steps 1-9 are as follows:
- Step 1: CH₂=CHC(O)CH₂R^{*IV*}, NaOMe, MeOH or EtOH, 0°C to 60°C
- Step 2: i) HCl, H₂O-HOAc, 80°C to 100°C
ii) AcCl, pyridine, CH₂Cl₂, 0°C
- Step 3: R^{*G*}CH=CHCH₂Br, NaH, DMF, 0° to rt
- Step 4: Pd(PPh₃)₄ or Pd(OAc)₂, PPh₃, Et₃N, DMAC, 90°C
- Step 5: NaOMe, MeOH, 60°C
- Step 6: R^{*E*}CH=CHSnBU₃, PdCl₂(PPh₃)₂, PhMe, 100°C
- Step 7: NaOH, H₂O-EtOH, rt to 100°C
- Step 8: PdCl₂(MeCN)₂, LiCl, benzoquinone, THF, 70°C to 80°C
- Step 9: NCS, CH₂Cl₂, rt R^{*F*} = Cl
HNO₃, CH₂Cl₂, rt R^{*F*} = NO₂

The principal method for constructing the tetracyclic 8,9,9a,10-tetrahydroindeno[2,1-*e*]indazol-7(3*H*)-one compounds of the present invention is summarized in Scheme IV. A 4-methyl-1-indanone (4c) reacts with a vinyl ketone under basic conditions (step 1) to provide the diketone (18). The diketone is then cyclized and deacylated under acidic or basic conditions (step 2) to afford the 7-amino-8-methyl-tetrahydrofluorenone intermediate (19). If excess vinyl ketone and DBU are used in step 1, the R^{*O*}NH group also reacts to form a R^{*O*}NCH₂CH₂C(O)CH₂R^{*IV*} derivative. Both the *N*-acyl and *N*-alkyl groups are removed in step 2 to give the amino product. Formation of the fused pyrazole ring is accomplished by treating (19) with a diazotizing reagent followed by cyclization of the diazo intermediate with KOAc and dibenzo-18-crown-6 (step 3). The tetracyclic product (20) can be further substituted at the 1-position by exposure to a suitable electrophilic reagent (step 4) in the presence of a base to provide products of type (21). 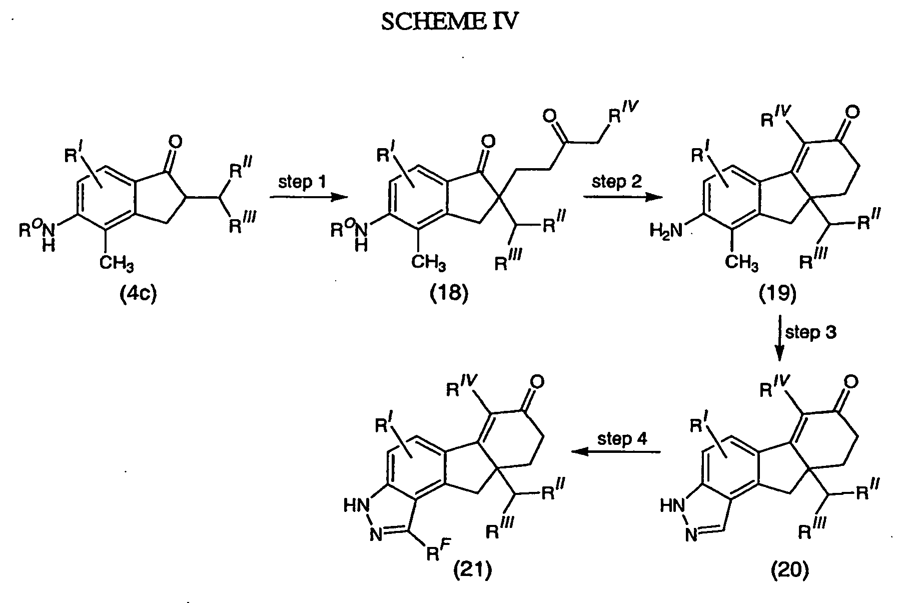 where
- R^{*F*}: is F, Cl, Br, or I

Representative reagents and reaction conditions indicated in Scheme IV as steps 1-4 are as follows:
- Step 1: CH₂=CHC(O)CH₂R^{*IV*}, DBU, THF, rt to 60°C or
CH₂=CHC(O)CH₂R^{*IV*}, NaOMe, MeOH or EtOH, 0°C to 60°C
- Step 2: HCl, H₂O-HAc, 80°C to 100°C or
i) pyrrolidine, HOAc, THF and/or PhMe, 80°C to 100°C
ii) NaOMe, MeOH-EtOH, rt or NaOH, H₂O-EtOH, 90-100°C
- Step 3: i) NOBF₄, CH₂Cl₂, -45°C to 10°C
ii) KOAc, dibenzo-18-crown-6, CH₂Cl₂, -40°C to rt or

i) NaNO₂, HCl, H₂O-HOAc, 0°C
ii) KPF₆
iii) KOAc, dibenzo-18-crown-6, CDCl₃, rt
- Step 4: NCS, NaOH, EtOH, rt R^{*F*} = Cl
Br₂, NaOH, EtOH, rt R^{*F*} = Br

Scheme V shows a method of synthesis of tetrahydroindeno[2,1-*e*]indazol-7(3*H*)-one compounds in which the R^{*IV*} substituent is introduced onto a preformed tricyclic ring system. The 4-unsubstituted tetrahydrofluorenone intermediate (22), which itself is prepared by cyclization (see Scheme IV, step 2 i) of intermediate (18) wherein R^{*IV*} is hydrogen, undergoes chlorination, bromination, or iodination (step 1) to afford the 4-halo intermediates (23). Deacylation (step 2) followed by pyrazole ring formation affords the 6-halo-tetrahydroindeno[2,1-*e*]indazol-7(3*H*)-one products (20a). The pyrazole group undergoes *N*-protection (step 3) to give a mixture of 2- and 3-substituted derivatives (24a) and (24b) which can be used as is or which can be separated and used independently. The *N*-protected intermediates are converted by established methods (step 4) into a variety of new derivatives (25) wherein R^{*IVb*} is, inter alia, an alkyl, alkenyl, alkynyl, aryl, heteroaryl or arylalkyl group. Removal of the *N*-protection (step 5) affords the products (20b). Alternatively, the tetracyclic products (20a) undergo addition-elimination reactions (step 6) at the 6-position to provide products (20b) wherein *R*^{*IVb*} is, inter alia, a CN, OR^{*a*}, NR^{*a*}R^{*b*}, or SR^{*a*} group. If the group *R*^{*IVb*} is, or contains, a functional group capable of further modification, such modifications can be carried out to produce additional derivatives. For example, if *R*^{*IVb*} is an alkenyl group, it can be reduced by catalytic hydrogenation to an alkyl group.

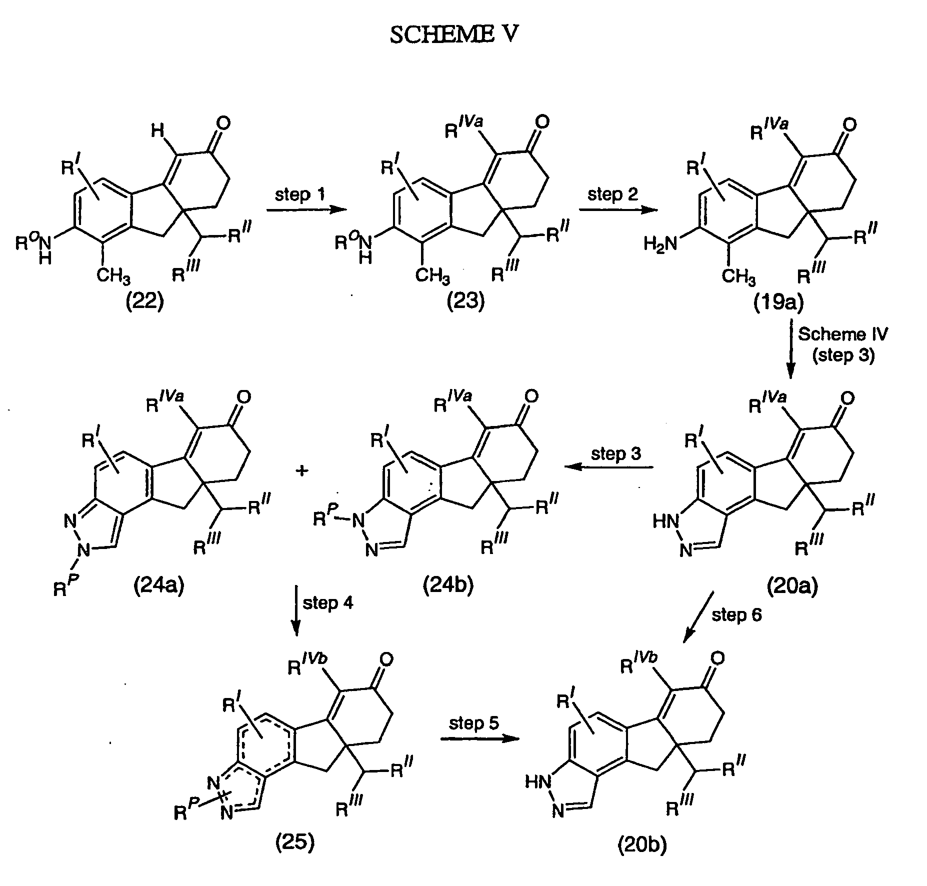

Representative reagents and reaction conditions indicated in Scheme V as stps 1-6 are as follows:

| | | |
|---|---|---|
| Step 1 | NCS, CCl₄, rt | *R*^{*IV*a} = Cl |
| | Br₂, NaHCO₃, CH₂Cl₂ or CCl₄, 0°C to rt | R^{*IVa*} = Br |
| | I₂, NaHCO₃, H₂O, CH₂Cl₂, rt | R^{*IVa*} = I |
| | | |
| Step 2 | NaOMe, MeOH and/or EtOH, rt to 80°C or NaOH, H₂O-EtOH, 100°C | |
| Step 3 | TsCl, DMAP, CH₂Cl₂, 0°C to rt | R^{*P*} = Ts |
| | SEM-Cl, NaH, DMF, 0°C to rt | R^{*P*} = SEM |
| | | |
| Step 4 | R^{*IVb*}SnBu₃, PdCl₂(PPh₃)₂, PhMe, 100-110°C or | R^{*IVb*} = alkenyl, aryl, or heteroaryl |
| | R^{*IVb*}SnBu₃, Pd(PPh₃)₄, PhMe, 100°C or | |
| | R^{*IVb*}B(OH)₂, PdCl₂(PPh₃)₂, Cs₂CO₃, DMF, 100°C or | |
| | R^{*IVb*}B(OH)₂, Pd(PPh₃)₄, aq Na₂CO₃, PhMe, 80°C | |
| | | |
| | (R^{*IVb*})₃B, PdCl₂(dppf) • CH₂Cl₂, Ph₃As, Cs₂CO₃, H₂O, THF, DMF, rt to 60°C | R^{*IVb*} = alkyl |
| | | |
| | R^{*IVb*}Sn(CH₂CH₂CH₂)₃N, Pd(PPh₃)₄, PhMe, 100°C | R^{*IVb*} = alkenyl, alkyl, or arylalkyl |
| | | |
| | R^{*IVb*}H, Pd(OAc)₂(PPh₃)₂, Et₃N, dioxane, 80°C to 100°C | R^{*IVb*} = alkenyl, |
| | | |
| Step 5 | NaOH, H₂O-EtOH rt for R^{*P*} = Ts | |
| | Bu₄NF, THF, rt to 50°C or | |
| | HCl, H₂O-MeOH, 65°C to 85°C for R^{*P*} = SEM | |
| | | |
| Step 6 | CuCN, NMP or DMAC, 160°C | R^{*IVb*} = CN |

The 8,9,9a,10-tetrahydrofluoreno[1,2-*d*]imidazol-7(3*H*)-one compounds of the present invention are prepared as outlined in Scheme VI. The 4,5-diamino-1-indanone (4f) reacts with an orthoacetate or equivalent reagent (step 1) to provide the 7,8-dihydroindeno[4,5-*d*]imidazol-6(3*H*)-one intermediate (26). This compound undergoes *N*-protection (step 2), Michael reaction with a vinyl ketone (step 3), and cyclization (step 4) to afford the tetracyclic product (29). If the product is unsubstituted at the 6-position (see 29a), it can be brominated (step 5) and the 6-bromo product (29b) converted to a variety of 6-substituted products (29c) by the methods previously described in Scheme V.

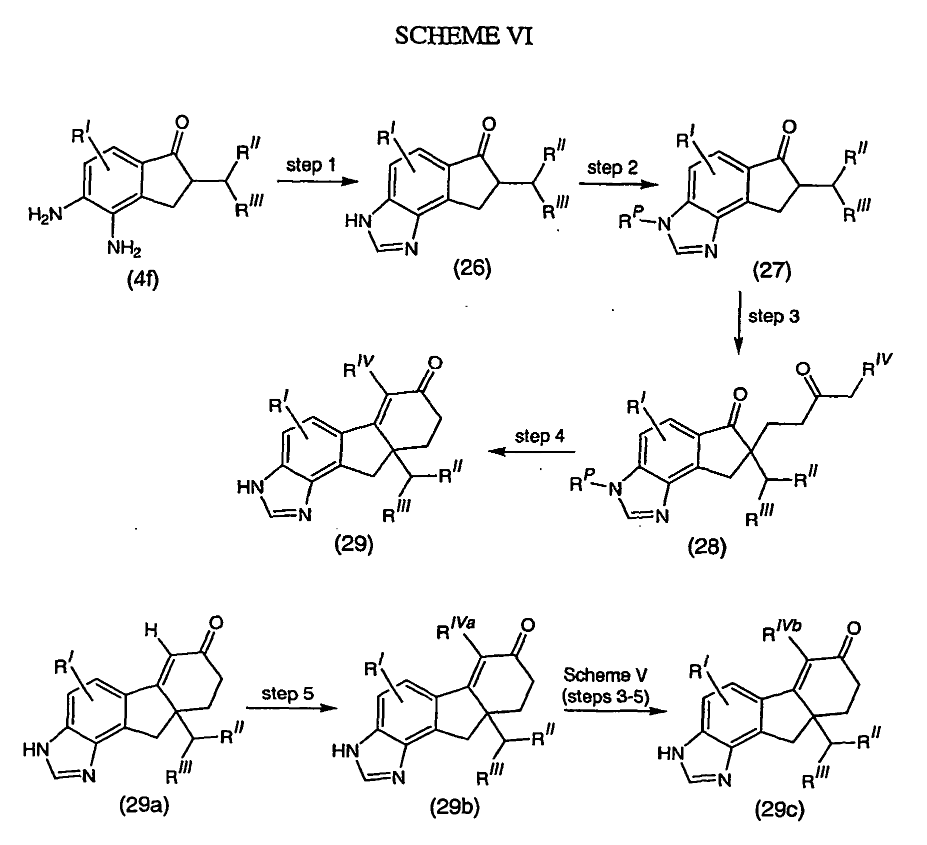

Representative reagents and reaction conditions indicated in Scheme VI as steps 1-5 are as follows:
- Step 1: (EtO)₃CH, EtOH, 80°C
- Step 2: (Boc)₂O, Et₃N, DMAC, THF, rt R^{*P*} = Boc
- Step 3: CH₂=CHC(O)CH₂R^{*IV*}, DBU, THF, 60°C to 70°C
- Step 4: HCl, H₂O-HOAc, 100°C or
i) pyrrolidine, HOAc, PhMe, 85°C to 100°C
ii) HCl, H₂O-EtOAc, rt
- Step 5: Br₂, NaHCO₃, CCl₄, 0°C R^{*IVa*} = Br

Two methods for constructing the tetracyclic 8,9,9a,10-tetrahydrofluoreno[1,2-*d*][1,2,3]triazol-7(3*H*]-one compounds of the present invention are illustrated in Scheme VII. Diazotization (step 1) of the 4,5-diamino-1-indanone (4f) produces a 7,8-dihydroindeno[4,5-*d*][1,2,3]triazol-6(3*H*)-one intermediate (30). This material reacts with a vinyl ketone in the presence of base (step 2) to provide the diketone (31) which is cyclized under acidic conditions (step 3) to afford the tetracyclic product (32). Alternatively, the tricyclic triazole (30) is *N*-protected to give a mixture of products (33). This mixture can be resolved into its individual isomers (33a), (33b) and (33c) and each processed separately, or mixtures of the isomers can be used in the subsequent steps. Structure (33) implies either of these possibilities. Michael reaction of intermediate (33) with a vinyl ketone (step 5) followed by diketone cyclization under mild conditions (step 6) yields the *N*-protected tetracyclic intermediate (35) which is deblocked (step 7) to afford the product (32).

Representative reagents and reaction conditions indicated in Scheme VII as steps 1-7 are as follows:
- Step 1: NaNO₂, HCl, H₂O-EtOH, 0°C
- Step 2: CH₂=CHC(O)CH₂R^{*IV*}, NaOMe, MeOH, 70°C to 75°C
- Step 3: HCl, H₂O-HOAc, 100°C
- Step 4: SEM-Cl, NaH, DMF, 0°C to rt R^{*P*}= SEM
PMB-Cl, NaH, DMF, 0°C to rt R^{*P*} = PMB
- Step 5: CH₂=CHC(O)CH₂R^{*IV*} , DBU, THF, 50°C to 60°C
- Step 6: pyrrolidine, HOAc, PhMe, 90°C to 100°C
- Step 7: Bu₄NF, THF, rt to 50°C or
HCl, H₂O-HOAc, 65°C to 85°C for R^{*P*} = SEM
CF₃CO₂H, 65°C to 75°C for R^{*P*} = PMB

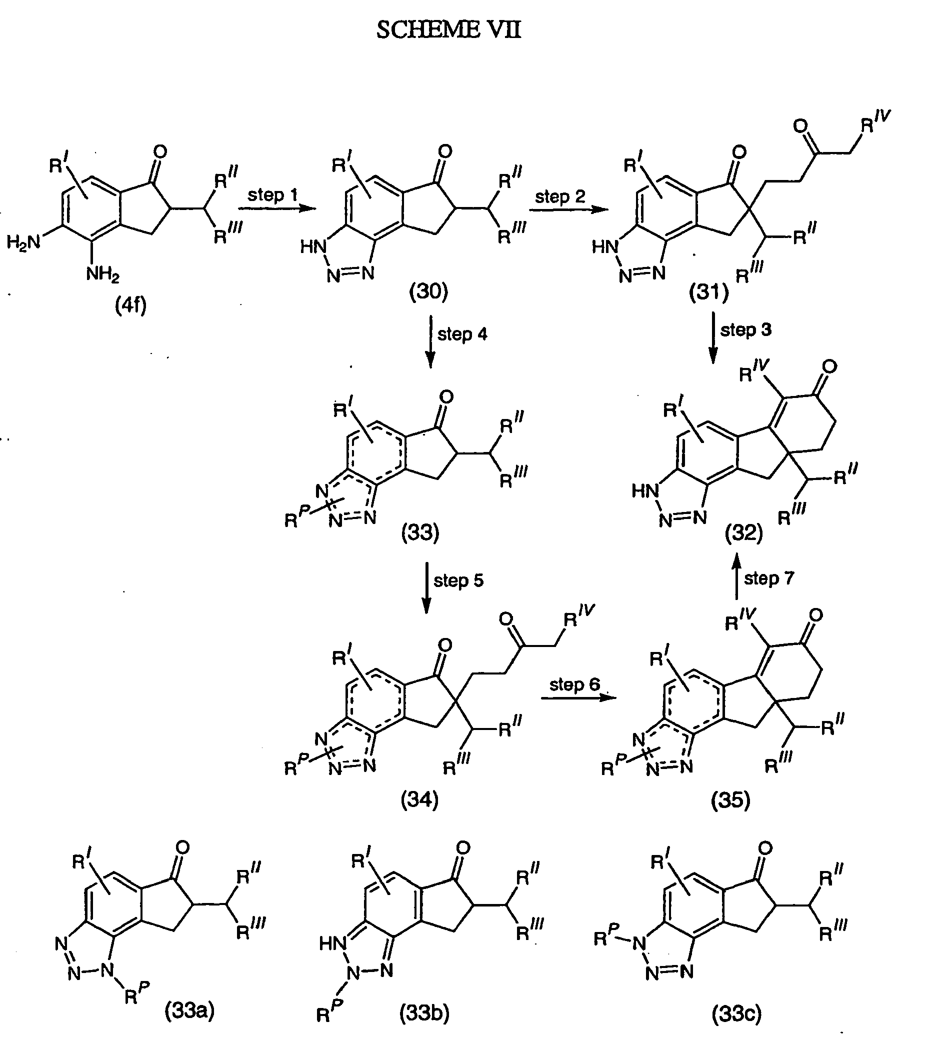

Scheme VIII illustrates alternative methods for preparing 6-substituted tetrahydrofluoreno[1,2-*d*][1,2,3]triazol-7(3*H*)-one compounds from *N*-protected-6-unsubstituted tetracyclic intermediates of type (35) wherein R^{*IV*} is hydrogen. Halogenation of (35a) affords the 6-halo intermediates (35b) that are deblocked to products (32a). As previously described in Scheme V, the halo intermediates also serve as precursors to a wide variety of 6-substituted intermediates (35c) and deblocked products thereof (32b) wherein R^{*IVb*} is, inter alia, an alkyl, alkenyl, alkynyl, aryl, heteroaryl, arylalkyl, or cyano group.

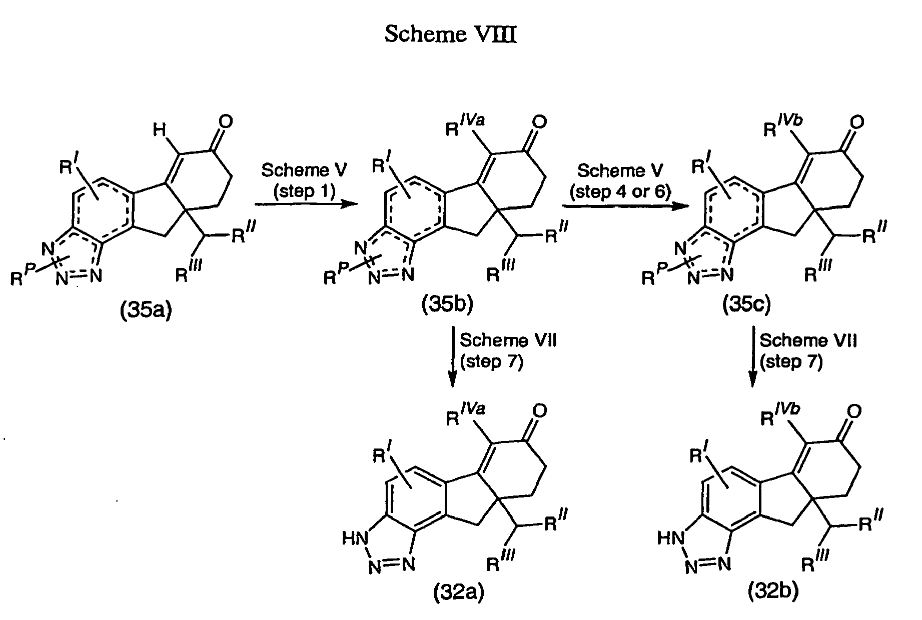

Methods for introduction of substituents at the 8-position of the four classes of tetracyclic products are illustrated in Schemes IX and X. In step 1 of Scheme IX, the fused pyrrole (Y=CR^{e}, Z=CR^{f}), pyrazole (Y=N, Z=CR^{f}), imidazole (Y=CR^{e}, Z=N), or triazole (Y=N, Z=N) ring is *N*-protected using any of a number of well known groups. When the fused heteroaryl ring contains more than one nitrogen atom, positional isomers as indicated by the structures (37a), (37b) and (37c) are possible. These isomers can be used as mixtures or they can be separated and used independently in the following steps. Structure (37) represents these possibilities. In general terms, the *N*-protected-8-unsubstituted tetracyclic intermediate (37) is treated with a strong base and the resulting ketone enolate is trapped with an appropriate electrophilic reagent (step 2). If the electrophile is an alkylating agent of the type R^{*Va*}X, then both monoalkylated (38) and dialkylated (39) products are obtained, depending on the specifics of the reaction conditions. The monoalkylated derivatives can be converted to disubstituted products (40) using the same or a different electrophilic reagent (step 3). Subsequent removal of the *N*-protecting group leads to a variety of products (41)-(43) wherein R^{*Va*} and R^{*Vb*} are, inter alia, alkyl, alkenyl, hydroxy, bromo, and iodo groups. Where appropriate, the newly introduced 8-substituent can be further manipulated to produce additional derivatives. For example, an allyl group can be oxidized to CH₂CHO group which can be reduced to a CH₂CH₂OH group in a second step.

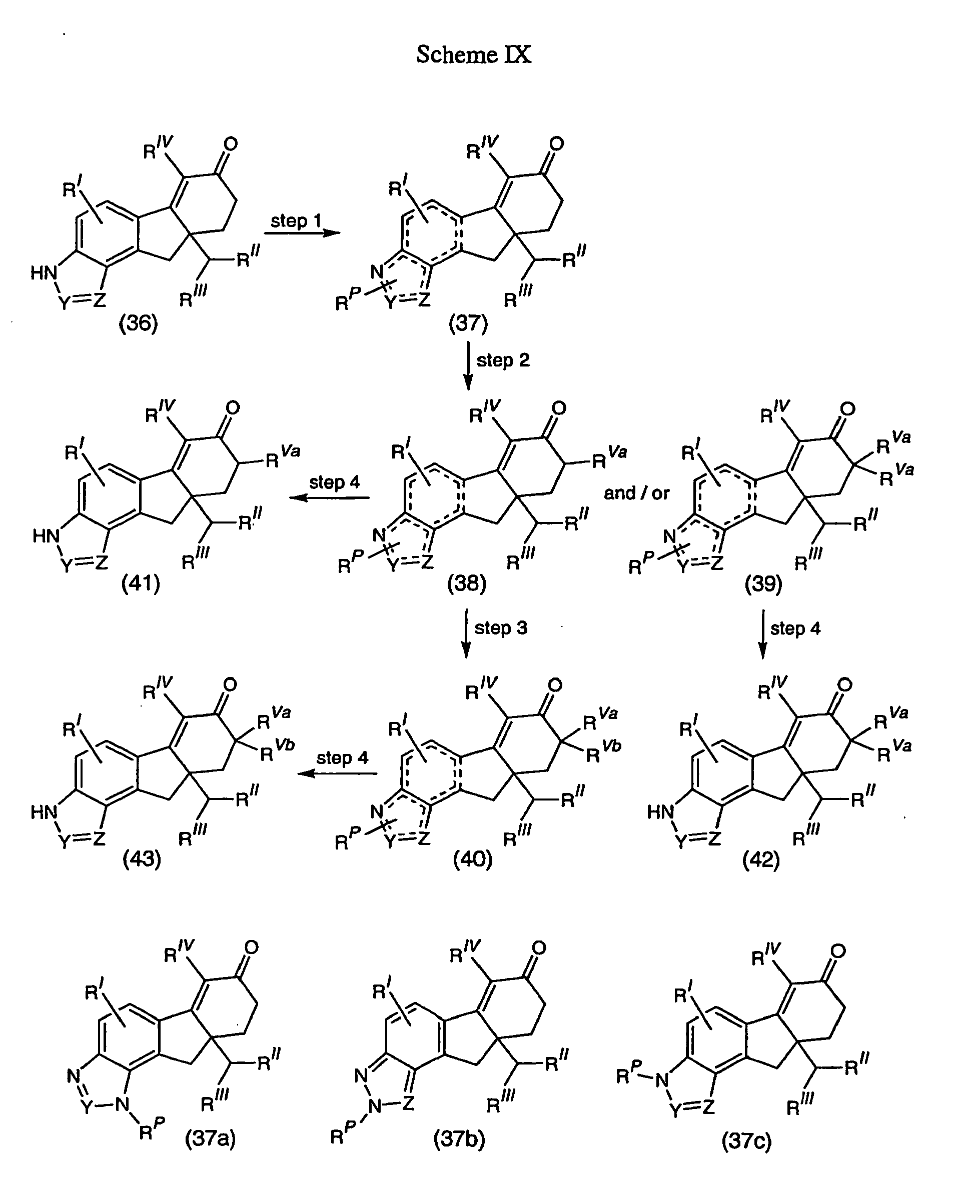

Representative reagents and reaction conditions indicated in Scheme IX as steps 1-4 are as follows:

| | | |
|---|---|---|
| Step 1 | TsCl, DMAP, CH₂Cl₂, 0°C to rt | R^{*P*} = Ts |
| | SEM-Cl, NaH, DMF, 0°C to rt | R^{P} = SEM |
| | PMB-CI, NaH, DMF, 0°C to rt | R^{*P*} = PMB |
| Step 2 | R^{*Va*}X, NaH, DMF, 0°C to rt (X = Br, I) or LDA, THF, 0°C then R^{*Va*}X, -78°C to rt | R^{*Va*} = alkyl, alkenyl |
| | i) LDA, THF, 0°C then TMSCI, -78°C to rt | R^{*va*} = OH |
| | ii) MCPBA, NaHCO₃, CH₂Cl₂, rt | |
| | I₂; pyridine, CH₂Cl₂, rt to 60°C or LDA, THF, 0°C then I₂, -78°C to rt | R^{*Va*} = I |
| Step 3 | same as step 2 except use R^{*Vb*}*X* to introduce a different alkyl or alkenyl group | |
| Step 4 | NaOH, H₂O-EtOH rt for R^{*P*} = Ts | |
| | Bu₄NF, THF, rt to 50°C or | |
| | aq HCl, MeOH or HOAc, 65°C to 85°C for R^{*P*} = SEM | |
| | CF₃CO₂H, 65°C to 75°C or | |
| | aq HCl, HOAc, 100°C for R^{*P*} = PMB | |

As shown in Scheme X, the ketone enolate of (37) can also be trapped with aldehydes (step 1) to afford, after deblocking, 8-alkylidene (46) and 8-hydroxyalkyl (47) products. Scheme X also illustrates a special case of C-8 functionalization that provides for 8,9a-bridged products of type (50). In this case, R^{*II*} of (48) is an unsubstituted or substituted alkyl group containing an electrophilic moiety such as an iodo, bromo, methylsulfonyloxy, aldehyde or keto group. Enol generation at C-8 (step 2) is followed by intramolecular reaction at the R^{*II*} electrophilic center to afford a bridged product of type (49). This compound can be deblocked or modified and then deblocked to provide the product (50). For example, if R^{*IId*} is a hydroxy group, modifications include acylation, oxidation, dehydration, or dehydration followed by reduction. 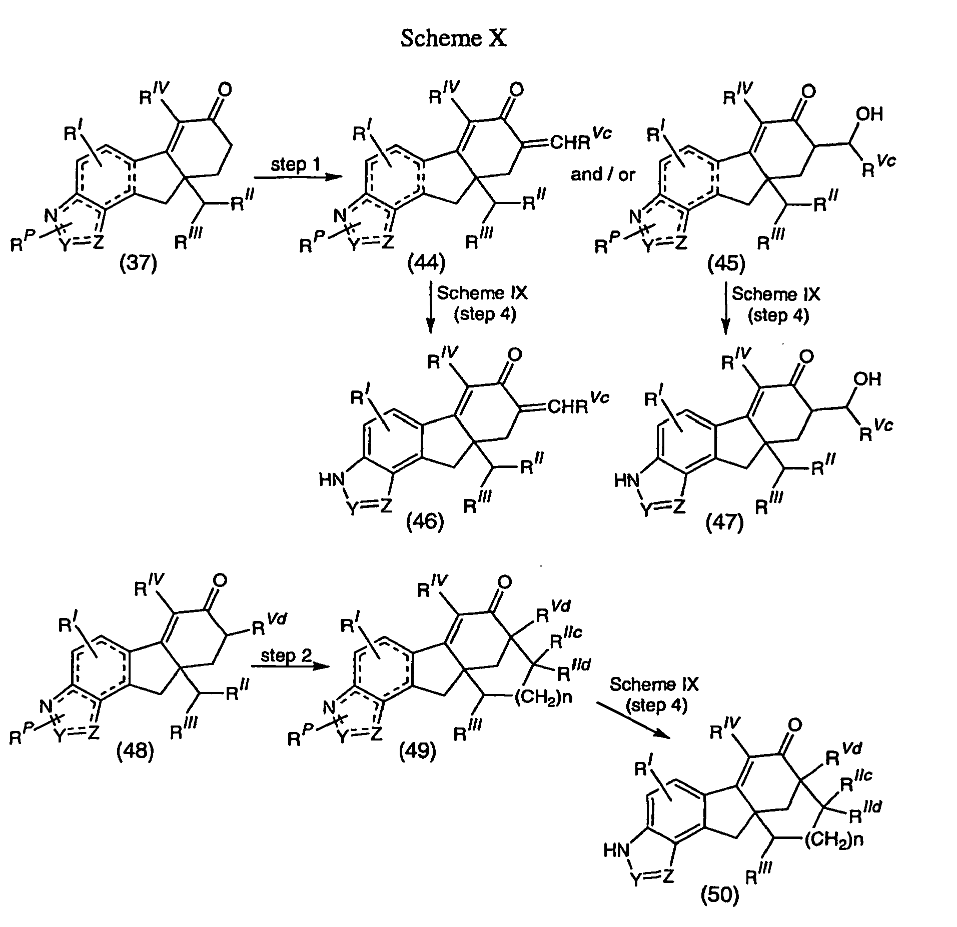 where
- R^{*Vd*}: is H, C₁₋₆alkyl, or C₂₋₆alkenyl
- R^{*IIc*}: is H or C₁₋₆alkyl
- R^{*IId*}: is H or OH

Representative reagents and reaction conditions indicated in Scheme X as steps 1 and 2 are as follows:
- Step 1: R^{*V*c}CHO, KOH, MeOH or EtOH, rt or
LDA, THF, 0°C then R^{*V*c}CHO, -78°C to rt or
EtOCHO, NaH, PhH, rt (gives 14, R^{*V*c} = OH)
- Step 2: LDA, THF, -78°C to rt or
NaH, DMF, 0°C to rt or
DBU, PhMe, 80°C to 100°C

Tetracyclic products bearing substituents at the 10-position are prepared by the methods summarized in Scheme XI. Fused pyrazole compounds (39, Y=N, Z=CR^{f}) are oxidized (step 1) by *N*-halosuccinimide reagents and the like to afford 10-halo (51) and 10-oxo (52) products. Alternatively, the 10-oxo product (52) is available by potassium persulfate oxididation of (39). The 10-halo products undergo displacement reactions with suitable nucleophilic reagents (step 2) to afford additional products of type (53). 10-Alkyl, alkenyl and alkynyl products of structure (55) are best prepared starting from the appropriate 3-substituted indanone (54) and using the procedures described in Schemes III-VIII. If desired, this methodology can be extended to the preparation of 10,10-disubstituted products.

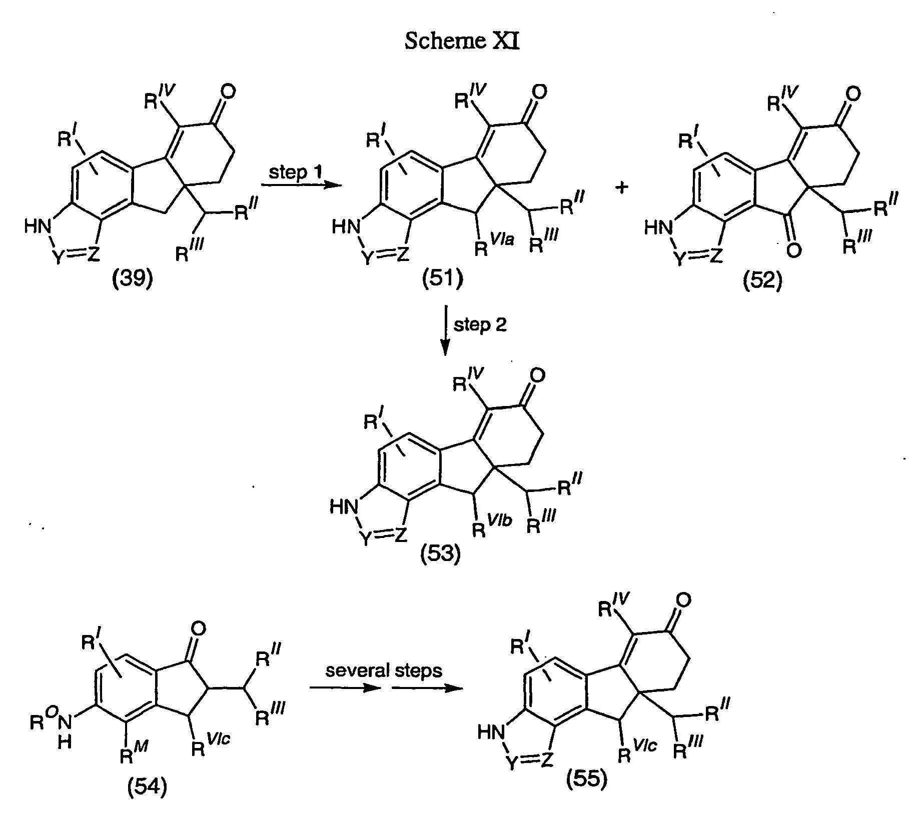

Representative reagents and reaction conditions indicated in Scheme XI as steps 1 and 2 are as follows:

| | | |
|---|---|---|
| Step 1 | i) NCS or NBS, CH₂Cl₂, rt | R^{*VIa*} = Cl or Br |
| | ii) aqueous workup or | |
| | K₂S₂O₈, H₂O, MeCN, rt | R^{*VIa*} = OH + (52) |
| Step 2 | i) NaN₃, DMF, rt to 80°C | R^{*VIb*} = N₃ |
| | ii) H₂S, piperidine, EtOH, rt | R^{*VIb*} = NH₂ |
| | NaOMe, MeOH, rt to 70°C | R^{*VIb*} = OMe |

Modifications to the C-7 ketone are outlined in Scheme XII for the generic tetracyclic compound (56) in which R^{*Q*} is hydrogen or an *N*-protecting group. In step 1, the ketone is reacted with a hydroxylamine, alkoxyamine, or hydrazine reagent to yield the 8-imino products (57). These compounds are typically obtained as separable mixture of *E*- and *Z*-isomers about the imino double bond. Ketone (56) also reacts with ylide reagents (step 2) to afford 3-alkylidene derivatives (59). If necessary, deblocking of the *N*-protecting group affords the products (58) and (60).

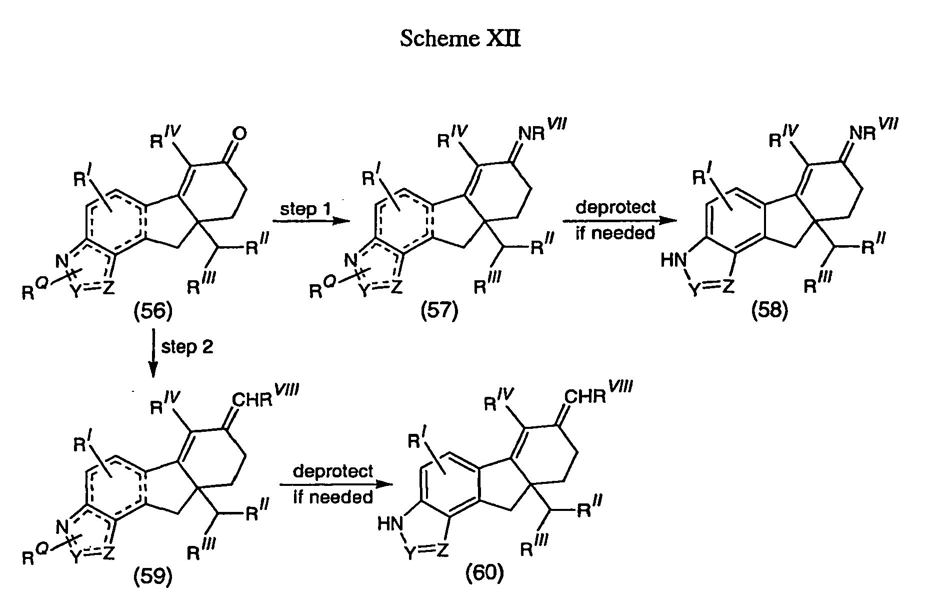

Representative reagents and reaction conditions indicated in Scheme XII as steps 1 and 2 are as follows:
- Step 1: NH₂OR^{a}·HCl, pyridine, rt to 60°C R^{*VII*} = OR^{a}
NH₂NR^{a}R^{b}, EtOH, rt R^{*VII*} = NR^{a}R^{b}
- Step 2: Ph₃P⁺CH₂R^{*VIII*} Br⁻, BuLi, THF, 0 to 50°C

Methods for the preparation of tetracyclic products bearing an alkenyl substituent at the 9a-position are outlined in Scheme XIII. A 2-alkylidene-1-indanone intermediate of type (8), wherein R^{*II*} is a carbon atom substituted with at least one hydrogen atom, reacts with a vinyl ketone in the presence of base to afford the diketone (61). Alternatively, indanone (8) is converted to the tricyclic dione (64) via intermediate (63). Diones (61) and (64) can be processed according to the methods outlined in Schemes III, IV, VI, and VII into tetracyclic products of type (62).

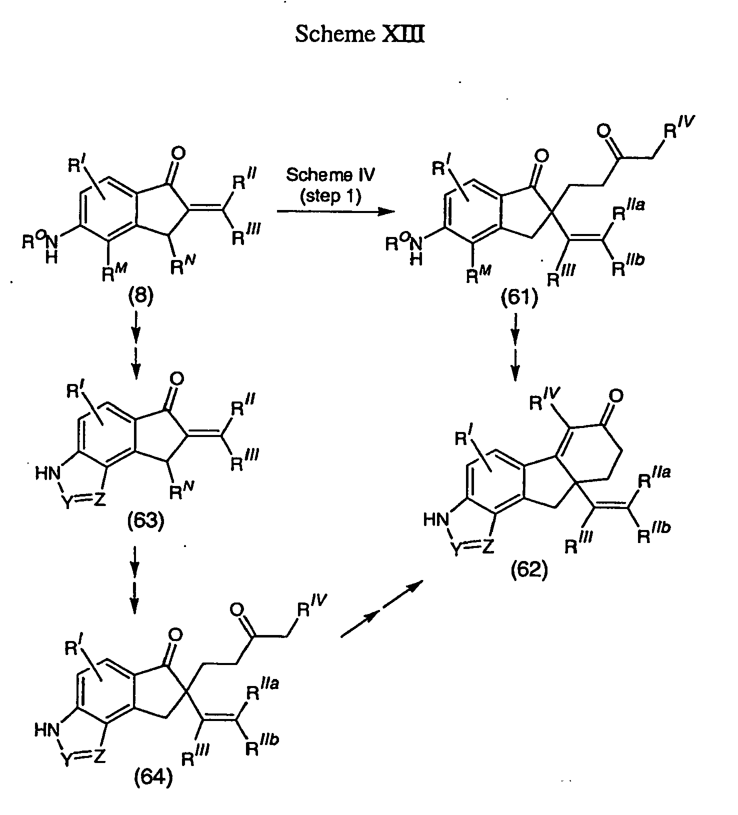

Also implied, but not discussed, in the foregoing schemes is an option for the preparation of 9-substituted tetracyclic products. These are obtained by substituting R^{*IX*}CH=CHC(O)CH₂R^{*IV*}, where R^{*IX*} is a C₁₋₁₀alkyl or C₂₋₁₀alkenyl group, for the CH₂=CHC(O)CH₂R^{*IV*} reagent in Schemes III, IV, VI, and VII. In general, use of the R^{*IX*} substituted vinyl ketone requires more vigorous reaction conditions, that islonger reaction times and/or higher temperatures, when compared to reactions using the unsubstituted vinyl ketone.

In Schemes I-XIII, the various R groups often contain protected functional groups which are deblocked by conventional methods. The deblocking procedure can occur at the last step or at an intermediate stage in the synthetic sequence. For example, if one of R^{*I*} is a methoxyl group, it can be converted to a hydroxyl group by any of a number of methods. These include exposure to BBr₃ in CH₂Cl₂ at -78°C to room temperature, heating with pyridine hydrochloride at 190-200°C, or treatment with EtSH and AlCl₃ in CH₂Cl₂ at 0°C to room temperature. Another example involves the use of methoxymethyl (MOM) protection of alcohols and phenols. The MOM group is conveniently removed by exposure to hydrochloric acid in aqueous methanol. Other well known protection-deprotection schemes can be used to prevent unwanted reactions of functional groups contained in the various R substituents.

The following specific examples, while not limiting, serve to illustrate the methods of preparation of the tetracyclic compounds of the present invention. All compounds prepared are racemic, but can be resolved if desired using known methodologies.

### EXAMPLE 1

### SYNTHESIS OF 9a-ETHYL-1,6-DIMETHYL-8,9,9a,10-TETRAHYDROINDENO[2,1-e]INDOL-7(3H)-ONE

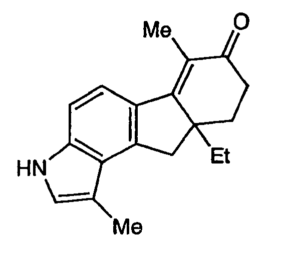

### Step 1: 5-(acetylamino)-2-ethyl-1-indanone

A suspension of 5-(acetylamino)-1-indanone (2.00 g, 10.46 mmol) in ethanol (42 mL) was treated with a 0.5M solution of sodium methoxide in methanol (4.20 mL, 2.1 mmol). The mixture was warmed to give a solution which was treated with 20% palladium hydroxide on carbon (200 mg). The mixture was sonicated for 10 seconds, then cooled in an ice bath, placed under a hydrogen atmosphere, and treated with acetaldehyde (1.17 mL, 20.9 mmol). The cooling bath was removed and the mixture was stirred under a hydrogen atmosphere at room temperature for 5.5 hours. The catalyst was removed by filtration and the filtrate was concentrated under vacuum to a gum. This material was taken up in EtOH (50 mL) and the mixture evaporated under vacuum. The residue was partitioned between EtOAc (50 mL) and brine (50 mL) containing aqueous 2N HCl (5 mL). The organic phase was dried over MgSO₄, filtered, and evaporated under vacuum to afford crude 5-(acetylamino)-2-ethyl-1-indanone (2.17 g) as a foam. The ¹H NMR spectrum revealed the presence of minor amounts of 5-acetamido-2-ethyl-2-(1-hydroxyethyl)-1-indanone diastereomers in the crude product.

### Step 2: 5-(acetylamino)-4-bromo-2-ethyl-1-indanone

A solution of crude 5-(acetylamino)-2-ethyl-1-indanone (2.17 g) in anhydrous dimethylformamide (10.5 mL) was treated with *N*-bromosuccinimide (1:86 g, 10.46 mmol). The resulting solution was stirred and heated in an oil bath at 60°C for 5 hours, then allowed to cool and stirred at room temperature overnight. The solvent was evaporated under vacuum. The residue in EtOAc (100 mL) was washed with water (4 x 100 mL) and brine (50 mL), dried over MgSO₄, filtered, and evaporated under vacuum to afford crude 5-(acetylamino)-4-bromo-2-ethyl-1-indanone (2.40 g) as a foam. The ¹H NMR spectrum revealed the presence of minor amounts of the 6-bromo and 2,4-dibromo byproducts in the crude product.

### Step 3: 5-amino-4-bromo-2-ethyl-2-(3-oxopentyl)-1-indanone

A solution of crude 5-(acetylamino)-4-bromo-2-ethyl-1-indanone (1.20 g) in anhydrous methanol (13 mL) was treated with ethyl vinyl ketone (0.65 mL, 6.54 mmol) and 0.5M sodium methoxide in methanol (2.6 mL, 1.3 mmol). The resulting solution was stirred under a nitrogen atmosphere and heated in an oil bath at 60°C for 4 hours and then stirred at room temperature for 2.5 days. The mixture was partitioned between EtOAc (50 mL) and water (50 mL). The organic phase was washed with brine (25 mL), dried over MgSO₄, filtered, and evaporated under vacuum to an oil (1.6 g). The crude product was purified by column chromatography on EM silica gel 60 (230-400 mesh, 2.75 x 26 cm) using 5% EtOAc in CH₂Cl₂ as the eluting solvent. The product containing fractions were combined and evaporated under vacuum to provide 5-amino-4-bromo-2-ethyl-2-(3-oxopentyl)-1-indanone (0.65 g) as an oil.

### Step 4: 7-amino-8-bromo-9a-ethyl-4-methyl-1,2,9,9a-tetrahydro-3H-fluoren-3-one

A solution of 5-amino-4-bromo-2-ethyl-2-(3-oxopentyl)-1-indanone (650 mg) in acetic acid (10 mL) was diluted with aqueous 6N HCl (10 mL). The mixture was stirred and heated in an oil bath at 80°C for 8 hours and then stirred at room temperature overnight. The mixture was partitioned between EtOAc (100 mL) and aqueous K₂CO₃ (200 mL). The organic phase was dried over MgSO₄, filtered, and evaporated under vacuum to provide 7-amino-8-bromo-9a-ethyl-4-methyl-1,2,9,9a-tetrahydro-3*H*-fluoren-3-one (568 mg) as an oil.

### Step 5: 7-(acetylamino)-8-bromo-9a-ethyl-4-methyl-1,2,9,9a-tetrahydro-3H-fluoren-3-one

An ice-cold solution of 7-amino-8-bromo-9a-ethyl-4-methyl-1,2,9,9a-tetrahydro-3*H*-fluoren-3-one (548 mg, 1.71 mmol) in anhydrous dichloromethane (6.8 mL) was treated successively with pyridine (0.208 mL, 2.57 mmol) and acetyl chloride (0.146 mL, 2.05 mmol). After stirring at 0°C for 30 minutes, the mixture was partitioned between EtOAc (50 mL) and water (50 mL) containing aqueous 2N HCl (5 mL), The organic phase was washed with brine, dried over MgSO₄, filtered, and evaporated under vacuum to afford 7-(acetylamino)-8-bromo-9a-ethyl-4-methyl-1,2,9,9a-tetrahydro-3*H*-fluoren-3-one (600 mg) as a solid.

### Step 6: 7-[N-allyl(acetylamino)1-8-bromo-9a-ethyl-4-methyl-1,2,9,9a-tetrahydro-3H-fluoren-3-one

Sodium hydride (6.5 mg of a 61.1 % dispersion in mineral oil, 0.165 mmol) and allyl bromide (0.013 mL, 0.152 mmol) were added to an ice-cold solution of 7-(acetylamino )-8-bromo-9a-ethyl-4-methyl-1,2,9,9a-tetrahydro-3*H*-fluoren-3-one (50 mg, 0.138 mmol) in anhydrous dimethylformamide (0.55 mL). The mixture was stirred under a nitrogen atmosphere with ice bath cooling for one hour. The mixture was partitioned between EtOAc (30 mL) and water (30 mL). The organic phase was washed with water (3 x 30 mL) and brine (10 mL), dried over MgSO₄, filtered, and evaporated under vacuum to afford 7-[*N*-allyl(acetylamino)]-8-bromo-9a-ethyl-4-methyl-1,2,9,9a-tetrahydro-3*H*-fluoren-3-one (50 mg) as an oil.

### Step 7: 3-(acetylamino)-9a-ethyl-1,6-dimethyl-8,9,9a,10-tetrahydroindeno[2,1-e]indol-7(3H)-one

A solution of 7-[*N*-allyl(acetylamino)]-8-bromo-9a-ethyl-4-methyl-1,2,9,9a-tetrahydro-3*H*-fluoren-3-one (50 mg, 0.124 mmol) in anhydrous *N,N-*dimethylacetamide (0.5 mL) was treated with triethylamine (0.035 mL, 0.249 mmol), tetrakis(triphenylphosphine)palladium(0) (7 mg, 0.0062 mmol) and triphenylphosphine (6.5 mg, 0.025 mmol). The mixture was stirred under a nitrogen atmosphere and heated in an oil bath at 90°C for 20 hours. TLC showed mainly starting material. The mixture was treated with palladium(II) acetate (5 mg, 0.022 mmol) and triethylamine (0.100 mL, 0.72 mmol) and stirred under a nitrogen atmosphere and heated at 90°C for 3.25 hours. TLC showed partial conversion to products. Additional palladium(II) acetate (50 mg, 0.22 mmol), triphenylphosphine (25 mg, 0.095 mmol) and triethylamine (0.100 mL, 0.72 mmol) were added and the mixture was then stirred and heated at 90°C for an additional 1.75 hours. After cooling to room temperature, the mixture was evaporated under vacuum and the residue was partitioned between EtOAc (30 mL) and water (30 mL). The organic phase was washed with water (4 x 30 mL) and brine (10 mL), dried over MgSO₄, filtered, and evaporated under vacuum to an oil (100 mg). The crude product was purified by preparative layer chromatography on two 0.1 x 20 x 20 cm silica gel GF plates, developing with 5% MeOH in CH₂Cl₂. Two major UV visible bands were removed, eluted with 10% MeOH in CH₂Cl₂, and the eluants evaporated under vacuum. The slower moving band afforded an oil (20.2 mg) that was tentatively identified as 3-acetamido-9a-ethyl-6-methyl-1-methylene-2,3,8,9,9a,10-hexahydroindeno[2,1-*e*]indol-7(1*H*)-one by NMR. The faster moving band provided 3-(acetylamino)-9a-ethyl-1,6-dimethyl-8,9,9a,10-tetrahydroindeno[2,1*-e*]indol-7(3*H*)-one (19.3 mg) as an oil.

### Step 8: 9a-ethyl-1,6-dimethyl-8,9,9a,10-tetrahydroindeno[2,1-e]indol-7(3H)-one

A solution of 3-(acetylamino)-9a-ethyl-1,6-dimethyl-8,9,9a,10-tetrahydroindeno[2,1-*e*]indol-7(3*H*)-one (19 mg, 0.059 mmol) in 0.5M sodium methoxide in methanol (0.25 mL, 0.125 mmol) was stirred and heated in an oil bath at 60°C for 50 minutes. After cooling to room temperature, the reaction mixture was purified by preparative layer chromatography on a 0.1 x 20 x 20 cm silica gel GF plate, developing with 5% MeOH in CH₂Cl₂. The major UV visible band was extracted with 10% MeOH in CH₂Cl₂ and the extracts were evaporated under vacuum to an oil. The oil was lyophilized from benzene to afford 9a-ethyl-1,6-dimethyl-8,9,9a,10-tetrahydroindeno[2,1-*e*]indol-7(3*H*)-one (8.2 mg) as an amorphous solid.
¹H NMR (CDCl₃, 500 MHz) δ 0.88 (t, CH₂C*H*_{*3*}), 1.55 and 1.69 (two m, C*H*_{*2*}CH₃), 2.06 and 2.31 (two ddd, 9-C*H*_{*2*}), 2.16 (s, 6-C*H*_{*3*}), 2.48 (s, 1-C*H*_{*3*}), 2.50 and 2.61 (two ddd, 8-C*H*_{*2*}), 3.03 and 3.46 (two d, 10-C*H*_{*2*}), 6.99 (s, *H*-2), 7.28 (d, *H*-4), 7.59 (d, *H-*5) and 8.05 (br s, N*H*).

### EXAMPLE 2

### SYNTHESIS OF 9a-ETHYL-6-METHYL-8,9,9a,10-TETRAHYDROINDENO[2,1-e]INDOL-7(3H)-ONE

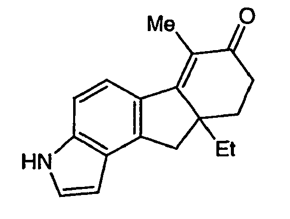

### Step 1: 7-(acetylamino)-9a-ethyl-4-methyl-8-vinyl-1,2,9,9a-tetrahydro-3H-fluoren-3-one

A mixture of 7-(acetylamino)-8-bromo-9a-ethyl-4-methyl-1,2,9,9a-tetrahydro-3*H*-fluoren-3-one. (200 mg, 0.55 mmol), tributyl(vinyl)tin (0.192 mL, 0.66. mmol), and dichlorobis(triphenylphosphine)palladium(II) (19 mg, 0.0275 mmol) in anhydrous toluene (1.1 mL) was placed under a nitrogen atmosphere and stirred with heating in a 100°C oil bath for 14.5 hours. After cooling to room temperature, the mixture was diluted with CH₂Cl₂ (1 mL) and the solution was streaked onto three 0.1 x 20 x 20 cm silica gel GF plates which were developed with 5% MeOH in CH₂Cl₂. The UV visible product band was extracted with 10% MeOH in CH₂Cl₂ and the extracts were evaporated under vacuum to provide 7-(acetylamino)-9a-ethyl-4-methyl-8-vinyl-1,2,9,9a-tetrahydro-3*H*-fluoren-3-one (143 mg, 84% yield) as an oil.

### Step 2: 7-amino-9a-ethyl-4-methyl-8-vinyl-1,2,9,9a-tetrahydro-3H-fluoren-3-one

Aqueous 5N sodium hydroxide (0.231 mL, 1.16 mmol) was added to a suspension of 7-(acetylamino)-9a-ethyl-4-methyl-8-vinyl-1,2,9,9a-tetrahydro-3*H* fluoren-3-one (143 mg, 0.46 mmol) in ethanol (3 mL). The mixture was stirred and heated in an oil bath at 80°C for 17.3 hours. After cooling to room temperature, the mixture was partitioned between EtOAc (20 mL) and brine (30 mL) containing aqueous 2N HCl (2 mL). The organic phase was dried over MgSO₄, filtered, and evaporated under vacuum to an oil (150 mg). The crude product was purified by preparative layer chromatography on two 0.1 x 20 x 20 cm silica gel GF plates that were developed with 5% MeOH in CH₂Cl₂. The UV visible product band was extracted with 10% MeOH in CH₂Cl₂ and the extracts were evaporated under vacuum. The residue was lyophilized from benzene to afford 7-amino-9a-ethyl-4-methyl-8-vinyl-1,2,9,9a-tetrahydro-3*H*-fluoren-3-one (92 mg, 75 % yield) as an amorphous solid.

### Step 3: 9a-ethyl-6-methyl-8,9,9a,10-tetrahydroindeno[2,1-e]indol-7(3H)-one

A mixture of 7-amino-9a-ethyl-4-methyl-8-vinyl-1,2,9,9a-tetrahydro-3*H*-fluoren-3-one (70 mg, 0.26 mmol), bis(acetonitrile)dichloropalladium(II) (13.6 mg, 0.052 mmol), lithium chloride (42 mg, 0.99 mmol), benzoquinone (33.7 mg, 0.312 mmol), and anhydrous tetrahydrofuran (2.6 mL) was placed under a nitrogen atmosphere. The mixture was stirred and heated in an oil bath at 70°C for 30 hours, then allowed to cool to room temperature. The solvent was evaporated under vacuum and the residue was purified by preparative layer chromatography on a 0.1 x 20 x 20 cm silica gel GF plate that was developed with 5% MeOH in CH₂Cl₂. The UV visible product band was eluted with 5% MeOH in CH₂Cl₂, the eluant evaporated under vacuum, and the residue lyophilized from benzene to afford 9a-ethyl-6-methyl-8,9,9a,10-tetrahydroindeno[2,1-*e*]indol-7(3*H*)-one (27.9 mg, 40% yield) as an amorphous solid.
¹H NMR (CDCl₃, 500 MHz) δ 0.88 (t, CH₂*CH*_{*3*}), 1.53 and 1.69 (two m, C*H*_{*2*}CH₃), 2.06 and 2.32 (two ddd, 9-C*H*_{*2*}), 2.16 (s, 6-C*H*_{*3*}), 2.50 and 2.61(two ddd, 8-C*H*_{*2*}), 2.88 and 3.24 (two d, 10-C*H*_{*2*}), 6.59 (m, *H*-1), 7.28 (m, *H*-2), 7.37 (d, *H*-4), 7.64 (d, *H*-5) and 8.37 (br s, N*H*).

### EXAMPLE 3

### SYNTHESIS OF 1-CHLORO-9a-ETHYL-6-METHYL-8,9,9a,10-TETRAHYDROINDENO[2,1-e]INDOL-7(3H)-ONE

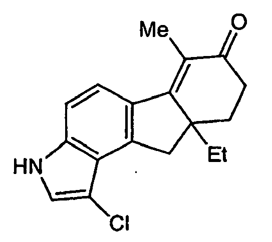

A solution of 9a-ethyl-6-methyl-8,9,9a,10-tetrahydroindeno[2,1-*e*]indol-7(3*H*)-one (15 mg, 0.057 mmol) and *N*-chlorosuccinimide (NCS, 8.4 mg, 0.063 mmol) in anhydrous dichloromethane (0.5 mL) was stirred at room temperature. After 145 minutes, additional NCS (1 mg, 0.0075 mmol) was added and stirring at room temperature was continued for 40 minutes. The reaction mixture was combined with a similar reaction using 5.2 mg (0.019 mmol) of starting material and the total crude was purified by preparative layer chromatography on a 0.1 x 20 x 20 cm silica gel GF plate. The plate was developed with 20% EtOAc in hexanes. The product containing band was eluted with EtOAc, the eluant evaporated under vacuum, and the residue lyophilized from benzene to afford 1-chloro-9a-ethyl-6-methyl-8,9,9a,10-tetrahydroindeno[2,1-*e*]indol-7(3*H*)-one (15 mg, 66% yield) as an amorphous solid.
¹H NMR (CDCl₃, 500 MHz) δ 0.89 (t, CH₂C*H*_{*3*}), 1.53 and 1.68 (two m, C*H*_{*2*}CH₃), 2.05 and 2.32 (two ddd, 9-C*H*_{*2*}), 2.15 (s, 6-C*H*_{*3*}), 2.50 and 2.61(two ddd, 8-C*H*_{*2*}), 3.04 and 3.63 (two d, 10-C*H*_{*2*}), 7.20 (d, *H*-2), 7.31 (d, *H*-4), 7.64 (d, *H*-5) and 8.36 (br s, N*H*).

### EXAMPLE 4

### SYNTHESIS OF 9a-ETHYL-6-METHYL-1-NITRO-8,9,9a,10-TETRAHYDROINDENO[2,1-e]ENDOL-7(3H)-ONE

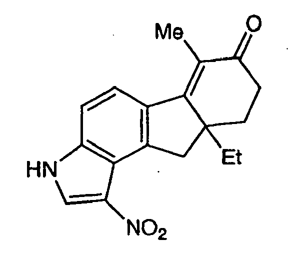

A solution of 9a-ethyl-6-methyl-8,9,9a,10-tetrahydroindeno[2,1-*e*]indol-7(3*H*)-one (11.8 mg, 0.044 mmol) in anhydrous dichloromethane (0.5 mL) was treated with 90% nitric acid (0.004 mL). The resulting dark solution was stirred at room temperature for 10 minutes and then partitioned between EtOAc (6 mL) and water (4 mL). The organic phase was washed with brine (5 mL), dried over MgSO₄ filtered, and evaporated under vacuum. The crude product was purified by preparative layer chromatography on two 0.025 x 20 x 20 cm silica gel GF plates which were developed with 5% MeOH in CH₂Cl₂. The product band was extracted with 10% MeOH in CH₂Cl₂, the extracts were evaporated under vacuum, and the residue was lyophilized from benzene to provide 9a-ethyl-6-methyl-1-nitro-8,9,9a,10-tetrahydroindeno[2,1-*e*]indol-7(3*H*)-one (1.9 mg) as an amorphous, brown solid.
¹H NMR (CDCl₃, 500 MHz) δ 0.87 (t, CH₂C*H*_{*3*}), 1.50 and 1.66 (two m, C*H*_{*2*}CH₃), 2.07 and 2.35 (two ddd, 9-C*H*_{*2*}), 2.15 (s, 6-C*H*_{*3*}), 2.52 and 2.62(two ddd, 8-C*H*_{*2*}), 3.27 and 3.80 (two d, 10-C*H*_{*2*}), 7.40 (d, *H*-4), 7.77 (d, *H*-5), 8.27 (d, *H*-2), and 9.05 (br s, N*H*).

### EXAMPLE 5

### SYNTHESIS OF 6-ACETYL-9a-BUTYL-4-FLUORO-8,9,9a,10-TETRAHYDROINDENO[2,1-e]INDOL-7(3H)-ONE

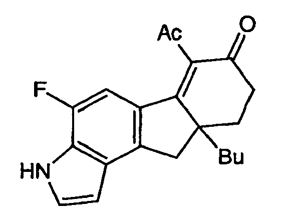

### Step 1: N-(4-bromo-2-fluorophenyl)-2,2-dimethylpropanamide

Pivaloyl chloride (72 mL, 580 mmol) was added slowly (caution - exothermic reaction) to an ice cold solution of 4-bromo-2-fluoroaniline (100 g, 526 mmol) in pyridine (200 mL). After the addition, the ice bath was removed and the hot reaction mixture was stirred at room temperature for 30 minutes. The mixture was poured into cold water (500 mL) and the precipitated product was collected by filtration and washed with 1N HCl. The solid was dissolved in EtOAc (1 L), dried over MgSO₄, filtered, and concentrated under vacuum to provide *N*-(4-bromo-2-fluorophenyl)-2,2-dimethylpropanamide (125 g).

### Step2: N-[2-fluoro-4-(1-hydroxyhexyl)phenyl]-2,2-dimethylpropanamide

A solution of *N*-(4-bromo-2-fluorophenyl)-2,2-dimethylpropanamide (22.6 g, 82.5 mmol) in anhydrous tetrahydrofuran (410 mL) was placed under a nitrogen atmosphere, cooled in a dry ice-acetone bath, and stirred while butyllithium (83 mL of a 2.5M solution in hexanes, 207.5 mmol) was added dropwise by syringe pump over 2.5 hours. The resulting mixture was aged at -78°C for 30 minutes and then treated with hexanal (25 mL, 208 mmol) added dropwise by syringe pump over 135 minutes. After stirring at -78°C for an additional 70 minutes, the mixture was removed from the cooling bath, treated with aqueous 50% saturated NH₄Cl, and the layers separated. The aqueous portion was extracted with EtOAc. The combined organics were washed with aqueous NaHCO₃ and brine, dried over MgSO₄, filtered, and concentrated under vacuum to an oil. The crude product was purified by flash chromatography on a Biotage 75L KP-Sil column, eluting with 4:1 hexanes-EtOAc (25 x 400 mL fractions). Fractions 9-22 were combined and evaporated under vacuum to afford *N*-[2-fluoro-4-(1-hydroxyhexyl)phenyl]-2,2-dimethylpropanamide (18.5 g, 76%) as a clear oil.

### Step 3: N-(2-fluoro-4-hexanoylphenyl)-2,2-dimethylpropanamide

A solution of *N*-[2-fluoro-4-(1-hydroxyhexyl)phenyl]-2,2-dimethylpropanamide (18.5 g, 62.6 mmol) in CH₂Cl₂ (300 mL) was treated with 4-methylmorpholine *N*-oxide (7.35 g, 62.6 mmol) followed by tetrapropylammonium perruthenate (TPAP, 0.22 g, 0.63 mmol). The mixture was stirred at room temperature for 30 minutes, then treated with additional TPAP (0.88 g, 2.5 mmol) and stirred an additional 30 minutes at room temperature. The mixture was filtered through a pad of MgSO₄ atop a pad of silica gel, washing through with EtOAc (500 mL). The filtrate was evaporated under vacuum to afford *N*-(2-fluoro-4-hexanoylphenyl)-2,2-dimethylpropanamide (17.8 g, 97%) as a white solid.

### Step 4: N-[4-(2-butylacryloyl)-2-fluorophenyl]-2,2-dimethylpropanamide,N-{2-fluoro-4-[2-(hydroxymethyl)hexanoyl]phenyl]-2,2-dimethyl]propanamide, and N-{2-fluoro-4-[2-(methoxymethyl)hexanoyl]phenyl]-2,2-dimethylpropanamide

A solution of *N*-(2-fluoro-4-hexanoylphenyl)-2,2-dimethylpropanamide (17.8 g, 60.7 mmol) in methanol (75 mL) was treated successively with K₂CO₃ (8.4 g, 60.7 mmol) and formaldehyde (37 wt. % solution in water, 5.0 mL, 67 mmol). The mixture was placed under a nitrogen atmosphere, stirred, and heated in an oil bath at 55°C for 4 hours. After cooling to room temperature, the mixture was diluted with CH₂Cl₂ (300 mL), dried over MgSO₄, and filtered through a pad of silica gel, using more CH₂Cl₂ (200 mL) to wash the pad. The filtrate and washings were concentrated under vacuum to provide a mixture (19.5 g) of *N*-[4-(2-butylacryloyl)-2-fluorophenyl]-2,2-dimethylpropanamide, *N*-{2-fluoro-4-[2-(hydroxymethyl)hexanoyl]phenyl]-2,2-dimethylpropanamide, and *N*-{2-fluoro-4-[2-(methoxymethyl)hexanoyl]phenyl]-2,2-dimethylpropanamide.

### Step 5: 5-amino-2-butyl-6-fluoro-1-indanone

A solution of the product mixture from step 4 (19.5 g, approx. 60.7 mmol) in CH₂Cl₂ (10 mL) was cooled in an ice bath and treated with ice-cold, conc. H₂SO₄ (300 mL). The resulting mixture was removed from the cooling bath and stirred at room temperature for 18 hours. The mixture was cautiously added to an ice-cold mixture of CH₂Cl₂ (300 mL) and chopped ice (1 L). Excess acid was neutralized by portionwise addition of saturated Na₂CO₃ solution (approx. 1 L) followed by solid Na₂CO₃ (approx. 500 g). Additional water and CH₂Cl₂ were occasionally added to dissolve the red/purple solids that formed during the neutralization. When the pH was neutral, the organic phase was separated and the aqueous portion was extracted with more CH₂Cl₂. The combined organics were washed with brine, dried over MgSO₄, filtered, and concentrated under vacuum. The residual dark red oil was purified by flash chromatography on a Biotage 75L KP-Sil column, eluting with 4:1 hexanes-EtOAc. The product containing fractions were combined and evaporated under vacuum to provide 5-amino-2-butyl-6-fluoro-1-indanone (5.5 g, 37%) as a yellow solid.

### Step 6: 5-amino-2-butyl-6-fluoro-2-(3-oxobutyl)-1-indanone

A solution of 5-amino-2-butyl-6-fluoro-1-indanone (0.98 g, 4.64 mmol) in ethanol (17 mL) was treated with sodium methoxide (0.5M solution in MeOH, 1.86 mL, 0.928 mmol) and methyl vinyl ketone (0.579 mL, 6.97 mmol). The resulting solution was stirred at room temperature and under a nitrogen atmosphere for 2 days. The mixture was diluted with CH₂Cl₂ and filtered through a pad of silica gel. The product washed off with EtOAc and the filtrate was evaporated under vacuum. The residue was purified by flash chromatography on a Biotage 40M KP-Sil column, eluting with 3:1 to 2.5:1 hexanes-EtOAc. The product containing fractions were evaporated under vacuum to provide to afford 5-amino-2-butyl-6-fluoro-2-(3-oxobutyl)-1-indanone (0.6 g, 46%) as a yellow foam.

### Step 7: 7-amino-9a-butyl-6-fluoro-1,2,9,9a-tetrahydro-3H-fluoren-3-one

The diketone from step 6 (0.6 g, 2.14 mmol) was dissolved in toluene (15 mL) and treated with acetic acid (0.236 mL, 4.12 mmol) and pyrrolidine (0.344 mL, 4.12 mmol). The resulting solution was stirred and heated in an oil bath at 100°C for 1.5 hours. After cooling to room temperature, the mixture was filtered through a pad of silica gel and the product was washed off with EtOAc. The filtrate and washings were concentrated under vacuum. The residue was purified by flash chromatography on a Biotage 40M KP-Sil column, eluting with 4:1 to 3:1 hexanes-EtOAc. The product containing fractions were evaporated under vacuum to provide 7-amino-9a-butyl-6-fluoro-1,2,9,9a-tetrahydro-3*H*-fluoren-3-one (0.45 g, 80%) as a yellow solid.

### Step 8: 7-(acetylamino)-9a-butyl-6-fluoro-1,2,9,9a-tetrahydro-3H-fluoren-3-one

A solution of 7-amino-9a-butyl-6-fluoro-1,2,9,9a-tetrahydro-3*H-*fluoren-3-one (1.12 g, 4.1 mmol) in anhydrous CH₂Cl₂ (13.7 mL) was purged with N₂, cooled in an ice bath, and treated with pyridine (0.33 mL, 4.1 mmol) followed by acetyl chloride (0.65 mL, 9 mmol). After stirring at 0-5°C for 4.5 hours, the reaction mixture was partitioned between water and EtOAc. The organic portion was washed with brine, dried over MgSO₄, filtered, and concentrated under vacuum to provide 7-(acetylamino)-9a-butyl-6-fluoro-1,2,9,9a-tetrahydro-3*H*-fluoren-3-one (1.1 g) as an orange foam. This material was used without further purification.

### Step 9: 7-(acetylamino)-4-bromo-9a-butyl-6-fluoro-1,2,9,9a-tetrahydro-3H-fluoren-3-one

The product from step 8 (1.1 g, approx. 3.5 mmol) was dissolved in anhydrous CH₂Cl₂ (11 mL) and the solution was purged with N₂, cooled in an ice bath, and treated with *N*-bromosuccinimide (0.498 g, 2.8 mmol). After stirring at 0-5°C for one hour, the reaction mixture was partitioned between water (150 mL) and EtOAc (150 mL). The organic portion was washed with aqueous 5% NaHCO₃ and brine, dried over MgSO₄, filtered, and evaporated under vacuum to provide crude 7-(acetylamino)-4-bromo-9a-butyl-6-fluoro-1,2,9,9a-tetrahydro-3*H*-fluoren-3-one (1.35 g).

### Step 10: 7-(acetylamino)-9a-butyl-4-(1-ethoxyvinyl)-6-fluoro-1,2,9,9a-tetrahydro-3H-fluoren-3-one

A partial solution of 7-(acetylamino)-4-bromo-9a-butyl-6-fluoro-1,2,9,9a-tetrahydro-3*H*-fluoren-3-one (495 mg, 1.26 mmol) in anhydrous toluene (6.3 mL) was purged with N₂, treated with dichlorobis(triphenylphosphine)palladium(II) (177 mg, 0.25 mmol), purged with N₂, and treated with tributyl(1-ethoxyvinyl)tin (0.600 mL, 1.9 mmol). The resulting mixture was stirred under a N₂ atmosphere and heated in an oil bath at 100°C for two hours. After cooling to room temperature, the mixture was filtered through a pad of silica gel, washing the product off with EtOAc. The filtrate and washings were evaporated under vacuum to an oil. The crude product was purified by flash chromatography on a Biotage 40S KP-Sil column, eluting with 4:1 hexanes-EtOAc (1 L) followed by 2:1 hexanes-EtOAc (1 L). The product containing fractions were concentrated under vacuum to afford 7-(acetylamino)-9a-butyl-4-(1-ethoxyvinyl)-6-fluoro-1,2,9,9a-tetrahydro-3*H* fluoren-3-one (238 mg, 49%) as a yellow foam.

### Step 11: 4-acetyl-7-amino-9a-butyl-6-fluoro-1,2,9,9a-tetrahydro-3H-fluoren-3-one

A solution of 7-(acetylamino)-9a-butyl-4-(1-ethoxyvinyl)-6-fluoro-1,2,9,9a-tetrahydro-3*H*-fluoren-3-one (205 mg, 0.53 mmol) in methanol (7.5 mL) was treated with aqueous 6N HCl (7.5 mL). The resulting mixture was placed under a N₂ atmosphere and stirred with heating in an oil bath at 80°C for 50 minutes. After cooling to room temperature, the mixture was partitioned between EtOAc and aqueous 5% NaHCO₃. The organic solution was washed with water and brine, dried over MgSO₄, filtered, and evaporated under vacuum to afford 4-acetyl-7-amino-9a-butyl-6-fluoro-1,2,9,9a-tetrahydro-3*H*-fluoren-3-one (157 mg, 93%) as a foam.

### Step 12: 4-acetyl-7-amino-8-bromo-9a-butyl-6-fluoro-1,2,9,9a-tetrahydro-3H-fluoren-3-one

A solution of 4-acetyl-7-amino-9a-butyl-6-fluoro-1,2,9,9a-tetrahydro-3*H*-fluoren-3-one (157 mg, 0.5 mmol) in anhydrous *N,N*-dimethylformamide (1.5 mL) was purged with N₂, cooled in an ice bath, and treated with *N*-bromosuccinimide (89 mg, 0.5 mmol). The resulting solution was stirred at 0-5°C for one hour, and then partitioned between EtOAc and water. The aqueous portion was extracted with more EtOAc. The combined organics were washed with water and brine, dried over MgSO₄, filtered, and concentrated under vacuum. The oily residue was purified by flash chromatography on a Biotage 12M column, eluting with 4:1 hexanes-EtOAc. The product containing fractions were evaporated under vacuum to afford 4-acetyl-7-amino-8-bromo-9a-butyl-6-fluoro-1,2,9,9a-tetrahydro-3*H*-fluoren-3-one (147 mg, 75%) as a yellow oil.

### Step 13: 4-acetyl-8-allyl-7-amino-9a-butyl-6-fluoro-1,2,9,9a-tetrahydro-3H-fluoren-3-one

A sample of 4-acetyl-7-amino-8-bromo-9a-butyl-6-fluoro-1,2,9,9a-tetrahydro-3*H*-fluoren-3-one (123.5 mg, 0.31 mmol) and bis(tri-t-butylphosphine)-palladium(0) (6.5 mg, 0.013 mmol) were dissolved in anhydrous toluene (1.5 mL). The solution was purged with N₂, treated with allyltributyltin (0.150 mL, 0.48 mmol), and stirred with heating in an oil bath at 100°C for 2.5 hours. After cooling, the mixture was filtered through a plug of silica gel with EtOAc. The filtrate was concentrated under vacuum to a residue that was purified by preparative layer chromatography (0.1 x 20 x 20 cm silica gel GF plate developed with 2:1 hexanes-EtOAc). The product band was eluted with EtOAc and the eluant evaporated under vacuum to afford 4-acetyl-8-allyl-7-amino-9a-butyl-6-fluoro-1,2,9,9a-tetrahydro-3*H-*fluoren-3-one (31 mg, 28%) as an orange foam.

### Step 14: 4-acetyl-7-(acetylamino)-8-allyl-9a-butyl-6-fluoro-1,2,9,9a-tetrahydro-3H fluoren-3-one

Pyridine (0.007 mL, 0.087 mmol) and acetyl chloride (0.012 mL, 0.174 mmol) were added to an ice-cold solution of 4-acetyl-8-allyl-7-amino-9a-butyl-6-fluoro-1,2,9,9a-tetrahydro-3*H*-fluoren-3-one (30.8 mg, 0.087 mmol) in anhydrous CH₂Cl₂ (0.3 mL). The resulting mixture was stirred at room temperature and under a N₂ atmosphere for 19 hours. The mixture was partitioned between EtOAc and 1N HCl. The organic phase was washed with 5% NaHCO₃ and brine, dried over MgSO₄, filtered, and concentrated under vacuum to provide crude 4-acetyl-7-(acetylamino)-8-allyl-9a-butyl-6-fluoro-1,2,9,9a-tetrahydro-3*H*-fluoren-3-one (31.6 mg).

### Step 15: 4-acetyl-7-(acetylamino)-9a-butyl-6-fluoro-8-(2-oxoethyl)-1.2.9.9a-tetrahydro-3H-fluoren-3-one

A solution of 4-acetyl-7-(acetylamino)-8-allyl-9a-butyl-6-fluoro-1,2,9,9a-tetrahydro-3*H*-fluoren-3-one (31.6 mg, 0.08 mmol) in THF (0.8 mL) was treated sequentially with OsO₄ in THF (0.2 mL of a 10 mg/mL solution, 0.008 mmol) and NaIO₄ (51 mg, 0.24 mmol). The mixture was stirred at room temperature for one hour, then treated with water and stirred an additional hour at room temperature. The mixture was partitioned between EtOAc and water. The organic phase was washed with brine, dried over MgSO₄, filtered, and evaporated under vacuum to provide crude 4-acetyl-7-(acetylamino)-9a-butyl-6-fluoro-8-(2-oxoethyl)-1,2,9,9a-tetrahydro-3*H*-fluoren-3-one (40 mg).

### Step 16: 6-acetyl-9a-butyl-4-fluoro-8,9,9a,10-tetrahydroindeno[2,1-e]indol-7(3H)-one

The crude product from step 15 was dissolved in CH₂Cl₂ (~1 mL) and treated with a catalytic amount of p-toluenesulfonic acid monohydrate. The reaction was stirred at room temperature for 5 minutes to affect cyclization to the *N-*acetylindole intermediate. The reaction mixture was diluted with EtOH (2 mL), treated with aqueous 5N NaOH (0.150 mL), and stirred at room temperature for 20 minutes to affect deacetylation. The mixture was diluted with CH₂Cl₂ and filtered through a plug of silica gel. The filtrate was evaporated under vacuum and the residue purified by preparative layer chromatography (0.1 x 20 x 20 silica GF plate developed with 2:1 hexanes-EtOAc). The product band was eluted with EtOAc, the eluant evaporated under vacuum, and the residue lyophilized from benzene to afford 6-acetyl-9a-butyl-4-fluoro-8,9,9a,10-tetrahydroindeno[2,1-*e*]indol-7(3*H*)-one (17 mg) as an amorphous solid.
¹H NMR (CDCl₃, 500 MHz) δ 0.83 (t, CH₂CH₂CH₂C*H*_{*3*}), 1.19-1.32 (m, CH₂C*H*_{*2*}C*H*_{*2*}CH₃), 1.49 and 1.71 (two m, C*H*_{*2*}CH₂CH₂CH₃), 2.10 and 2.34 (two ddd, 9-CH₂), 2.43 (s, COC*H*_{*3*}), 2.52 and 2.61 (two ddd, 8-C*H*_{*2*}), 2.86 and 3.22 (two d, 10-C*H*_{*2*}), 6.61 (dd, *H*-1), 7.03 (d, *H*-5), 7.30 (dd, *H*-2), and 8.70 (br s, N*H*).

### EXAMPLE 6

### SYNTHESIS OF 9a-ETHYL-6-METHYL-8,9,9a,10-TETRAHYDROINDENO[2,1-e]INDAZOL-7(3H)-ONE

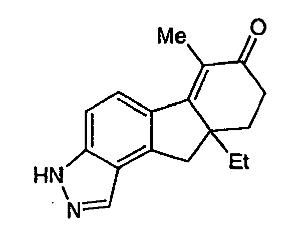

### Step 1: 5-(acetylamino)-4-bromo-1-indanone

A suspension of 5-(acetylamino)-1-indanone (10.0 g, 52.3 mmol) in anhydrous acetonitrile (52.3 mL) was treated with *N*-bromosuccinimide (9.8 g, 54.9 mmol). The resulting mixture was stirred and heated in an oil bath at 60°C for 24 hours. After cooling to room temperature, the mixture was concentrated under vacuum to approximately half volume and the resulting white suspension was stirred overnight at room temperature. The mixture was filtered and the solid portion was washed with cold MeCN (10 mL) and dried under a stream of nitrogen to afford 5-(acetylamino)-4-bromo-1-indanone (10.4 g, 74% yield) as white crystals.

### Step 2: 5-(acetylamino)-4-methyl-1-indanone

A mixture of 5-(acetylamino)-4-bromo-1-indanone (65.16 g, 0.243 mol), dichlorobis(triphenylphosphine)palladium(II) (8.50 g, 0.012 mol), triphenylphosphine (6.37 g, 0.024 mol), and lithium chloride (20.6 g, 0.486 mol) in anhydrous *N,N* dimethylformamide (DMF, 486 mL) was purged with nitrogen and treated with tetramethyltin (37.1 mL, 0.267 mol). The mixture was stirred under a nitrogen atmosphere and heated in an oil bath at 100°C for 22.3 hours. After cooling to room temperature, the mixture was filtered and the solid portion washed with DMF (20 mL). The filtrate and washings were evaporated under vacuum to a residue that was partitioned between 5% MeOH in CH₂Cl₂ (500 mL) and brine (500 mL). The aqueous phase was back-extracted with more 5% MeOH in CH₂Cl₂ (500 mL). The combined organics were dried over MgSO₄, filtered, and evaporated under vacuum to a semi-solid. This material was suspended in Et₂O (250 mL) and the mixture was filtered. The solid was washed with Et₂O (250 ml) and dried under a stream of nitrogen to afford crude product (46 g). The crude product, plus 3 gm of additional crude from a previous reaction, was purified by recrystallization from hot MeCN (400 mL). After cooling the mixture to room temperature, the solid was collected, washed with MeCN (50 mL) and Et₂O (200 mL), and dried under a stream of nitrogen to afford 5-(acetylamino)-4-methyl-1-indanone (37.9 g) as pale yellow crystals. The mother liquors and washings, after concentration to approximately 100-125 mL, gave additional product (6.0 g) as yellow needles.

### Step 3: 5-(acelylamino)-2-ethyl-4-methyl-1-indanone

A suspension of 5-(acetylamino)-4-methyl-1-indanone (0.50.g, 2.46 mmol) and 20% palladium hydroxide on carbon (50 mg) in ethanol (10 mL) was sonicated for 0.5 minute, then cooled in an ice bath and treated with 0.5M sodium methoxide in methanol (0.98 mL, 0.49 mmol). The mixture was placed under a hydrogen atmosphere and treated with acetaldehyde (0.28 mL, 4.92 mmol). The resulting mixture was stirred under a hydrogen atmosphere for one hour 0°C and then stirred at room temperature for three hours. The mixture was filtered and the filtrate evaporated under vacuum. The residue was dissolved in ethanol (10 mL) and reevaporated under vacuum. The residue in EtOAc (20 mL) was washed with water (20 mL) containing 2N HCl (1.5 mL), washed with brine (10 mL), dried over MgSO₄, filtered, and evaporated under vacuum to provide crude 5-(acetylamino)-2-ethyl-4-methyl-1-indanone (512 mg) as a foam.

### Step 4: 5-(acetylamino)-2-ethyl-4-methyl-2-(3-oxopentyl)-1-indanone

The crude 5-(acetylamino)-2-ethyl-4-methyl-1-indanone (500 mg, approx. 2.1 mmol) from step 3 was dissolved in methanol (5.4 mL) and the solution was treated with ethyl vinyl ketone (0.27 mL, 2.7 mmol) and 0.5M sodium methoxide in methanol (0.98 mL, 0.49 mmol). The mixture was stirred and heated in an oil bath at 60°C for 6 hours and then stirred at room temperature for 12 hours. The solvent was evaporated under vacuum and the residue was partitioned between EtOAc (20 mL) and water (20 will) made basic with K₂CO₃. The EtOAc phase was washed with brine (10 mL), dried over MgSO₄, filtered, and evaporated under vacuum to afford crude 5-(acetylamino)-2-ethyl-4-methyl-2-(3-oxopentyl)-1-indanone (633 mg) as an oil. The NMR spectrum revealed that the product contained approximately 20-25% of the corresponding deacetylated derivative.

### Step 5: 7-amino-9a-ethyl-4,8-dimethyl-1,2,9,9a-tetrahydro-3H-fluoren-3-one

The crude mixture (0.63 g) of 5-(acetylamino)-2-ethyl-4-methyl-2-(3-oxopentyl)-1-indanone and 5-amino-2-ethyl-4-methyl-2-(3-oxopentyl)-1-indanone from step 4 was dissolved in acetic acid (9 mL) and the solution was diluted with aqueous 6N HCl (9 mL). The mixture was stirred and heated in an oil bath at 80°C for 4.5 hours, then allowed to cool to room temperature and evaporated under vacuum to an oily residue. The residue was partitioned between EtOAc (50 mL) and saturated aqueous K₂CO₃. The organic phase was dried over MgSO₄, filtered, and evaporated under vacuum to provide 7-amino-9a-ethyl-4,8-dimethyl-1,2,9,9a-tetrahydro-3*H-*fluoren-3-one (0.50 g) as a yellow-brown foam.

### Step 6: 9a-ethyl-6-methyl-8,9,9a,10-tetrahydroindeno[2,1-e]indazol-7(3H)-one

An ice-cold solution of 7-amino-9a-ethyl-4,8-dimethyl-1,2,9,9a-tetrahydro-3*H*-fluoren-3-one (0.50 g, 1.96 mmol) in anhydrous dichloromethane (9.8 mL) was treated with nitrosonium tetrafluoroborate (252 mg, 2.16 mmol). The resulting dark solution was stirred at 0°C for 15 minutes, and then treated with potassium acetate (385 mg, 3.92 mmol) and dibenzo-18-crown-6 (35 mg, 0.098 mmol). After stirring an additional 5 minutes at 0°C, the mixture was briefly sonicated at which time gas evolution was observed. After stirring an additional 10 minutes at 0°C, the mixture was diluted with CH₂Cl₂ (20 mL), washed with water (20 mL), dried over MgSO₄, filtered, and concentrated under vacuum to an oil (550 mg). The crude product was purified by column chromatography on EM silica gel 60 (20 g, 230-400 mesh) using 10% EtOAc in CH₂Cl₂ as the eluting solvent. The product containing fractions were combined and evaporated under vacuum to a red-brown solid (269 mg). This material was dissolved in hot EtOH (4 mL) and the solution was decolorized with activated carbon (208 mg). Filtration and evaporation of the filtrate under vacuum gave a yellow oil (228 mg) that crystallized from benzene (2 mL) to provide 9a-ethyl-6-methyl-8,9,9a,10-tetrahydroindeno[2,1-*e*]indazol-7(3*H*)-one (214 mg) as a pale yellow solid. The proton NMR indicated that the product was a benzene hemi-solvate. The product can be freed of benzene by dissolving in ethanol, evaporating the solvent under vacuum, triturating the residue with water, and drying the resulting solid under high vacuum.
¹H NMR (CDCl₃, 500 MHz) δ 0.87 (t, CH₂C*H*_{*3*}), 1.53 and 1.69 (two m, C*H*_{*2*}CH₃), 2.08 and 2.34 (two ddd, 9-C*H*_{*2*}), 2.16 (s, 6-C*H*_{*3*}), 2.52 and 2.62 (two ddd, 8-C*H*_{*2*}), 2.94 and 3.29 (two d, 10-C*H*_{*2*}), 7.46 (d, *H*-4), 7.82 (d, *H*-5), 8.13 (s, *H-*1), and 10.32 (br s, N*H*).

### EXAMPLE 7

### SYNTHESIS OF 6-BROMO-9a-ETHYL-8,9,9a,10-TETRAHYDROINDENO[2,1-e]INDAZOL-7(3H)-ONE

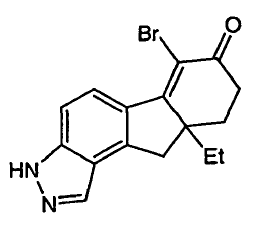

### Step 1: 5-(acetylamino)-2-ethyl-4-methyl-1-indanone

A mixture of 5-(acetylamino)-4-methyl-1-indanone (5.0 g, 24.6 mmol), 20% palladium hydroxide on carbon (0.5 g), ethanol (100 mL), and 0.5M sodium methoxide in methanol (9.84 mL, 4.92 mmol)was cooled in an ice bath, placed under a hydrogen atmosphere, and treated with acetaldehyde (2.8 mL, 49.2 mmol). The resulting mixture was stirred under a hydrogen atmosphere at 0°C for 0.5 hours followed by 2.7 hours at room temperature. The mixture was filtered and the filtrate evaporated under vacuum. The residue was dissolved in ethanol (100 mL) and reevaporated under vacuum. The residue of crude 5-(acetylamino)-2-ethyl-4-methyl-1-indanone plus sodium ethoxide was used directly in the next step.

### Step 2: 5-(acetylamino)-2-ethyl-4-methyl-2-(3-oxobutyl)-1-indanone

The crude mixture of sodium ethoxide and 5-(acetylamino)-2-ethyl-4-methyl-1-indanone from step 1 was dissolved in ethanol (61.5 mL) and the solution was treated with methyl vinyl ketone (2.56 mL, 30.75 mmol). The mixture was stirred at room temperature for 39 hours. The solvent was evaporated under vacuum and the residue was partitioned between EtOAc (100 mL) and brine (100 mL). The aqueous phase was back-extracted with EtOAc (50 mL). The combined organics were dried over MgSO₄, filtered, and evaporated under vacuum to afford crude 5-(acetylamino)-2-ethyl-4-methyl-2-(3-oxobutyl)-1-indanone (8.5 g) as a foam.

### Step 3: 7-(acetylamino)-9a-ethyl-8-methyl-1,2,9,9a-tetrahydro-3H-fluoren-3-one

A mixture of crude 5-(acetylamino)-2-ethyl-4-methyl-2-(3-oxobutyl)-1-indanone (8.5 g, approx. 24.6 mmol)), toluene (225 mL), and tetrahydrofuran (20 mL) was treated with pyrrolidine (2.06 mL, 24.6 mmol) and acetic acid (1.41 mL, 24.6 mmol). The resulting mixture was stirred and heated in an oil bath at 100°C for 75 minutes, then cooled to room temperature and evaporated under vacuum. The residue was partitioned between EtOAc (200 mL) and water (100 mL) and the aqueous phase was back-extracted with EtOAc(50 mL). The combined organics were washed with brine (100 mL), dried over MgSO₄, filtered, and evaporated under vacuum to afford crude 7-(acetylamino)-9a-ethyl-8-methyl-1,2,9,9a-tetrahydro-3*H-*fluoren-3-one as a foam.

### Step 4: 7-(acetylamino)-4-bromo-9a-ethyl-8-methyl-1,2,9,9a-tetrahydro-3H-fluoren-3-one

The crude 7-(acetylaniino)-9a-ethyl-8-methyl-1,2,9,9a-tetrahydro-3*H-*fluoren-3-one (approx. 22-23 mmol) from step 3 was dissolved in anhydrous dichloromethane (220 ml) and the solution was treated with sodium bicarbonate (9.3 g, 110.7 mmol). The mixture was cooled in an ice bath and swirled by hand while bromine (1.14 mL, 22.1 mmol) was added dropwise over 3 minutes. The mixture was stirred at 0°C for 25 minutes, after which time additional bromine (0.05 mL, 1 mmol) was added. The mixture was stirred an additional 15 minutes at 0°C, then diluted with water (100 mL) and shaken. The organic phase was dried over MgSO₄, filtered, and evaporated under vacuum to provide crude 7-(acetylamino)-4-bromo-9a-ethyl-8-methyl-1,2,9,9a-tetrahydro-3*H*-fluoren-3-one (9.0 g) as a dark foam.

### Step 5: 7-amino-4-bromo-9a-ethyl-8-methyl-1,2,9,9a-tetrahydro-3H-fluoren-3-one

The crude 7-(acetylamino)-4-bromo-9a-ethyl-8-methyl-1,2,9,9a-tetrahydro-3*H*-fluoren-3-one (9.0 g) from step 4 was dissolved in ethanol (125 mL) and the solution was treated with 0.5M sodium methoxide in methanol (123 mL, 61.5 mmol). The resulting solution was stirred under a nitrogen atmosphere and heated in an oil bath at 80°C for 12 hours. After cooling to room temperature, the mixture was evaporated under vacuum and the residue was partitioned between EtOAc (200 mL) and brine (100 mL). The organic phase was dried over MgSO₄, filtered ,and evaporated under vacuum to afford crude 7-amino-4-bromo-9a-ethyl-8-methyl-1,2,9,9a-tetrahydro-3*H*-fluoren-3-one (7.0 g) as a dark foam.

### Step 6: 6-bromo-9a-ethyl-8,9,9a,10-tetrahydroindeno[2,1-e]indazol-7(3H)-one

The crude 7-amino-4-bromo-9a-ethyl-8-methyl-1,2,9,9a-tetrahydro-3*H*-fluoren-3-one (7.0 g, approx. 22 mmol) from step 5 was dissolved in anhydrous dichloromethane (125 mL) and the solution was cooled in a dry ice-acetonitrile bath at -35°C. The cold solution was treated with nitrosonium tetrafluoroborate (2.59 g, 22.1 mmol) and then stirred with gradual warming to 0°C (bath temperature) over 70 minutes. Additional nitrosonium tetrafluoroborate (0.22 g, 1.9 mmol) was added and stirring was continued for 50 minutes, at which time the bath temperature was 7°C. The cooling bath was brought to -35°C and the reaction mixture was diluted with dichloromethane (125 ml). Potassium acetate (4.35 g, 44.3 mmol) and dibenzo-18-crown-6 (0.40 g, 1.1 mmol) were added and the resulting mixture was removed from the cooling bath and stirred at room temperature over 2 hours. The mixture was shaken with water (100 mL) and filtered through a celite pad. The organic portion of the filtrate was dried over MgSO4, filtered, and evaporated under vacuum to an oil (7.6 g). The crude product was purified by column chromatography on EM silica gel 60 (230-400 mesh, 4.5 x 31 cm column) using 20% EtOAc in CH₂Cl₂ as the eluting solvent. The product containing fractions were combined and evaporated under vacuum to a residue that crystallized on addition of benzene (20 mL). The solid was collected and dried under vacuum to afford 6-bromo-9a-ethyl-8,9,9a,10-tetrahydroindeno[2,1-*e*]indazol-7(3*H*)-one (2.7 g) as a tan solid.
¹H NMR (CDCl₃, 500 MHz) δ 0.90 (t, CH₂C*H*_{*3*}), 1.63 and 1.76 (two m, C*H*_{*2*}CH₃), 2.17 and 2.36 (two ddd, 9-C*H*_{*2*}), 2.72-2.84 (m, 8-*CH*₂), 3.02 and 3.34 (two d, 10-C*H*_{*2*}), 7.51 (d, *H*-4), 8.15 (s, *H*-1), and 8.66 (d, *H*-5).

### EXAMPLE 8

### SYNTHESIS OF 9a-ETHYL-6-TRIFLUOROMETHYL-8,9,9a,10-TETRAHYDROINDENO[2,1-e] INDAZOL-7(3H)-ONE

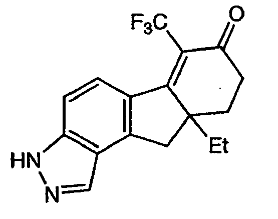

### Step 1: 7-(acetylamino)-9a-ethyl-8-methyl-4-trifluoromethyl-1,2,9,9a-tetrahydro-3H-fluoren-3-one

7-(Acetylamino)-4.-bromo-9a-ethyl-8-methyl-1,2,9,9a-tetrahydro-3*H* fluoren-3-one (1.2 g, 3.32 mmol) and copper(I) iodide (0.76 g, 4.0 mmol) were taken up in anhydrous *N,N*-dimethylformamide (66.4 mL). The resulting mixture was placed under a N₂ atmosphere, treated with methyl (fluorosulfonyl)difluoroacetate (3.2 mL, 25.2 mmol), and then stirred with heating in an oil bath at 75-80°C for 15.5 hours. After cooling to room temperature, the mixture was filtered through a pad of solka floc which was rinsed with EtOAc (50 mL): The combined filtrate and rinsate were diluted with EtOAc (500 mL) and H₂O (500 mL) and the mixture was filtered through a solka floc pad. The organic phase was washed with water (8 x 500 mL, brine added to separate the layers) and brine (200 mL), dried over MgSO₄, filtered, and concentrated under vacuum to a yellow oil (1.6 g). The crude product was purified by column chromatography on EM silica gel 60 (230-400 mesh, 2.75 x 20 cm), eluting sequentially with 10% EtOAc in CH₂Cl₂ (200 mL), 20% EtOAc in CH₂Cl₂ (200 mL), and 50 % EtOAc in CH₂Cl₂. The product containing fractions were combined and evaporated under vacuum to afford 7-(acetylamino)-9a-ethyl-8-methyl-4-trifluoromethyl-1,2,9,9a-tetrahydro-3*H* fluoren-3-one (0.73 g) as an oil.

### Step 2: 7-amino-9a-ethyl-8-methyl-4-trifluoromethyl-1,2,9,9a-tetrahydro-3H-fluoren-3-one

A solution of 7-(acetylamino)-9a-ethyl-8-methyl-4-trifluoromethyl-1,2,9,9a-tetrahydro-3*H*-fluoren-3-one (730 mg) in EtOH (20 mL) was treated with water (5 mL) and conc. HCI (3 mL). The resulting yellow solution was stirred and heated in an oil bath at 80°C for 4.8 hours. After cooling to room temperature, the mixture was partitioned between EtOAc (100 mL) and aq. saturated NaHCO₃. The organic phase was washed with brine (100 mL), dried over MgSO₄, filtered, and evaporated under vacuum to provide 7-amino-9a-ethyl-8-methyl-4-trifluoromethyl-1,2,9,9a-tetrahydro-3*H*-fluoren-3-one (650 mg) as a yellow foam.

### Step 3: 9a-ethyl-6-trifluoromethyl-8,9,9a,10-tetrahydroindeno[2,1,e]indazol-7(3H)-one

A portion of the crude 7-amino-9a-ethyl-8-methyl-4-trifluoromethyl-1,2,9,9a-tetrahydro-3*H*-fluoren-3-one (409 mg, 1.32 mmol) from step 2 was dissolved in anhydrous CH₂Cl₂ (10 mL) and the solution was cooled in a dry ice-acetonitrile bath at -30°C. The cold solution was treated with nitrosonium tetrafluoroborate (155 mg, 1.32 mmol) and then stirred with gradual warming to -5°C (bath temperature) over 100 minutes. The reaction mixture was treated with KOAc (260 mg, 2.64 mmol) and dibenzo-18-crown-6 (24 mg, 0.066 mmol) and stirred at-5°C for 5 minutes. The mixture was removed from the cooling bath and stirred at room temperature for 15 minutes. The mixture was partitioned between CH₂Cl₂ (50 mL) and water (50 mL). The organic phase was dried over MgSO₄, filtered, and evaporated under vacuum to a red oil (468 mg). The crude product was purified by column chromatography on EM silica gel 60 (230-400 mesh, 3.5 x 19 cm column) using 5% EtOAc in CH₂Cl₂ as the eluting solvent. The product containing fractions were combined and evaporated under vacuum to afford 9a-ethyl-6-trifluoromethyl-8,9,9a,10-tetrahydroindeno[2,1-*e*]indazol-7(3*H*)-one (190 mg) as a red oil.
¹H NMR (CDCl₃, 500 MHz) δ 0.86 (t, CH₂C*H*_{*3*}), 1.45 and 1.65 (two dq, C*H*_{*2*}CH₃), 2.16 and 2.33 (two ddd, 9-C*H*₂), 2.54-2.67 (m, 8-C*H*₂), 3.06 and 3.32 (two d, 10-C*H*₂), 7.45 (d, *H*-4), 7.84 (qd, *H*-5), and 8.15 (s, *H*-1); mass spectrum m/z 321.1 (M+1).

### EXAMPLE 9

### SYNTHESIS OF 9a-ETHYL-6-14-{2-(1-PIPERIDINYL)ETHOXY]PHENYL}-8,9,9a,10-TETRAHYDROINDENO[2,1-e]INDAZOL-7(3H)-ONE HYDROCHLORIDE SALT

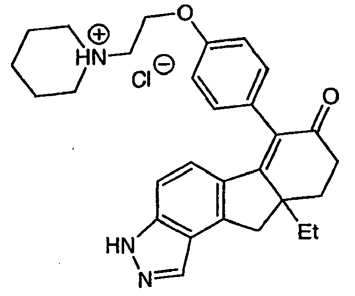

### Step 1: 6-bromo-9a-ethyl-3-[(4-methylphenyl)sulfonyl]-8,9,9a,10-tetrahydroindeno[2,1-e]indazol-7(3H)-one and 6-bromo-9a-ethyl-2-[(4-methylnhenyl)sulfonyl]-8,9,9a,10-tetrahydroindeno[2,1-e]indazol-7(2H)-one

A solution of 6-bromo-9a-ethyl-8,9,9a,10-tetrahydroindeno[2,1-*e*]indazol-7(3*H*)-one hemi-benzene solvate (250 mg, 0.675 mmol) in anhydrous dichloromethane (3.4 mL) was cooled in an ice bath and treated with 4-(dimethylamino)pyridine (124 mg, 1.015 mmol) andp-toluenesulfonyl chloride (155 mg, 0.813 mmol). The resulting solution was stirred under a nitrogen atmosphere for one hour at 0°C followed by two hours at room temperature, then diluted with dichloromethane (30 mL) and washed with water (20 mL), 1M pH 3 phosphate (20 mL), 5% aq: NaHCO₃ (20 mL), and brine (20 mL). The dichloromethane solution was dried over MgSO₄, filtered, and evaporated under vacuum to a foam (320 mg). The crude product was purified by chromatography on a Biotage Flash 40S KP-Sil column, eluting with 7:3 hexanes-ethyl acetate, to afford 6-bromo-9a-ethyl-2-[(4-methylphenyl)sulfonyl]-8,9,9a,10-tetrahydroindeno[2,1-*e*]indazol-7(2*H*)-one (188 mg, contains 14% of the 3-tosyl isomer) and 6-bromo-9a-ethyl-3-[(4-methylphenyl)sulfonyl]-8,9,9a,10-tetrahydroindeno[2,1-*e*]indazol-7(3*H*)-one (107 mg, contains trace amounts of the 2-tosyl isomer) as solids.

### Step 2: 9a-ethyl-6-[4-(methoxymethoxy)phenyl]-3-[(4-methylphenyl)sulfonyl]-8,9,9a,10-tetrahydroindeno[2,1-e]lindazol-7(3H)-one

A mixture of 6-bromo-9a-ethyl-3-[(4-methylphenyl)sulfonyl]-8,9,9a,10-tetrahydroindeno[2,1-*e*]indazol-7(3*H*)-one (107 mg, 0.220 mmol) and tetrakis(triphenylphosphine)palladium(0) 12.7 mg, 0.011 mmol) was treated with a solution of tributyl[4-(methoxymethoxy)phenyl]stannane (103 mg, 0.241 mmol) in anhydrous toluene (1.6 mL). The mixture was purged with nitrogen, sonicated to give a suspension, then stirred with heating in an oil bath at 100°C for 15 hours.. After cooling to room temperature, the mixture was evaporated under vacuum to a violet oil (236 mg). The crude product was purified by chromatography on a Biotage 12M KP-Sil column, eluting with 7:3 hexanes-EtOAc, to afford 9a-ethyl-6-[4-(methoxymethoxy)phenyl]-3-[(4-methylphenyl)sulfonyl]-8,9,9a,10-tetrahydroindeno[2,1-*e*]indazol-7(3*H*)-one (113 mg, 95% yield) as a pale yellow gum.

### Step 3: 9a-ethyl-6-(4-hydroxyphenyl)-3-[(4-methylphenyl)sulfonyl]-8.9,9a,10-tetrahydroindeno[2,1-e]indazol-7(3H)-one

A mixture of 9a-ethyl-6-[4-(methoxymethoxy)phenyl]-3-[(4-methylphenyl)sulfonyl]-8,9,9a,10-tetrahydroindeno[2,1-*e*]indazol-7(3*H*)-one (113 mg, 0.208 mmol) and methanol (5.2 mL) was sonicated to give a suspension. Aqueous 2N HCl (0.52 mL) was added dropwise and the resulting mixture was stirred in a capped flask and heated in an oil bath at 80°C. After 10 minutes, the suspension gave way to a pale yellow solution. After 45 minutes, the mixture was allowed to cool to room temperature and the solvent was evaporated under vacuum. The residue was dissolved in EtOAc (10 mL), washed with water (5 mL) and brine (5 mL), dried over MgSO₄, filtered, and evaporated under vacuum. The oily residue was lyophilized from benzene to provide 9a-ethyl-6-(4-hydroxyphenyl)-3-[(4-methylphenyl)sulfonyl]-8,9,9a,10-tetrahydroindeno[2,1-*e*]indazol-7(3*H*)-one (95 mg, 92% yield) as an off-white amorphous solid.

### Step 4: 9a-ethyl-3-[(4-methylphenyl)sulfonyl]-6-{4-[2-(1-piperidinyl)ethoxy]Phenyl}-8,9,9a,10-tetrahydroindeno[2,1-e]indazol-7(3H)-one

A solution of 9a-ethyl-6-(4-hydroxyphenyl)-3-[(4-methylphenyl)sulfonyl]-8,9,9a,10-tetrahydroindeno[2,1-*e*]indazol-7(3*H*)-one (95 mg, 0.19 mmol), triphenylphosphine (150 mg, 0.57 mmol) and piperidineethanol (0.076 mL, 0.57 mmol) in anhydrous THF (1.5 mL) was cooled in an ice bath, placed under a nitrogen atmosphere, and stirred while a solution of diisopropyl azodicarboxylate (95%,0.118 mL, 0.57 mmol) in THF (0.4 mL) was added dropwise over 4 minutes. The reaction mixture was stirred at 0°C for one hour followed by 5 hours at room temperature, then evaporated under vacuum to a yellow gum (450 mg). The residue was purified by chromatography on a Biotage Flash12M KP-Sil column, eluting with 5% MeOH in CH₂Cl₂, to afford enriched product (143 mg) as a pale yellow oil. This material was crystallized from Et₂O to give 9a-ethyl-3-[(4-methylphenyl)sulfonyl]-6-{4-[2-(1-piperidinyl)ethoxy]phenyl}-8,9,9a,10-tetrahydroindeno[2,1-*e*]indazol-7(3*H*)-one (94 mg, 81% yield) as a pale yellow solid.

### Step 5: 9a-ethyl-6-{4-[2-(1-piperidinyl)ethoxy]phenyl}-8,9,9a,10-tetrahydroindeno[2,1-e]indazol-7(3H)-one hydrochloride salt

A suspension of 9a-ethyl-3-[(4-methylphenyl)sulfonyl]-6-{4-[2-(1-piperidinyl)ethoxy]phenyl}-8,9,9a,10-tetrahydroindeno[2,1-*e*]indazol-7(3*H*)-one (82 mg, 0.134 mmol) in EtOH (2.0 mL) was treated with aqueous 1N sodium hydroxide (0.67 mL, 0.67 mmol). The resulting mixture was stirred at room temperature for one hour, then treated with HOAc (0.05 mL), and evaporated under vacuum. The residue was partitioned between CH₂Cl₂ (30 mL) and brine (20 mL). The organic layer was dried over MgSO₄, filtered, and evaporated under vacuum to a clear gum (73 mg). NMR analysis of this material showed EtOTs and a 74:26 mixture of the *N*-tosyl starting material to destosyl product.

A solution of the above gum (73 mg) in EtOH (2.0 mL) was treated dropwise with aqueous 1N NaOH (0.67 mL) to give a suspension. This mixture was stirred in a capped flask and heated in an oil bath at 60°C. After 5 minutes, the suspension gave way to a yellow solution. After 20 minutes, the mixture was removed from the heating bath, stirred at room temperature for 45 minutes, then acidified with HOAc (0.05 mL) and evaporated under vacuum. The residue was partitioned between EtOAc (30 mL) and brine (10 mL) plus water (5 mL). The EtOAc phase was washed with brine (10 mL) plus 5% aqueous NaHCO₃ (5 ml) followed by brine (15 mL) alone, dried over MgSO₄, filtered, and evaporated under vacuum to a pale yellow gum (51 mg). NMR analysis of this material showed EtOTs and a 87:6:7 mixture of the desired product and two *N*-ethyl by-products. Attempted purification of this mixture by preparative layer chromatography (0.1 x 20 x 20 cm silica gel GF plate developed with 5% MeOH +1% Et₃N in EtOAc) gave a 86:7:7 mixture (38 mg) of the product and the two *N*-ethyl by-products as a pale yellow gum.

The mixture was resolved by preparative HPLC using a YMC-Pack ODS column (100 x 20 mm i.d., S-5 µm, 120A), a solvent gradient of 80:20 A:B to 50:50 A:B where A is 0.1% TFA in H₂O and B is 0.1% TFA in MeCN, a flow rate of 20 mL/min, and UV detection at 254 nm. One major and two minor components were separated. The pooled fractions from the major peak were evaporated under vacuum to afford the trifluoroacetic acid salt of 9a-ethyl-6-{4-[2-(1-piperidinyl)ethoxy]phenyl}-8,9,9a,10-tetrahydroindeno[2,1-*e*]indazol-7(3*H*)-one (32.3 mg) as a pale yellow foam. This material was partitioned between CH₂Cl₂ (2 mL) and 5% aqueous NaHCO₃ (0.5 ml). The organic phase was washed with brine, dried over MgSO₄, filtered, and evaporated under vacuum to give the free base form of the product (25.4 mg) as a pale yellow oil. The oil was taken up in Et₂O (1 mL) containing a few drops of EtOAc and the solution was treated with 1M HCl in Et₂O (0.1 mL). The resulting precipitate was collected, washed with Et₂O, and dried under vacuum to afford 9a-ethyl-6-{4-[2-(1-piperidinyl)ethoxy]phenyl}-8,9,9a,10-tetrahydroindeno[2,1-*e*]indazol-7(3*H*)-one hydrochloride salt (22.8 mg) as a pale yellow powder.
¹H NMR (CD₃CN, 500 MHz) δ 0.89 (t, CH₂C*H*_{*3*}), 1.46, 1.83, and 2.05 (three br m, C*H*_{*2*}(C*H*_{*2*}CH₂)₂N), 1.60 and 1.82 (two m, C*H*_{*2*}CH₃), 2.18 and 2.37 (two m, 9-C*H*_{*2*}), 2.44 and 2.67 (two ddd, 8-C*H*_{*2*}), 3.14 and 3.53 (two br m, CH₂(CH₂C*H*_{*2*})₂N), 3.15 and 3.35 (two d, 10-C*H*_{*2*}), 3.38 (m, NC*H*_{*2*}CH₂O), 4.48 (m, NCH₂C*H*_{*2*}O), 6.37 (d, *H-*5), 6.7-7.2 (br m, C₆*H*_{*4*}), 7.13 (d, *H*-4), 8.07 (s, *H*-1), and 11.67 (br s, N*H*); mass spectrum m/z 456.4 (M+1).

### EXAMPLE 10

### SYNTHESIS OF 9a-ETHYL-6-(4-HYDROXYPHENYL)-8,9,9a,10-TETRAHYDROINDENO[2,1-e]INDAZOL-7(3H)-ONE

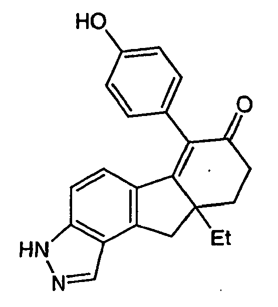

A solution of 9a-ethyl-6-(4-hydroxyphenyl)-3-[(4-methylphenyl)sulfonyl]-8,9,9a,10-tetrahydroindeno[2,1-*e*]indazol-7(3*H*)-one (10.0 mg, 0.02 mmol) in EtOH (0.4 mL) was treated with aqueous 1N NaOH (0.1 mL). The resulting yellow-orange solution was stirred at room temperature for 45 minutes, then acidified with HOAc (0.006 mL) and evaporated under vacuum. The residue was partitioned between EtOAc (5 mL) and water (2 mL). The organic phase was washed with brine (2 mL), dried over MgS04, filtered, evaporated under vacuum, and the residue lyophilized from benzene to provide an amorphous, off-white solid (7.8 mg). The crude product was purified by preparative layer chromatography on a 0.025 x 20 x 20 cm silica gel GF plate which was developed with 3:2 EtOAc-hexanes. The major UV visible band at R_{f} 0.17-0.29 was extracted with EtOAc and the solvent evaporated under vacuum to afford 9a-ethyl-6-(4-hydroxyphenyl)-8,9,9a,10-tetrahydroindeno[2,1-*e*]indazol-7(3*H)*-one (6.2 mg, 90% yield) as a pale yellow solid. A minor UV visible band at R_{f} 0.83-0.89 gave EtOTs (1.3 mg) as a clear oil.
¹H NMR (10:1 CDCl₃-CD₃OD, 500 MHz) δ 0.89 (t, CH₂C*H*_{*3*}), 1.63 and 1.9 (two m, C*H*_{*2*}CH₃), 2.16 and 2.37 (two m, 9-C*H*_{*2*}), 2.58 and 2.70 (two m, 8-C*H*_{*2*}), 2.95 and 3.28 (two d, 10-C*H*_{*2*}), 6.45 (d, *H*-5), 6.7-7.2 (br m, C₆*H*_{*4*}), 7.02 (d, *H*-4), and 8.02 (s, *H*-1); ¹³C NMR (10:1 CDCl₃-CD₃OD, 125 MHz) δ 9.7, 30.9, 31.0, 33.7, 41.2, 47.8, 109.0, 115.8, 120.1, 124.9, 126.8, 128.2, 130.0, 131.2, 133.6, 141.2, 142.8, 156.0, 169.3, and 199.1; mass spectrum m/z 345.4 (M+1),

### EXAMPLE 11

### SYNTHESIS OF 9a-ETHYL-6-METHYL-8,9.9a,10-TETRAHYDROINDENO[2.1-e]INDAZOL-7(3H)-ONE

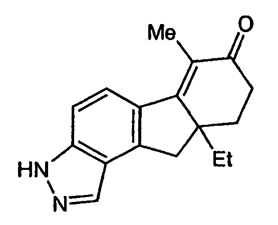

### Step 1: 9a-ethyl-6-methyl-2-[(4-methylphenyl)sulfonyl]-8;9,9a,10-tetrahydroindeno[2,1-e]indazol-7(2H)-one and 9a-ethyl-6-methyl-3-[(4-methylphenyl)sulfonyl]-8,9,9a,10-tetrahydroindeno[2,1-e]indazol-7(3H)-one

A 57:43 mixture (37.0 mg, 0.0762 mmol) of 6-bromo-9a-ethyl-2-[(4-methylphenyl)sulfonyl]-8,9,9a,10-tetrahydroindeno[2,1-*e*]indazol-7(2*H*)-one and 6-bromo-9a-ethyl-3-[(4-methylphenyl)sulfonyl]-8,9,9a,10-tetrahydroindeno[2,1-*e*]indazol-7(3*H*)-one was taken up in anhydrous toluene (1.17 mL, 0.065M) and 1.0 mL of this solution was added to 5-methyl-1-aza-5-stannabicyclo[3.3.3]undecane (17.5 mg, 0.064 mmol) and tetrakis(triphenylphosphine)palladium(0) (7.5 mg, 0.0065 mmol). The resulting mixture was purged with nitrogen, then stirred under a nitrogen atmosphere and heated in an oil bath at 100°C for 2 hours. The reaction mixture was allowed to cool to room temperature, then cooled in an ice bath and filtered to remove the precipitate of 5-bromo-1-aza-5-stannabicyclo[3.3.3]undecane. The filtrate was evaporated under vacuum to a dark amber oil (41.7 mg). This material was purified by chromatography on a Biotage Flash 12S KP-Sil column, eluting with 7:3 hexanes-EtOAc, to afford a mixture (22.1 mg, 69% yield) of 9a-ethyl-6-methyl-2-[(4-methylphenyl)sulfonyl]-8,9,9a,10-tetrahydroindeno[2,1-*e*]indazol-7(2*H*)-one and 9a-ethyl-6-methyl-3-[(4-methylphenyl)sulfonyl]-8,9 ,9a,10-tetrahydroindeno[2,1-*e*]indazol-7(3*H*)-one as a yellow oil.

The 5-methyl-1-aza-5-stannabicyclo[3.3.3]undecane used in this step was prepared as follows. A suspension of 5-chloro-1-aza-5-stannabicyclo[3.3.3]-undecane (29.4 mg, 0.1 mmol) in anhydrous THF (0.5 mL) was cooled in an *i*PrOH-dry ice bath (-45°C) and stirred under a nitrogen atmosphere while 1.4M MeLi in Et₂O (0.10 mL, 0.14 mmol) was added dropwise by syringe. Approximately two minutes following the addition, the suspension gave way to a clear solution. The solution was stirred under nitrogen with gradual warming to 0°C over 2 hours, then diluted with Et₂O to 8 mL, washed with water (3 mL), 1M pH 3 phosphate (3 mL), water (3 mL), and brine (3 mL), dried over MgSO₄, filtered, and evaporated under vacuum to afford 5-methyl-1-aza-5-stannabicyclo[3.3.3]undecane (22.8 mg, 83% yield) as a white solid.

### Step 2: 9a-ethyl-6-methyl-8,9,9a,10-tetrahydroindeno[2,1-e]indazol-7(3H)-one

A suspension of the *N*-tosyl products from step 1 (22.1 mg, 0.0526 mmol) in EtOH (1.0 mL) was treated with aqueous 5N NaOH (0.053 mL, 0.265 mmol) and the mixture was swirled by hand for approximately one minute to give a yellow solution. The solution was stirred at room temperature for 30 minutes, then acidified with HOAc (0.020 mL) and evaporated under vacuum. The residue in EtOAc (5 mL) was washed with water (2 mL), 5% NaHCO₃ (2 mL) and brine (2 mL), dried over MgSO₄, filtered, and evaporated under vacuum to a yellow oil. The oil was purified by preparative layer chromatography on a 0.05 x 20 x 20 silica gel GF plate using 7:3 hexanes-EtOAc as the developing solvent. The major UV visible band at Rf 0.08-0.19 was eluted with EtOAc and the solvent evaporated under vacuum to give a gum. This material was swirled with benzene to afford 9a-ethyl-6-methyl-8,9,9a,10-tetrahydroindeno[2,1-*e*]indazol-7(3*H*)-one (13.8 mg, 86% yield) as an off-white solid following evaporation of the solvent. NMR analysis revealed that the product was isolated as a hemi-benzene solvate.
¹H NMR (CDCl₃, 500 MHz) δ 0.87 (t, CH₂C*H*_{*3*}), 1.52 and 1.69 (two m, C*H*_{*2*}CH₃), 2.07 and 2.33 (two ddd, 9-C*H*_{*2*}), 2.16 (s, 6-C*H*_{*3*}), 2.51 and 2.62 (two ddd, 8-C*H*_{*2*}), 2.94 and 3.28 (two d, 10-C*H*_{*2*}), 7.36 (s, PhH solvate), 7.46 (d, *H*-4), 7.82 (d, *H-*5), and 8.12 (s, *H*-1).

### EXAMPLE 12

### SYNTHESIS OF 9a-ETHYL-6-VINYL-8,9,9a,10-TETRAHYDROINDENO[2,1-e]INDAZOL-7(3H)-ONE

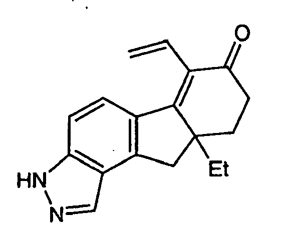

### Step 1: 9a-ethyl-2-[(4-methylphenyl)sulfonyl]-6-vinyl-8,9,9a,10-tetrahydroindeno[2,1-e]indazol-7(2H)-one and 9a-ethyl-3-[(4-methylphenyl)sulfonyl]-6-vinyl-8,9,9a,10-tetrahydroindeno[2,1-e]indazol-7(3H)-one

A mixture (150 mg, 0.31 mmol) of 6-bromo-9a-ethyl-2-[(4-methylphenyl)sulfonyl]-8,9,9a,10-tetrahydroindeno[2,1-*e*]indazol-7(2*H*)-one and 6-bromo-9a-ethyl-3-[(4-methylphenyl)sulfonyl]-8,9,9a,10-tetrahydroindeno[2,1-*e*]indazol-7(3*H*)-one was dissolved in anhydrous toluene (3.1 mL) and the solution was treated with tributyl(vinyl)tin (0.180 mL, 0.62 mmol) and dichlorobis(triphenylphosphine)palladium(II) (44 mg, 0.062 mmol). The resulting mixture was purged with nitrogen, then stirred under a nitrogen atmosphere and heated in an oil bath at 100°C for 24 hours. After cooling, the reaction mixture was purified by preparative layer chromatography on three 0.1 x 20 x 20 cm silica gel GF plates, developing with 5% EtOAc in CH₂Cl₂. The product bands were extracted with EtOAc and the extracts were evaporated under vacuum to afford a mixture (143 mg) of 9a-ethyl-2-[(4-methylphenyl)sulfonyl]-6-vinyl-8,9,9a,10-tetrahydroindeno[2,1-*e*]indazol-7(2*H*)-one and 9a-ethyl-3-[(4-methylphenyl)sulfonyl]-6-vinyl-8,9,9a,10-tetrahydroindeno[2,1-*e*]indazol-7(3*H*)-one as an oil.

### Step 2: 9a-ethyl-6-vinyl-8,9,9a,10-tetrahydroindeno[2,1-e]indazol-7(3H)-one

The mixture of the *N*-tosyl products from step 1 (143 mg) was dissolved in ethanol (2.5 mL) and the solution was diluted with water (0.25 mL) and aqueous 5N sodium hydroxide (0.186 mL, 0.93 mmol). The resulting mixture was stirred at room temperature for one hour, and then partitioned between EtOAc (20 mL) and brine (20 mL). The organic phase was dried over MgSO₄ filtered, and evaporated under vacuum. The residue was purified by preparative layer chromatography on two 0.1 x 20 x 20 cm silica gel GF plates, developing with 5% MeOH in CH₂Cl₂. The product bands were extracted with 10% MeOH in CH₂Cl₂, the extracts were evaporated under vacuum, and the residue was lyophilized from benzene to afford 9a-ethyl-6-vinyl-8,9,9a,10-tetrahydroindeno[2,1-*e*]indazol-7(3*H*)-one (66 mg, 76% yield over two steps) as an amorphous solid.
¹H NMR (CDCl₃, 500 MHz) δ 0.88 (t, CH₂C*H*_{*3*}), 1.55 and 1.73 (two m, C*H*_{*2*}CH₃), 2.11 and 2.34 (two ddd, 9-C*H*_{*2*}), 2.55 and 2.65 (two ddd, 8-C*H*_{*2*}), 2.98 and 3.30 (two d, 10-C*H*_{*2*}), 5.58 and 5.81 (two dd, CH=C*H*_{*2*}), 6.61 (dd, C*H*=CH₂), 7.45 (d, *H*-4), 7.96 (d, *H*-5), 8.17 (s, *H*-1), and 10.2 (br s, N*H*).

### EXAMPLE 13

### SYNTHESIS OF 6,9a-DIETHYL-8,9,9a,10-TETRAHYDROINDENO[2,1-e]INDAZOL-7(3H)-ONE

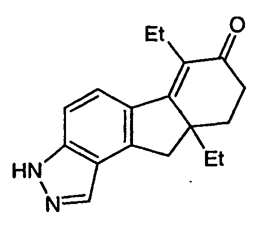

A mixture of 9a-ethyl-6-vinyl-8,9,9a,10-tetrahydroindeno[2,1-*e*]indazol-7(3*H*)-one (approx. 30 mg), 10% palladium on carbon (30 mg), and ethanol (3 mL) was stirred under an atmosphere of hydrogen for 4 hours. The mixture was filtered through a pad of celite and the filtrate was evaporated under vacuum. The residue was lyophilized from benzene to afford 6,9a-diethyl-8,9,9a,10-tetrahydroindeno[2,1-*e*]indazol-7(3*H*)-one as an amorphous solid.
¹H NMR (CDCl₃, 500 MHz) δ 0.86 (t, 9a-CH₂C*H*_{*3*}), 1.15 (t, 6-CH₂C*H*_{*3*}), 1.54 and 1.68 (two m, 9a-C*H*_{*2*}CH₃), 2.06 and 2.32 (two ddd, 9-C*H*_{*2*}), 2.49 and 2.60 (two ddd, 8-C*H*_{*2*}), 2.58 and 2.73 (two m, 6-C*H*_{*2*}CH₃), 2.95 and 3.28 (two d, 10-C*H*_{*2*}), 7.48 (br d, *H*-4), 7.78 (d, *H*-5), and 8.15 (br s, *H*-1).

### EXAMPLE 14

### SYNTHESIS OF 6-ALLYL-9a-ETHYL-8,9,9a,10-TETRAHYDROINDENO[2.1-e]INDAZOL-7(3H)-ONE

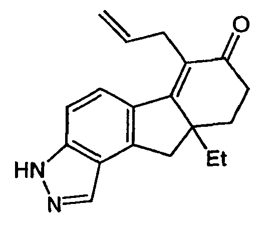

### Step 1: 6-allyl-9a-ethyl-2-[(4-methylphenyl)sulfonyl]-8,9,9a,10-tetrahydroindeno[2,1-e]indazol-7(2H)-one and 6-allyl-9a-ethyl-3-[(4-methylphenyl)sulfonyl]-8,9,9a,10-tetrahydroindeno[2,1-e]indazol-7(3H)-one

A mixture (100 mg, 0.206 mmol) of 6-bromo-9a-ethyl-2-[(4-methylphenyl)sulfonyl]-8,9,9a,10-tetrahydroindeno[2,1-*e*]indazol-7(2*H*)-one and 6-bromo-9a-ethyl-3-[(4-methylphenyl)sulfonyl]-8,9,9a,10-tetrahydroindeno[2,1-*e*]indazol-7(3*H*)-one was dissolved in anhydrous toluene (2.0 mL) and the solution was treated with allyltributyltin (0.128 mL, 0.412 mmol) and dichlorobis(triphenylphosphine)palladium(II) (29 mg, 0.041 mmol). The resulting mixture was purged with nitrogen, then stirred under a nitrogen atmosphere and heated in an oil bath at 100°C for 24 hours. After cooling, the reaction mixture was purified by preparative layer chromatography on three 0.1 x 20 x 20 cm silica gel GF plates, developing with 5% EtOAc in CH₂Cl₂. The product bands were extracted with EtOAc, the extracts were evaporated under vacuum, and the residue was lyophilized from benzene to afford a mixture (69 mg) of 6-allyl-9a-ethyl-2-[(4-methylphenyl)sulfonyl]-8,9,9a,10-tetrahydroindeno[2,1-*e*]indazol-7(2*H*)-one and 6-allyl-9a-ethyl-3-[(4-methylphenyl)sulfonyl]-8,9,9a,10-tetrahydroindeno[2,1-*e*]indazol-7(3*H*)-one.

### Step 2: 6-allyl-9a-ethyl-8,9,9a,10-tetrahydroindeno[2,1-e]indazol-7(3H)-one

The mixture of the *N*-tosyl products from step 1 (69 mg) was dissolved in ethanol (3.0 mL) and the solution was diluted with water (0.10 mL) and aqueous 5N sodium hydroxide (0.103 mL, 0.515 mmol). The resulting mixture was stirred at room temperature for 30 minutes, and then partitioned between EtOAc (20 mL) and water (20 mL) containing aqueous 2N HCl (1mL). The organic phase was washed with brine (10 mL), dried over MgSO₄, filtered, and evaporated under vacuum. The residue was purified by preparative layer chromatography on a 0.1 x 20 x 20 cm silica gel GF plates, developing with 5% MeOH in CH₂Cl₂. The product band was extracted with 10% MeOH in CH₂Cl₂, the extracts were evaporated under vacuum, and the residue was lyophilized from benzene to afford 6-allyl-9a-ethyl-8,9,9a,10-tetrahydroindeno[2,1-*e*]indazol-7(3*H*)-one (39 mg) as an amorphous solid.
¹H NMR (CDCl₃, 500 MHz) δ 0.90 (t, CH₂C*H*_{*3*}), 1.59 and 1.75 (two m, C*H*_{*2*}CH₃), 2.11 and 2.36 (two ddd, 9-C*H*_{*2*}), 2.53 and 2.65 (two ddd, 8-C*H*_{*2*}), 2.96 and 3.30 (two d, 10-C*H*_{*2*}), 3.23 and 3.55 (two m, C*H*_{*2*}CH=CH₂), 5.03-5.09 (m, CH=C*H*_{*2*}), 6.03 (m, C*H*=CH₂), 7.42 (d, *H*-4), 7.73 (d, *H*-5), 8.12 (s, *H*-1), and 10.21 (br s, N*H*).

### EXAMPLE 15

### SYNTHESIS OF 6-CYCLOPENTYL-9a-ETHYL-8,9,9a,10-TETRAHYDROINDENO[2,1-e]INDAZOL-7(3H)-ONE

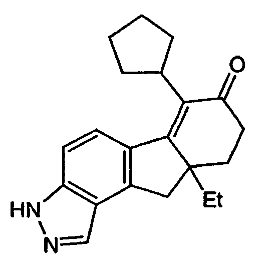

### Step 1: 6-(2-cyclopenten-1-yl)-9a-ethyl-2-[(4-methylphenyl)sulfonyl]-8,9,9a,10-tetrahydroindeno[2,1-e]indazol-7(2H)-one and 6-(2-cyclopenten-1-yl)-9a-ethyl-3-[(4-methylphenyl)sulfonyl]-8,9,9a,10-tetrahydroindeno[2,1-e]indazol-7(3H)-one

A mixture (25 mg, 0.056 mmol) of 6-bromo-9a-ethyl-2-[(4-methylphenyl)sulfonyl]-8,9,9a,10-tetrahydroindeno[2,1-*e*]indazol-7(2*H*)-one and 6-bromo-9a-ethyl-3-[(4-methylphenyl)sulfonyl]-8,9,9a,10-tetrahydroindeno[2,1-*e*]indazol-7(3*H*)-one was dissolved in anhydrous 1,4-dioxane (0.5 mL) and the solution was treated with cyclopentene (0.049 mL, 0.56 mmol), triethylamine (0.039 mL, 0.28 mmol), and bis(acetato)bis(triphenylphosphine)palladium(II) (21 mg, 0.028 mmol). The resulting mixture was stirred under a nitrogen atmosphere and heated in an oil bath at 100°C for 3 days. After cooling, the reaction mixture was diluted with CH₂Cl₂ and passed through a pad of silica gel. The filtrate was evaporated under vacuum to provide a crude mixture of 6-(2-cyclopenten-1-yl)-9a-ethyl-2-[(4-methylphenyl)sulfonyl]-8,9,9a,10-tetrahydroindeno[2,1-*e*]indazol-7(2*H*)-one and 6-(2-cyclopenten-1-yl)-9a-ethyl-3-[(4-methylphenyl)sulfonyl]-8,9,9a,10-tetrahydroindeno[2,1-*e*]indazol-7(3*H*)-one.

### Step 2: 6-(2-cyclopenten-1-yl)-9a-ethyl-8,9,9a,10-tetrahydroindeno[2,1-e]indazol-7(3H)-one

The mixture of the *N*-tosyl products from step 1 was dissolved in ethanol (0.5 mL) and the solution was diluted with aqueous 2.5N sodium hydroxide (0.05 mL). The resulting mixture was stirred at room temperature for 30 minutes, then diluted with CH₂Cl₂ and dried over MgSO₄. The CH₂Cl₂ solution was filtered through a pad of silica gel and the filtrate was evaporated under vacuum. The residue was purified by preparative layer chromatography on a 0.1 x 20 x20 cm silica gel GF plate, developing with 1:1 EtOAc-hexanes. The UV visible product band was extracted with EtOAc and the extracts were evaporated under vacuum to provide 6-(2-cyclopenten-1-yl)-9a-ethyl-8,9,9a,10-tetrahydroindeno[2,1-*e*]indazol-7(3*H*)-one as a mixture of diastereomers.

### Step 3: 6-cyclopentyl-9a-ethyl-8,9,9a,10-tetrahydroindeno[2,1-e]indazol-7(3H)-one

A mixture of 6-(2-cyclopenten-1-yl)-9a-ethyl-8,9,9a,10-tetrahydroindeno[2,1-*e*]indazol-7(3*H*)-one (4.5 mg), 10% palladium on carbon (4.5 mg), and ethanol (1 mL) was stirred under an atmosphere of hydrogen at room temperature for 6 hours. The mixture was filtered through a pad of celite which was rinsed with EtOAc. The filtrate was evaporated under vacuum to afford 6-cyclopentyl-9a-ethyl-8,9,9a,10-tetrahydroindeno[2,1-*e*]indazol-7(3*H*)-one (4 mg) as an oil.
¹H NMR (CDCl₃, 500 MHz) δ 0.84 (t, CH₂C*H*_{*3*}), 1.36-1.71 and 1.82-2.11 (two m, C*H*_{*2*}CH₃, four cyclopentyl C*H*_{*2*}, one of 9-C*H*_{*2*}), 2.36 (m, one of 9-C*H*_{*2*}), 2.47 (m, 8-C*H*_{*2*}), 2.96 and 3.23 (two d, 10-C*H*_{*2*}), 3.36 (m, cyclopentyl CH), 7.44 (d, *H*-4), 7.72 (d, *H*-5), and 8.14 (br s, *H*-1); mass spectrum m/z 321.2 (M+1).

### EXAMPLE 16

### SYNTHESIS OF 6-CYANO-9a-ETHYL-8,9,9a,10-TETRAHYDROINDENO[2,1-e]INDAZOL-7(3H)-ONE

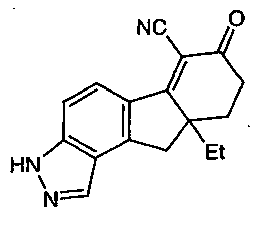

A mixture of 6-bromo-9a-ethyl-8,9,9a,10-tetrahydroindeno[2,1-e]indazol-7(3*H*)-one (200 mg, 0.60 mmol), copper(1) cyanide (200 mg, 2.23 mmol), and anhydrous *N,N*-dimethylacetamide (2 mL) was stirred under a nitrogen atmosphere and heated in an oil bath at 160°C for 20 minutes. After cooling to room temperature, the mixture was partitioned between EtOAc (75 mL) and water (75 mL) containing aqueous 2N HCl (10 mL). The organic phase was washed with water (4 x 75 mL) and brine (50 mL), dried over MgSO₄ filtered, and evaporated under vacuum to an oil (109 mg). The crude product was purified by preparative layer chromatography on a 0.1 x 20 x20 cm silica gel GF plate, developing with 5% MeOH in CH₂Cl₂. The product band was extracted with 10% MeOH in CH₂Cl₂ ,the extracts were evaporated under vacuum, and the residue was lyophilized from benzene to provide 6-cyano-9a-ethyl-8,9,9a,10-tetrahydroindeno[2,1-*e*]indazol-7(3*H*)-one as a beige colored, amorphous solid.
¹H NMR (CDCl₃, 500 MHz) δ 0.93 (t, CH₂C*H*_{*3*}), 1.60 and 1.78 (two m, C*H*_{*2*}CH₃), 2.11 and 2.42 (two ddd, 9-C*H*_{*2*}), 2.66 (m, 8-C*H*_{*2*}), 3.02 and 3.41 (two d, 10-C*H*_{*2*}), 7.58 (d, *H*-4), 8.27 (br s, *H*-1), and 8.45 (d, *H*-5).

### EXAMPLE 17

### SYNTHESIS OF 1-CHLORO-9a-ETHYL-6-METHYL-8,9,9a,10-TETRAHYDROINDENO[2,1-e]INDAZOL-7(3H)-ONE

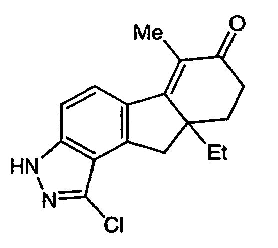

A solution of 9a-ethyl-6-methyl-8,9,9a,10-tetrahydroindeno[2,1-*e*]indazol-7(3*H*)-one (20 mg, 0.075 mmol) in ethanol (0.38 mL) was treated with aqueous 5N sodium hydroxide (0.018 mL, 0.09 mmol) and *N*-chlorosuccinimide (10 mg, 0.075 mmol). The mixture was stirred at room temperature for 30 minutes, and then partitioned between EtOAc (5 mL) and water (5 mL) containing aqueous 2N HCl (0.5 mL). The organic phase was washed with brine (5 mL), dried over MgSO₄, filtered, and evaporated under vacuum. The residue was purified by preparative layer chromatography on a 0.1 x 20 x 20 cm silica gel GF plate, developing with 5% MeOH in CH₂Cl₂. The UV visible product band was extracted with 10% MeOH in CH₂Cl₂ (10 mL), the extracts were evaporated under vacuum, and the residue was lyophilized from benzene (3 mL) to afford 1-chloro-9a-ethyl-6-methyl-8,9,9a,10-tetrahydroindeno[2,1-*e*]indazol-7(3*H*)-one (12 mg, 53% yield) as an amorphous solid.
¹H NMR (CDCl₃, 500 MHz) δ 0.89 (t, CH₂C*H*_{*3*}), 1.53 and 1.69 (two m, C*H*_{*2*}CH₃), 2.07 and 2.34 (two ddd, 9-C*H*_{*2*}), 2.16 (s, 6-C*H*_{*3*}), 2.53 and 2.63 (two ddd, 8-C*H*_{*2*}), 3.02 and 3.56 (two d, 10-C*H*_{*2*}), 7.42 (d, *H*-4), and 7.84 (d, *H*-5).

### EXAMPLE 18

### SYNTHESIS OF 1-BROMO-9a-ETHYL-6-METHYL-8,9,9a,10-TETRAHYDROINDENO[2,1-e]INDAZOL-7(3H)-ONE

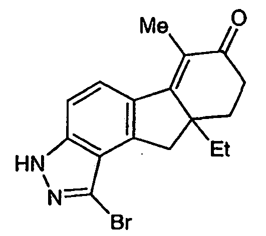

A solution of 9a-ethyl-6-methyl-8,9,9a,10-tetrahydroindeno[2,1-*e*]indazol-7(3*H*)-one (100 mg, 0.375 mmol) in ethanol (2 mL) was treated with aqueous 5N sodium hydroxide (0.152 mL, 0.76 mmol) and bromine (0.021 mL, 0.41 mmol). The mixture was stirred at room temperature for 18 minutes, treated with additional bromine (0.003 mL, 0.06 mmol), and stirred at room temperature for 5 more minutes. The mixture was partitioned between EtOAc (20 mL) and water (20 mL) containing aqueous 2N HCl (2 mL). The organic phase was washed with brine (10 mL), dried over MgSO₄, filtered, and evaporated under vacuum. The residue was purified by preparative layer chromatography on two 0.1 x 20 x 20 cm silica gel GF plates, developing with 5% MeOH in CH₂Cl₂. The UV visible product bands were extracted with 10% MeOH in CH₂Cl₂, the extracts were evaporated under vacuum, and the residue was lyophilized from benzene (3 mL) to afford 1-bromo-9a-ethyl-6-methyl-8,9,9a,10-tetrahydroindeno[2,1-*e*]indazol-7(3*H*)-one (50 mg, 39% yield) as an amorphous solid.
¹H NMR (CDCl₃, 500 MHz) δ 0.89 (t, CH₂C*H*_{*3*}), 1.53 and 1.68 (two m, C*H*_{*2*}CH₃), 2.07 and 2.35 (two ddd, 9-C*H*_{*2*}), 2.15 (s, 6-C*H*_{*3*}), 2.52 and 2.62 (two ddd, 8-C*H*_{*2*}), 3.04 and 3.63 (two d, 10-C*H*_{*2*}), 7.42 (d, *H*-4), and 7.84 (d, *H*-5).

### EXAMPLE 19

### SYNTHESIS OF 9a-ETHYL-6-METHYL-9,9a-DEHYDROINDENO[2.1-e]INDAZOLE-7,10(3H,8H)-DIONE AND (RAC)-(9aR,10R)-10-CHLORO-9a-ETHYL-6-METHYL-8,9,9a,10-TETRAHYDROINDENO[2,1-e]INDAZOL-7(3H)-ONE

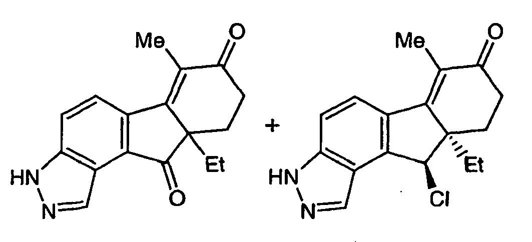

A mixture of 9a-ethyl-6-methyl-8,9,9a,10-tetrahydroindeno[2,1-*e*]indazol-7(3*H*)-one (26 mg, 0.10 mmol), *N*-chlorosuccinimide (16 mg, 0.12 mmol), and dichloromethane (0.25 mL) was stirred at room temperature for 20.5 hours. The mixture was diluted with EtOAc (6 mL), washed with water (4 ml) and brine (5 mL), dried over MgSO₄, filtered, and evaporated under vacuum to an oil (22 mg). The crude product mixture was purified by preparative layer chromatography on a 0.1 x 20 x 20 cm silica gel GF plate, developing with 25 % EtOAc in CH₂Cl₂. Two major UV visible bands were removed, separately extracted with EtOAc, and the extracts were concentrated under vacuum. The slower moving band afforded 9a-ethyl-6-methyl-9,9a-dihydroindeno[2,1-*e*]indazole-7,10(3*H*,8*H*)-dione (4.6 mg) and the faster moving band provided (*rac*)-(9a*R*,10*R*)-10-chloro-9a-ethyl-6-methyl-8,9,9a,10-tetrahydroihdeno[2,1-*e*]indazol-7(3*H*)-one (4.5 mg).
10-Keto product: ¹H NMR (CDCl₃, 500 MHz) δ 0.77 (t, CH₂C*H*_{*3*}), 1.81 and 1.98 (two m, C*H*_{*2*}CH₃), 1.98 and 2.43 (two ddd, 9-C*H*_{*2*}), 2.26 (s, 6-C*H*_{*3*}), 2.62 and 2.76 (two ddd, 8-C*H*_{*2*}), 7.92 (d, *H*-4 or *H*-5), 8.02 (d, *H*-5 or *H*-4), and 8.66 (s, *H*-1); mass spectrum m/z 281.4 (M+1).
10-Chloro product: ¹H NMR (CDCl₃, 500 MHz) δ 0.94 (t, CH₂C*H*_{*3*}), 1.49 and 1.87 (two m, C*H*_{*2*}CH₃), 2.16 (m, one of 9-C*H*_{*2*}), 2.19 (s, 6-C*H*_{*3*}), 2.58-2.67 (m, one of 9-C*H*_{*2*} and 8-C*H*_{*2*}), 5.52 (s, *H*-10), 7.60 (d, *H*-4), 7.82 (d, *H*-5, and 8.29 (d, *H*-1): mass spectrum m/z 301.2 (M+1).

### EXAMPLE 20

### SYNTHESIS OF (RAC)-(9aR,10R)-10-AZIDO-9a-ETHYL-6-METHYL-8,9,9a,10-TETRAHYDROINDENO[2,1-e]INDAZOL-7(3H)-ONE AND (RAC)-(9aR,10S)-10-AZIDO-9a-ETHYL-6-METHYL-8,9,9a,10-TETRAHYDROINDENO[2,1-e]INDAZOL-7(3H)-ONE

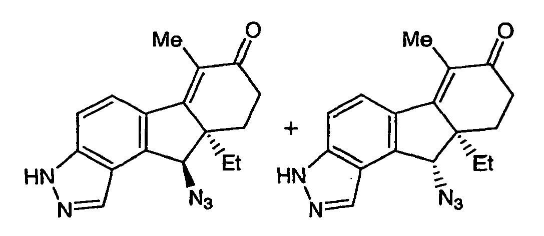

A mixture of 9a-ethyl-6-methyl-8,9,9a,10-tetrahydroindeno[2,1-*e*]indazol-7(3*H*)-one (104 mg, 0.40 mmol), *N*-chlorosuccinimide (64 mg, 0.48 mmol), and dichloromethane (1 mL) was stirred at room temperature for 6 hours. The solvent was evaporated under a stream of nitrogen and the crude (*rac*)-(9a*R*,10*R*)-10-chloro-9a-ethyl-6-methyl-8,9,9a, 10-tetrahydroindeno[2,1-*e*]indazol-7(3*H*)-one product thus formed was immediately dissolved in anhydrous *N,N*-dimethylformamide (1 mL). One-half of this solution was treated with sodium azide (16 mg, 0.24 mmol) and the resulting mixture was stirred at room temperature for 67 hours and then heated in an oil bath at 80°C for 1.5 hours. After cooling, the mixture was diluted with EtOAc (20 mL), washed with water (5 x 10 mL) and brine (5 mL), dried over MgSO₄, filtered, and evaporated under vacuum. The residue was purified by preparative layer chromatography on a 0.1 x 20 x 20 cm silica gel GF plate, developing with 20% EtOAc in CH₂Cl₂. The UV visible product band was extracted with EtOAc and the extracts were evaporated under vacuum to afford a 9:1 mixture (10.5 mg) of (*rac*)-(9a*R*,10*R*)-10-azido-9a-ethyl-6-methyl-8,9,9a,10-tetrahydroindeno[2,1-*e*]indazol-7(3*H*)-one and (*rac*)-(9a*R*,10*S*-10-azido-9a-ethyl-6-methyl-8,9,9a,10-tetrahydroindeno[2,1-*e*]indazol-7(3*H*)one.
¹H NMR (CDCl₃, 500 MHz) δ 0.92 (t, CH₂C*H*_{*3*}), 1.45 and 1.79 (two m, C*H*_{*2*}CH₃), 2.16 and 2.41 (two ddd, 9-C*H*_{*2*}), 2.18 (s, 6-C*H*_{*3*})*,* 2-61-2.66 (m, 8-C*H*_{*2*})*,* 4.86 (s, *H-*10), 7.64 (d, *H*-4), 7.85(d, *H*-5), and 8.27 (s, *H*-1); mass spectrum m/z 308.4 (M+1). The minor isomer showed resonances at δ 0.59 (t, CH₂C*H*_{*3*}), 2.16 (s, 6-C*H*_{*3*}), 5.07 (s, 10-*H*), 7.56 (d, *H*-4), 7.77(d, *H*-5), and 8.35 (s, *H*-1) in the ¹H NMR spectrum.

### EXAMPLE 21

### SYNTHESIS OF 6-BROMO-9a-ETHYL-9,9a-DIHYDROINDENO[2.1-e]INDAZOLE-7,10(3H,8H)-DIONE

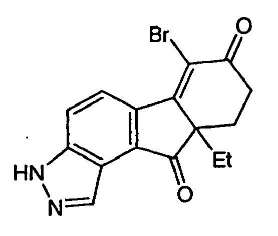

A mixture of 6-bromo-9a-ethyl-8,9,9a,10-tetrahydroindeno[2,1-*e*]indazol-7(3*H*)-one (31 mg. 0.094 mmol), potassium persulfate (K₂S₂O₈, 674 mg, 0.248 mmol), water (1 mL), and MeCN (1 mL) was stirred at room temperature for 16.8 hours. The mixture was then stirred with heating at 50°C (oil bath temperature) for one hour followed by heating at 80°C for one hour. The mixture was briefly sonicated to disperse the K₂S₂O₈ and then stirred with heating at 80°C for an additional 3.5 hours. After cooling to room temperature, the mixture was partitioned between EtOAc (30 mL), and water (30 mL). The organic phase was washed with brine (10 mL), dried over MgSO₄, filtered, and concentrated under vacuum to an oil (20 mg). The crude was purified by preparative layer chromatography on a 0.05 x 20 x20 cm silica gel GF plate, developing with 1:1 hexanes-EtOAc. The UV visible product band was extracted with EtOAc and the eluant evaporated under vacuum to provide 6-bromo-9a-ethyl-9,9a-dihydroindeno[2,1-*e*]indazole-7,10(3*H*,8*H*)-dione (5.0 mg). Two minor UV visible bands provided samples of 6-bromo-9a-ethyl-10-hydroxy-8,9,9a,10-tetrahydroindeno[2,1-*e*]indazol-7(3*H*)-one (2.1 mg) and 9-bromo-5a-ethyl-6,7-dihydroisochromeno[3,4-*e*]indazole-5,8(1*H*,5a*H*)-dione (3.7 mg, tentative assignment).
10-Keto product: ¹H NMR (CDCl₃, 500 MHz) δ 0.80 (t, CH₂C*H*_{*3*}), 1.90 and 2.03 (two dq, C*H*_{*2*}CH₃), 2.08 and 2.48 (two ddd, 9-C*H*_{*2*}), 2.84 and 2.95 (two ddd, 8-C*H*_{*2*}), 7.97 (dd, *H*-4), 8.68 (d, *H*-1), 8.86 (d, *H*-5), and 10.72 (br s, N*H*); mass spectrum m/z 345.0 (M+1) and 347.0 (M+3).

### EXAMPLE 22

### SYNTHESIS OF 9a-BUTYL-8,9,9a,10-TETRAHYDROINDENO[2,1-e]INDAZOL-7(3H)-ONE

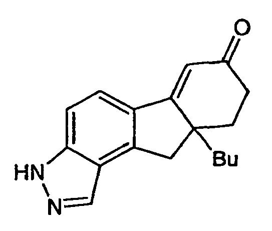

### Step 1: 5-(acetylamino)-2-butyl-1-indanone

Potassium hydroxide (85%, 14 mg, 0.21 mmol) was added to a mixture of 5-(acetylamino)-1-indanone (200 mg, 1.05 mmol) and butyraldehyde (0.141 mL, 1.57 mmol) in ethanol (1 mL). The suspension rapidly gave way to a solution. The solution was stirred at room temperature for 10 minutes and then treated with 10% palladium on carbon (20 mg), placed under a hydrogen atmosphere, and stirred at room temperature for 50 minutes. The mixture was filtered and the filtrate was concentrated under vacuum to an oily residue. This material was purified by preparative layer chromatography on three 0.1 x 20 x 20 cm silica gel GF plates, developing with 5% MeOH in CH₂Cl₂. The UV visible product band was eluted with 10% MeOH in CH₂Cl₂ and the eluant was evaporated under vacuum to provide 5-(acetylamino)-2-butyl-1-indanone (151 gm, 59% yield) as an oil.

### Step 2: 5-(acetylamino)-4-bromo-2-butyl-1-indanone

A solution of 5-(acetylamino)-2-butyl-1-indanone (1.19 g, 4.85 mmol) and *N*-bromosuccinimide (0.95 g, 5.34 mmol) in anhydrous *N,N*-dimethylformamide (4.85 mL) was stirred under a nitrogen atmosphere and heated in an oil bath at 60°C for 2.5 hours. The mixture was evaporated under vacuum to a residue that was partitioned between EtOAc (100 mL) and water (100 mL). The organic phase was washed with water (3 x 100 mL) and brine (50 mL), dried over MgSO₄, filtered, and evaporated under vacuum to a solid. This material was purified by chromatography on EM silica gel 60 (230-400 mesh, 2.75 x 27 cm column), eluting with 5% EtOAc in CH₂Cl₂. The product containing fractions were evaporated under vacuum to provide 5-(acetylamino)-4-bromo-2-butyl-1-indanone (0.836 g, 56% yield) as a solid.

### Step 3: 5-(acetylamino)-2-butyl-4-methyl-1-indanone

A mixture of 5-(acetylamino)-4-bromo-2-butyl-1-indanone (836 mg, 2.7 mmol), triphenylphosphine (142 mg, 0.54 mmol), lithium chloride (315 mg, 7.43 mmol), tetramethyltin (0.750 mL, 5.4 mmol), dichlorobis(triphenylphosphine)-palladium(II) (142 mg, 0.2 mmol), and anhydrous *N,N*-dimethylformamide (14 mL) was purged with nitrogen and then stirred under a nitrogen atmosphere and heated in an oil bath at 100°C for 16.5 hours. After cooling to room temperature, the mixture was concentrated under vacuum and the residue was partitioned between EtOAc (100 mL), and water (100 mL). The organic phase was washed with water (2 x 100 mL) and brine (50 mL), dried over MgSO₄, filtered, and evaporated under vacuum to a solid (720 mg). A 420 mg sample of the crude product was purified by preparative layer chromatography on four 0.1 x 20 x 20 cm silica gel GF plates which were developed twice with 25% EtOAc in CH₂Cl₂. The UV visible product band was extracted with EtOAc and the extracts were evaporated under vacuum to provide 5-(acetylamino)-2-butyl-4-methyl-1-indanone (300 mg, 74% yield) as a white solid.

### Step 4: 5-(acetylamino)-2-butyl-4-methyl-2-(3-oxobuty)-1-indanone

A suspension of 5-(acetylamino)-2-butyl-4-methyl-1-indanone (300 mg, 1.16 mmol) in anhydrous tetrahydrofuran (1 mL) was treated with methyl vinyl ketone (0.116 mL, 1.39 mmol) and 1,8-diazabicyclo[5.4.0]undec-7-ene (0.035 mL, 0.232 mmol). The mixture was stirred under a nitrogen atmosphere at room temperature. After 20 minutes, the heavy suspension gave way to a solution. After 18 hours, the mixture was partitioned between EtOAc (20 mL) and water (20 mL) containing aqueous 2N HCl (2 mL). The organic portion was washed with water (10 mL) and brine (5 mL), dried over MgSO₄, filtered, and evaporated under vacuum to provide crude 5-(acetylamino)-2-butyl-4-methyl-2-(3-oxobutyl)-1-indanone (400 mg) as an oil. The ¹H NMR spectrum of this material revealed that the desired product was accompanied by the *N*-(3-oxobutyl) derivatives of both the starting material and the product.

### Step 5: 7-(acetylamino)-9a-butyl-8-methyl-1,2,9,9a-tetrahydro-3H-fluoren-3-one

The product mixture (400 mg, approx. 1.16 mmol) form step 4 was dissolved in toluene (2.5 mL) and the solution was treated with pyrrolidine (0.097 mL, 1.16 mmol) and acetic acid (0.066 mL, 1.16 mmol). The mixture was stirred and heated in an oil bath at 100°C for 3 hours. After cooling to room temperature, the mixture was partitioned between EtOAc (50 mL) and water(50 mL) containing aqueous 2N HCl (3 mL). The organic phase was washed with water (50 mL), aqueous NaHCO₃ (50 mL) and brine (10 mL), dried over MgSO₄, filtered, and evaporated under vacuum to an oil (220 mg). The crude product was purified by preparative layer chromatography on two 0.1 x 20 x 20 cm silica gel GF plates, developing with 5% MeOH in CH₂Cl₂. The UV visible product band was eluted with 10% MeOH in CH₂Cl₂ and the eluant was evaporated under vacuum to afford 7-(acetylamino)-9a-butyl-8-methyl-1,2,9,9a-tetrahydro-3*H*-fluoren-3-one (75 mg, 21% yield over two steps) as an oil.

### Step 6: 7-amino-9a-butyl-8-methyl-1,2,9,9a-tetrahydro-3H-fluoren-3-one

A mixture of 7-(acetylamino)-9a-butyl-8-methyl-1,2,9,9a-tetrahydro-3*H*-fluoren-3-one (75 mg, 0.24 mmol), ethanol (1 mL), aqueous 5N sodium hydroxide (0.10 mL, 0.5 mmol), and water (0.25 mL) was stirred and heated in an oil bath at 90°C for 4 hours. After cooling to room temperature, the mixture was partitioned between EtOAc (10 mL) and water (10 mL) containing aqueous 2N HCl (0.25 mL). Th e organic phase was washed with brine (10 mL), dried over MgSO₄, filtered, and evaporated under vacuum to an oil. This material was lyophilized from benzene (2 mL) to afford 7-amino-9a-butyl-8-methyl-1,2,9,9a-tetrahydro-3*H*-fluoren-3-one (60 mg, 92% yield) as an amorphous solid.

### Step 7: 9a-butyl-8-methy-3-oxo-2,3,9,9a-tetrahydro-1H-fluorene-7-diazonium hexafluorophosphate

A solution of 7-amino-9a-butyl-8-methyl-1,2,9,9a-tetrahydro-3*H-*fluoren-3-one (20 mg, 0.074 mmol) in acetic acid (0.25 mL) was treated with aqueous 2N HCl (0.25 mL) and the resulting dark solution was cooled in an ice bath. After 3 minutes, sodium nitrite (5.6 mg, 0.082 mmol) was added and the dark solution was stirred at 0°C for 20 minutes. The reaction mixture was then treated with aqueous 0.543M potassium hexafluorophosphate (0.273 mL, 0.148 mmol) to give a gummy precipitate. The mixture was centrifuged and the insoluble portion was washed with cold water (2 x 4 mL) and dried under vacuum to afford crude 9a-butyl-8-methyl-3-oxo-2,3,9,9a-tetrahydro-1*H*-fluorene-7-diazonium hexafluorophosphate as a semi-solid.

### Step 8: 9a-butyl-8,9,9a,10-tetrahydroindeno[2,1-e]indazol-7(3H)-one

The crude diazonium salt from step 7 was dissolved in chloroform-*d* (CDCl₃, 1.0 mL) and the solution was treated with potassium acetate (15 mg, 0.148 mmol) and dibenzo-18-crown-6 (2.7 mg, 0.0074 mmol). The resulting dark suspension was sonicated for one minute, stirred at room temperature for 30 minutes, and then partitioned between EtOAc (10 mL) and water (10 mL) containing aqueous 2N HCl (1 mL). The organic phase was washed with brine (5 mL), dried over MgSO₄, filtered, and evaporated under vacuum. The residue was purified by preparative layer chromatography on a 0.05 x 20 x 20 cm silica gel GF plate that was developed with 5% MeOH in CH₂Cl₂. The UV visible product band was extracted with 10% MeOH in CH₂Cl₂ and the extracts were evaporated under vacuum to afford 9a-butyl-8,9,9a,10-tetrahydroindeno[2,1-*e*]indazol-7(3*H*)-one (6.7 mg).
¹H NMR (DMSO-*d*₆, 500 MHz) δ 0.77 (t, CH₂CH₂CH₂C*H*_{*3*}), 1.09-1.29 (m, CH₂C*H*_{*2*}CH₂CH₃), 1.41 and 1.64 (two m, C*H*_{*2*}CH₂CH₂CH₃), 1.99 and 2.25 (two ddd, 9-C*H*_{*2*}), 2.30 and 2.52 (two ddd, 8-C*H*_{*2*}), 2.91 and 3.30 (two d, 10-C*H*_{*2*}), 6.19 (s, *H*-6), 7.49 (d, *H*-4 or *H*-5), 7.66 (d, *H*-5 or *H*-4), 8.19 (s, *H*-1), and 13.40 (br s, N*H*).

### EXAMPLE 23

### SYNTHESIS OF 6-BROMO-9a-BUTYL-8,9,9a,10-TETRAHYDROINDENO[2,1-e]INDAZOL-7(3H)-ONE

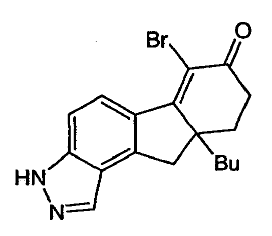

### Step 1: 7-(acetylamino)-4-bromo-9a-butyl-8-methyl-1,2,9,9a-tetrahydro-3H-fluoren-3-one

Sodium bicarbonate (32 mg, 0.384 mmol) and bromine (6.6 µL, 0.128 mmol) were added to a suspension of 7-(acetylamino)-9a-butyl-8-methyl-1,2,9,9a-tetrahydro-3*H*-fluoren-3-one (40 mg, 0.128 mmol) in carbon tetrachloride (1 mL). The mixture was stirred at room temperature for 15 minutes, and then partitioned between EtOAc (20 mL) and dilute aqueous Na₂S₂O₃. The organic phase was washed with brine (10 mL), dried over MgSO₄, filtered, and evaporated under vacuum to an oil. The ¹H NMR spectrum of this material showed starting material and product. The oil was taken up in carbon tetrachloride (1 mL) and dichloromethane (0.5 mL), treated with sodium bicarbonate (32 mg, 0.384 mmol) and bromine (3 µL, 0.058mmol), and the mixture was stirred at room temperature for 5 minutes. Workup as described above gave an oil that was lyophilized from benzene (3 mL) to afford crude 7-(acetylamino)-4-bromo-9a-butyl-8-methyl-1,2,9,9a-tetrahydro-3*H*-fluoren-3-one (50 mg) as an amorphous solid.

### Step 2: 7-amino-4-bromo-9a-butyl-8-methyl-1,2,9,9a-tetrahydro-3H-fluoren-3-one

A mixture of crude 7-(acetylamino)-4-bromo-9a-butyl-8-methyl-1,2,9,9a-tetrahydro-3*H*-fluoren-3-one (50 mg), ethanol (1.75 mL), water (0.25 mL), and aqueous 5N NaOH (0.1 mL) was stirred and heated in an oil bath at 100°C for 3.5 hours. After cooling to room temperature, the mixture was diluted with EtOAc (20 mL), washed with water (20 mL) containing aqueous 2N HCl (0.25 mL), and washed with brine (10 mL). The combined aqueous washes were back-extracted with EtOAc (10 mL). The combined EtOAc solution was dried over MgSO₄, filtered, and evaporated under vacuum to an oil (44 mg). The crude product was purified by preparative layer chromatography on a 0.1 x 20 x 20 cm silica gel GF plate, developing with10% EtOAc in CH₂Cl₂. The UV visible product band was extracted with EtOAc, the extracts were evaporated under vacuum, and the residue was lyophilized from benzene to afford 7-amino-4-bromo-9a-butyl-8-methyl-1,2,9,9a-tetrahydro-3*H*-fluoren-3-one (20 mg) as an amorphous solid.

### Step 3: 4-bromo-9a-butyl-8-methyl-3-oxo-2,3,9,9a-tetrahydro-1H-fluorene-7-diazonium hexafluorophosphate

Sodium nitrite (3.3 mg, 0.047 mmol) was added to an ice-cold suspension of 7-amino-4-bromo-9a-butyl-8-methyl-1,2,9,9a-tetrahydro-3*H*-fluoren-3-one (15 mg, 0.043 mmol) in acetic acid (0.3 mL) and aqueous 2N HCl (0.2 mL). After stirring for 2 minutes at 0°C, the suspension gave way to a yellow solution. After stirring for an additional 45 minutes at 0°C, the solution was treated with aqueous 0.543M potassium hexafluorophosphate (0.160 mL, 0.086 mmol) and ice-cold water (1 mL). The precipitate that formed was collected by centrifugation, washed with cold water (2 x 3 mL), and dried under vacuum to provide crude 4-bromo-9a-butyl-8-methyl-3-oxo-2,3,9,9a-tetrahydro-1*H*-fluorene-7-diazonium hexafluorophosphate.

### Step 4: 6-bromo-9a-butyl-8,9,9a,10-tetrahydroindeno[2,1-e]indazol-7(3H)-one

The crude diazonium salt from step 3 was dissolved in chloroform-*d* (CDCl₃, 1.0 mL) and the solution was treated with potassium acetate (8.5 mg, 0.086 mmol) and dibenzo-18-crown-6 (0.7 mg, 0.002 mmol). The resulting suspension was sonicated for one minute and stirred at room temperature for 130 minutes. The mixture was directly purified by preparative layer chromatography on a 0.05 x 20 x 20 cm silica gel GF plate, developing with 5% MeOH in CH₂Cl₂. The UV visible product band was extracted with 10% MeOH in CH₂Cl₂, the extracts were evaporated under vacuum, and the residue was lyophilized from benzene (3 mL) to afford 6-bromo-9a-butyl-8,9,9a,10-tetrahydroindeno[2,1-*e*]indazol-7(3*H*)-one (8.5 mg) as an amorphous solid.
¹H NMR (DMSO-*d*₆, 500 MHz) δ 0.76 (t, CH₂CH₂CH₂C*H*_{*3*}), 1.07-1.26 (m, CH_{Z}C*H*_{*2*}C*H*_{*2*}CH₃), 1.41 and 1.67 (two m, CH₂C*H*_{*2*}CH₂CH₃), 2.14 and 2.24 (two ddd, 9-C*H*_{*2*}), 2.59 and 2.79 (two ddd, 8-C*H*_{*2*}), 3.04 and 3.36 (two d, 10-C*H*_{*2*}), 7.58 (d, *H*-4), 8.27 (s, *H*-1), 8.45 (d, *H*-5), and 13.49 (br s, N*H*).

### EXAMPLE 24

### SYNTHESIS OF 9a-BUTYL-6-TRIFLUOROMETHYL-8,9,9a,10-TETRAHYDROINDENO[2,1-e]INDAZOL-7(3H)-ONE

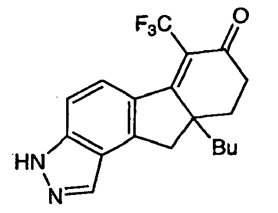

### Step 1: 7-(acetylamino)-9a-butyl-8-methyl-4-trifluoromethyl-1,2,9,9a-tetrahydro-3H fluoren-3-one

7-(Acetylamino)-4-bromo-9a-butyl-8-methyl-1,2,9,9a-tetrahydro-3*H-*fluoren-3-one (500 mg, 1.29 mmol) and copper(I) iodide (294 mg, 1.54 mmol) were taken up in anhydrous *N,N*-dimethylformamide (25.8 mL). The resulting mixture was placed under a N₂ atmosphere, treated with methyl (fluorosulfonyl)difluoroacetate (1.2 mL, 9.42 mmol), and then stirred with heating in an oil bath at 80°C for 7 hours. After cooling to room temperature, the mixture was filtered. The filtrate was washed with water (5 x 200 mL) and brine (50 mL), dried over MgSO₄, filtered, and concentrated under vacuum to an orange oil (0.7 g). The crude product was purified by column chromatography on EM silica gel 60 (230-400 mesh, 2.5 x 20 cm column), eluting with CH₂Cl₂ (32 x 8 mL fractions) followed by 20% EtOAc in CH₂Cl₂ (28 x 8 mL fractions). The product containing fractions were combined and evaporated under vacuum to afford 7-(acetylamino)-9a-butyl-8-methyl-4-trifluoromethyl-1,2,9,9a-tetrahydro-3*H*-fluoren-3-one (230 mg) as an oil.

### Step 2: 7-amino-9a-butyl-8-methyl-4-trifluoromethyl-1,2,9,9a-tetrahydro-3H-fluoren-3-one

A solution of 7-(acetylamino)-9a-butyl-8-methyl-4-trifluoromethyl-1,2,9,9a-tetrahydro-3*H*-fluoren-3-one (210 mg) in EtOH (7.6 mL) was treated with water (2 mL) and conc. HCl (1 mL). The resulting solution was stirred and heated in an oil bath at 80°C for 2.4 hours. After cooling to room temperature, the mixture was filtered to remove a solid precipitate. The solid was rinsed with EtOH (0.5 mL) and dried under vacuum to provide an orange solid (87 mg). This material was identified as the HCl salt of the desired product. The filtrate and washings were added to EtOAc (20 mL), washed with aqueous NaHCO₃ (20 mL) and brine (10 mL), dried over MgS04, filtered, and concentrated under vacuum to an oil (104 mg). This material was purified by preparative layer chromatography (PLC) on a 0.1 x 20 x20 cm silica gel GF plate, developing with 5% MeOH in CH₂Cl₂. The UV visible product band was eluted with 10% MeOH in CH₂Cl₂ and the eluant evaporated under vacuum to provide the product free base.

The HCl salt (87 mg) described above was partitioned between EtOAc (10 mL) and aqueous NaHCO₃ (10 mL). The EtOAc phase was washed with brine (10 mL), dried over MgSO₄, filtered, and evaporated under vacuum to provide the product free base (80 mg) as an oil. This material was combined with the free base isolated after PLC and the combination lyophilized from benzene to afford 7-amino-9a-butyl-8-methyl-4-trifluoromethyl-1,2,9,9a-tetirahydro-3*H*-fluoren-3-one (136 mg) as an amorphous, orange solid.

### Step 3: 9a=butyl-6-trifluorometyl-8-9,9a,10-tetrahydroindeno[2,1-e]indazol-7(3H)-one

A solution of 7-amino-9a-butyl-8-methyl-4-trifluoromethyl-1,2,9,9a-tetrahydro-3*H*-fluoren-3-one (50 mg, 0.148 mmol) in anhydrous CH₂Cl₂ (0.74 mL) was cooled in a dry ice-acetonitrile bath (approx. -30°C). The cold solution was treated with nitrosonium tetrafluoroborate (17.7 mg, 0.151 mmol) and the mixture was stirred in the cold for 55 minutes. The reaction mixture was treated with KOAc (29 mg, 0.296 mmol) and dibenzo-18-crown-6 (2.7 mg, 0.0074 mmol) and then stirred with gradual warming from -25°C to 0°C over 25 minutes. The mixture was removed from the cooling bath and stirred at room temperature for 15 minutes. The mixture was partitioned between CH₂C1₂ (20 mL) and water (20 mL). The organic phase was dried over MgSO₄, filtered, and evaporated under vacuum to an orange oil (57 mg). The crude product was purified by preparative layer chromatography on a 0.1 x 20 x 20 cm silica gel GF plate, developing with 10% EtOAc in CH₂Cl₂. The product band was eluted with EtOAc, the eluant evaporated under vacuum, and the residue lyophilized from benzene to afford 9a-butyl-6-trifluoromethyl-8,9,9a,10-tetrahydroindeno[2,1-*e*]indazol-7(3*H*)-one (20 mg) as an amorphous solid.
¹H NMR (CDCl₃, 500 MHz) δ 0.81 (t, CH₂CH₂CH₂C*H*_{*3*}), 1.16-1.26 (m, CH₂C*H*_{*2*}C*H*_{*2*}CH₃), 1.33 and 1.60 (two m, C*H*_{*2*}CH₂CH₂CH₃), 2.15 and 2.32 (two ddd, 9-C*H*_{*2*}), 2.55-2.67 (m, 8-C*H*_{*2*}), 3.07 and 3.32 (two d, 10-C*H*_{*2*}), 7.45 (d, *H-*4)*,* 7.84 (qd, *H*-5), 8.15 (s, *H*-1), and 10.31 (br s, N*H*); mass spectrum m/z 349.2 (M+1).

### EXAMPLE 25

### SYNTHESIS OF 9a-BUTYL-6-METHYL-8,9,9a,10-TETRAHYDROMENO[2,1-e]INDAZOL-7(3H)-ONE

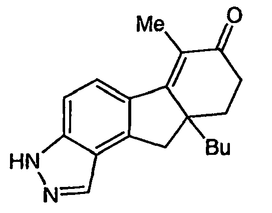

### Step 1: 5-[N-(3-oxopentyl)acetylamino]-2-butyl-4-methyl-2-(3-oxopentyl)-1-indanone

A suspension of 5-(acetylamino)-2-butyl-4-methyl-1-indanone (169 mg, 0.65 mmol) in anhydrous tetrahydrofuran (1.3 mL) was treated with ethyl vinyl ketone (EVK, 0.078 mL, 0.78 mmol) and 1,8-diazabicyclo[5.4.0]undec-7-ene (0.0194 mL, 0.13 mmol). The mixture was stirred under a nitrogen atmosphere and heated in an oil bath at 60°C. After 5 minutes, the suspension gave way to a solution. After 18.7 hours, the mixture was treated with additional EVK (0.078 mL, 0.78 mmol) and DBU (0.02 mL, 0.13 mmol) and then heated at 60°C for an additional 4 hours. The reaction mixture, which contains the 5-[*N*-(3-oxopentyl)acetylamino]-2-butyl-4-methyl-2-(3-oxopentyl)-1-indanone product, was cooled to room temperature and used directly in the next step.

### Step 2: 7-[N-(3-oxopentyl)acegylamino]-9a-butyl-4,8-dimethyl-1,2,9,9a-tetrahydro-3H-fluoren-3-one

The reaction mixture from step 1 was diluted with acetic acid (3 mL) and aqueous 6N HCl (3 mL) and the resulting mixture was stirred and heated in an oil bath at 100°C for 19.8 hours. After cooling to room temperature, the mixture was partitioned between EtOAc (20 mL) and water (20 mL) containing aqueous 2N HCl (2 mL). The organic portion was washed with water (20 mL), aqueous 5% NaHCO₃ (50 ml) and brine (20 mL), dried over MgSO₄, filtered, and evaporated under vacuum to an oil (300 mg). The crude product was purified by preparative layer chromatography on three 0.1 x 20 x 20 cm silica gel GF plates, developing with 10% EtOAc in CH₂Cl₂. The UV visible product band was extracted with EtOAc and the extracts were evaporated under vacuum to afford 7-[N (3-oxopentyl)acetylamino]-9a-butyl-4,8-dimethyl-1,2,9,9a-tetrahydro-3*H*-fluoren-3-one (130 mg) as an oil. The ¹H NMR spectrum of this material showed a mixture of two amide rotational isomers. A second UV visible band provided a by-product identified as 9a-butyl-4-ethyl-6,11-dimethyl-8,9,9a,10-tetrahydro-7*H*-indeno[1,2-*g*]quinolin-7-one by ¹H NMR.

### Step 3: 7-amino-9a-butyl-4,8-dimethyl-1,2,9,9a-tetrahydro-3H-fluoren-3-one

A solution of 7-[*N*-(3-oxopentyl)acetylamino]-9a-butyl-4,8-dimethyl-1,2,9,9a-tetrahydro-3*H*-fluoren-3-one (130 mg) in ethanol (4 mL) was treated with aqueous 5N NaOH (7 drops). The resulting mixture was stirred and heated in an oil bath at 100°C for 3 hours, then treated with more 5N NaOH (3 drops) and heated an additional one hour at 100°C. After cooling to room temperature, the mixture was diluted with EtOAc (20 mL), washed with water (20 mL) containing 2N HCl (1 mL), and washed with brine (10 mL). The combined aqueous washes were back-extracted with EtOAc (5 mL). The combined organics were dried over MgSO₄, filtered, and evaporated under vacuum to a gum. This material was purified by preparative layer chromatography on a 0.1 x 20 x 20 cm silica gel GF plate, developing with 5% MeOH in CH₂Cl₂. The UV visible product band was extracted with 10% MeOH in CH₂Cl₂, the extracts were evaporated under vacuum, and the residue was lyophilized from benzene to afford 7-amino-9a-butyl-4,8-dimethyl-1,2,9,9a-tetrahydro-3*H*-fluoren-3-one (65 mg) as an amorphous solid.

### Step 4: 9a-butyl-4,8-dimethyl-3-oxo-2,3,9,9a-tetrahydro-1H-fluorene-7-diazonium hexafluorophosphate

A solution of 7-amino-9a-butyl-4,8-dimethyl-1,2,9,9a-tetrahydro-3*H-*fluoren-3-one (20 mg, 0.071 mmol) in acetic acid (0.25 mL) was treated with aqueous 2N HCl (0.25 mL) and the resulting solution was cooled in an ice bath. Sodium nitrite(5.4 mg, 0.078 mmol) was added and the solution was stirred at 0°C for 50 minutes. The reaction mixture was then treated with aqueous 0.543M potassium hexafluorophosphate (0.26 mL, 0.141 mmol) to give a solid precipitate. The mixture was diluted with cold water (1 mL) and centrifuged. The solid pellet was washed with cold water (3 mL) and dried under vacuum to afford crude 9a-butyl-4,8-dimethyl-3-oxo-2,3,9,9a-tetrahydro-1*H*-fluorene-7-diazonium hexafluorophosphate as a powder.

### Step 5: 9a-butyl-6-methyl-8,9,9a,10-tetrahydroindeno[2,1-e]indazol-7(3H)-one

The crude diazonium salt from step 4 was dissolved in chloroform-*d* (CDCl₃, 1.0 mL) and the solution was treated with potassium acetate (14 mg, 0.142 mmol) and dibenzo-18-crown-6 (1.3 mg, 0.0036 mmol). The resulting suspension was sonicated for one minute and then stirred at room temperature for 15 minutes. The mixture was purified by preparative layer chromatography on a 0.1 x 20 x 20 cm silica gel GF plate that was developed with 5% MeOH in CH₂Cl₂. The UV visible product band was extracted with 10% MeOH in CH₂Cl₂, the extracts were evaporated under vacuum, and the residue was lyophilized from benzene (3 mL) to afford 9a-butyl-6-methyl-8,9,9a,10-tetrahydroindeno[2,1-*e*]indazol-7(3*H*)-one (14 mg) as a yellow-orange, amorphous solid.
¹H NMR (DMSO-*d*₆, 500 MHz) δ 0.76 (t, CH₂CH₂CH₂C*H*_{*3*}), 1.09-1.26 (m, CH₂C*H*_{*2*}CH₂CH₃), 1.32 and 1.59 (two m, C*H*_{*2*}CH₂CH₂CH₃), 2.02 and 2.22 (two ddd, 9-C*H*_{*2*}), 2.03 (s, 6-C*H*_{*3*}), 2.35 and 2.55 (two ddd, 8-C*H*_{*2*}), 2.92 and 3.27 (two d, 10-C*H*_{*2*}), 7.51 (d, *H*-4), 7.75 (d, *H*-5), 8.20 (s, *H*-1), and 13.33 (s, N*H*).

### EXAMPLE 26

### SYNTHESIS OF 6-METHYL-9a-PROPYL-8,9,9a,10-TETRAHYDROINDENO[2,1-e]INDAZOL-7(3H)-ONE OXIME

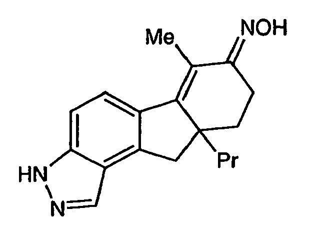

A mixture of 6-methyl-9a-propyl-8,9,9a,10-tetrahydroindeno[2,1-*e*]indazol-7(3*H*)-one (10 mg, 0.036 mmol), hydroxylamine hydrochloride (12.4 mg, 0.178 mmol), and pyridine (0.18 mL) was stirred at room temperature for 4 days. The mixture was evaporated under vacuum and the residue was partitioned between EtOAc (10 mL) and water (10 mL) containing aqueous 2N HCl (5 mL). The organic phase was dried over MgSO₄, filtered, and evaporated under vacuum to a solid. The crude product was purified by preparative layer chromatography on a 0.025 x 20 x 20 cm silica gel GF plate, developing twice with 5% MeOH in CH₂Cl₂. The UV visible product band was extracted with 10 % MeOH in CH₂Cl₂, the extracts were evaporated under vacuum, and the residue was lyophilized from benzene to afford 6-methyl-9a-propyl-8,9,9a,10-tetrahydroindeno[2,1-*e*]indazol-7(3*H*)-one oxime (7.8 mg) as an amorphous solid. The NMR spectrum revealed a 96:4 oxime isomers, the major isomer presumable having the (7E)-configuration.
¹H NMR (DMSO-*d*₆, 500 MHz) δ 0.73 (t, CH₂CH₂C*H*_{*3*}), 1.09 and 1.14 (two m, CH₂C*H*_{*2*}CH₃), 1.21 and 1.33 (two dt, C*H*_{*2*}CH₂CH₃), 1.61 and 2.09 (two ddd, 9-C*H*_{*2*}), 2.14 (s, 6-C*H*_{*3*}), 2.29 and 2.79 (two ddd; 8-C*H*_{*2*}), 2.82 and 3.17 (two d, 10-C*H*_{*2*}), 7.43 (d, *H*-4), 7.67 (d, *H*-5), 8.09 (s, *H*-1)*,* 10.83 (s, OH), and 13.17 (s, N*H*). The minor isomer showed δ 10.28 (s, O*H*).

### EXAMPLE 27

### SYNTHESIS OF (RAC)-(9aS,10S)-9a-ETHYL-6,10-DIMETHYL-8,9,9a,10-TETRAHYDROINDENO[2,1-e]INDAZOL-7(3H)-ONE

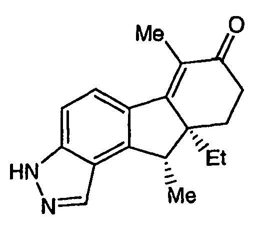

### Step 1: 1-(2-methyl-3-nitrophenyl)ethanone

A solution of 2-methyl-3-nitrobenzoyl chloride (4.00 g, 20.0 mmol) and palladium (II) acetate (150 mg, 0.67 mmol) in anhydrous tetrahydrofuran (20 mL) was placed under a nitrogen atmosphere and cooled in an ice bath. The cold solution was treated with 2.0M dimethylzinc in toluene (11 mL, 22.0 mmol) over 3 minutes. The reaction mixture was stirred at 0°C for 30 minutes and at room temperature for 30 minutes, then added to aqueous 2N HCl (50 mL) and extracted with EtOAc (50 mL). The organic phase was washed with water (50 mL) and brine (50 mL), dried over MgSO₄, filtered, and evaporated under vacuum to an oil (4.49 g). The crude product was purified by chromatography on a Biotage Flash 40S KP-Sil column using 9:1 hexanes-EtOAc as the eluting solvent. The product containing fractions were combined and concentrated to approximately one third volume to give a suspension. The mixture was filtered and the solid portion washed with hexanes and dried under vacuum to afford 1-(2-methyl-3-nitrophenyl)ethanone (0.96 g, 27% yield) as a white solid. The filtrate and washings were evaporated under vacuum and the residue was crystallized from warm 9:1 hexanes-EtOAc to give additional product (0.59 g, 16%) as white solid.

### Step 2: tert-butyl (2Z)-3-(2-methyl-3-nitrophenyl)-2-butenoate and tert-butyl (2E)-3-(2-methyl-3-nitrophenyl)-2-butenoate

A solution of 1-(2-methyl-3-nitrophenyl)ethanone (0.78g, 4.35 mmol) and (tert-butoxycarbonylmethylene)triphenylphosphorane (1.80 g, 4.79 mmol) in anhydrous toluene (5.0 mL) was placed under a nitrogen atmosphere, stirred, and heated at reflux for 10 hours. After storing at room temperature for two days, the reaction mixture was treated with additional phosphorane (1.00 g, 2.66 mmol) and heated at reflux for an additional 24 hours. After cooling, the mixture was applied to a Biotage Flash 40S KP-Sil column which was eluted with 10% EtOAc in hexanes. The product containing fractions were combined and evaporated under vacuum to afford a 7:3 mixture of *tert*-butyl (2*Z*)-3-(2-methyl-3-nitrophenyl)-2-butenoate and *tert*-butyl (2*E*)-3-(2-methyl-3-nitrophenyl)-2-butenoate (517 mg, 43% yield) as an oil. The major isomer is tentatively assigned the (2*Z*)-configuration.

### Step 3: tert-butyl (2Z)-3-(3-amino-2-methylphenyl)-2-butenoate and tert-butyl (2E)-3-(3-amino-2-methylphenyl)-2-butenoate

A mixture of mixture of *tert*-butyl (2Z)-3-(2-methyl-3-nitrophenyl)-2-butenoate and *tert*-butyl (2*E*)-3-(2-methyl-3-nitrophenyl)-2-butenoate (517 mg, 1.86 mmol) and 10% palladium on carbon (51 mg) in methanol ((5 mL) was hydrogenated on a Parr shaker at 50 psi for 16 hours at room temperature. The reaction mixture was filtered and the filtrate evaporated under vacuum to afford a crude mixture of *tert-*butyl (2Z)-3-(3-amino-2-methylphenyl)-2-butenoate and *tert-*butyl (2*E*)-3-(3-amino-2-methylphenyl)-2-butenoate (480 mg) as an oil.

### Step 4: tert-butyl 3-(3-amino-2-methylphenyl)butanoate

The crude product mixture (480 mg) from Step 3 and 10% palladium on carbon (50 mg) were mixed with methanol (5 mL) and the resulting mixture was hydrogenated at 500 psi and 110°C for 18 hours. After cooling to room temperature, the mixture was filtered through a 0.45 µm Acrodisc, washing the filter with MeOH. The filtrate and washings were evaporated under vacuum to provide crude *tert-butyl* 3-(3-amino-2-methylphenyl)butanoate (328 mg) as an oil.

### Step 5: tert-butyl 3-[3-(acetylamino)-2-methylphenyl]butanoate

Acetyl chloride (0.140 mL, 1.97 mmol) was added to a solution of crude *tert*-butyl 3-(3-amino-2-methylphenyl)butanoate (328 mg, 1.32 mmol) and triethylamine (0.367 mL, 2.64 mmol) in anhydrous dichloromethane (4.0 mL). The resulting solution was stirred at room temperature for 20 minutes, and then evaporated under vacuum to an oil which was partitioned between EtOAc (15 mL) and water (15 mL). The organic phase was washed with brine, dried over MgSO₄ filtered, and evaporated under vacuum to afford crude *tert*-butyl 3-[3-(acetylamino)-2-methylphenyl]butanoate (400 mg) as an oil.

### Step 6: 5-(acetylamino)-3,4-dimethyl-1-indanone

A mixture of crude *tert*-butyl 3-[3-(acetylamino)-2-methylphenyl]butanoate (400 mg, 1.37 mmol) and polyphosphoric acid (9.0 g) was placed under a nitrogen atmosphere and heated in an oil bath at 80°C for 75 minutes. After cooling to room temperature, the mixture was partitioned between water (15 mL) and EtOAc (15 mL). The aqueous phase was back-extracted with EtOAc (15 mL). The combined organics were washed with brine (15 mL), dried over MgSO₄, filtered, and evaporated under vacuum to an oil (255 mg). The crude product was purified by chromatography on a Biotage Flash 12M KP-Sil column, eluting with 2:1 EtOAc-hexanes. The product containing fractions were combined and evaporated under vacuum to provide 5-(acetylamino)-3,4-dimethyl-1-indanone (91 mg, 31% yield) as a semi-solid.

### Step 7: 5-(acetyamino)-3,4-dimethyl-2-ethyl-1-indanone

A solution of 5-(acetylamino)-3,4-dimethyl-1-indanone (91 mg, 0.42 mmol) in methanol (2.0 mL) was stirred under a nitrogen atmosphere with ice-bath cooling, treated with 10% palladium on carbon (10 mg), and then treated with 0.5M sodium methoxide in methanol (0.168 mL, 0.083 mmol). The mixture was purged with hydrogen, placed under a hydrogen atmosphere, and treated with acetaldehyde (0.050 mL, 0.84 mmol). The resulting mixture was stirred rapidly under hydrogen while allowing the cooling bath to gradually warm to room temperature. After 17 hours, the reaction mixture was concentrated under vacuum and the residue was partitioned between EtOAc (10 mL) and aqueous 0.5N HCl (5 mL). The organic phase was washed with water (5 mL) and brine (5 mL), dried over MgSO₄, filtered, and evaporated under vacuum to a clear oil (96 mg). The NMR spectrum of this material shows mainly a single isomer of the 5-(acetylamino)-3,4-dimethyl-2-ethyl-1-indanone product.

### Step 8: 5-(acetylamino)-3,4-dimethyl-2-ethyl-2-(3-oxopentyl)-1-indanone

An ice-cold solution of crude 5-(acetylamino)-3,4-dimethyl-2-ethyl-1-indanone (96 mg, 0.42 mmol) in methanol (1.0 mL) was stirred under nitrogen and treated with ethyl vinyl ketone (EVK, 0.052 mL, 0.52 mmol) followed by 0.5M sodium methoxide in methanol (0.168 mL, 0.084 mmol). The ice bath was removed and the reaction mixture was stirred at room temperature for 30 minutes and then heated in an oil bath at 60°C. Additional EVK (0.050 mL, 0.50 mmol) was added at 5 hours and the mixture was stirred and heated at 60°C for a total of 21 hours. After cooling, the mixture was partitioned between EtOAc (10 mL) and aqueous 1N HCl (5 mL). The organic phase was washed with water (5 mL) and brine (5 mL), dried over MgSO₄, filtered, and evaporated under vacuum to an oil (148 mg). NMR and LC-MS of this material revealed a complex mixture that contained 5-(acetylamino)-3,4-dimethyl-2-ethyl-2-(3-oxopentyl)-1-indanone as a major component.

### Step 9: (rac)-(9S,9aS)-7-amino-9a-ethyl-4,8,9-trimethyl-1,2,9,9a-tetrahydro-3H-fluoren-3-one

A solution of crude 5-(acetylamino)-3,4-dimethyl-2-ethyl-2-(3-oxopentyl)-1-indanone (148 mg) in acetic acid (1.75 mL) was diluted with aqueous 6N HCl (1.75 mL) and the resulting mixture was stirred and heated in an oil bath at 80°C for 5.5 hours. After cooling, the reaction mixture was partitioned between saturated aqueous K₂CO₃ (5 mL) and EtOAc (10 mL). The organic phase was washed with water (5 mL) and brine (10 mL), dried over MgSO₄, filtered, and evaporated under vacuum to a film (100 mg). The crude product was purified by chromatography on a Biotage Flash 12M KP-Sil column, eluting with 30% EtOAc in hexanes. The product containing fractions were evaporated under vacuum to provide (*rac*)-(9*S*,9a*S*)-7-amino-9a-ethyl-4,8,9-trimethyl-1,2,9,9a-tetrahydro-3*H*-fluoren-3-one (30.4 mg) as an oil.

### Step 10: (rac)-(9aS,10S)-9a-ethyl-6,10-dimethyl-8,9,9a.10-tetrahydroindeno[2,1-e]indazol-7(3H)-one

A solution of (*rac*)-(9*S*,9a*S*)-7-amino-9a-ethyl-4,8,9-trimethyl-1,2,9,9a-tetrahydro-3*H*-fluoren-3-one (29 mg, 0.108 mmol) in anhydrous dichloromethane (1.1 mL) was placed under a nitrogen atmosphere, cooled in an acetonitrile-dry ice bath at -42°C, and treated with nitrosonium tetrafluoroborate (15 mg, 0.128 mmol). The mixture was stirred in the cooling bath with gradual warming to -15°C over 45 minutes. Immediately following the NOBF₄ addition and at 30 minutes thereafter, the mixture was briefly sonicated to solubilize small grains of solids. After 45 minutes, the temperature was readjusted to -30°C and stirring was continued. At 56 minutes, the mixture was cooled to -42°C, stirred 10 minutes, and then treated with dibenzo-18-crown-6 (2.3 mg, 0.0064 mmol) followed by potassium acetate (25.5 mg, 0.260 mmol). The resulting mixture was stirred with gradual warming to room temperature over two hours and then stirred at room temperature for one hour. The mixture was partitioned between EtOAc (10 mL) and water (5 mL). The organic phase was washed with brine (5 mL), dried over MgSO₄, filtered, and evaporated under vacuum to a film (35.7 mg). The crude product was purified by chromatography on a Biotage Flash 12M KP-Sil column, eluting with 2:1 hexanes-EtOAc. The product containing fractions were combined and evaporated under vacuum to afford (*rac*)-(9a*S*,10*S*)-9a-ethyl-6,10-dimethyl-8,9,9a,10-tetrahydroindeno[2,1-*e*]indazol-7(3*H*)-one (22 mg, 73% yield) as a film. This material was lyophilized from benzene to provide the product as a yellow, amorphous solid.
¹H NMR (CDCl₃, 500 MHz) δ 0.47 (t, CH₂C*H*_{*3*}), 1.60 and 1.82 (two m, C*H*_{*2*}CH₃), 1.65 (d, 10-C*H*_{*3*}), 2.11 and 2.25 (two ddd, 9-C*H*_{*2*}), 2.17 (s, 6-C*H*_{*3*}), 2.53 and 2.71 (two ddd, 8-C*H*_{*2*}), 3.37 (q, *H*-10), 7.44 (d, *H*-4), 7.77 (d, *H*-5), 8.27 (s, *H*-1), and 10.37 (br s, N*H*); mass spectrum m/z 281.4 (M+1).

### EXAMPLE 28

### SYNTHESIS OF 9a-BUTYL-6-ETHYL-4-FLUORO-8,9,9a,10-TETRAHYDROINDENO[2,1-e]INDAZOL-7(3H)-ONE

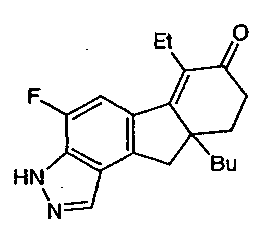

### Step 1: tert-butyl 2-fluorophenylcarbamate

A solution of di-*tert*-butyl dicarbonate (104 g, 477 mmol) and 2-fluoroaniline (53 g, 477 mmol) in tetrahydrofuran (500 mL) was heated at 60°C for 3. days. After the solution was cooled to room temperature, THF was removed under vacuum. The residue was dissolved in EtOAc, washed twice with 1M citric acid, dried over MgSO₄, and filtered through a pad of silica. The filtrate was diluted with hexane and the precipitate was filtered off. The new filtrate concentrated under vacuum to provide *tert*-butyl 2-fluorophenylcarbamate (99.5 g, 99%).

### Step 2: tert-butyl 2-fluoro-6-methylphenylcarbamate

A solution of *tert*-butyl 2-fluorophenylcarbamate (99.5 g, 471 mmol) in anhydrous tetrahydrofuran (410 mL) was placed under a nitrogen atmosphere, cooled in a dry ice-acetone bath, and stirred while t-butyllithium (666 mL of a 1.7M solution in pentanes, 207.5 mmol) was added dropwise. The resulting mixture was slowly warmed to -15°C over 2 hours, then cooled back to-78°C and treated with iodomethane (32.3 mL, 518 mmol). After warming to -15°C in 30 minutes, the mixture was treated with aqueous saturated NH₄Cl (20 mL) and diluted with dichloromethane (2 L). The organic phase was dried over MgSO₄, filtered through a pad of silica, and concentrated under vacuum to afford *tert*-butyl 2-fluoro-6-methylphenylcarbamate (105 g, 99%) as a foam.

### Step 3: 2-fluoro-6-methylaniline

Trifluoroacetic acid (TFA, 500 mL) was carefully added to *tert*-butyl 2-fluoro-6-methylphenylcarbamate (105 g, 467 mmol) and the resulting solution was stirred at room temperature for one hour. The TFA was removed under vacuum and the residue was diluted with dichloromethane (1 L), carefully quenched with solid sodium carbonate, and filtered through a pad of silica. The filtrate was evaporated under vacuum to provide crude 2-fluoro-6-methylaniline.

### Step 4: 4-bromo-2-fluoro-6-methylaniline

A solution of the crude 2-fluoro-6-methylaniline from step 3 in *N,N-*dimethylformamide (DMF, 400 mL) was cooled in a ice bath, treated with *N-*bromosuccinimide (83.7 g, 470 mmol), and stirred for one hour. The DMF was evaporated under vacuum. The residue was dissolved in EtOAc, washed three times with brine, dried over MgSO₄, and filtered through a pad of silica, and the filtrate evaporated under vacuum to provide 4-bromo-2-fluoro-6-methylaniline (80 g, 84%).

### Step 5: N-(4-bromo-2-fluoro-6-methylphenyl)-2,2-dimethylpropanamide

Pivaloyl chloride (56 mL, 459 mmol) was added slowly (caution - exothermic reaction) to an ice cold solution of 4-bromo-2-fluoro-6-methylaniline (78 g, 382 mmol) in pyridine (200 mL). After the addition, the ice bath was removed and the hot reaction mixture was stirred at room temperature for 30 minutes. The mixture was dissolved in EtOAc (1 L), washed with 1N HCl (3 times), dried over MgSO₄, filtered, and concentrated under vacuum. The crude product was purified by flash chromatography on a Biotage 75L KP-Sil column, eluting with 20:1 to 10:1 hexanes-EtOAc. The product containing fractions were combined and evaporated under vacuum to provide *N*-(4-bromo-2-fluoro-6-methylphenyl)-2,2-dimethylpropanamide (49 g).

### Step 6: N-(2-fluoro-4-hexanoyl-6-methylphenyl)-2,2-dimethylpropanamide

A solution of *N*-(4-bromo-2-fluoro-6-methylphenyl)-2,2-dimethylpropanamide (20.9 g, 72.5 mmol) in anhydrous tetrahydrofuran (400 mL) was placed under a nitrogen atmosphere, cooled in a dry ice-acetone bath, and stirred while butyllithium (113 mL of a 1.6M solution in hexanes, 181 mmol) was added dropwise by syringe pump over 2.5 hours. The resulting mixture was aged at -78°C for 30 minutes and then treated with *N,N*-dimethylhexanamide (25.9 g, 181 mmol) added dropwise. After warming to -30°C in 30 minutes, the mixture was removed from the cooling bath, treated with aqueous saturated NH₄Cl and diluted with dichloromethane (2 L). The organic phase was dried over MgSO₄, filtered through a pad of silica, and the filtrate concentrated under vacuum. The crude product was purified by flash chromatography on a Biotage 75L KP-Sil column, eluting with 20:1 to 10:1 hexanes-EtOAc. The product containing fractions were evaporated under vacuum to afford *N*-[2-fluoro-4-hexanoyl-6-methylphenyl]-2,2-dimethylpropanamide (17 g with about 5g of side products based on NMR).

### Step 7: N-[4-(2-bugylacryloyl)-2-fluoro-6-methylphenyl]-2,2-dimethylpropanamide, N-{2-fluoro-4-[2-(hydroxymethyl)hexanoyl]-6-metylphenyl]-2,2-dimethylpropanamide, and N-{2-fluoro-4-[2-(methoxymethyl)hexanoyl]-6-metylphenyl]-2,2-dimethylpropanamide

The crude *N*-(2-fluoro-4-hexanoyl-6-methylphenyl)-2,2-dimethylpropanamide from step 6 was dissolved in methanol (40 mL) and the solution was treated successively with K₂CO₃ (5.38 g, 39 mmol) and formaldehyde (37 wt. % solution in water, 3.22 mL, 43 mmol). The mixture was placed under a nitrogen atmosphere, stirred, and heated in an oil bath at 50°C for 2 hours. After cooling to room temperature, the mixture was diluted with CH₂Cl₂ (300 mL), dried over MgSO₄, and filtered through a pad of silica gel, using more CH₂Cl₂ (200 mL) to wash the pad. The filtrate and washings were concentrated under vacuum to provide a mixture (19.5 g) of *N*-[4-(2-butylacryloyl)-2-fluoro-6-methylphenyl]-2,2-dimethylpropanamide, *N*-{2-fluoro-4-[2-(hydroxymethyl)hexanoyl]-6-metylphenyl]-2,2-dimethylpropanamide, and *N*-{2-fluoro-4-[2-(methoxymethyl)hexanoyl]-6-methyl-phenyl]-2,2-dimethylpropanamide.

### Step 8: 5-amino-2-butyl-6-fluoro-4-methyl-1-indanone

A solution of the product mixture from step 7 in CH₂Cl₂ (10 mL) was cooled in an ice bath and treated with ice-cold, conc. H₂SO₄ (400 mL). The resulting mixture was removed from the cooling bath and stirred at room temperature for 20 hours. The mixture was cautiously added to an ice-cold mixture of CH₂Cl₂ (300 mL) and chopped ice (1 L). Excess acid was neutralized by portionwise addition of saturated Na₂CO₃ solution (approx. 1 L) followed by solid Na₂CO₃ (approx. 500 g). Additional water and CH₂Cl₂ were occasionally added to dissolve the red/purple solids that formed during the neutralization. When the pH was neutral, the organic phase was separated and the aqueous portion was extracted with more CH₂Cl₂. The combined organics were washed with brine, dried over MgSO₄, filtered, and concentrated under vacuum. The residual dark red oil was purified by flash chromatography on a Biotage 75L KP-Sil column, eluting with 20:1 to 10:1 hexanes-EtOAc. The product containing fractions were combined and evaporated under vacuum to provide 5-amino-2-butyl-6-fluoro-4-methyl-1-indanone (4.2 g) as a yellow solid.

### Step 9: 5-amino-2-butyl-6-fluoro-4-methyl-2-(3-oxohexyl)-1-indanone

A solution of 5-amino-2-butyl-6-fluoro-4-methyl-1-indanone (0.235 g, 1 mmol) in ethanol (5 mL) was treated with sodium methoxide (0.5M solution in MeOH, 0.4. mL, 0.2 mmol) and propyl vinyl ketone (147 mg, 1.5 mmol). The resulting solution was stirred at 75°C and under a nitrogen atmosphere for 20 hours. The mixture was diluted with CH₂Cl₂, filtered through a pad of silica gel, and the filtrate evaporated under vacuum to provide 5-amino-2-butyl-6-fluoro-4-methyl-2-(3-oxohexyl)-1-indanone.

### Step 10: 7-amino-9a-butyl-6-fluoro-8-methyl-1,2,9,9a-tetrahydro-3H-fluoren-3-one

The crude diketone from step 9 was treated with acetic acid (2.5 mL) and 6N HCl (2.5 mL). The resulting solution was stirred and heated in an oil bath at 100°C for 1.5 hours. After cooling to room temperature, the mixture was diluted with CH₂Cl₂ and neutralized with sodium bicarbonate, filtered through a pad of silica gel and the product was washed off with EtOAc. The filtrate and washings were concentrated under vacuum. The residue was purified by flash chromatography on a Biotage 40M KP-Sil column, eluting with 3:1 hexanes-EtOAc. The product containing fractions were evaporated under vacuum to provide 7-amino-9a-butyl-6-fluoro-8-methyl-1,2,9,9a-tetrahydro-3*H*-fluoren-3-one (0.260 g) as a yellow solid.

### Step 11: 9a-butyl-6-ethyl-4-fluoro-8,9,9a,10-tetrahydroindeno[2,1-e]indazol-7(3H)-one

An ice cold solution of 7-amino-9a-butyl-6-fluoro-8-methyl-1,2,9,9a-tetrahydro-3*H*-fluoren-3-one (0.165 g, 0.54 mmol) in HOAc (2.4 mL) and 2N HCl (1.5 mL) was treated with NaNO₂ (37 mg, 0.54 mmol). The resulting solution was stirred in an ice bath and under a nitrogen atmosphere for one hour, and then treated with KPF₆ (100mg/mL in water, 1.99 mL, 1.08 mmol). The mixture was extracted with EtOAc, and the organic portion was washed with water (3 times), dried over Na₂SO₄, filtered , and evaporated under vacuum.

The diazonium salt from above in CH₂Cl₂ (6 mL) at -78°C was treated with KOAc (106 mg, 1.08 mmol) and dibenzo-18-crown-6 (catalytic amount). The resulting solution was slowly warmed to 0°C over 40 min. The mixture was filtered through a pad of silica gel and the product was washed off with EtOAc. The filtrate was concentrated under vacuum to a residue that was purified by preparative layer chromatography (0.1 x 20 x 20 cm silica gel GF plate developed with 2:1 hexanes-EtOAc). The product band was eluted with EtOAc and the eluant evaporated under vacuum to afford 9a-butyl-6-ethyl-4-fluoro-8,9,9a,10-tetrahydroindeno[2,1-e]indazol-7(3*H*)-one (22 mg) as an orange foam.
¹H NMR (CDCl₃, 500 MHz) δ 0.82 (t, CH₂CH₂CH₂C*H*_{*3*}), 1.15 (t, 6-CH₂C*H*_{*3*}), 1.22 (m, CH₂C*H*_{*2*}C*H*_{*2*}CH₃), 1.45 and 1.63 (two m, C*H*_{*2*}CH₂CH₂CH₃), 2.06 and 2.30 (two ddd, 9-C*H*_{*2*}), 2.51 and 2.61 (two ddd, 8-C*H*_{*2*}), 2.55 and 2.69 (two dq, 6-C*H*_{*2*}CH₃), 2.90 and 3.23 (two d, 10-C*H*_{*2*}), 7.46 (d, *H*-5), and 8.17 (s, *H-*1); mass spectrum m/z 327.2 (M+1), 390.2 (M+Na+MeCN), and 675.3 (2M+Na).

### EXAMPLE 29

### SYNTHESIS OF 6-ACETYL-9a-BUTYL-4-FLUORO-8,9,9a,10-TETRAHYDROINDENO[2,1-e]INDAZOL-7(3H)-ONE

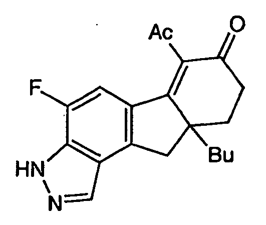

### Step1: 5-amino-2-butyl-6-fluoro-4-methyl-2-(3-oxobutyl)-1-indanone

A solution of 5-amino-2-butyl-6-fluoro-4-methyl-1-indanone (2.7 g, 11.5 mmol) in ethanol (60 mL) was treated with sodium methoxide (0.5M solution in MeOH, 4.6 mL, 2.3 mmol) and methyl vinyl ketone (1.43 mL, 17.2 mmol). The resulting solution was stirred at 40°C and under a nitrogen atmosphere for 20 hours. The mixture was diluted with CH₂Cl₂ and filtered through a pad of silica gel, washing the product off with EtOAc. The filtrate and washings were evaporated under vacuum to provide crude 5-amino-2-butyl-6-fluoro-4-methyl-2-(3-oxobutyl)-1-indanone.

### Step 2: 7-amino-9a-butyl-6-fluoro-8-methyl-1,2.9.9a-tetrahydro-3H-fluoren-3-one

The diketone from step 1 was dissolved in toluene (100 mL) and treated with acetic acid (1.31 mL, 23 mmol) and pyrrolidine (1.92 mL, 23 mmol). The resulting solution was stirred and heated in an oil bath at 100°C for 2 hours. After cooling to room temperature, the mixture was filtered through a pad of silica gel and the product was washed off with EtOAc. The filtrate and washings were concentrated under vacuum. The residue was purified by flash chromatography on a Biotage 40M KP-Sil column, eluting with 4:1 to 3:1 hexanes-EtOAc. The product containing fractions were evaporated under vacuum to provide 7-amino-9a-butyl-6-fluoro-8-methyl-1,2,9,9a-tetrahydro-3*H*-fluoren-3-one (2.56 g) as a yellow solid.

### Step 3: 7-arnino-4-bromo-9a-butyl-6-fluoro-8-methyl-1,2,9,9a-tetrahydro-3H-fluoren-3-one

A solution of 7-amino-9a-butyl-6-fluoro-8-methyl-1,2,9,9a-tetrahydro-3*H*-fluoren-3-one (1.4 g, 4.88 mmol) in anhydrous DMF (20 mL) was purged with N₂, cooled in an ice bath, and treated with *N*-bromosuccinimide (0.868 g, 4.88 mmol). After stirring at 0°C for 2 hour, the reaction mixture was partitioned between water (150 mL) and EtOAc (150 mL). The organic portion was washed with diluted brine (3 times), dried over MgSO₄, filtered, and evaporated under vacuum. The residue was purified by flash chromatography on a Biotage 40M KP-Sil column, eluting with 4:1 hexanes-EtOAc. The product containing fractions were evaporated under vacuum to provide 7-amino-4-bromo-9a-butyl-6-fluoro-8-methyl-1,2,9,9a-tetrahydro-3*H-*fluoren-3-one (1.5 g) as a yellow solid.

### Step 4: 7-(acetylamino)-4-bromo-9a-butyl-6-fluoro-8-methyl-1,2,9,9a-tetrahydro-3H-fluoren-3-one

A solution of 7-amino-4-bromo-9a-butyl-6-fluoro-8-methyl-1,2,9,9a-tetrahydro-3*H*-fluoren-3-one (0.47 g, 1.28 mmol) in anhydrous CH₂Cl₂ (4 mL) was purged with N₂; cooled in an ice bath, and treated with pyridine (0.206 mL, 2.56 mmol) followed by acetyl chloride (0.137 mL, 1.93 mmol). After stirring at 0-5°C for 2 hours, the reaction mixture was diluted with EtOH (8 mL) and treated with 5N NaOH (1.28 mL). After stirring at room temperature for 10 min, the mixture was further diluted with CH₂Cl₂ (20 mL), dried over MgSO₄, filtered, and concentrated under vacuum. The residue was purified by flash chromatography on a Biotage 40M KP-Sil column, eluting with 4:1 hexanes-EtOAc. The product containing fractions were evaporated under vacuum to provide 7-(acetylamino)-4-bromo-9a-butyl-6-fluoro-8-methyl-1,2,9,9a-tetrahydro-3*H*-fluoren-3-one (0.47 g) as a yellow solid.

### Step 5: 4-acetyl-7-amino-9a-butyl-6-fluoro-8-methyl-1,2,9,9a-tetrahydro-3H-fluoren-3-one

A partial solution of 7-(acetylamino)-4-bromo-9a-butyl-6-fluoro-8-methyl-1,2,9,9a-tetrahydro-3*H*-fluoren-3-one (49 mg, 0.12 mmol) in anhydrous toluene (1 mL) was purged with N₂, treated with dichlorobis(triphenylphosphine)-palladium(II) (17 mg, 0.024 mmol), purged with N₂, and treated with tributyl(1-ethoxyvinyl)tin (0.057 mL, 0.18 mmol). The resulting mixture was stirred under a N₂ atmosphere and heated in an oil bath at 100°C for two hours. After cooling to room temperature, the mixture was purified by preparative layer chromatography (0.1 x 20 x 20 cm silica gel GF plate developed with 1:1 hexanes-EtOAc). The product band was eluted with EtOAc and the eluant evaporated under vacuum to afford 7-(acetylamino)-9a-butyl-4-(1-ethoxyvinyl)-6-fluoro-8-methyl-1,2,9,9a-tetrahydro-3*H-*fluoren-3-one as an orange foam.

### Step 6: 4-acetyl-7-amino-9a-butyl-6-fluoro-8-methyl-1,2,9,9a-tetrahydro-3H-fluoren-3-one

The enol ether from step 5 was dissolved in AcOH (1 mL) and the solution was treated with aqueous 6N HCl (1 mL). The resulting mixture was placed under a N₂ atmosphere and stirred with heating in an oil bath at 100°C for 3 h. After cooling to room temperature, the mixture was diluted with CH₂Cl₂ and neutralized with Na₂CO₃, filtered through a pad of silica gel, and the product was washed off with EtOAc. The filtrate and washings were concentrated under vacuum. The mixture was purified by preparative layer chromatography (0.1 x 20 x 20 cm silica gel GF plate developed with 3:2 hexanes-EtOAc). The product band was eluted with EtOAc and the eluant evaporated under vacuum to afford 4-acetyl-7-amino-9a-butyl-6-fluoro-8-methyl-1,2,9,9a-tetrahydro-3*H*-fluoren-3-one (22 mg) as an orange foam

### Step 7: 6-acetyl-9a-butyl-4-fluoro-8,9,9a,10-tetrahydroindeno[2,1-e]indazol-7(3H)-one

An ice cold solution of 4-acetyl-7-amino-9a-butyl-6-fluoro-8-methyl-1,2,9,9a-tetrahydro-3*H*-fluoren-3-one (0.022 g,0.067 mmol) in HOAc (0.8 mL) and 2N HCl (0.5 mL) was treated with NaNO₂ (4.6 mg, 0.067 mmol). The resulting solution was stirred in a ice bath under a nitrogen atmosphere for 1 hour, then treated with KPF₆ (100mg/mL in water, 0.246 mL, 0.134 mmol). The mixture was extracted with EtOAc and the extracts were washed with water (3 times), dried over Na₂SO₄, filtered, and evaporated under vacuum to provide the crude diazonium salt intermediate.

The diazonium salt in CH₂Cl₂ (2 mL) at -78°C was treated with KOAc (13 mg, 0.134 mmol) and dibenzo-18-crown-6 (catalytic amount). The resulting solution was slowly warmed up to 0°C over 40 min. The mixture was purified by preparative layer chromatography (0.1 x 20 x 20 cm silica gel GF plate developed with 2:1 hexanes-EtOAc). The product band was eluted with EtOAc and the eluant evaporated under vacuum to afford 6-acetyl-9a-butyl-4-fluoro-8,9,9a,10-tetrahydroindeno[2,1-*e*]indazol-7(3*H*)-one (4.5 mg) as an orange foam.
¹H NMR (CDCl₃, 500 MHz) δ 0.84 (t, CH₂CH₂CH₂C*H*_{*3*}), 1.18-1.32 (m, CH₂C*H*_{*2*}C*H*_{*2*}CH₃), 1.49 and 1.72 (two m, C*H*_{*2*}CH₂CH₂CH₃), 2.12 and 2.36 (two ddd, 9-C*H*_{*2*}), 2.45 (s, COC*H*_{*3*}), 2.56 and 2.63 (two ddd, 8-C*H*_{*2*}), 2.93 and 3.28 (two d, 10-C*H*_{*2*}), 7.21 (d, *H*-5), and 8.15 (d, *H-*1*);* mass spectrum m/z 341.2 (M+1).

### EXAMPLE 30

### SYNTHESIS OF 6-ETHYL-3,9,10,11-TETRAHYDRO-8,10a-METHANOZULENO[2,1-e]INDAZOL-7(8H)-ONE

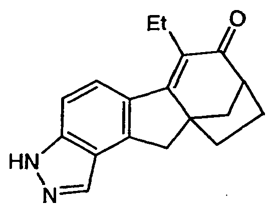

### Step 1: 5-(acetylamino)-2-(2-hydroxyethyl)4-methyl-1-indanone

Potassium hydroxide (5 g, 85% weight pure) and 10% palladium on activated carbon (5 g) were added to a mixture of 5-(acetylamino)-4-methyl-1-indanone (25.4 g, 125 mmol) and glycolaldehyde dimer (10 g, 83.3 mmol) in methanol (500 mL). The resulting mixture was stirred under an atmosphere of hydrogen at room temperature for 30 hours. The mixture was diluted with CH₂Cl₂ (1 L), treated with 5 mL saturated aqueous NH₄Cl, dried over MgSO₄, filtered through a pad of silica, and the filtrate evaporated under vacuum to afford crude 5-(acetylamino)-2-(2-hydroxyethyl)-4-methyl-1-indanone (34.0 g) as a foam.

### Step 2: 5-(acetylamino)-2-(2-hydroxyethyl)-4-methyl-2-(3-oxohexyl)-1-indanone

A solution of 5-(acetylamino)-2-(2-hydroxyethyl)-4-methyl-1-indanone (5.1 g, 20.6 mmol) in methanol (100 mL) was treated with sodium methoxide (0.5M solution in MeOH, 41.2 mL, 20.6 mmol) and propyl vinyl ketone (3.04 g, 31 mmol). The resulting solution was stirred at 40°C and under a nitrogen atmosphere for 20 hours. The mixture was diluted with CH₂Cl₂, filtered through a pad of silica gel, and the filtrate was concentrated under vacuum. The crystalline portion of 5-(acetylamino)-2-(2-hydroxyethyl)-4-methyl-2-(3-oxohexyl)-1-indanone was collected and the filtrate was evaporated under vacuum. The residue was purified by preparative layer chromatography (0.1 x 20 x 20 cm silica gel GF plate developed with EtOAc) to provide additional 5-(acetylamino)-2-(2-hydroxyethyl)-4-methyl-2-(3-oxohexyl)-1-indanone.

### Step 3: 7-amino-4-ethyl-9a-(2-hydroxyethyl)-8-methyl-1,2,9,9a-tetrahydro-3H-fluoren-3-one

The diketone from step 2 was treated with acetic acid (60 mL) and 6N HCl (60 mL). The resulting solution was stirred and heated in an oil bath at 100°C for 1.5 hours. After cooling to room temperature, the mixture was diluted with CH₂Cl₂ and neutralized with Na₂CO₃, filtered through a pad of silica gel, and the product was washed off with 7:3 CH₂Cl₂-MeOH. The filtrate and washings were concentrated under vacuum. The residue was purified by flash chromatography on a Biotage 40M KP-Sil column, eluting with 30:1 to 9:1 CH₂Cl₂-MeOH. The product containing fractions were evaporated under vacuum to provide 7-amino-4-ethyl-9a-(2-hydroxyethyl)-8-methyl-1,2,9,9a-tetrahydro-3*H*-fluoren-3-one (5 g) as a yellow solid.

### Step 4: 6-ethyl-9a-(2-hydroxyethyl)-8,9,9a,10-tetrahydroindeno[2,1-e]indazol-7(3H)-one

A solution of 7-amino-4-ethyl-9a-(2-hydroxyethyl)-8-methyl-1,2,9,9a-tetrahydro-3*H*-fluoren-3-one (0.050 g, 0.175 mmol) in anhydrous CH₂Cl₂ (1 mL) and DMF (1 mL) was purged with N₂, cooled in an dry ice bath, and treated with NOBF₄ (0.023 g, 0.193 mmol). The mixture was warmed to -10°C over 40 minutes to give the diazonium salt intermediate.

The diazonium salt mixture from above was cooled to -78°C and treated with KOAc (34 mg, 0.35 mmol) and dibenzo-18-crown-6 (catalytic amount). The resulting solution was slowly warmed to 0°C over 40 min. The mixture was filtered through a pad of silica gel and the product was washed off with 9:1 CH₂Cl₂₋MeOH. The filtrate was concentrated under vacuum to a residue that was purified by preparative layer chromatography (0.1 x 20 x 20 cm silica gel GF plate developed with 12:1 CH₂Cl₂-MeOH. The product band was eluted with 9:1 CH₂Cl₂-MeOH and the eluant evaporated under vacuum to afford 6-ethyl-9a-(2-hydroxyethyl)-8,9,9a,10-tetrahydroindeno[2,1-*e*]indazol-7(3*H*)-one (6 mg) as an orange foam.

### Step 5: 6-ethyl-3-methylsulfonyloxy-9a-[2-(methylsulfonyloxyl)ethyl]-8,9,9a,10-tetrahydroindeno[2,1-e]indazol-7(3H)-one

A solution of 6-ethyl-9a-(2-hydroxyethyl)-8,9,9a,10-tetrahydroindeno[2,1-*e*]indazol-7(3*H*)-one (20 mg, 0.0676 mmol) in anhydrous CH₂Cl₂ (1 mL) was purged with N₂ and treated with triethyl amine (0.038 mL, 0.27 mmol) followed by methanesulfonyl chloride (0.0156 mL, 0.203 mmol). After stirring for 2 hours, the mixture was purified by preparative layer chromatography (0.1 x 20 x 20 cm silica gel GF plate developed with 2:3 hexanes-EtOAc). The product band was eluted with EtOAc and the eluant evaporated under vacuum to afford 6-ethyl-3-methylsulfonyloxy-9a-[2-(methylsulfonyloxy)ethyl]-8,9,9a,10-tetrahydroindeno[2,1-*e*]indazol-7(3*H*)-one as an orange foam.

### Step 6: 6-ethyl-3.9,10,11-tetrahydro-8,10a-methanoazuleno[2,1-e]indazol-7(8H)-one

A solution of 6-ethyl-3-methylsulfonyloxy-9a-[2-(methylsulfonyloxy)ethyl]-8,9,9a,10-tetrahydroindeno[2,1-*e*]indazol-7(3*H*)-one from step 5 in toluene (1 mL) was treated with DBU (0.020 mL). The resulting mixture was placed under a N₂ atmosphere and stirred with heating in an oil bath at 90°C for 6 h. After cooling to room temperature, the reaction mixture was diluted with MeOH (2 mL) and treated with 5N NaOH (0.1 mL). After stirring at room temperature for 20 min, the mixture was further diluted with CH₂Cl₂ (20 mL), treated with aqueous saturated NH₄Cl (0.5 mL), and dried over MgSO₄. The mixture was filtered through a pad of silica and the filtrate was concentrated under vacuum. The residue was purified by preparative layer chromatography (0.1 x 20 x 20 cm silica gel GF plate developed with 1:1 hexanes-EtOAc). The product band was eluted with EtOAc and the eluant evaporated under vacuum to 6-ethyl-3,9,10,11-tetrahydro-8,10a-methanoazuleno[2,1-*e*]indazol-7(8*H*)-one (5 mg) as an pale yellow foam.
¹H NMR (CDCl₃, 500 MHz) δ 1.11 (t, CH₂C*H*_{*3*}), 1.69 and 1.91 (two ddd, 10-C*H*_{*2*}), 1.84 and 2.30 (two m, 9-C*H*_{*2*}), 1.99 and 2.03 (two dd, 12-C*H*_{*2*}), 2.60 and 2.74 (two dq, C*H*_{*2*}CH₃), 3.10 (dd, *H*-8), 3.32 and 3.48 (two d, 11-C*H*_{*2*}), 7.51 (d, *H*-4), 7.80 (d, *H*-5), and 8.16 (s, *H*-1); mass spectrum m/z 279.3 (M+1.

### EXAMPLE 31

### SYNTHESIS OF 9a-ETHYL-6-METHYL-8,9,9a,10-TETRAHYDROFLUORENO[1,2-d]IMAZOL-7(3H)-ONE

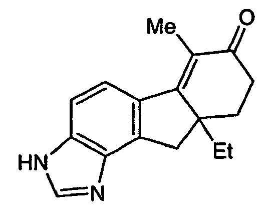

### Step 1: 5-(acetylamino)-2-ethyl-1-indanone

A solution of 5-(acetylamino)-1-indanone (5.00 g, 26.1 mmol) in ethanol (100 mL, required heating to affect solution) was treated with 0.5M sodium methoxide in methanol (10.5 mL, 5.2 mmol) and moist 20% palladium hydroxide on carbon (500 mg). The mixture was cooled in an ice bath, placed under a hydrogen atmosphere, and treated with acetaldehyde (2.9 mL, 52 mmol). The resulting mixture was stirred under a hydrogen atmosphere for one hour at 0°C followed by 23 hours at room temperature. The mixture was filtered through a pad of silica gel (100 mL) which was further eluted with EtOAc (300 mL). The filtrate was washed with 1N HCl (300 mL) and the aqueous layer back-extracted with EtOAc (150 mL). The combined organics were washed with aqueous 5% NaHCO₃ (300 mL) and brine (300 mL), dried over MgSO₄, and filtered. The solution was treated with EM silica gel 60 (ca. 35 mL) and the mixture evaporated under vacuum. The silica gel was loaded onto a Sample Injection Module, placed in tandem with a Biotage Flash 40M KP-Sil column, and eluted with 2:1 hexanes-EtOAc (1000 mL) followed by 1:1 hexanes-EtOAc (2000 mL). The product containing fractions were combined and evaporated under vacuum to afford 5-(acetylamino)-2-ethyl-1-indanone (2.88 g, 51% yield) as a white solid.

### Step 2: 5-(acetylamino)-2-ethyl-4-nitro-1-indanone

Nitric acid (≥90% by weight, 22 mL) and 5-(acetylamino)-2-ethyl-1-indanone (2.88 g, 13 mmol) were separately cooled in an ice bath and then combined. The mixture was placed under a nitrogen atmosphere and stirred at 0°C for 35 minutes, then partitioned between EtOAc (150 mL) and water (150 mL). The aqueous phase was extracted with EtOAc (2 x 50 mL). The combined organics were washed with water (100 mL) and brine (100 mL), dried over MgSO₄, filtered, and concentrated under vacuum to approximately half-volume. The solution was treated with EM silica gel 60 (ca. 20 mL) and evaporated under vacuum. The silica gel was loaded onto a Sample Injection Module, placed in tandem with a Biotage Flash 40M KP-Sil column, and eluted with 4:1 hexanes-EtOAc (3000 mL). The product containing fractions were combined and evaporated under vacuum to afford 5-(acetylamino)-2-ethyl-4-nitro-1-indanone (825 mg, 24% yield) as a yellow solid. Earlier fractions from the column chromatography provided the isomeric 6-nitro product.

### Step 3: 5-amino-2-ethyl-4-nitro-1-indanone

A partial solution/suspension of 5-(acetylamino)-2-ethyl-4-nitro-1-indanone (807 mg, 3.1 mmol) in methanol (10.5 mL) was treated with aqueous 6N hydrochloric acid (10.5 mL). The resulting mixture was placed under a nitrogen atmosphere and stirred with heating in an oil bath at 80°C for 45 minutes. After cooling, the mixture was partitioned between EtOAc (200 mL) and aqueous 5% NaHCO₃ (200 mL). The aqueous phase was back-extracted with EtOAc (50 mL). The combined organics were washed with brine, dried over MgSO₄, filtered, and concentrated under vacuum to afford 5-amino-2-ethyl-4-nitro-1-indanone (683 mg, 100% yield) as a yellow solid.

### Step 4: 4,5-diamino-2-ethyl-1-indanone

5-Amino-2-ethyl-4-nitro-1-indanone (674 mg, 3.06 mmol) was dissolved in EtOAc (13 mL) and EtOH (13 mL) with warming. After cooling to room temperature, the yellow solution was purged with nitrogen and treated with 10% palladium on carbon (263 mg). The resulting mixture was placed under a hydrogen atmosphere and stirred at room temperature for 5 hours. The mixture was filtered through a pad of silica gel (75 mL) which was further washed with EtOAc (500 mL). The filtrate and washings were evaporated under vacuum to provide 4,5-diamino-2-ethyl-1-indanone (560 mg, 96% yield) as a yellow solid.

### Step 5: 7-ethyl-7,8-dihydroindeno[4,5-d]imidazol-6(3H)-one

Triethyl orthoformate (0.58 mL, 3.5 mmol) was added to a suspension of 4,5-diamino-2-ethyl-1-indanone (441 mg, 2.32 mmol) in warm ethanol (4 mL). The resulting orange solution was stirred with heating in an oil bath at 80°C for 16 hours. After cooling, the solution was diluted with EtOAc (300 mL), washed with water (300 mL) and brine (300 mL), dried over MgSO₄, filtered, and evaporated under vacuum to afford crude 7-ethyl-7,8-dihydroindeno[4,5-*d*]imidazol-6(3*H*)-one 420 mg, 91% yield) as an orange solid. This material was used without further purification.

### Step 6: 3-(tert-butyloxycarbonyl)-7-ethyl-7,8-dihydroindeno[4,5-d]imidazol-6(3H)-one

A mixture of 7-ethyl-7,8-dihydroindeno[4,5-*d*]imidazol-6(3*H*)-one (366 mg, 1.83 mmol) and 4-(dimethylamino)pyridine (224 mg, 1.83 mmol) in anhydrous tetrahydrofuran (7 mL) was placed under a nitrogen atmosphere and treated with triethylamine (0.255 mL, 1.83 mmol). The suspension was then treated with di-tert-butyl dicarbonate (0.452 mL, 1.97 mmol) over two minutes to give a clear orange solution. After stirring at room temperature for 14 hours, the solution was diluted with EtOAc (150 mL), washed with aqueous 1N HCl, aqueous 5% NaHCO₃, and brine, dried with MgSO₄, filtered, and evaporated under vacuum to provide 3-(*tert-*butyloxycarbonyl)-7-ethyl-7,8-dihydroindeno[4,5-*d*]imidazol-6(3*H*)-one (525 mg, 96% yield) as an oil.

### Step 7: 3-(tert-butyloxycarbonyl)-7-ethyl-7-(3-oxopentyl)-7,8-dihydroindeno[4,5-d]imidazol-6(3H)-one

A solution of 3-(*tert*-butyloxycarbonyl)-7-ethyl-7,8-dihydroindeno[4,5-*d*]imidazol-6(3*H*)-one (525 mg, 1.75 mmol) in anhydrous tetrahydrofuran (4 mL) was placed under a nitrogen atmosphere and treated with 1,8-diazabicyclo[5.4.0]undec-7-ene (0.053 mL, 0.35 mmol) and ethyl vinyl ketone (0.350 mL, 3.5 mmol). The resulting solution was stirred and heated at 60°C for 22 hours. After cooling, the reaction mixture was diluted with EtOAc (150 mL) and washed with water (150 mL) containing some brine. The aqueous phase was back-extracted with EtOAc (50 mL). The combined organics were washed with aqueous 1N HCl, aqueous 5% NaHCO₃, and brine, dried over MgSO₄, filtered, and evaporated under vacuum to a gum (523 mg). NMR analysis of this material showed a 1.5:1 mixture of product and starting material.

A solution of the above mixture (523 mg) in anhydrous tetrahydrofuran (3.5 mL) was placed under a nitrogen atmosphere and treated with 1,8-diazabicyclo[5.4.0]undec-7-ene (0.026 mL, 0.17 mmol) and ethyl vinyl ketone (0.175 mL, 1.76 mmol). The resulting solution was stirred and heated at 60°C for 24 hours. After cooling, the reaction mixture was partitioned between EtOAc (200 mL) and aqueous 1N HCl (200 mL), and the aqueous portion was back-extracted with EtOAc (50 mL). The combined organics were washed with aqueous 5% NaHCO₃ (150 mL) and brine (150 mL), dried over MgSO₄, filtered, and evaporated under vacuum. The crude product (489 mg) was purified by chromatography on a Biotage Flash 40S KP-Sil column, eluting with 4:1 hexanes-EtOAc (2350 mL) followed by 2:1 hexanes-EtOAc (750 mL). The product containing fractions were combined and evaporated under vacuum to provide 3-(*tert*-butyloxycarbonyl)-7-ethyl-7-(3-oxopentyl)-7,8-dihydroindeno[4,5-*d*]imidazol-6(3*H*)-one (158 mg, 24% yield) as a clear oil.

### Step 8: 9a-ethyl-6-methyl-8,9,9a,10-tetrahydrofluoreno[1,2-d]imidazol-7(3H)-one

A solution of 3-(*tert*-butyloxycarbonyl)-7-ethyl-7-(3-oxopentyl)-7,8-dihydroindeno[4,5-*d*]imidazol-6(3*H*)-one (158 mg, 0.41 mmol) in acetic acid (1.6 mL) was placed under a nitrogen atmosphere and treated with aqueous 37% hydrochloric acid (1.6 mL) followed shortly thereafter with water (1.6 mL). The resulting mixture was stirred and heated in an oil bath at 100°C for 2 hours. After cooling, the mixture was partitioned between EtOAc (100 mL) and aqueous 5% NaHCO₃ (100 mL). The organic phase was washed with water (100 mL) and brine (100 mL), dried over MgSO₄, filtered, and concentrated under vaccum. The residue was purified by preparative layer chromatography on a 0.1 x 20 x 20 cm silica gel GF plate, developing with 5% MeOH in CH₂Cl₂. The UV visible band at R_{f} 0.1-0.23 afforded 9a-ethyl-6-methyl-8,9,9a,10-tetrahydrofluoreno[1,2-*d*]imidazol-7(3*H*)-one (84 mg, 77% yield) as a pale yellow oil. This material was lyophilized from benzene to afford the product as an amorphous solid.
¹H NMR (CDCl₃, 500 MHz) δ 0.84 (t, CH₂C*H*_{*3*}), 1.50 and 1.67 (two m, C*H*_{*2*}CH₃), 2.05 and 2.31 (two m, 9-C*H*_{*2*}), 2.17 (s, C*H*_{*3*}), 2.51 and 2.62 (two ddd, 8-C*H*_{*2*}), 2.92 and 3.37 (two d, 10-C*H*_{*2*}), 7.59 (d, *H*-4 or *H*-5), 7.73 (d, *H*-5 or *H*-4), and 8.24 (s, *H-*2); mass spectrum m/z 267.2 (M+1).

### EXAMPLE 32

### SYNTHESIS OF 6-BROMO-9a-ETHYL-8,9,9a,10-TETRAHYDROFLUORENO[1,2-d]MIDAZOL-7(3H)-ONE

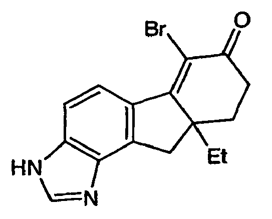

### Step 1: 3-(tert-butyloxycarbonyl)-7-ethyl-7-(3-oxobutyl)-7,8-dihydroindeno[4,5-d]imidazol-6(3H)-one

A solution of 3-(*tert*-butyloxycarbonyl)-7-ethyl-7,8-dihydroindeno[4,5-*d*]imidazol-6(3*H*)-one (118 mg, 0.39 mmol) in anhydrous tetrahydrofuran (0.8 mL) was placed under a nitrogen atmosphere and treated with 1,8-diazabicyclo[5.4.0]undec-7-ene (0.012 mL, 0.08 mmol) and methyl vinyl ketone (0.065 mL, 0.78 mmol). The resulting solution was stirred and heated in an oil bath at 70°C for 23 hours. After cooling, the reaction mixture was partitioned between EtOAc (80 mL) and aqueous 1N HCl (80 mL). The organic phase was washed with aqueous 5% NaHCO₃ and brine, dried over MgSO₄, filtered, and evaporated under vacuum to an oil. The crude product was purified by chromatography on a Biotage Flash 12M KP-Sil column, eluting with 3:1 hexanes-EtOAc (300 mL) followed by 2:1 hexanes-EtOAc (300 mL). The product containing fractions were combined and evaporated under vacuum to provide 3-(*tert*-butyloxycarbonyl)-7-ethyl-7-(3-oxobutyl)-7,8-dihydroindeno[4,5-*d*]imidazol-6(3*H*)-one (31 mg, 22% yield) as a clear oil.

### Step 2: 9a-ethyl-8,9,9a,10-tetrahydrofluoreno[1,2-d]imidazol-7(3H)-one

A solution of 3-(*tert*-butyloxycarbonyl)-7-ethyl-7-(3-oxobutyl)-7,8-dihydroindeno[4,5-*d*]imidazol-6(3*H*)-one (31 mg, 0.084 mmol) in toluene (0.8 mL) was placed under a nitrogen atmosphere and treated pyrrolidine (0.007 mL, 0.084 mmol) and acetic acid (0.005 mL, 0.087 mmol). The resulting mixture was stirred and heated in an oil bath at 85-100°C for 2 hours. After cooling, the reaction mixture was diluted with EtOAc (1 mL), aqueous 6N HCl (1 mL) and water (1 mL) and then stirred at room temperature for one hour. The mixture was partitioned between EtOAc (50 mL) and aqueous 3% NaHCO₃ (50 mL). The organic phase was washed with 5% NaHCO₃ and brine, dried over MgSO₄, filtered, and concentrated under vacuum to provide 9a-ethyl-8,9,9a,10-tetrahydrofluoreno[1,2-*d*]imidazol-7(3*H*)-one (15 mg, 71% yield) as a yellow oil.

### Step 3: 6-bromo-9a-ethyl-8,9,9a,10-tetrahydrofluoreno[1,2-d]imidazol-7(3H)-one

An ice-cold mixture of 9a-ethyl-8,9,9a,10-tetrahydrofluoreno[1,2-*d*]imidazol-7(3*H*)-one (14.1 mg, 0.055 mmol), carbon tetrachloride (0.2 mL) and sodium bicarbonate (25 mg, 0.3 mmol) was placed under a nitrogen atmosphere and treated with bromine (0.003 mL, 0.058 mmol). The mixture was vigorously stirred at 0°C for 35 minutes, and then partitioned between CH₂Cl₂ (30 mL) and water (30 mL). The aqueous phase was back-extracted with CH₂Cl₂ (10 mL). The combined organics were washed with saturated aqueous Na₂S₂O₃ (25 mL) and brine (25 mL), dried over MgSO₄, filtered ,and concentrated under vacuum. The residue was purified by preparative layer chromatography on a 0.1 x 10 x 20 cm silica gel GF plate, developing with 7.5% MeOH in CH₂Cl₂. The major UV visible band was extracted with 5% MeOH in CH₂Cl₂ and the extracts were evaporated under vacuum to afford 6-bromo-9a-ethyl-8,9,9a,10-tetrahydrofluoreno[1,2-*d*]imidazol-7(3*H*)-one 7.8 mg, 43% yiel) as an oil. The oil was lyophilized from benzene (ca. 1 mL) containing a few drops of MeOH to give the product as a white amorphous solid.
¹H NMR (CDCl₃, 500 MHz) δ 0.90 (t, CH₂C*H*_{*3*}), 1.61 and 1.75 (two m, C*H*_{*2*}CH₃), 2.17 and 2.37 (two ddd, 9-C*H*_{*2*}), 2.72-2.84 (m, 8-C*H*_{*2*}), 3.03 (d, one of 10-C*H*_{*2*}), 3.50 (br s, one of 10-C*H*_{*2*}), 7.57 (br s, *H*-4), 8.16 (s, *H*-2), 8.59 (d, *H*-5), and 9.76 (br s, N*H*); mass spectrum m/z 331.3 (M+1) and 333.3 (M+3).

### EXAMPLE 33

### SYNTHESIS OF 9a-BUTYL-6-ETHYL-4-FLUORO-8,9,9a,10-TETRAHYDROFLUORENO[1,2-d]IMIDAZOL-7(3H)-ONE

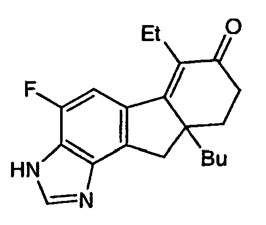

### Step 1: 5-amino-2-butyl-6-fluoro-2-(3-oxohexyl)-1-indanone

A solution of 5-(acetylamino)-6-fluoro-2-butyl-1-indanone (943 mg, 4.3 mmol) in anhydrous MeOH (6.9 mL) was treated with 0.5M NaOMe in MeOH (1.72 mL, 0.86 mmol) followed by propyl vinyl ketone (632 mg, 6.45 mmol). The resulting mixture was purged with N₂ and then stirred in a capped flask with heating in an oil bath at 70°C for 24 hours. After cooling to room temperature, the mixture was partitioned between EtOAc (75 mL) and aqueous saturated NH₄Cl (75 mL). The organic portion was washed with 5% NaHCO₃ and brine, dried over MgSO₄, filtered, and evaporated under vacuum to give an orange oil (1.51 g). The crude product was purified by chromatography on a Biotage Flash 40S column (4.0 x 7.0 cm, KP-Sil), eluting with 4:1 hexanes-EtOAc (0.5 L) followed by 2:1 hexanes-EtOAc (1 L). The product containing fractions were combined and evaporated under vacuum to afford 5-amino-2-butyl-6-fluoro-2-(3-oxohexyl)-1-indanone (569 mg) as an orange oil.

### Step 2: 7-amino-9a-butyl-4-ethyl-6-fluoro-1,2,9,9a-tetrahydro-3H-fluoren-3-one

A solution of 5-amino-2-butyl-6-fluoro-2-(3-oxohexyl)-1-indanone (569 mg, 1.78 mmol) in HOAc (1.8 mL) was diluted with aqueous 6N HCl (1.8 mL). The resulting mixture was purged with N₂ and then stirred in a capped flask while heating in an oil bath at 100°C for 2 hours. After cooling, the mixture was partitioned between EtOAc (75 mL) and aqueous 5% NaHCO₃ (75 mL). The organic phase was washed with brine, dried over MgSO₄, filtered, and concentrated under vacuum. The crude product was purified by chromatography on a Biotage Flash 12M column (12 mm x 15 cm, KP-Sil), eluting with 4:1 hexanes-EtOAc. The product containing fractions were combined and evaporated under vacuum to afford 7-amino-9a-butyl-4-ethyl-6-fluoro-1,2,9,9a-tetrahydro-3*H*-fluoren-3-one (400 mg, 75%) as an orange foam.

### Step 3: 7-amino-9a-butyl-4-ethyl-6-fluoro-8-nitro-1,2,9.9a-tetrahydro-3H-fluoren-3-one

A solution of 7-amino-9a-butyl-4-ethyl-6-fluoro-1,2,9,9a-tetrahydro-3*H*-fluoren-3-one (400 mg, 1.33 mmol) in trifluoroacetic acid (2.6 ml) was treated with 2,3,5,6-tetrabromo-4-methyl-4-nitro-2,5-cyclohexadien-1-one (623 mg, 1.33 mmol). The resulting suspension was purged with N₂, sonicated briefly, and then stirred at room temperature for three hours. The mixture was diluted with EtOAc (50 mL) and washed with water, 5% NaHCO₃, and brine. The organic solution was dried over MgSO4, filtered, and evaporated under vacuum to provide a brown oil (1.033 g). The crude product was purified by chromatography on a Biotage 40M column (4.0 x 15.0 cm, KP-Sil), eluting with 10:1 hexanes-EtOAc (700 mL) followed by 4:1 hexanes-EtOAc (1 L). The product containing fractions were combined and evaporated under vacuum to leave 7-amino-9a-butyl-4-ethyl-6-fluoro-8-nitro-1,2,9,9a-tetrahydro-3*H*-fluoren-3-one (125.5 mg, 27%) as an orange film. The product was lyophilized from benzene to give an amorphous solid.

### Step 4: 7.8-diamino-9a-butyl-4-ethyl-6-fluoro-1,2.9.9a-tetrahydro-3H-fluoren-3-one

A sample of 7-amino-9a-butyl-4-ethyl-6-fluoro-8-nitro-1,2,9,9a-tetrahydro-3*H*-fluoren-3-one (114 mg, 0.33 mmol) was dissolved in EtOH (13 mL) with warming. After cooling, the solution was treated with KOAc (32 mg, 0.33 mmol) and 10% palladium on carbon (32 mg). The resulting mixture was stirred under a hydrogen atmosphere and at room temperature for 3.5 hours. The mixture was filtered through a plug of silica gel with the aid of 5% MeOH in CH₂Cl₂. The filtrate was concentrated to a residue (130 mg) that was purified by preparative layer chromatography (two 0.1 x 20 x 20 cm silica gel GF plates, developed with 1:1 hexanes-EtOAc). The UV visible product band was eluted with EtOAc and the eluant evaporated under vacuum to an orange oil. This material was lyophilized from benzene to provide 7,8-diamino-9a-butyl-4-ethyl-6-fluoro-1,2,9,9a-tetrahydro-3*H-*fluoren-3-one (76 mg, 73%) as an amorphous solid.

### Step 5: 9a-butyl-6-ethyl-4-fluoro-8,9,9a,10-tetrahydrofluoreno[1,2-d]imidazol-7(3H)-one

A solution of 7,8-diamino-9a-butyl-4-ethyl-6-fluoro-1,2,9,9a-tetrahydro-3*H*-fluoren-3-one (24 mg, 0.076 mmol) in EtOH (0.32 mL) was treated with triethyl orthoformate (0.020 mL, 0.11 mmol). The resulting solution was placed under a N₂ atmosphere, stirred, and heated at 80°C in a capped flask for one hour. After cooling, the mixture was concentrated under vacuum. The residue was purified by preparative layer chromatography on a 0.1 x 20 x 20 cm silica gel GF plate, developing with 5% MeOH in CH₂Cl₂. The product band was eluted with 5% MeOH in CH₂Cl₂, the eluant evaporated under vacuum, and the residual yellow oil (5.4 mg) lyophilized from benzene to afford 9a-butyl-6-ethyl-4-fluoro-8,9,9a,10-tetrahydrofluoreno[1,2-*d*]imidazol-7(3*H*)-one as an amorphous solid.
¹H NMR (CDCl₃, 500 MHz) δ 0.80 (t, CH₂CH₂CH₂C*H*_{*3*}), 1.16 (t, 6-CH₂C*H*_{*3*}), 1.14-1.28 (m, CH₂C*H*_{*2*}C*H*_{*2*}CH₃), 1.43 and 1.62 (two m, C*H*_{*2*}CH₂CH₂CH₃), 2.04 and 2.29 (two ddd, 9-C*H*_{*2*}), 2.50 and 2.60 (two ddd, 8-C*H*_{*2*}), 2.55 and 2.71 (two qd, 6-C*H*_{*2*}CH₃), 2.90 and 3.33 (two d, 10-C*H*_{*2*}), 7.41 (d, *H*-5), and 8.42 (s, *H*-2); mass spectrum m/z 327.2 (M+1), 390.2 (M+Na+MeCN), and 675.3 (2M+Na).

### EXAMPLE 34

### SYNTHESIS OF 9a-ETHYL-6-METHYL-8,9,9a,10-TETRAHYDROFLUORENO[1,2-d][1,2,3]TRIAZOL-7(3H)-ONE

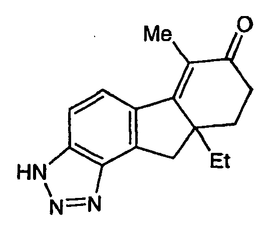

### Step 1: 7-ethyl-7,8-dihydroindeno[4,5-d][1,2,3]triazol-6(3H)-one

An ice-cold solution of 4,5-diamino-2-ethyl-1-indanone (190 mg, 1 mmol) in ethanol (13 mL) was treated with aqueous 37% HCl (1 ml, 12 mmol) and water (0.25 mL). The resulting orange solution was stirred with ice-bath cooling while a solution of NaNO₂ (276 mg, 4 mmol) in water (1.3 mL) was added dropwise over one minute. The solution darkened and a precipitate formed. The mixture was stirred an additional 35 minutes at 0°C and then partitioned between EtOAc (150 mL) and water (150 mL). The organic phase was washed with brine, dried over MgSO₄, filtered, and evaporated under vacuum to afford 7-ethyl-7,8-dihydroindeno[4,5-*d*][1,2,3]triazol-6(3*H*)-one (183 mg, 91% yield) as an orange solid.

### Step 2: 3-(tert-butyloxycarbonyl)-7-ethyl-7,8-dihydroindeno[4,5-d][1,2,3]triazol-6(3H)-one

A solution of 7-ethyl-7,8-dihydroindeno[4,5-*d*][1,2,3]triazol-6(3*H*)-one (180 mg, 0.89 mmol) and 4-(dimethylamino)pyridine (109 mg, 0.89 mmol) in anhydrous tetrahydrofuran (3.6 mL) was placed under nitrogen and stirred at room temperature while triethylamine (0.125 mL, 0.89 mmol) and di-*tert*-butyl dicarbonate (0.225 mL, 0.98 mmol) were added by syringe. After stirring an additional 30 minutes at room temperature, the reaction mixture was partitioned between EtOAc (150 mL) and aqueous 1N HCl (150 mL). The organic phase was washed with aqueous 5% NaHCO₃ (150 mL) and brine (150 mL), dried over MgSO₄, filtered, and concentrated under vacuum, to an orange-pink solid (288 mg). The crude product was purified by chromatography on a Biotage Flash 12M KP-Sil column, eluting with 4:1 hexanes-EtOAc. The product containing fractions were evaporated under vacuum to provide 3-(*tert*-butyloxycarbonyl)-7-ethyl-7,8-dihydroindeno[4,5-*d*][1,2,3]triazol-6(3*H*)-one (246 mg, 92% yield) as a white solid.

### Step 3: 7-ethyl-2-(3-oxopentyl)-7,8-dihydroindenor[4,5-d][1.2.3]triazol-6(2H)-one and 7-ethyl-3-(3-oxopentyl)-7,8-dihydroindeno[4,5-d][1,2,3]triazol-6(3H)-one

A solution of 3-(*tert*-butyloxycarbonyl)-7-ethyl-7,8-dihydroindeno[4,5-*d*][1,2,3]triazol-6(3*H*)-one (246 mg, 0.82 mmol) in anhydrous tetrahydrofuran (2.1 mL) was treated with 1,8-diazabicyclo[5.4.0]undec-7-ene (DBU, 0.024 mL, 0.16 mmol) and ethyl vinyl ketone (EVK, 0.160 mL, 1.6 mmol). The resulting solution was placed under a nitrogen atmosphere and stirred with heating in a 60°C oil bath. NMR and LC-MS analysis of an aliquot removed after heating for 22 hours showed the presence of starting material and two major products. After 22.5 hours at 60°C, the reaction mixture was treated with additional DBU (0.012 mL, 0.08 mmol) and EVK (0.08 mL, 0.8 mmol). The resulting mixture was stirred under nitrogen and heated in an oil bath at 60°C for an additional 16 hours. After cooling, the mixture was diluted with EtOAc (150 mL) and washed with aqueous 1N HCl (150 mL). The aqueous was back-extracted with EtOAc (50 mL). The combined organics were washed with aqueous 5% NaHCO₃ (150 ml) and brine (150 ml), dried over MgSO₄, filtered, and concentrated under vacuum. The residue was purified by chromatography on a Biotage Flash 12M KP-Sil column, eluting with 4:1 hexanes-EtOAc (350 mL) followed by 2:1 hexanes-EtOAc (100 mL). Early fractions provided 7-ethyl-2-(3-oxopentyl)-7,8-dihydroindeno[4,5-*d*][1,2,3]triazol-6(2*H*)-one (70 mg, 30% yield) as an oil, whereas later fractions provided mainly 7-ethyl-3-(3-oxopentyl)-7,8-dihydroindeno[4,5-*d*][1,2,3]triazol-6(3*H*)-one (110 mg, 47% yield) as an oil. The latter product contained approximately 10% of the isomeric 7-ethyl-1-(3-oxopentyl)-7,8-dihydroindeno[4,5-*d*][1,2,3]triazol-6(1*H*)-one product. The structural assignments of the three products are based on analogy to related compounds and are not definitively proven.

### Step 4: 7-ethyl-7-(3-oxopentyl)-7,8-dihydroindeno[4,5-d][1,2,3]triazol-6(3H)-one

A solution of 7-ethyl-2-(3-oxopentyl)-7,8-dihydroindeno[4,5-*d*][1,2,3]triazol-6(2*H*)-one(70 mg, 0.25 mmol) in methanolic 0.5M NaOMe (0.60 mL, 0.3 mmol) was treated with ethyl vinyl ketone (EVK, 0.025 mL, 0.25 mmol). The resulting mixture was stirred and heated in an oil bath at 70°C. After 17 hours, the reaction mixture was treated with additional 0.5M NaOMe in MeOH (0.05 mL, 0.025 mmol) and EVK (0.01 mL, 0.1 mmol), and heating was continued for 4 hours. The mixture was partitioned between EtOAc (150 mL) and aqueous 1N HCl (150 mL), and the aqueous phase was back-extracted with EtOAc (50 mL). The combined organics were washed with aqueous 5% NaHCO₃ (100 mL) and brine (100 mL), dried over MgSO₄, filtered, and evaporated under vacuum. The residue was purified by chromatography on a Biotage Flash 12M KP-Sil column which was eluted with 2:1 hexanes-EtOAc. The product containing fractions were evaporated under vacuum to afford 7-ethyl-7-(3-oxopentyl)-7,8-dihydroindeno[4,5-*d*][1,2,3]triazol-6(3*H*)-one (16 mg, 23% yield) as a clear oil.

### Step 5: 9a-ethyl-6-methyl-8,9,9a,10-tetrahydrofluoreno[1,2-d][1,2,3]triazol-7(3H)-one

A solution of 7-ethyl-7-(3-oxopentyl)-7,8-dihydroindeno[4,5-*d*][1,2,3]triazol-6(3*H*)-one (16 mg, 0.056 mmol) in acetic acid (0.25 mL) was diluted with aqueous 6N HCl (0.25 mL) and the resulting solution was stirred and heated in an oil bath at 100°C for one hour. After cooling to room temperature, the reaction mixture was partitioned between EtOAc (20 mL) and aqueous 5% NaHCO₃ (20 mL). The organic phase was washed with brine (20 mL), dried over MgSO₄, and concentrated under vacuum. The residue was purified by preparative layer chromatography on a 0.1 x 10 x 20 cm silica gel GF plate, developing with 5% MeOH in CH₂Cl₂. The major UV visible band at R_{f} 0.11-0.18 was eluted with 5% MeOH in CH₂Cl₂ and the eluant evaporated under vacuum to leave 9a-ethyl-6-methyl-8,9,9a,10-tetrahydrofluoreno[1,2-*d*][1,2,3]triazol-7(3*H*)-one (13.7 mg, 92% yield) as a yellow oil. This material was lyophilized from benzene to provide the product as a pale yellow, amorphous solid.
¹H NMR (CDCl₃, 500 MHz) δ 0.87 (t, CH₂C*H*_{*3*}), 1.53 and 1.70 (two m, C*H*_{*2*}CH₃), 2.11 and 2.36 (two m, 9-C*H*_{*2*}), 2.20 (s, C*H*_{*3*}), 2.59 and 2.68 (two ddd, 8-C*H*_{*2*}), 3.37 and 3.56 (two d, 10-C*H*_{*2*}), 7.83 (d, *H*-4 or *H*-5), and 7.89 (d, *H*-5 or *H*-4); mass spectrum m/z 268.4 (M+1).

### EXAMPLE 35

### SYNTHESIS OF 6-ALLYL-9a-ETHYL-8,9,9a,10-TETRAHYDROFLUORENO-[1,2-d][1,2,3]TRIAZOL-7(3H]-ONE

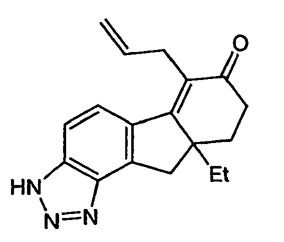

### Step 1: 7-ethyl-2-{[2-(trimethylsilyl)ethoxy]methyl}-7.8-dihydroindeno[4,5-d][1,2,3]triazol-6(2H)-one, 7-ethyl-3-{[2-(trimethylsilyl)ethoxy]methyl}-7.8-dihydroindeno[4,5-d][1,2,3]triazol-6(3H)-one, and 7-ethyl-1-{[2-(trimethylsilyl)ethoxy]methyl}-7,8-dihydroindeno[4,5-d][1,2,3]triazol-6(1H)-one

Sodium hydride (NaH, 115 mg of a 61% dispersion in mineral oil, 2.9 mmol) was added to an ice-cold solution 7-ethyl-7,8-dihydroindeno[4,5-*d*][1,2,3]triazol-6(3*H*)-one (538 mg, 2.67 mmol) in anhydrous dimethylformamide (8 mL). The mixture was stirred under a nitrogen atmosphere and cooled in an ice bath while 2-(trimethylsilyl)ethoxymethyl chloride (SEM-Cl, 0.54 mL, 3.1 mmol) was added over one minute. The resulting mixture was stirred with gradual warming to room temperature. After 16.5 hours, additional NaH (13 mg, 0.33 mmol) and SEM-Cl (0.05 mL, 0.28 mmol) were added to the mixture and stirring was continued for 30 minutes at room temperature. The mixture was partitioned between EtOAc (200 mL) and water (200 mL). The organic phase was washed with brine, dried over MgSO₄, filtered, and concentrated under vacuum to a brown oil (1.2 g). The crude product was purified by chromatography on a Biotage Flash 40S column, eluting with 4:1 hexanes-EtOAc (1000 mL). The early fractions gave 7-ethyl-2-{[2-(trimethylsilyl)ethoxy]methyl}-7,8-dihydroindeno[4,5-*d*] [1,2,3]triazol-6(2*H*)-one (298 mg, 34% yield) as an orange oil. The later fractions provided a 58:42 mixture (464 mg, 52% yield) of 7-ethyl-3-{[2-(trimethylsilyl)ethoxy]methyl}-7,8-dihydroindeno[4,5-*d*][1,2,3]triazol-6(3*H*)-one and 7-ethyl-1-{[2-(trimethylsilyl)ethoxy]methyl}-7,8-dihydroindeno[4,5-*d*][1,2,3]triazol-6(1*H*)-one as a yellow oil. The structural assignments for the three isomeric products are based on analogy to related compounds and are not definitively proven.

### Step 2: 7-ethyl-7-(3-oxobutyl)-3-{[2-(trimethylsilyl)ethoxy]methyl}-7,8-dihydroindeno[4,5-d][1,2,3]triazol-6(3H)-one and 7-ethyl-7-(3-oxobutyl)-1-{[2-(trimethylsilyl)ethoxy]methyl}-7,8-dihydroindeno[4,5-d][1,2,3]triazol-6(1H)-one

A 58:42 mixture of 7-ethyl-3-{[2-(trimethylsilyl)ethoxy]methyl}-7,8-dihydroindeno[4,5-*d*][1,2,3]triazol-6(3*H*)-one and 7-ethyl-1-{[2-(trimethylsilyl)ethoxy]methyl}-7,8-dihydroindeno[4,5-*d*][1,2,3]triazol-6(1*H*)-one (464 mg, 1.40 mmol) was dissolved in anhydrous tetrahydrofuran (2.8 mL). The solution was placed under a nitrogen atmosphere and treated with 1,8-diazabicyclo[5.4.0]undec-7-ene (0.042 mL, 0.28 mmol) and methyl vinyl ketone (0.230 mL, 2,8 mmol). The resulting solution was stirred and heated in an oil bath at 55°C for 135 minutes. After cooling, the mixture was partitioned between EtOAc (250 mL) and aqueous 1N HCl (200 mL), and the aqueous phase was back-extracted with EtOAc (50 mL). The combined organics were washed with aqueous 5% NaHCO₃ (200 mL), water (200 mL), and brine (200 mL), dried over MgSO₄, filtered, and concentrated under vacuum to provide a 58:42 mixture (543 mg, 97% yield) of 7-ethyl-7-(3-oxobutyl)-3-{[2-(trimethylsilyl)ethoxy]methyl}-7,8-dihydroindeno[4,5-*d*][1,2,3]triazol-6(3*H*)-one and 7-ethyl-7-(3-oxobutyl)-1-{[2-(trimethylsilyl)ethoxy]methyl}-7,8-dihydroindeno[4,5-*d*][1,2,3]triazol-6(1*H*)-one as an oil.

### Step 3: 9a-ethyl-3-{[2-trimethylsilyl)ethoxy]methyl}-8,9,9a,10-tetrahydrofluoreno[1,2-d][1,2,3]triazol-7(3H)-one and 9a-ethyl-1-{[2-trimethylsilyl)ethoxy]methyl}-8,9,9a,10-tetrahydrofluoreno[1,2-d][1,2,3]triazol-7(1H)-one

A 58:42 mixture (543 mg, 1.35 mmol) of 7-ethyl-7-(3-oxobutyl)-3-{[2-(trimethylsilyl)ethoxy]methyl}-7,8-dihydroindeno[4,5-*d*][1,2,3]triazol-6(3*H*)-one and 7-ethyl-7-(3-oxobutyl)-1-{[2-(trimethylsilyl)ethoxy]methyl}-7,8-dihydroindeno[4,5-*d*][1,2,3]triazol(1*H*)-one was dissolved in anhydrous toluene (14 mL). The solution was stirred under a nitrogen atmosphere while pyrrolidine (0.120 mL, 1.44 mmol) and acetic acid (0.080 mL, 1.40 mmol) were added by syringe. The resulting solution was warmed to 90°C over 20 minutes, then heated at 90°C for 4 hours. The reaction mixture was diluted with EtOAc (250 mL) and washed with water (250 mL). The aqueous phase was back-extracted with EtOAc (50 mL) and the combined organics were washed with brine, dried over MgSO₄, filtered, and concentrated under vacuum to an oil (510 mg). The crude product was purified by chromatography on a Biotage Flash 40S KP-Sil column, eluting with 4:1 hexanes-EtOAc (1000 mL) followed by 2:1 hexanes-EtOAc (300 mL). The product containing fractions were combined and evaporated under vacuum to afford a 58:42 mixture (406 mg, 78% yield) of 9a-ethyl-3-{[2-trimethylsilyl)ethoxy]methyl}-8,9,9a,10-tetrahydrofluoreno[1,2-*d*] [1,2,3]triazol-7(3*H*)-one and 9a-ethyl-1-{[2-trimethylsilyl)ethoxy]methyl}-8,9,9a,10-tetrahydrofluoreno[1,2-*d*][1,2,3]triazol-7(1*H*)-one as an oil.

### Step 4: 6-bromo-9a-ethyl-3-{[2-trimethylsilyl)ethoxy]methyl}-8,9,9a,10-tetrahydrofluoreno[1,2-d][1,2,3]triazol-7(3H)-one and 6-bromo-9a-ethyl-1-{[2-trimethylsilyl)ethoxy]methyl}-8,9,9a,10-tetrahydrofluoreno[1,2-d][1,2,3]triazol-7(1H)-one

A 58:42 mixture (406 mg, 1.06 mmol) of 9a-ethyl-3-{[2-trimethylsilyl)ethoxy]methyl}-8,9,9a,10-tetrahydrofluoreno[1,2-*d*][1,2,3]triazol-7(3*H*)-one and 9a-ethyl-1-{[2-trimethylsilyl)ethoxy]methyl}-8,9,9a,10-tetrahydrofluoreno[1,2-*d*][1,2,3]triazol-7(1*H*)-one was dissolved in carbon tetrachloride (3.1 mL) and the solution was treated with solid NaHCO₃ (445 mg, 5.3 mmol). The mixture was cooled in an ice bath, placed under a nitrogen atmosphere, and stirred while bromine (0.055 mL, 1,06 mmol) was added by syringe. After stirring for 10 minutes at 0°C, the mixture was sonicated for approximately one minute to break up a gummy orange deposit. The mixture was returned to the cooling bath and stirred an additional 45 minutes at 0°C. More bromine (0.020 mL, 0.39 mmol) was added and the mixture was stirred at 0°C for an additional 15 minutes. The mixture was then partitioned between CH₂Cl₂ (200 mL) and water (100 mL). The organic phase was washed with dilute aqueous Na₂S₂O₃ and brine, dried over MgSO₄, filtered, and concentrated under vacuum to a yellow oil. This material was purified by chromatography on a Biotage Flash 40S KP-Sil column, eluting with 4:1 hexanes-EtOAc (1000 mL) followed by 2:1 hexanes-EtOAc (300 mL) and collecting two 100 mL fractions followed by 25 mL fractions. Fractions 7-19 were combined and evaporated under vacuum to afford a 77:23 mixture (322 mg, 65% yield) of 6-bromo-9a-ethyl-3-{[2-trimethylsilyl)ethoxy]methyl}-8,9,9a,10-tetrahydrofluoreno[1,2-*d*][1,2,3]triazol-7(3*H*)-one and 6-bromo-9a-ethyl-1-{[2-trimethylsilyl)ethoxy]methyl}-8,9,9a,10-tetrahydrofluoreno[1,2-*d*][1,2,3]triazol-7(1*H*)-one as an oil. Fractions 32-38 afforded recovered starting material (85 mg, 22%) as a mixture of isomers.

### Step 5: 6-allyl-9a-ethyl-3-{[2-trimethylsilyl)ethoxy]methyl}-8,9,9a,10-tetrahydrofluoreno[1,2-d][1,2,3]triazol-7(3H)-one and 6-allyl-9a-ethyl-1-{[2-trimethylsilyl)ethoxy]methyl}-8,9,9a,10-tetrahydrofluoreno[1,2-d][1,2,3]triazol-7(1H)-one

A 77:23 mixture (93 mg, 0.20 mmol) of 6-bromo-9a-ethyl-3-{[2-trimethylsilyl)ethoxy]methyl}-8,9,9a,10-tetrahydrofluoreno[1,2-*d*][1,2,3]triazol-7(3*H*)-one and 6-bromo-9a-ethyl-1-{[2-trimethylsilyl)ethoxy]methyl}-8,9,9a,10-tetrahydrofluoreno[1,2-*d*][1,2,3]triazol-7(1*H*)-one and dichloro-bis(triphenylphosphine)palladium(II) (28 mg, 0.04 mmol) were dissolved in anhydrous toluene (2 mL). The solution was purged with nitrogen, treated with allyltributyltin (0.130 mL, 0.40 mmol), gradually heated to 100°C over 20 minutes, and then stirred and heated at 100°C for 18 hours. After cooling to room temperature, the mixture was concentrate under vacuum. The residue was dissolved in EtOAc (50 mL) and the solution filtered through a pad of silica gel (7.5 mL). Evaporation of the filtrate left an oil which was purified by chromatography on a Biotage Flash 12M KP-Sil column, using 4:1 hexanes-EtOAc as eluting solvent. The product containing fractions were combined and evaporated under vacuum to provide a 4:1 mixture (87 mg) of 6-allyl-9a-ethyl-3-{[2-trimethylsilyl)ethoxy]methyl}-8,9,9a,10-tetrahydrofluoreno[1,2-*d*][1,2,3]triazol-7(3*H*)-one and 6-allyl-9a-ethyl-1-{[2-trimethylsilyl)ethoxy]methyl}-8,9,9a,10-tetrahydrofluoreno[1,2-*d*][1,2,3]triazol-7(1*H*)-one. The product was contaminated with minor amounts of triphenylphosphine oxide.

### Step 6: 6-allyl-9a-ethyl-8,9,9a,10-tetrahydrofluoreno[1,2-d]triazol-7(3H)-one

A 4:1 mixture (36 mg, 0.085 mmol)) of 6-allyl-9a-ethyl-3-{[2-trimethylsilyl)ethoxy]methyl}-8,9,9a,10-tetrahydrofluoreno[1,2-*d*][1,2,3]triazol-7(3*H*)-one and 6-allyl-9a-ethyl-1-{[2-trimethylsilyl)ethoxy]methyl}-8,9,9a,10-tetrahydrofluoreno[1,2-*d*][1,2,3]triazol-7(1*H*)-one was dissolved in anhydrous tetrahydrofuran (0.50 mL) and the solution was treated with tetrabutylammonium fluoride (0.10 mL of a 1.0M solution in tetrahydrofuran, 0.1 mmol). The resulting solution was placed under a nitrogen atmosphere and stirred at room temperature for 1.5 hours followed by 16 hours at 50°C. After cooling, the reaction mixture was partitioned between EtOAc (50 mL) and aqueous 1N HCl (50 mL). The organic phase was washed with aqueous 5% NaHCO₃ (50 mL), water (50 mL), and brine (50 mL), dried over MgSO₄, filtered, and concentrated under vacuum to a yellow oil (28 mg). This material was purified by preparative HPLC using a YMC-Pack ODS column (100 x 20 mm i.d., S-5 µm, 120A) and 0.1% TFA in aqueous MeCN as eluting solvent. The product containing fractions were evaporated under vacuum. The residue was partitioned between EtOAc (50 mL) and aqueous 5% NaHCO₃ (50 mL) and the organic phase was washed with brine, dried over MgSO₄, filtered, and evaporated under vacuum to a white film (9 mg). This material was further purified by preparative layer chromatography on a 0.1 x 10 x 20 cm silica gel GF plate which was developed three times with 2:1 hexanes-EtOAc. The major UV visible band was eluted with EtOAc and the solvent evaporated under vacuum to afford 6-allyl-9a-ethyl-8,9,9a,10-tetrahydrofluoreno[1,2-*d*][1,2,3]triazol-7(3*H*)-one (4.7 mg, 25% yield) as an oil.
¹H NMR (CDCl₃, 500 MHz) δ 0.91 (t, CH₂C*H*_{*3*}), 1.61 and 1.77 (two m, C*H*_{*2*}CH₃), 2.15 and 2.41 (two m, 9-C*H*_{*2*}), 2.60 and 2.70 (two ddd, 8-C*H*_{*2*}), 3.10 and 3.59 (two d, 10-C*H*_{*2*}), 3.26 and 3.59 (two m, CH₂=CHC*H*_{*2*}), 5.07 (m, C*H*_{*2*}=CHCH₂), 6.04 (m, CH₂=C*H*CH₂), 7.75 (br s, *H*-4 or *H*-5), and 7.82 (d, *H*-5 or *H*-4); the broadened signals at δ 3.59 and 7.75 presumably reflect the presence of triazole tautomers.

### EXAMPLE 36

### SYNTHESIS OF 9a-ETHYL-6-PROPYL-8,9,9a,10-TETRAHYDROFLUORENO-[1,2-d][1,2,3]TRIAZOL-7(3H)-ONE

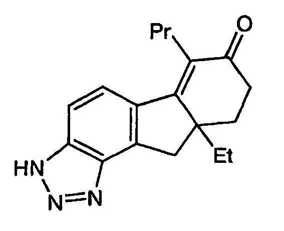

### Step 1: 9a-ethyl-6-propyl-3-{[2-trimethylsilyl)ethoxy]methyl}-8,9,9a,10-tetrahydrofluoreno[1,2-d]1,2,3]triazol-7(3H)-one and 9a-ethyl-6-propyl-1-{[2-trimethylsilyl)ethoxy]methyl}-8,9,9a,10-tetrahydrofluoreno[1,2-d][1,2,3]triazol-7(1H)-one

A 4:1 mixture (50 mg, 0.12 mmol) of 6-allyl-9a-ethyl-3-{[2-trimethylsilyl)ethoxy]methyl}-8,9,9a,10-tetrahydrofluoreno[1,2-*d*][1,2,3]triazol-7(3*H*)-one and 6-allyl-9a-ethyl-1-{[2-trimethylsilyl)ethoxy]methyl}-8,9,9a,10-tetrahydrofluoreno[1,2-*d*][1,2,3]triazol-7(1*H*)-one was dissolved in ethanol (1 mL). The solution was treated with 10% palladium on carbon (15 mg), placed under a hydrogen atmosphere, and stirred at room temperature for 22 hours. The mixture was filtered through a pad of silica gel (7.5 mL) and the silica gel washed with EtOAc (50 mL). The filtrate and washings were evaporated under vacuum to an oil (45 mg) which was purified by preparative layer chromatography on a 0.1 x 20 x 20 cm silica gel GF plate using 4:1 hexanes-EtOAc as developing solvent. The UV visible band at R_{f} 0.3-0.4 was extracted with EtOAc and the extracts concentrated under vacuum to afford a 4:1 mixture (29 mg, 57% yield) of 9a-ethyl-6-propyl-3-{[2-trimethylsilyl)ethoxy]methyl}-8,9,9a,10-tetrahydrofluoreno[1,2-*d*][1,2,3]triazol-7(3*H*)-one and 9a-ethyl-6-propyl-1-{[2-trimethylsilyl)ethoxy]methyl}-8,9,9a,10-tetrahydrofluoreno[1,2-*d*][1,2,3]triazol-7(1*H*)-one as a clear oil.

### Step 2: 9a-ethyl-6-propyl-8,9,9a,10-tetrahydrofluoreno[1,2-d][1,2,3]triazol-7(3H)-one

A 4:1 mixture (29 mg, 0.07 mmol)) of 9a-ethyl-6-propyl-3-{[2-trimethylsilyl)ethoxy]methyl}-8,9,9a,10-tetrahydrofluoreno[1,2-*d*][1,2,3]triazol-7(3*H*)-one and 9a-ethyl-6-propyl-1-{[2-trimethylsilyl)ethoxy]methyl}-8,9,9a,10-tetrahydrofluoreno[1,2-*d*][1,2,3]triazol-7(1*H*)-one was dissolved in methanol (0.50 mL) and the solution was diluted with aqueous 6N HCl (0.50 mL). The resulting milky suspension was placed under a nitrogen atmosphere and stirred with heating in an oil bath at 65°C for 50 minutes. After cooling, the reaction mixture was diluted with EtOAc (10 mL) and aqueous 5% NaHCO₃ (1 mL) and then partitioned between EtOAc (50 mL) and water (40 mL). The organic phase was washed with brine, dried over MgSO₄, filtered, and evaporated under vacuum to a clear oil (25 mg). NMR analysis of this material revealed a 1:1 mixture of starting materials and product.

A solution of the crude mixture in methanol (0.25 mL) and aqueous 6N HCl (0.25 mL) was stirred with heating in an oil bath at 80°C for 40 minutes. Workup as described above afforded a clear oil which was purified by preparative layer chromatography (0.1 x 20 x 20 cm silica gel GF plate developed with 2:1 hexanes-EtOAc). The UV visible band at R_{f} 0.10-0.26 was extracted with EtOAc and the extracts evaporated under vacuum to afford 9a-ethyl-6-propyl-8,9,9a,10-tetrahydrofluoreno[1,2-*d*][1,2,3]triazol-7(3*H*)-one (14 mg, 68% yield) as an oil.
¹H NMR (CD₃OD, 500 MHz) δ 0.86 and 1.02 (two t, CH₂C*H*_{*3*} and CH₂CH₂C*H*_{*3*}), 1.46, 1.56 and 1.70 (three m, C*H*_{*2*}CH₃ and CH₂C*H*_{*2*}CH₃), 2.11 and 2.39 (two m, 9-C*H*_{*2*}), 2.47 and 2.65 (two m, 8-C*H*_{*2*}), 2.57 and 2.67 (two m, C*H*_{*2*}CH₂CH₃), 3.04 and 3.46 (two d, 10-C*H*_{*2*}), and 7.04 (m, *H*-4 and *H*-5); mass spectrum m/z 296.2 (M+1).

### EXAMPLE 37

### SYNTHESIS OF 9a-ETHYL-6-TRIFLUOROMETHYL-8,9,9a,10-TETRAHYDROFLUORENO-[1,2-d][1,2,3]TRIAZOL-7(3H)-ONE

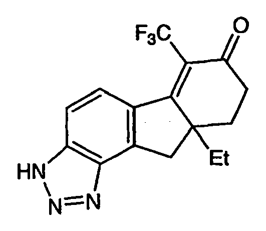

### Step 1: 9a-ethyl-6-trifluoromethyl-3-{[2-trimethylsilyl)ethoxy]methyl}-8,9,9a,10-tetrahydrofluoreno[1,2-d][1,2,3]triazol-7(3H)-one and 9a-ethyl-6-trifluoromethyl-1-{[2-trimethylsilyl)ethoxy]methyl}-8,9,9a,10-tetrahydrofluoreno[1,2-d][1,2,3]triazol-7(1H)-one

Copper(I) iodide (37 mg, 0.20 mmol) and a mixture (62.3 mg, 0.135 mmol) of 6-bromo-9a-ethyl-3-{[2-trimethylsilyl)ethoxy]methyl}-8,9,9a,10-tetrahydrofluoreno[1,2-*d*][1,2,3]triazol-7(3*H*)-one and 6-bromo-9a-ethyl-1-{[2-trimethylsilyl)ethoxy]methyl}-8,9,9a,10-tetrahydrofluoreno[1,2-*d*][1,2,3]triazol-7(1*H*)-one were dissolved in anhydrous *N*,*N*-dimethylformamide (3.2 mL). The solution was treated with methyl difluoro(fluorosulfonyl)acetate (MFSDA, 0.150 mL, 1.17 mmol), placed under a nitrogen atmosphere, stirred at room temperature for one hour, and then heated in an oil bath at 70°C for two hours. Additional MFSDA (0.150 mL, 1.17 mmol) was added and the mixture was heated at 70°C overnight (16.5 hours). After cooling, the mixture was diluted with EtOAc (60 mL), washed with water (3 x 50 ml) and brine, dried over MgSO₄, filtered, and concentrated under vacuum. The residue was purified by preparative layer chromatography on a 0.1 x 20 x 20 cm silica gel GF plate, developing wit 2:1 hexanes-EtOAc. The major UV visible band was eluted with EtOAc and the eluant evaporated under vacuum to provide a mixture (32 mg, 55%) of 9a-ethyl-6-trifluoromethyl-3-{[2-trimethylsilyl)ethoxy]methyl}-8,9,9a,10-tetrahydrofluoreno[1,2-*d*][1,2,3]triazol-7(3*H*)-one and 9a-ethyl-6-trifluoromethyl-1-{[2-trimethylsilyl)ethoxy]methyl}-8,9,9a,10-tetrahydrofluoreno[1,2-*d*][1,2,3]triazol-7(1*H*)-one as an off-white solid.

### Step 2: 9a-ethyl-6-trifluoromethyl-8,9,9a,10-tetrahydrofluoreno[1,2-d][1,2,3]triazol-7(3H)-one

The product mixture from step 1 (32 mg, 0.07 mmol) was dissolved in MeOH (~1 mL) and treated with aqueous 2N HCl (~1 mL). The resulting white suspension was stirred and heated in an oil bath at 80°C for 70 minutes. After cooling, the mixture was partitioned between EtOAc and aqueous 5% NaHCO₃. The organic phase was washed with brine, dried over MgSO₄, filtered, and concentrated under vacuum. The residue was purified by preparative layer chromatography (PLC, 0.1 x 20 x 20 cm silica gel GF plate), developing with 5% MeOH in CH₂Cl₂. Two UV visible bands were removed and eluted with 5% MeOH in CH₂Cl₂. The slower moving band afforded the deblocked product whereas the faster moving band returned starting material.

The recovered starting material was dissolved in MeOH (~1mL), treated with aqueous 6N HCl (~ 1mL), and the mixture was heated in an oil bath at 80°C for 75 minutes. Workup as above followed by PLC afforded only deblocked product. The two product samples were combined and lyophilized from benzene to afford 9a-ethyl-6-trifluoromethyl-8,9,9a,10-tetrahydrofluoreno[1,2-*d*][1,2,3]triazol-7(3*H*)-one (18 mg, 80%) as an amorphous solid.
¹H NMR (CD₃OD, 500 MHz) δ 0.86 (t, CH₂*CH*_{*3*}), 1.45 and 1.65 (two dq, C*H*_{*2*}CH₃), 2.24 and 2.40 (two ddd, 9-C*H*_{*2*}), 2.62 (m, 8-C*H*_{*2*}), 3.22 and 3.55 (two d, 10-C*H*_{*2*}), 3.30 (p, C*H*D₂OD), 7.78 (d, *H*-4), and 7.86 (qd, *H*-5); mass spectrum m/z 322.2 (M+1), 385.2 (M+Na+MeCN), and 665.2 (2M+1).

### EXAMPLE 38

### SYNTHESIS OF 6-BROMO-9a-ETHYL-8,9,9a,10-TETRAHYDROFLUORENO-[1,2-d][1,2,3]TRIAZOL-7(3H)-ONE

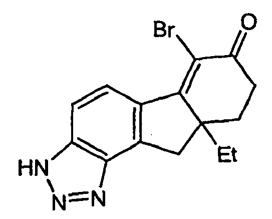

A crude mixture (55 mg, contains ca. 20 mole % of the 6-protio precursors) of 6-bromo-9a-ethyl-3-{[2-trimethylsilyl)ethoxy]methyl}-8,9,9a,10-tetrahydrofluoreno[1,2-*d*][1,2,3]triazol-7(3*H*)-one and 6-bromo-9a-ethyl-1-{[2-trimethylsilyl)ethoxy]methyl}-8,9,9a,10-tetrahydrofluoreno[1,2-*d*] [1,2,3]triazol-7(1*H*)-one was dissolved in methanol (0.5 mL) and the solution was diluted with aqueous 6N HCl (0.50 ml) to give a milky suspension. The mixture was stirred and heated in an oil bath at 80°C. After heating for 5 minutes, the mixture was sonicated for one minute to partially solidify an un-stirrable gummy precipitate. The mixture was then stirred and heated in an oil bath at 87°C for 55 minutes. After cooling, the mixture was diluted with EtOAc (10 mL) and aqueous 5% NaHCO₃ and then partitioned between EtOAc (50 ML) and water (40 mL). The aqueous phase was washed with brine, dried over MgSO₄, filtered, and evaporated under vacuum to an oil (44 mg). This material was purified by preparative layer chromatography on a 0.1 x 20 x 20 cm silica gel GF plate, developing with 2:1 hexanes-EtOAc. The UV visible band at R_{f} 0.13-0.20 was extracted with EtOAc and the extracts were evaporated under vacuum. The residue was lyophilized from benzene to afford 6-bromo-9a-ethyl-8,9,9a,10-tetrahydrofluoreno[1,2-*d*][1,2,3]triazol-7(3*H*)-one (21 mg) as an amorphous solid.
¹H NMR (CD₃OD, 500 MHz) δ 0.88 (t, CH₂C*H*_{*3*}), 1.62 and 1.79 (two m, C*H*_{*2*}CH₃), 2.22 and 2.42 (two ddd, 9-C*H*_{*2*}), 2.71 and 2.87 (two ddd, 8-C*H*_{*2*}), 3.15 and 3.55 (two d, 10-C*H*_{*2*}), 7.84 (d, *H*-4 or *H*-5), and 8.70 (d, *H*-5 or *H*-4); mass spectrum m/z 332.3 (M+1), 334.3 (M+3), 373.3 (M+1+MeCN), and 375.3 (M+3+MeCN).

### EXAMPLE 39

### SYNTHESIS OF 6,9a-DIETHYL-8,9,9a,10-TETRAHYDROFLUORENO-[1,2-d][1,2,3]TRIAZOL-7(3H)-ONE

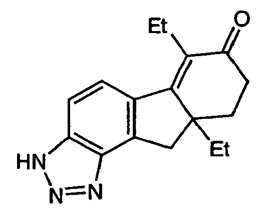

### Step 1: 7-ethyl-7-(3-oxohexyl)-7,8-dihydoindeno[4,5-d][1,2,3]triazol-6(3H)-one

A solution of 7-ethyl-7,8-dihydoindeno[4,5-*d*][1,2,3]triazol-6(3*H*)-one (100 mg, 0.5 mmol) in methanolic 0.5M sodium methoxide (1.2 mL, 0.6 mmol) was treated with propyl vinyl ketone (PVK, 90% pure, 82 mg, 0.75 mmol). The resulting solution was placed under a nitrogen atmosphere and stirred while heating in a 75°C oil bath for 20 hours. Additional 0.5M NaOMe in MeOH (0.25 mL, 0.125 mmol) and PVK (approx. 30 mg, 0.28 mmol) were added and the mixture was stirred at 70°C for an additional 19 hours. The mixture was partitioned between EtOAc (60 mL) and water (60 mL) that had been adjusted to pH 4 with 1N HCl. The organic phase was washed with brine, dried over MgSO₄, filtered, and evaporated under vacuum to provide crude 7-ethyl-7-(3-oxohexyl)-7,8-dihydoindeno[4,5-*d*][1,2,3]triazol-6(3*H*)-one (183 mg) as an oil. This material was used in the next step without purification.

### Step 2: 6,9a-diethyl-8,9,9a,10-tetrahydrofluoreno[1,2-d][1,2,3]triazol-7(3H)-one

A solution of crude 7-ethyl-7-(3-oxohexyl)-7,8-dihydoindeno[4,5-*d*][1,2,3]triazol-6(3*H*)-one (183 mg, approx. 0.5 mmol) in acetic acid (2 mL) was diluted with aqueous 6N HCl (2 mL) and the resulting mixture was placed under a nitrogen atmosphere and stirred while heating in an oil bath at 100°C for 3 hours. The mixture was partitioned between EtOAc (50 mL) and aqueous 5% NaHCO₃ (50 mL). The organic phase was washed with brine, dried over MgSO₄, filtered, and evaporated under vacuum to a red oil. This material was purified by preparative HPLC on a YMC-Pack ODS column (100 x 20 mm i.d., S-5 µm, 120A) using a solvent gradient of 90:10 A:B to 100% B where A is 0.1% TFA in H₂O and B is 0.1% TFA in MeCN. The product containing fractions were concentrated under vacuum to a residue that was taken up in EtOAc (50 mL) The solution was washed with water (50 mL) containing 5% NaHCO₃ (approx. 1 mL), washed with brine, dried over MgSO₄, filtered, and evaporated under vacuum to an orange film (55 mg). NMR analysis of this material showed product and starting material. Further purification of this material by preparative layer chromatography (0.1 x 20 x 20 cm silica gel GF plate developed with 5% MeOH in CH₂Cl₂) gave a major UV visible band that provided product (40 mg) still contaminated with starting material.

The crude mixture from above (36 mg) was dissolved in acetic acid (0.50 mL) and aqueous 6N HCl (0.50 mL) and the solution was stirred and heated in an oil bath at 100°C for 1.5 hours. Workup as described below afforded an oil which was shown by NMR to still contain about 5% starting material. The reaction was repeated with 0.50 mL each of HOAc and 6N HCl, but this time the mixture was heated at 100°C for 3 hours and then stirred at room temperature overnight. The reaction mixture was partitioned between EtOAc (60 mL) and aqueous 5% NaHCO₃ (60 mL). The organic phase was washed with brine, dried over MgSO₄, filtered, and concentrated under vacuum to an oil (35 mg). The crude product was purified by preparative layer chromatography on a 0.1 x 20 x 20 cm silica gel GF plate which was developed with 5% MeOH in CH₂Cl₂. The UV visible band at R_{f} 0.23-0.34 was extracted with 5% MeOH in CH₂Cl₂ and the extracts evaporated under vacuum to afford a clear oil. The oil was lyophilized from benzene (2 mL) to provide 6,9a-diethyl-8,9,9a,10-tetrahydrofluoreno[1,2-*d*][1,2,3]triazol-7(3*H*)-one (28 mg) as an amorphous white solid.
¹H NMR (CDCl₃, 500 MHz) δ 0:89 (t, 9a-CH₂C*H*_{*3*}), 1.18 (t, 6-CH₂C*H*_{*3*}), 1.56 and 1.70 (two m, 9a-C*H*_{*2*}CH₃), 2.13 and 2.37 (two ddd, 9-C*H*_{*2*}), 2.56 and 2.65 (two m, 8-C*H*_{*2*}), 2.64 and 2.77 (two m, 6-C*H*_{*2*}CH₃), 3.08 and 3.55 (two d, 10-C*H*_{*2*}), 7.80 (br s, *H*-4 or *H*-5), and 7.86 (d, *H*-5 or *H*-4); the broadened signals at δ 3.55 and 7.80 presumably reflect the presence of triazole tautomers; mass spectrum m/z 282.2 (M+1).

### EXAMPLE 40

### SYNTHESIS OF 9a-BUTYL-6-ETHYL-4-FLUORO-8,9,9a,10-TETRAHYDROFLUORENO[1,2-d][1,2,3]TRIAZOL-7(3H)-ONE

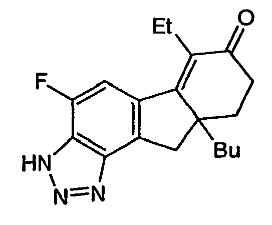

A solution of 7,8-diamino-9a-butyl-4-ethyl-6-fluoro-1,2,9,9a-tetrahydro-3*H*-fluoren-3-one (30 mg, 0.095 mmol) in EtOH (1.25 mL) was treated with water (0.027 mL, 1.8 mmol). The resulting solution was purged with N₂, cooled in an ice bath, stirred, and treated with conc. HCl (0.095 mL, 1.14 mmol) followed by aqueous 3M NaNO₂ (0.126 mL, 0.38 mmol) added dropwise over two minutes. After stirring at 0-5°C for one hour, the solution was partitioned between EtOAc (30 mL) and water (30 mL). The organic phase was washed with brine, dried over MgSO₄, filtered, and concentrated under vacuum. The residue (34 mg) was purified by preparative layer chromatography on a 0.1 x 20 x 20 cm silica gel GF plate, developing with 5% MeOH in CH₂Cl₂. The UV visible product band was eluted with 5% MeOH in CH₂Cl₂, the eluant concentrated under vacuum, and the residue lyophilized from benzene plus MeOH to afford 9a-butyl-6-ethyl-4-fluoro-8,9,9a,10-tetrahydrofluoreno[1,2-*d*][1,2,3]triazol-7(3*H*)-one (29 mg, 94%) as an amorphous solid.
¹H NMR (CDCl₃, 500 MHz) δ 0.82 (t, CH₂CH₂CH₂C*H*_{*3*}), 1.18 (t, 6-CH₂C*H*_{*3*}), 1.16-1.31 (m, CH₂C*H*_{*2*}C*H*_{*2*}CH₃), 1.46 and 1.65 (two m, C*H*_{*2*}CH₂CH₂CH₃), 2.10 and 2.35 (two ddd, 9-C*H*_{*2*}), 2.58 and 2.67 (two ddd, 8-C*H*_{*2*}), 2.58 and 2.74 (two qd, 6-C*H*_{*2*}CH₃), 3.03 and 3.48 (two d, 10-C*H*_{*2*}), and 7.51 (d, *H*-5); mass spectrum m/z 328.2 (M+1), 391.2 (M+Na+MeCN), and 677.3 (2M+Na).

### EXAMPLE 41

### SYNTHESIS OF (9aS)-9a-BUTYL-6-ETHYL-8,9,9a,10-TETRAHYDROFLUORENO[1,2-d][1,2,3]TRIAZOL-7(3H)-ONE AND (9aR)-9a-BUTYL-6-ETHYL-8,9,9a,10-TETRAHYDROFLUORENO[1,2-d][1,2,3]TRIAZOL-7(3H)-ONE

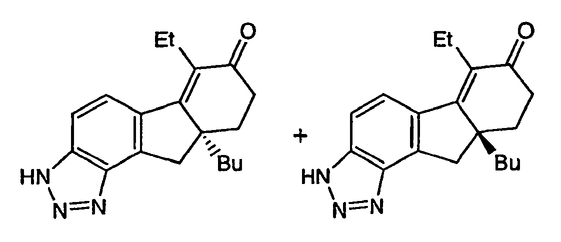

### Step 1: 5-(acetylamino)-2-butyl-1-indanone

A suspension of 5-(acetylamino)-1-indanone (1.892 g, 10 mmol) in EtOH (40 mL) was treated with butyraldehyde (1.35 mL, 15 mmol) and powdered KOH (87.7%, 128 mg, 2 mmol). The mixture was stirred at room temperature for 10 minutes to give a yellow solution. The solution was treated with 10% palladium on carbon (190 mg), placed under a hydrogen atmosphere, and stirred at room temperature for 3.5 hours. The mixture was treated with more butyraldehyde (0.18 mL, 2 mmol) and stirred under hydrogen for an additional hour. The mixture was filtered through a celite pad to remove the catalyst which was washed with EtOH. The filtrate and washings were evaporated under vacuum and the residue stripped with EtOH (2 x 50 mL). The oily residue ws partitioned between EtOAc (50 mL) and brine (40 mL) containing 2N HCl (10 mL). The organic phase was dried over MgSO₄, filtered, and the filtrate evaporated under vacuum to afford a pale yellow solid (2.405 g). The crude product was purified by chromatography on a Biotage Flash 40M KP-Sil column (4.0 x 15 cm), loading the sample as a CH₂Cl₂ solution and eluting the column with 1:1 EtOAc-hexanes (25 mL fractions). Fractions 25-45 were combined and evaporated under vacuum to afford 5-(acetylamino)-2-butyl-1-indanone (1.826 g, 74%) as an off-white solid.

### Step 2: 5-(acetylamino)-2-butyl-(3-oxohexyl)-1-indanone and 5-amino-2-butyl-(3-oxohexyl)-1-indanone

A solution of 5-(acetylamino)-2-butyl-1-indanone (1.805 g, 7.36 mmol) in anhydrous MeOH (14.7 mL) was treated with propyl vinyl ketone (1.25 mL, 11.09 mmol) and 0.5M NaOMe in MeOH (2.94 mL, 1.47 mmol). The resulting mixture was stirred in a capped flask and heated in an oil bath at 60°C for 7 hours. After cooling to room temperature, the mixture was treated with HOAc (0.090 mL, 1.57 mmol) and concentrated under vacuum. The residue was partitioned between EtOAc (100 mL) an water (50 mL). The organic portion was washed with brine, dried over MgSO₄, filtered, and evaporated under vacuum to give an amber oil (2.775 g). NMR showed a 78:22 mixture of 5-(acetylamino)-2-butyl-(3-oxohexyl)-1-indanone and 5-amino-2-butyl-(3-oxohexyl)-1-indanone accompanied by several minor contaminants.

### Step 3: 7-amino-9a-butyl-4-ethyl-1,2,9,9a-tetrahydro-3H-fluoren-3-one

The crude diketone mixture from step 2 (2.77 g, ca. 7.3 mmol) was taken up in HOAc (31.5 mL) and diluted with 6N HCl (31.5 mL). The resulting solution was stirred in a capped flask and heated in an oil bath at 100°C for 2 hours. After cooling to room temperature, the mixture was diluted with EtOAc (200 mL) and water (200 mL) and stirred rapidly while K2CO₃ (60 g) was added portionwise. The phases were separated and the aqueous portion extracted with more EtOAc (2 x 50 mL). The combined EtOAc solution was washed with brine (50 ml), dried over MgSO₄, filtered, and evaporated under vacuum to provide a brown oil (2.23 g). The crude product was purified by chromatography on a Biotage Flash 40M column (4.0 x 15 cm, KP-Sil), loading the sample as a CH₂Cl₂ solution and eluting the column with 3:1 hexanes-EtOAc (25 mL fractions). Fractions 23-34 were combined and evaporated under vacuum to afford 7-amino-9a-butyl-4-ethyl-1,2,9,9a-tetrahydro-3*H-*fluoren-3-one (1.516 g) as a pale brown solid.

### Step 4: 7-amino-6-bromo-9a-butyl-4-ethyl-1,2,9,9a-tetrahydro-3H-fluoren-3-one

A solution of 7-amino-9a-butyl-4-ethyl-1,2,9,9a-tetrahydro-3*H* fluoren-3-one (1.510 g, ca. 97% pure, 5.33 mmol) in anhydrous *N,N* dimethylformamide (DMF, 22 mL) was placed under a nitrogen atmosphere, stirred, and cooled in a dry ice-iPrOH bath maintained at -25 to -30°C. A solution of *N-*bromosuccinimide (0.977 g, 5.49 mmol) in DMF was added dropwise over 8 minutes. The resulting mixture was stirred at -25 to -30°C for 3 hours, with a few brief excursions to -20°C. The DMF solvent was evaporated under vacuum. The residue in EtOAc (200 ml) was washed with 10% aqueous K₂CO₃ (2 x 100 mL) and brine (100 mL), dried over MgSO₄, filtered, and evaporated under vacuum to give a yellow-brown foam (1.958 g). NMR analysis revealed an 84:9:7 mixture of the 6-bromo, 8-bromo, and 6,8-dibromo products. The mixture was crystallized from hot EtOAc-hexanes. The crystals were collected, washed with hexanes, and dried under vacuum to provide 7-amino-6-bromo-9a-butyl-4-ethyl-1,2,9,9a-tetrahydro-3*H*-fluoren-3-one (1.158 g, 60%) as a pale gold-brown solid.

### Step 5: 7-amino-6-bromo-9a-butyl-4-ethyl-8-nitro-1,2,9,9a-tetrahydro-3H-fluoren-3-one

A solution of 7-amino-6-bromo-9a-butyl-4-ethyl-1,2,9,9a-tetrahydro-3*H*-fluoren-3-one (725 mg, 2 mmol) in trifluoroacetic acid (TFA, 4 ml) was treated with 2,3,5,6-tetrabromo-4-methyl-4-nitro-2,5-cyclohexadien-1-one (937 mg, 2 mmol). The resulting suspension was sonicated briefly and then stirred at room temperature. The mixture was periodically sonicated in order to remove solids from the side of the flask. After 3 hours, the mixture was cooled in an ice bath and filtered to remove the solids, which were washed with cold TFA and dried under vacuum. The dried powder (820 mg) was identified as 2,3,5,6-tetrabromo-4-methyl-phenol by NMR spectroscopy. The TFA filtrate was diluted with CH₂Cl₂ (50 mL) and carefully treated with 10% aqueous K₂CO₃ (100 mL). The layers were separated and the aqueous portion was extracted with more CH₂Cl₂ (2 x 25 mL). The combined CH₂Cl₂ solution was washed with 5% NaHCO₃ (25 ml) and brine (25 ml), dried over MgSO₄, filtered, and evaporated under vacuum to a dark amber foam. The crude product was purified by chromatography on a Biotage 40S column (4.0 x 7.0 cm, KP-Sil), loading the sample as a CH₂Cl₂ solution and eluting the product with 9:1 hexanes-EtOAc (20 mL fractions). Fractions 15-28 were combined and evaporated under vacuum to afford 7-amino-6-bromo-9a-butyl-4-ethyl-8-nitro-1,2,9,9a-tetrahydro-3*H*-fluoren-3-one (493 mg, 61%) as a red-orange solid.

### Step 6: 7,8-diamino-9a-butyl-4-ethyl-1,2,9,9a-tetrahydro-3H-fluoren-3-one

A solution of 7-amino-6-bromo-9a-butyl-4-ethyl-8-nitro-1,2,9,9a-tetrahydro-3*H*-fluoren-3-one (490 mg, 1.20 mmol) in EtOH (24 mL) and EtOAc (24 mL) was treated with KOAc (177 mg, 1.80 mmol) and 10% palladium on carbon (70 mg). The mixture was stirred under a balloon of hydrogen for 135 minutes. The mixture was filtered through a celite pad to remove the catalyst which was washed with EtOAc. The filtrate and washings were evaporated under vacuum. The residue in EtOAc (50 mL) was washed with 5% aqueous NaHCO₃ (25 ml) and brine (25 mL), dried over MgSO₄, filtered, and evaporated under vacuum to afford 7,8-diamino-9a-butyl-4-ethyl-1,2,9,9a-tetrahydro-3*H*-fluoren-3-one (344 mg, 96%) as an orange solid.

### Step 7: (9aS)-7,8-diamino-9a-bulyl-4-ethyl-1.2,9,9a-tetrahydro-3H-fluoren-3-one and (9aR)-7,8-diamino-9a-butyl-4-ethyl-1,2,9,9a-tetrahydro-3H-fluoren-3-one

The racemic 7,8-diamino-9a-butyl-4-ethyl-1,2,9,9a-tetrahydro-3*H-*fluoren-3-one from step 6 was resolved into its individual enantiomers by chiral HPLC on a Chiracel OJ column (2.5 x 37.5 cm). The column was eluted with 1:3 EtOH-heptane at a flow rate of 10 mL/min. Fifteen 1 mL sample injections of a 20 mg/mL solution in EtOH were made. The enantiomers were detected by UV at 280 and 370 nm. Enantiomers A and B had retention times of 15.9 and 18.7 minutes, respectively. All enantiomer A containing fractions were combined and evaporated under vacuum to an orange solid (102 mg, 100% pure by analytical chiral HPLC). Similarly, all enantiomer B containing fractions were combined and evaporated under vacuum to provide an orange solid (106 mg, 97.5% pure by analytical, chiral HPLC),

### Step 8: (9aS)-9a-butyl-6-ethyl-8,9,9a,10-tetrahydrofluoreno[1,2-d]1,2,3]triazol-7(3H)-one and (9aR)-9a-butyl-6-ethyl-8,9,9a,10-tetrahydrofluoreno[1,2-d][1,2,3]triazol-7(3H)-one

Enantiomer A of the diamine from step 7 (99 mg, 0.0332) was dissolved in EtOH (4.4) and the solution was stirred under a nitrogen atmosphere with ice bath cooling. Water (0.084 mL, 4.67 mmol) and conc. HCl (0.340 mL, 4.11 mmol) were added followed by the dropwise addition of aqueous 3M NaN02 (0.443 mL, 1.33 mmol) over 2 minutes. The resulting mixture was stirred at 0 to 5°C for 40 minutes, and then partitioned between EtOAc (40 mL) and water (25 mL). The aqueous phase was extracted with more EtOAc (10 mL). The combined organics were washed with brine (25 mL), dried over MgS04, filtered, and evaporated under vacuum. The residue was lypohilized from benzene (4 ml) to afford enantiomer A (100 mg, 97%) of the 9a-butyl-6-ethyl-8,9,9a,10-tetrahydrofluoreno[1,2-*d*][1,2,3]triazol-7(3*H*) product as a pale tan, amorphous solid.
¹H NMR (CDCl₃, 500 MHz) δ 0.82 (t, CH₂CH₂CH₂C*H*_{*3*}), 1.18 (t, 6-CH₂C*H*_{*3*}), 1.15-1.33 (m, CH₂C*H*_{*2*}C*H*_{*2*}CH₃), 1.45 and 1.65 (two dt, C*H*_{*2*}CH₂CH₂CH₃), 2.10 and 2.36 (two ddd, 9-C*H*_{*2*}), 2.55 and 2.65 (two ddd, 8-C*H*_{*2*}), 2.61 and 2.75 (two dq, 6-C*H*_{*2*}CH₃), 3.08 and 3.54 (two d, 10-C*H*_{*2*}), 7.77 (d, *H*-4 or *H*-5), and 7.85 (d, *H*-5 or *H*-4); mass spectrum m/z 310.3 (M+1).

Similarly, enantiomer B of the diamine from step 7 (103.4 mg, 0.347 mmol) was converted to enantiomer B (102 mg, 95%) of the 9a-butyl-6-ethyl-8,9,9a,10-tetrahydrofluoreno[1,2-*d*][1,2,3]triazol-7(3*H*) product.

| | |
|---|---|
| Enantiomer A: | [α]_{D} = - 389 ± 5° (c 0.104, CHCl₃, 20°C). |
| Enantiomer B: | [α]_{D} = + 402 ± 4° (c 0.123, CHCl₃, 20°C). |

### EXAMPLE 42

### SYNTHESIS OF 2-(2,2-DIMETHYLPROPANOYL)-9a-ETHYL-6-METHYL-8,9,9a,10-TETRAHYDROIDENO[2,1-e]INDAZOL-7(2H)-ONE AND 3-(2,2-DIMETHYLPROPANOYL)-9a-ETHYL-6-METHYL-8,9,9a,10-TETRAHYDROINDENO[2,1-e]INDAZOL-7(3H)-ONE

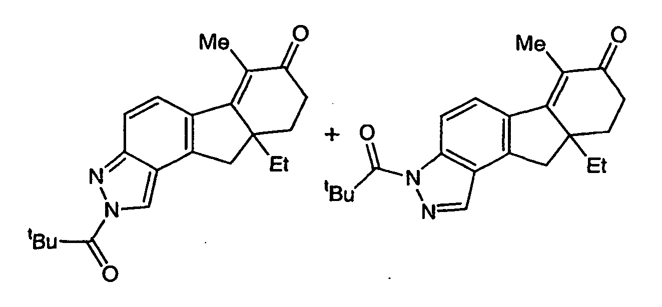

A mixture of 9a-ethyl-6-methyl-8,9,9a,10-tetrahydroindeno[2,1-*e*]indazol-7(3*H*)-one (30 mg, 0.113 mmol), 4-(dimethylamino)pyridine (21 mg, 0.169 mmol), trimethylacetyl chloride (0.017 mL, 0.138 mmol), and anhydrous dichloromethane (0.45 mL) was stirred at room temperature for 2 hours. The mixture was purified by preparative layer chromatography on a 0.1 x 20 x 20 cm silica gel GF plate, developing with 5% EtOAc in CH₂Cl₂. The product band was extracted with EtOAc, the extracts were evaporated under vacuum , and the residue was lyophilized from benzene (3 mL) to afford a 55:45 mixture (25.3 mg) of 2-(2,2-dimethylpropanoyl)-9a-ethyl-6-methyl-8,9,9a,10-tetrahydroindeno [2,1-*e*]indazol-7(2*H*)-one and 3-(2,2-dimethylpropanoyl)-9a-ethyl-6-methyl-8,9,9a,10-tetrahydroindeno[2,1-*e*]indazol-7(3*H*)-one as an amorphous white solid.
2-Pivaloyl isomer. ¹H NMR (CDCl₃, 500 MHz) δ 0.86 (t, CH₂C*H*_{*3*}), 1.53 and 1.67 (two m, C*H*_{*2*}CH₃), 1.63 (s, C(C*H*_{*3*})₃), 2.05 and 2.31 (two m, 9-C*H*_{*2*}), 2.16 (s, 6-C*H*_{*3*}), 2.51 and 2.61 (two m, 8-C*H*_{*2*}), 2.84 and 3.13 (two d, 10-C*H*_{*2*}), 7.60 (d, *H*-4 or *H*-5), 7.78 (d, *H*-5 or *H*-4), and 8.78 (s, *H*-1).
3-Pivaloyl isomer: ¹H NMR (CDCl₃, 500 MHz) δ 0.87 (t, CH₂C*H*_{*3*}), 1.53 and 1.67 (two m, C*H*_{*2*}CH₃), 1.58 (s, C(C*H*_{*3*})₃), 2.08 and 2.34 (two m, 9-C*H*_{*2*}), 2.17 (s, 6-CH₃), 2.53 and 2.62 (two m, 8-C*H*_{*2*}), 2.93 and 3,28 (two d, 10-C*H*_{*2*}), 7.91 (d, *H*-4 or *H*-5), 8.14 (s, *H-*1), and 8.44 (d, *H*-5 or *H*-4).

### EXAMPLE 43

### RESOLUTION OF RACEMIC 9a-ETHYL-6-METHYL-8,9,9a,10-TETRAHYDROINDENO[2,1-e]INDAZOL-7(3H)-ONE BY CHIRAL HPLC

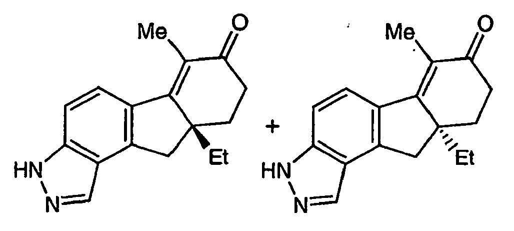

Racemic 9a-ethyl-6-methyl-8,9,9a,10-tetrahydroindeno[2,1-*e*]indazol-7(3*H*)-one (120 mg) was dissolved in ethanol to a final volume of 2.4 mL. The solution was injected in seven portions (0.20, 0.25, 0.30, 0.40, 0.40, 0.40, and 0.55 mL) onto a Daicel semi-prep ChiralCel OJ column (20 x 250 mm) which was eluted with 85:15 hexane-ethanol at a flow rate of 5 mL/minute. The enantiomers were detected by UV at 230 nm.. Each run was allowed to proceed until complete elution of the two enantiomers prior to the next injection. The pooled fractions from the faster eluting component were evaporated under vacuum and the oily residue was lyophilized from benzene to afford enantiomer A (44.1 mg) as an amorphous white solid. Similarly, the pooled fractions from the slower eluting component were evaporated under vacuum and the oily residue was lyophilized from benzene to afford enantiomer B (43.7 mg) as an amorphous white solid.

| | |
|---|---|
| Enantiomer A: | [α]_{D} = + 468° (c 0.515, CHCl₃). |
| Enantiomer B: | [α]_{D} = - 486° (c 0.487, CHCl₃). |

### EXAMPLE 44

### RESOLUTION OF RACEMIC 9a-ETHYL-6-TRIFLUOROMETHYL-8,9,9a,10-TETRAHYDROINDENO[2,1-e]INDAZOL-7(3H)-ONE BY CHIRAL HPLC

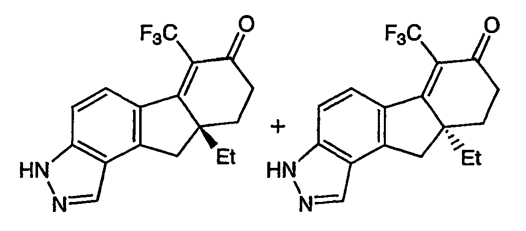

Racemic 9a-ethyl-6-trifluoromethyl-8,9,9a,10-tetrahydroindeno[2,1-*e*]indazol-7(3*H*)-one (190 mg) was dissolved in ethanol (19 mL). The solution was injected in six separate portions onto a Daicel ChiralCel OJ column (2.5 x 37.5 cm) column which was eluted with 85:15 heptane-ethanol at a flow rate of 7 mL/minute. The enantiomers were detected by UV at 220 and 335 nm.. The pooled fractions of the faster eluting component (ret. time approx. 25-32 minutes) from the six separate runs were evaporated under vacuum and residue was lyophilized from benzene to afford Enantiomer A (74.6 mg) as an amorphous white solid. Similarly, the pooled fractions from the slower eluting component (ret. time approx. 43-50 minutes) were evaporated under vacuum and the residue was lyophilized from benzene to afford Enantiomer B (60.5 mg) as an amorphous white solid.

Under analytical conditions (4.6 x 50 mm ChiralCel OJ 10 micron column, 85:15 heptane-EtOH, 1.5 mL/min., UV 220 nm), Enantiomers A and B had retention times of 1.28 and 1.84 minutes, respectively.

### EXAMPLE 45

### RESOLUTION OF RACEMIC 9a-BUTYL-6-TRIFLUOROMETIYL-8,9,9a,10-TETRAHYDROINDENO[2,1-e]INDAZOL-7(3H)-ONE BY CHIRAL HPLC

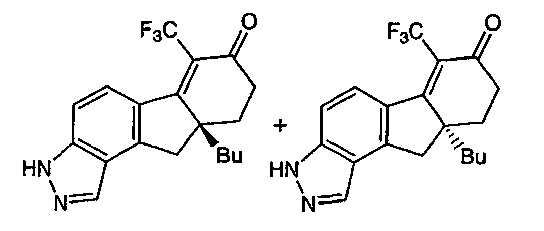

Racemic 9a-butyl-6-trifluoromethyl-8,9,9a,10-tetrahydroindeno[2,1-*e*]indazol-7(3*H*)-one (177 mg) was dissolved in ethanol. The solution was injected in six separate portions onto a Daicel ChiralCel OJ column (2.5 x 37.5 cm) column which was eluted with 90:10 heptane-ethanol at a flow rate of 7 mL/minute. The enantiomers were detected by UV at 220 and 335 nm.. The pooled fractions of the faster eluting component (ret. time approx. 25-36 minutes) from the six separate runs were evaporated under vacuum and residue was lyophilized from benzene to afford Enantiomer A (77 mg) as an amorphous white solid. Similarly, the pooled fractions from the slower eluting component (ret. time approx. 42-54 minutes) were evaporated under vacuum and the residue was lyophilized from benzene to afford Enantiomer B (76 mg) as an amorphous white solid.

Under analytical conditions (4.6 x 250 mm ChiralCel OJ 5 micron column, 90:10 heptane-EtOH, 0.75 mL/min., UV 220 nm), Enantiomers A and B had retention times of 13.04 and 18.38 minutes, respectively.

### EXAMPLE 46

### SYNTHESIS OF (RAC)-(8S,9aR)-9a-ETHYL-8-HYDROXY-6-METHYL-8,9,9a,10-TETRAHYDROINDENO[2,1-e]INDAZOL-7(3H)-ONE

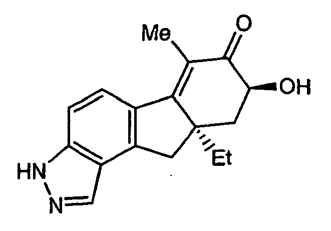

### Step 1: 9a-ethyl-6-methyl-2-{[2-(trimethylsilyl)ethoxy]methyl}-8,9,9a,10-tetrahydroindeno[2,1-e]indazol-7(2H)-one and 9a-ethyl-6-methyl-3-{[2-(trimethylsilyl)ethoxy]methyl}-8,9,9a,10-tetrahydroindeno[21-e]indazol-7(3H)-one

Sodium hydride (87 mg of a 61% dispersion in mineral oil, 2.2 mmol) is added to an ice-cold solution 9a-ethyl-6-methyl-8,9,9a,10-tetrahydroindeno[2,1-*e*]indazol-7(3*H*)-one (533 mg, 2.0 mmol) in anhydrous dimethylformamide (5 mL). The mixture is stirred under a nitrogen atmosphere and cooled in an ice bath while 2-(trimethylsilyl)ethoxymethyl chloride (SEM-Cl, 0.43 mL, 2.4 mmol) is added over one minute. The resulting mixture is stirred with gradual warming to room temperature. After 16.5 hours, the mixture is partitioned between EtOAc (100 mL) and water (100 mL). The organic phase is washed with water and brine, dried over MgSO₄, filtered, and concentrated under vacuum. The residue is purified by flash chromatography on EM silica gel 60, eluting with hexanes-EtOAc, to afford 9a-ethyl-6-methyl-2-{[2-(trimethylsilyl)ethoxy]methyl} -8,9,9a,10-tetrahydroindeno[2,1-*e*]indazol-7(2*H*)-one and 9a-ethyl-6-methyl-3-{ [2-(trimethylsilyl)ethoxy]methyl}-8,9,9a,10-tetrahydroindeno[2,1-*e*]indazol-7(3*H*)-one.

### Step 2: 9a-ethyl-6-methyl-3-{[2-(trimethylsilyl)ethoxy]methyl}-7-[(trimethylsilyl)oxy]-3,9,9a,10-tetrahydroindeno[2,1-e]indazole

A 0.4M solution of lithium diisopropylamide (LDA) in tetrahydrofuran (THF) is prepared by dissolving diisopropyl amine (0.56 mL, 4 mmol) in anhydrous THF (5 mL), cooling the solution to 0°C, adding either 1.6M (2.5 mL) or 2.5M (1.6 mL) butyllithium in hexanes, diluting the resulting solution to 10.0 mL total volume with anhydrous THF, and stirring the solution for at least 30 minutes at 0°C.

A solution of 9a-ethyl-6-methyl-3-{[2-(trimethylsilyl)ethoxy]methyl}-8,9,9a,10-tetrahydroindeno[2,1-*e*]indazol-7(3*H*)-one (79 mg, 0.20 mmol) in anhydrous THF (1 mL) is cooled in an ice bath and stirred under a nitrogen atmosphere while 0.4M LDA in THF (0.75 mL, 0.30 mmol) is added by syringe. After stirring at 0°C for 30 minutes, the solution is cooled to -78°C (dry ice-acetone bath) and treated with chlorotrimethylsilane (0.038 mL, 0.30 mmol). The resulting mixture is allowed to slowly warm to room temperature, and then stirred at room temperature overnight. The mixture is diluted with EtOAc (50 mL) and shaken with 5% aqueous NaHCO₃ (25 mL). The aqueous phase is separated and extracted with more EtOAc (20 mL). The combined organics are washed with saturated brine, dried over MgSO₄, filtered, and concentrated under vacuum to afford crude 9a-ethyl-6-methyl-3-{[2-(trimethylsilyl)ethoxy]methyl}-7-[(trimethylsilyl)oxy]-3,9,9a,10-tetrahydroindeno[2,1-*e*]indazole.

### Step 3: (rac)-(8S,9aR)-9a-ethyl-8-hydroxy-6-methyl-3-{[2-(trimethylsilyl)ethoxy]methyl}-8,9,9a,10-tetrahydroindeno[2,1-e]indazol-7(3H)-one

A solution of the crude trimethylsilylenolether from step 2 (approx. 0.2 mmol) in anhydrous dichloromethane (2 mL) is treated with sodium bicarbonate (25 mg, 0.3 mmol). The mixture is placed under a nitrogen atmosphere, stirred at room temperature, and treated over two minutes with three portions of 96% 3-chloroperoxybenzoic acid (3 x 15 mg, 0.25 mmol), purging with nitrogen after each addition. After stirring overnight at room temperature, the mixture is diluted with CH₂Cl₂ (2 mL), treated with saturated aqueous Na₂SO₃ (2 mL), and stirred at room temperature for 25 minutes. The mixture is partitioned between CH₂Cl₂ (20 mL) and water (5 mL), and the aqueous portion is back-extracted with more CH₂Cl₂ (5 mL). The combined organics are washed with water and brine, dried over MgSO₄, filtered, and concentrated under vacuum.. The residue is purified by preparative layer chromatography on a 0.1 x 20 x 20 cm silica gel GF plates using 4:1 hexanes-EtOAc as the developing solvent. The UV visible band product band is extracted with EtOAc and the extracts are evaporated under vacuum to afford (*rac*)-(8*S*,9a*R*)-9a-ethyl-8-hydroxy-6-methyl-3-{[2-(trimethylsilyl)ethoxy]methyl)-8,9,9a,10-tetrahydroindeno[2,1-*e*]indazol-7(3*H*)-one.

### Step 4: (rac)-(8S,9aR)-9a-ethyl-8-hydroxy-6-methyl-8,9,9a,10-tetrahydroindeno[2,1-e]indazol-7(3H)-one

A solution of (*rac*)-(8*S*,9a*R*)-9a-ethyl-8-hydroxy-6-methyl-3-{[2-(trimethylsilyl)ethoxy]methyl}-8,9,9a,10-tetrahydroindeno[2,1-*e*]indazol-7(3*H*)-one (58 mg, 0.14 mmol) in anhydrous tetrahydrofuran (1.2 mL) is treated with tetrabutylammonium fluoride (0.17 mL of a 1.0M solution in tetrahydrofuran, 0.17 mmol). The resulting solution is placed under a nitrogen atmosphere, stirred, and heated in an oil bath at 60°C for 16 hours. After cooling, the reaction mixture is partitioned between EtOAc (50 mL) and aqueous 1N HCl (50 mL). The organic phase is washed with aqueous 5% NaHCO₃ (50 mL), water (50 mL), and brine (50 mL), dried over MgSO₄, filtered, and concentrated under vacuum. The residue is purified by preparative layer chromatography on a 0.1 x 20 x 20 cm silica gel GF plates using 5% MeOH in CH₂Cl₂ as the developing solvent. The UV visible band product band is extracted with 10% MeOH in CH₂Cl₂ and the extracts are evaporated under vacuum to afford (*rac*)-(8*S*,9a*R*)-9a-ethyl-8-hydroxy-6-methyl-8,9,9a,10-tetrahydroindeno[2,1-*e*]indazol-7(3*H*)-one.

### EXAMPLE 47

### SYNTHESIS OF (RAC)-(8S,9aS)-9a-ETHYL-6,8-DIMETHYL-8,9,9a,10-TETRAHYDROINDENO[2,1-e]INDAZOL-7(3H)-ONE

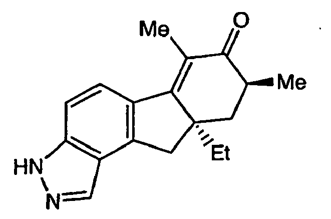

### Step 1: (rac)-(8S,9aS)-9a-ethyl-6.8-dimethyl-3-{[2-(trimethylsilyl)ethoxylmethyl}-8,9,9a,10-tetrahydroindeno[2,1-e]indazol-7(3H)-one

A solution of 9a-ethyl-3-{[2-(trimethylsilyl)ethoxy]methyl}-8,9,9a,10-tetrahydroindeno[2,1-*e*]indazol-7(3*H*)-one (79 mg, 0.2 mmol) in anhydrous tetrahydrofuran (1 mL) is cooled in an ice bath and stirred under a nitrogen atmosphere while 0.4M lithium diisopropylamide in tetrahydrofuran (0.6 mL, 0.24 mmol) is added by syringe. After stirring at 0°C for 30 minutes, the solution is cooled to -78°C (dry ice-acetone bath) and treated with iodomethane (0.062 mL, 1 mmol). The resulting mixture is allowed to slowly warm to room temperature, and then stirred at room temperature overnight. The mixture is diluted with EtOAc (25 mL) and shaken with saturated aqueous NH₄Cl (15 mL). The aqueous phase is extracted with more EtOAc (10 mL). The combined organics are washed with 5% NaHCO₃, water, and saturated brine, dried over MgSO₄, filtered, and concentrated under vacuum. The residue is purified by preparative layer chromatography on a 0.1 x 20 x 20 cm silica gel GF plate using 4:1 hexanes-EtOAc as developing solvent. The UV visible product band is extracted with EtOAc and the extracts are evaporated under vacuum to provide (*rac*)-(8*S*,9a*S*)-9a-ethyl-6,8-dimethyl-3-{[2-(trimethylsilyl)ethoxy]methyl}-8,9,9a,10-tetrahydroindeno[2,1-*e*]indazol-7(3*H*)-one.

### Step 2: (rac)-(8S,9aS)-9a-ethyl-6,8-dimethyl-8,9,9a,10-tetrahydroindeno[2,1-e]indazol-7(3H)-one

A mixture of (*rac*)-(8*S*,9a*S*)-9a-ethyl-6,8-dimethyl-3-{[2-(trimethylsilyl)ethoxy]methyl}-8,9,9a,10-tetrahydroindeno[2,1-*e*]indazol-7(3*H*)-one (30 mg, 0.073 mmol), methanol (0.5 mL) and aqueous 6N HCl (0.5 ml) is stirred and heated in an oil bath at 80°C for one hour. After cooling, the mixture is partitioned between EtOAc (20 mL) and aqueous 5% NaHCO₃ (5 mL). The organic phase is washed with brine, dried over MgSO₄, filtered, and evaporated under vacuum. The residue is purified by preparative layer chromatography on a 0.05 x 20 x 20 cm silica gel GF plate, developing with 5% MeOH in CH₂Cl₂. The UV visible product band is extracted with 10% MeOH in CH₂Cl₂ and the extracts are evaporated under vacuum to provide (*rac*)-(8*S*,9a*S*)-9a-ethyl-6,8-dimethyl-8,9,9a,10-tetrahydroindeno[2,1-*e*]indazol-7(3*H*)-one.

### EXAMPLE 48

### SYNTHESIS OF (RAC)-(8S,9aR)-9a-ETHYL-6,8-DIMETHYL-8-PROPYL-8,9,9a,10-TETRAHYDROINDENO[2,1-e]INDAZOL-7(3H)-ONE

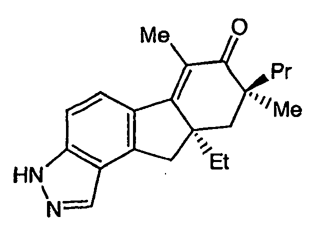

### Step 1: (rac)-(8S,9aR)-9a-ethyl-6,8-dimethyl-8-propyl-3-{[2-(trimethylsilyl)ethoxy]methyl}-8,9,9a,10-tetrahydroindeno[2,1-e]indazol-7(3H)-one

A solution of (*rac*)-(8*S*,9a*S*)-9a-ethyl-6,8-dimethyl-3-{[2-(trimethylsilyl)ethoxy]methyl}-8,9,9a,10-tetrahydroindeno[2,1-*e*]indazol-7(3*H*)-one (41 mg, 0.2 mmol) in anhydrous tetrahydrofuran (0.5 mL) is cooled in an ice bath and stirred under a nitrogen atmosphere while 0.4M lithium diisopropylamide in tetrahydrofuran (0.3 mL, 0.12 mmol) is added by syringe. After stirring at 0°C for 30 minutes, the solution is cooled to -78°C (dry ice-acetone bath) and treated with 1-iodopropane (0.049 mL, 0.5 mmol). The resulting mixture is allowed to slowly warm to room temperature, and then stirred at room temperature overnight. The mixture is diluted with EtOAc (10 mL) and shaken with saturated aqueous NH₄Cl (5 mL). The aqueous phase is extracted with more EtOAc (5 mL). The combined organics are washed with 5% NaHCO₃, water, and saturated brine, dried over MgSO₄, filtered, and concentrated under vacuum. The residue is purified by preparative layer chromatography on a 0.1 x 20 x 20 cm silica gel GF plate using 5:1 hexanes-EtOAc as developing solvent. The UV visible product band is extracted with EtOAc and the extracts are evaporated under vacuum to provide (*rac*)-(8*S*,9a*R*)-9a-ethyl-6,8-dimethyl-8-propyl-3-{[2-(trimethylsilyl)ethoxy]methyl}-8,9,9a,10-tetrahydroindeno[2,1-*e*]indazol-7(3*H*)-one

### Step 2: (rac)-(8S,9aR)-9a-ethyl-6,8-dimethyl-8-propyl-8,9.9a,10-tetrahydroindeno[2,1-e]indazol-7(3H)-one

A mixture of (*rac*)-(8*S*,9a*R*)-9a-ethyl-6,8-dimethyl-8-propyl-3-{[2-(trimethylsilyl)ethoxy]methyl}-8,9,9a,10-tetrahydroindeno[2,1-*e*]indazol-7(3*H*)-one (21 mg, 0.046 mmol), methanol (0.3 mL) and aqueous 6N HCl (0.3 ml) is stirred and heated in an oil bath at 80°C for one hour. After cooling, the mixture is partitioned between EtOAc (10 mL) and aqueous 5% NaHCO₃ (3 mL). The organic phase is washed with brine, dried over MgSO₄, filtered, and evaporated under vacuum. The residue is purified by preparative layer chromatography on a 0.05 x 20 x 20 cm silica gel GF plate, developing with 5% MeOH in CH₂Cl₂. The UV visible product band is extracted with 10% MeOH in CH₂Cl₂ and the extracts are evaporated under vacuum to provide (*rac*)-(8*S*,9a*R*)-9a-ethyl-6,8-dimethyl-8-propyl-8,9,9a,10-tetrahydroindeno[2,1-*e*]indazol-7(3*H*)-one.

### EXAMPLE 49

### SYNTHESIS OF (RAC)-(8R,10R,10aS)-6-ETHYL-10-PROPYL-3,9,10,11-TETRAHYDRO-8,10a-METHANOAZULENO[2,1-e]INDAZOL-7(8H)-ONE

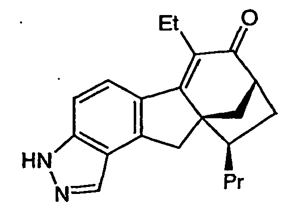

### Step 1: 9a-(2-acetoxyethyl)-7-amino-4-ethyl-8-methyl-1,2,9,9a-tetrahydro-3H-fluoren-3-one

A solution of 5-(acetylamino)-2-(2-hydroxyethyl)-4-methyl-1-indanone (7 g, 28.3 mmol) in MeOH (100 mL) was treated with NaOMe (0.5M solution in MeOH, 11.3 mL, 5.66 mmol) and propyl vinyl ketone (3.33 g, 34 mmol). The resulting solution was stirred at 60°C and under a nitrogen atmosphere for 8 hours. The mixture was diluted with CH₂Cl₂, filtered through a pad of silica gel, and the filtrate was evaporated under vacuum to provide crude 5-(acetylamino)-2-(2-hydroxyethyl)-4-methyl-2-(3-oxohexyl)-1-indanone.

The diketone from above was treated with acetic acid (50 mL) and 6N HCl (50 mL). The resulting solution was stirred and heated in an oil bath at 100°C for 1.5 hours. After cooling to room temperature, the mixture was diluted with CH₂Cl₂ and neutralized with NaHCO₃, filtered through a pad of silica gel and the product was washed off with 7:3 dichloromethane-methanol. The filtrate and washings were concentrated under vacuum. The residue was purified by flash chromatography on a Biotage 40M KP-Sil column, eluting with 3:1-1:1 hexane-ethyl acetate. The product containing fractions were evaporated under vacuum to provide 9a-(2-acetoxyethyl)-7-amino-4-ethyl-8-methyl-1,2,9,9a-tetrahydro-3*H*-fluoren-3-one (1 g) as a yellow solid.

### Step 2: 9a-(2-acetoxyethyl)-6-ethyl-8,9,9a,10-tetrahydroindeno[2,1-e]indazol-7(3H)-one

A solution of 9a-(2-acetoxyethyl)-7-amino-4-ethyl-8-methyl-1,2,9,9a-tetrahydro-3*H* fluoren-3-one (0.36 g, 1.1 mmol) in anhydrous CH₂Cl₂ (11 mL) was purged with N₂, cooled in an dry ice bath, and treated with NOBF₄ (0.129 g, 1.1 mmol). The mixture was warmed to -10°C over 40 minutes.

The diazonium salt from above in CH₂Cl₂ was cooled to -78°C and treated with KOAc (216 mg, 2.2 mmol) and dibenzo-18-crown-6 (19.8 mg, 0.055 mmol). The resulting solution was slowly warmed to room temperature over 40 min. The mixture was filtered through a pad of silica gel and the product was washed off with 9:1 dichloromethane-methanol. The filtrate was concentrated under vacuum to a residue that was purified by preparative layer chromatography (0.1 x 20 x 20 cm silica gel GF plate developed with 12:1 CH₂Cl₂-MeOH. The product band was eluted with 1:2 hexane-ethyl acetate and the eluant evaporated under vacuum to afford 9a-(2-acetoxyethyl)-6-ethyl-8,9,9a,10-tetrahydroindeno[2,1-*e*]indazol-7(3*H*)-one (223 mg) as an orange foam.

### Step 3: 6-ethyl-9a-(2-hydroxyethyl)-2-(methoxymethyl)-8,9,9a,10-tetrahydroindeno[2,1-e]indazol-7(3H)-one and 6-ethyl-9a-(2-hydroxyethyl)-3-(methoxymethyl)-8,9,9a,10-tetrahydroindeno[2,1-e]indazol-7(3H)-one

A solution of 9a-(2-acetoxyethyl)-6-ethyl-8,9,9a,10-tetrahydroindeno[2,1-*e*]indazol-7(3*H*)-one (0.041 g, 0.121 mmol) in anhydrous CH₂Cl₂ (1 mL) was purged with N₂ and treated with *N,N*-diisopropylethylamine (0.084 mL, 0.484 mmol) followed by chloromethyl methyl ether (0.027 mL, 0.363 mmol). After stirring for 5 minutes, the mixture was diluted MeOH (1 mL) and treated with NaOMe (1.5 mL of a 0.5M solution in MeOH, 0.75 mmol). After stirring 30 minutes, the mixture was further diluted with CH₂Cl₂ (20 mL), treated with sat. NH₄Cl (0.5 mL), dried over MgSO₄, filtered through a pad of silica, and the filtrate concentrated under vacuum. The residue was purified by preparative layer chromatography (0.1 x 20 x 20 cm silica gel GF plate developed with EtOAc). The product bands were eluted with EtOAc and the eluants evaporated under vacuum to provide 6-ethyl-9a-(2-hydroxyethyl)-2-(methoxymethyl)-8,9,9a,10-tetrahydroindeno[2,1-*e*]indazol-7(3*H*)-one and 6-ethyl-9a-(2-hydroxyethyl)-3-(methoxymethyl)-8,9,9a,10-tetrahydroindeno[2,1-*e*]indazol-7(3*H*)-one (two regioisomers, 5 and 16 mg each) as pale yellow foams.

### Step 4: 6-ethyl-3-(methoxymethyl)-9a-(2-oxoethyl)-8,9,9a,10-tetrahydroindeno[2,1-e]indazol-7(3H)-one

A solution of 6-ethyl-9a-(2-hydroxyethyl)-2-(methoxymethyl)-8,9,9a,10-tetrahydroindeno[2,1-*e*]indazol-7(3*H*)-one (0.012 g, 0.035 mmol) in anhydrous CH₂Cl₂ (1 mL) was purged with N₂ and treated successively with 4-methylmorpholine *N*-oxide (0.0062 mL, 0.053mmol), 3A molecular sieves, and tetrapropylammonium perruthenate (0.0012 mg, 0.0035 mmol). After stirring for one hour, the mixture was filtered through a pad of silica and the filtrate was concentrated under vacuum. The residue was purified by preparative layer chromatography (0.1 x 20 x 20 cm silica gel GF plate developed with 2:3 hexane- EtOAc). The product band was eluted with EtOAc and the eluant evaporated under vacuum to afford 6-ethyl-3-(methoxymethyl)-9a-(2-oxoethyl)-8,9,9a,10-tetrahydroindeno[2,1-*e*]indazol-7(3*H*)-one (8 mg) as an pale yellow foam.

### Step 5: 6-ethyl-9a-(1-formyl-3-butenyl)-3-(methoxymethyl)-8,9,9a,10-tetrahydroindeno[2,1-e]indazol-7(3H)-one

A solution of 6-ethyl-3-(methoxymethyl)-9a-(2-oxoethyl)-8,9,9a,10-tetrahydroindeno[2,1-*e*]indazol-7(3*H*)-one (0.200 g, 0.59 mmol) in anhydrous tetrahydrofuran (3 mL) at room temperature is purged with N₂ and treated with Pd(OAc)₂ (13.2 mg, 0.059mmol), PPh₃ (30 mg, 0.118 mmol), LiCl (30 mg, 0.71 mmol), allyl alcohol (0.048 mL, 0.71 mmol), Et₃B (1.0M solution in THF, 1.42 mL, 1.42 mmol), and Et₃N (0.099 mL, 0.71 mmol). After stirring for 48h, the mixture is filtered through a pad of Celite and the filtrate is concentrate under vacuum. The residue is purified by preparative layer chromatography (two 0.1 x 20 x 20 cm silica gel GF plates developed with 1:1 hexane- EtOAc). The product band is eluted with EtOAc and the eluant is evaporated under vacuum to provide 6-ethyl-9a-(1-formyl-3-butenyl)-3-(methoxymethyl)-8,9,9a,10-tetrahydroindeno[2,1-*e*]indazol-7(3*H*)-one.

### Step 6: 6-ethyl-9a-[1-(hydroxymethyl)-3-butenyl)-3-(methoxymethyl)-8.9,9a,10-tetrahydroindeno[2,1-e]indazol-7(3H)-one

A solution of 6-ethyl-9a-(1-formyl-3-butenyl)-3-(methoxymethyl)-8,9,9a,10-tetrahydroindeno[2,1-*e*]indazol-7(3*H*)-one (0.100 g, 0.264 mmol) in 2-propanol (2 mL) at -78°C is purged with N₂ and treated with NaBH₄ (10 mg, 0.264 mmol). After stirring for one hour, the mixture is treated with sat. NH₄Cl (0.5 mL), dried over MgSO₄, filtered through a pad of silica, and concentrated under vacuum. The residue is purified by preparative layer chromatography (0.1 x 20 x 20 cm silica gel GF plate developed with 1:3 hexane- EtOAc). The product band is eluted with EtOAc and the eluant evaporated under vacuum to provide 6-ethyl-9a-[1-(hydroxymethyl)-3-butenyl]-3-(methoxymethyl)-8,9,9a,10-tetrahydroindeno[2,1-*e*]indazol-7(3*H*)-one.

### Step 7: 6-ethyl-9a-{1-[(methylsulfonyloxy)methyl]-3-butenyl}-3-(methoxymethyl)-8,9,9a,10-tetrahydroindeno[2,1-e]indazol-7(3H)-one

A solution of 6-ethyl-9a-[1-(hydroxymethyl)-3-butenyl]-3-(methoxymethyl)-8,9,9a,10-tetrahydroindeno[2,1-*e*]indazol-7(3*H*)-one (0.080 g, 0.21 mmol) in anhydrous CH₂Cl₂ (1 mL) is purged with N₂ and treated with Et₃N (0.058 mL, 0.42 mmol) followed by methanesulfonyl chloride (0.024 mL, 0.315 mmol). After stirring for two hours, the mixture is purified by preparative layer chromatography (0.1 x 20 x 20 cm silica gel GF plate developed with 2:3 hexanes-EtOAc). The product band is eluted with EtOAc and the eluant evaporates under vacuum to afford 6-ethyl-9a-{1-[(methylsulfonyloxy)methyl]-3-butenyl}-3-(methoxymethyl)-8,9,9a,10-tetrahydroindeno[2,1-*e*]indazol-7(3*H*)-one

### Step 8: (rac)-(8R,10R,10aS)-10-allyl-6-ethyl-3-(methox zethyl)-3,9,10,11-tetrahydro-8,10a-methanoazuleno[2,1-e]indazol-7(8H)-on

A solution of 6-ethyl-9a-{1-[(methylsulfonyloxy)methyl]-3-butenyl}-3-(methoxymethyl)-8,9,9a,10-tetrahydroindeno[2,1-*e*]indazol-7(3*H*)-one (88 mg, 0.20 mmol) in toluene (1 mL) is treated with 1,8-diazabicyclo[5.4.0]undec-7-ene (0.020 mL). The resulting mixture is placed under a N₂ atmosphere and stirred with heating in an oil bath at 90°C for six hours to afford crude (*rac*)-(8*R*,10*R*,10a*S*)-10-allyl-6-ethyl-3-(methoxymethyl)-3,9,10,11-tetrahydro-8,10a-methanoazuleno[2,1-*e*]indazol-7(8*H*)-one. After cooling to room temperature, the reaction mixture is used as described in the next step.

### Step 9: (rac)-(8R,10R,10aS)-6-ethyl-10-propyl-3.9,10,11-tetrahydro-8,10a-methanoazuleno[2,1-e]lindazol-7(8H)-one

The reaction mixture from step 8 is diluted with MeOH (2 mL) and treated with 10% Pd/C (10 mg). After stirring at room temperature under hydrogen for 20 hours, the mixture is further treated with 2N HCl (1 mL) and stirred with heating in an oil bath at 60°C for one hour. After cooling to room temperature, the mixture is diluted with CH₂Cl₂, neutralized with NaHCO₃, filtered through a pad of silica gel, and concentrated under vacuum. The residue is purified by preparative layer chromatography (0.1 x 20 x 20 cm silica gel GF plate developed with 1:1 hexanes-EtOAc). The product band is eluted with EtOAc and the eluant is evaporated under vacuum to afford (*rac*)-(8*R*,10*R*,10a*S*)-6-ethyl-10-propyl-3,9,10,11-tetrahydro-8,10a-methanoazuleno[2,1-*e*]indazol-7(8*H*)-one.

### EXAMPLES 50-95

The following compounds were prepared using methods analogous to those described in the preceding examples:

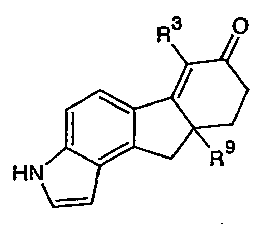

| | | |
|---|---|---|
| 50 | R³ = CH₃ | 6-methyl-9a-propyl-8,9,9a,10-tetrahydroindeno[2,1-*e*]indol-7(3*H*)-one |
| | R⁹ = CH₂CH₂CH₃ | |
| ¹H NMR (CDCl₃, 500 MHz) δ 0.81 (t, CH₂CH₂C*H*_{*3*}), 1.27 (m, CH₂C*H*_{*2*}CH₃), 1.46 and 1.62 (two dt, C*H*_{*2*}CH₂CH₃), 2.07 and 2.30 (two ddd, 9-C*H*_{*2*}), 2.16 (s, 6-C*H*_{*3*}), 2.50 and 2.64 (two ddd, 8-C*H*_{*2*}), 2.91 and 3.24 (two d, 10-C*H*_{*2*}), 6.58 (m, *H*-1), 7.28 (dd, *H*-2), 7.37 (d, *H*-4), and 7.63 (d, *H*-5). | | |

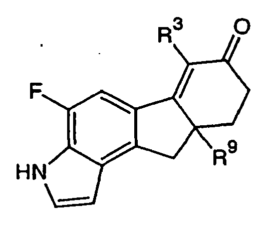

| | | |
|---|---|---|
| 51 | R³ = CH₃ | 9a-ethyl-4-fluoro-6-methyl-8,9,9a,10-tetrahydroindeno[2,1-*e*]indol-7(3*H*)-one |
| | R⁹ = CH₂CH₃ | |
| ¹H NMR (CDCl₃, 500 MHz) δ 0.87 (t, CH₂C*H*_{*3*}), 1.53 and 1.67 (two dq, C*H*_{*2*}CH₃), 2.05 and 2.30 (two ddd, 9-C*H*_{*2*}), 2.13 (s, 6-C*H*_{*3*}), 2.50 and 2.61 (two ddd, 8-C*H*_{*2*}), 2.83 and 3.18 (two d, 10-C*H*_{*2*}), 6.60 (ddd, *H*-1), 7.30 (dd, *H*-2), 7.34 (d, *H*-5), and 8.61 (br s, N*H*). | | |

| | | |
|---|---|---|
| 52 | R³ = CH₂CH₃ | 6,9a-diethyl-4-fluoro-8,9,9a,10-tetrahydroindeno[2,1-*e*]indol-7(3*H*)-one |
| | R⁹ = CH₂CH₃ | |
| ¹H NMR (CDCl₃, 500 MHz) δ 0.85 (t, 9a-CH₂C*H*_{*3*}), 1.15 (t, 6-CH₂C*H*_{*3*}), 1.54 and 1.66 (two dq, 9a-C*H*_{*2*}CH₃), 2.04 and 2.28 (two ddd, 9-C*H*_{*2*}), 2.48 and 2.59 (two ddd, 8-C*H*_{*2*}), 2.56 and 2.70 (two dq, 6-C*H*_{*2*}CH₃), 2.82 and 3.16 (two d, 10-C*H*_{*2*}), 6.60 (ddd, *H*-1), 7.28 (d, *H*-5), 7.30 (dd, *H*-2), and 8.78 (br s, N*H*); mass spectrum m/z 298.2 (M+1). | | |

| | | |
|---|---|---|
| 53 | R³ = CH₃ | 9a-butyl-4-fluoro-6-methyl-8,9,9a,10-tetrahydroindeno[2,1-*e*]indol-7(3*H*)-one |
| | R⁹ = CH₂CH₂CH₂CH₃ | |
| ¹H NMR (CDCl₃, 500 MHz) δ 0.82 (t, CH₂CH₂CH₂C*H*_{*3*}), 1.15-1.29 (m, CH₂C*H*_{*2*}C*H*_{*2*}CH₃), 1.43 and 1.63 (two m, C*H*_{*2*}CH₂CH₂CH₃), 2.05 and 2.29 (two ddd, 9-C*H*_{*2*}), 2.13 (s, 6-C*H*_{*3*}), 2.49 and 2.59 (two ddd, 8-C*H*_{*2*}), 2.84 and 3.18 (two d, 10-C*H*_{*2*}), 6.60 (ddd, *H*-1), 7.31 (dd, *H*-2), 7.33 (d, *H*-5), and 8.56 (br s, N*H*). | | |

| | | |
|---|---|---|
| 54 | R³ = CH₂CH₃ | 9a-butyl-6-ethyl-4-fluoro-8,9,9a,10-tetrahydroindeno[2,1-*e*]indol-7(3*H*)-one |
| | R⁹ = CH₂CH₂CH₂CH₃ | |
| ¹H NMR (CDCl₃, 500 MHz) δ 0.82 (t, CH₂CH₂CH₂C*H*_{*3*}), 1.15 (t, 6-CH₂C*H*_{*3*}), 1.17-1.31 (m, CH₂C*H*_{*2*}C*H*_{*2*}CH₃), 1.44 and 1.62 (two m, C*H*_{*2*}CH₂CH₂CH₃), 2.03 and 2.28 (two ddd, 9-C*H*_{*2*}), 2.47 and 2.60 (two ddd, 8-C*H*_{*2*}), 2.56 and 2.69 (two dq, 6-C*H*_{*2*}CH₃), 2.83 and 3.16 (two d, 10-C*H*_{*2*}), 6.60 (ddd, *H*-1), 7.28 (d, *H*-5), 7.30 (dd, *H*-2), and 8.66 (br s, N*H*). | | |

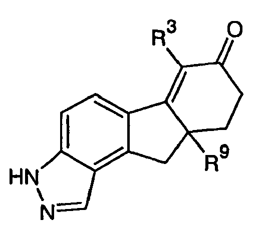

| | | |
|---|---|---|
| 55 | R³ = CH₃ | 6,9a-dimethyl-8,9,9a,10-tetrahydroindeno[2,1-*e*]indazol-7(3*H*)-one |
| | R⁹ = CH₃ | |
| ¹H NMR (CDCl₃, 500 MHz) δ 1.30 (s, 9a-C*H*_{*3*}), 2.15 (s, 6-C*H*_{*3*}), 2.20 (m, 9-C*H*_{*2*}), 2.58 and 2.73 (two ddd, 8-C*H*_{*2*}), 3.12 (ABq, 10-C*H*_{*2*}), 7.49 (d, *H*-4), 7.84 (d, *H*-5), and 8.15 (br s, *H*-1); mass spectrum m/z 253.1 (M+1). | | |

| | | |
|---|---|---|
| 56 | R³ = Br | 6-bromo-9a-methyl-8,9,9a,10-tetrahydroindeno[2,1-*e*]indazol-7(3*H*)-one |
| | R⁹ = CH₃ | |
| ¹H NMR (CDCl₃, 500 MHz) δ 1.38 (s, 9a-C*H*_{*3*}), 2.22 and 2.30 (two ddd, 9-C*H*_{*2*}), 2.82 and 2.88 (two ddd, 8-C*H*_{*2*}), 3.20 (s, 10-C*H*_{*2*}), 7.52 (d, *H*-4)*,* 8.15 (s, *H*-1), and 8.64 (d, *H*-5) ; mass spectrum m/z 317.0 (M+1), 319.0(M+3). | | |

| | | |
|---|---|---|
| 57 | R³ = H | 9a-ethyl-8,9,9a,10-tetrahydroindeno[2,1-*e*]indazol-7(3*H*)-one |
| | R⁹ = CH₂CH₃ | |
| ¹H NMR (CDCl₃, 500 MHz) δ 0.91 (t, CH₂C*H*_{*3*}), 1.62 and 1.75 (two m, C*H*_{*2*}CH₃), 2.07 and 2.39 (two ddd, 9-C*H*_{*2*}), 2.50 and 2.61 (two ddd, 8-C*H*_{*2*}), 2.94 and 3.33 (two d, 10-C*H*_{*2*}), 6.24 (s, *H*-6), 7.46 (d, *H*-4), 7.57 (d, *H*-5), and 8.12 (s, *H-*1)*.* | | |

| | | |
|---|---|---|
| 58 | R³ = CH(CH₃)₂ | 9a-ethyl-6-isopropyl-8,9,9a,10-tetrahydroindeno[2,1-*e*]indazol-7(3*H*)-one |
| | R⁹ = CH₂CH₃ | |
| ¹H NMR (CDCl₃, 500 MHz) δ 0.82 (t, CH₂C*H*_{*3*}), 1.22 and 1.48 (two d, CH(C*H*_{*3*})₂), 1.45 and 1.60 (two m, C*H*_{*2*}CH₃), 2.04 and 2.25 (two m, 9-C*H*_{*2*}), 2.44 and 2.52 (two m, *8-CH*_{*2*}*),* 2.96 and 3.23 (two d, 10-C*H*_{*2*}), 3.30 (m, C*H*(CH₃)₂), 7.44 (d, *H*-4), 7.71 (d, *H*-5), and 8.12 (s, *H*-1). | | |

| | | |
|---|---|---|
| 59 | R³ = CH₂CH₂CH₂CH₃ | 6-butyl-9a-ethyl-8,9,9a,10-tetrahydroindeno[2,1-*e*]indazol-7(3*H*)-one |
| | R⁹ = CH₂CH₃ | |
| ¹H NMR (CDCl₃, 500 MHz) δ 0.86 and 0.96 (two t, CH₂C*H*_{*3*} and CH₂CH₂CH₂C*H*_{*3*}), 1.23-1.59 (m, CH₂C*H*_{*2*}C*H*_{*2*}C*H*_{*3*}), 1.46 and 1.67 (two m, C*H*_{*2*}CH₃), 2.05 and 2.31 (two m, 9-C*H*_{*2*}), 2.45-2.73 (m, 8-C*H*_{*2*} and C*H*_{*2*}CH₂CH₂CH₃), 2.93 and 3.26 (two d, 10-C*H*_{*2*}), 7.46 (d, *H*-4), 7.72 (d, *H*-5), and 8.12 (s, *H*-1). | | |

| | | |
|---|---|---|
| 60 | R³ = OCH₃ | 9a-ethyl-6-methoxy-8,9,9a,10-tetrahydroindeno[2,1-*e*]indazol-7(3*H*)-one |
| | R⁹ = CH₂CH₃ | |
| ¹H NMR (CDCl₃, 500 MHz) δ 0.89 (t, CH₂C*H*_{*3*}), 1.63 and 1.73 (two m, C*H*_{*2*}CH₃), 2.09 and 2.30 (two ddd, 9-C*H*_{*2*}), 2.58 and 2.67 (two ddd, 8-C*H*_{*2*}), 2.97 and 3.30 (two d, 10-C*H*_{*2*}), 3.78 (s,OC*H*_{*3*}), 7.48 (d, *H*-4), 8.05 (d, *H*-5), and 8.11 (s, *H*-1). | | |

| | | |
|---|---|---|
| 61 | R³ = CH₃ | 6-methyl-9a-propyl-8,9,9a,10-tetrahydroindeno[2,1-*e*]indazol-7(3*H*)-one |
| | R⁹ = CH₂CH₂CH₃ | |
| ¹H NMR (DMSO-*d*₆, 500 MHz) δ 0.75 (t, CH₂CH₂C*H*_{*3*}), 1.12 and 1.21 (two m, CH₂C*H*_{*2*}CH₃), 1.33 and 1.56 (two dt, C*H*_{*2*}CH₂CH₃), 2.02 and 2.21 (two ddd, 9-C*H*_{*2*}), 2.03 (s, 6-C*H*_{*3*}), 2.34 and 2.57 (two ddd, 8-C*H*_{*2*}), 2.92 and 3.27 (two d, 10-C*H*_{*2*}), 7.51 (d, *H*-4), 7.75 (d, *H*-5), 8.19 (s, *H*-1), and 13.34 (br s, N*H*). | | |

| | | |
|---|---|---|
| 62 | R³ = Br | 6-bromo-9a-propyl-8,9,9a,10-tetrahydroindeno[2,1-*e*]indazol-7(3*H*)-one |
| | R⁹ = CH₂CH₂CH₃ | |
| ¹H NMR (DMSO-*d*₆, 500 MHz) δ 0.76 (t, CH₂CH₂C*H*_{*3*}), 1.10 and 1.21 (two m, CH₂C*H*_{*2*}CH₃), 1.42 and 1.64 (two dt, C*H*_{*2*}CH₂CH₃), 2.15 and 2.23 (two ddd, 9-C*H*_{*2*}),2.59 and 2.81 (two ddd, 8-C*H*_{*2*}), 3.05 and 3.36 (two d, 10-C*H*_{*2*}), 7.58 (d, *H*-4), 8.27 (s, *H*-1), 8.45 (d, *H*-5), and 13.49 (br s, N*H*). | | |

| | | |
|---|---|---|
| 63 | R³ = CN | 6-cyano-9a-propyl-8,9,9a,10-tetrahydroindeno[2,1-*e*]indazol-7(3*H*)-one |
| | R⁹ = CH₂CH₂CH₃ | |
| ¹H NMR (DMSO-*d*₆, 500 MHz) δ 0.78 (t, CH₂CH₂C*H*_{*3*}), 1.10 and 1.24 (two m, CH₂C*H*_{*2*}CH₃), 1.39 and 1.69 (two dt, C*H*_{*2*}CH₂CH₃), 2.14 and 2.26 (two m, 9-C*H*_{*2*}), 2.47 and 2.69 (two m, 8-C*H*_{*2*}), 3.05 and 3.44 (two d, 10-C*H*_{*2*}), 7.69 (d, *H*-4), 8.15 (d, *H*-5), and 8.33 (s, *H*-1). | | |

| | | |
|---|---|---|
| 64 | R³ = CH₂CH₃ | 9a-butyl-6-ethyl-8,9,9a,10-tetrahydroindeno[2,1-*e*]indazol-7(3*H*)-one |
| | R⁹ = CH₂CH₂CH₂CH₃ | |
| ¹H NMR (CDCl₃, 500 MHz) δ 0.82 (t, CH₂CH₂CH₂C*H*_{*3*}), 1.16 (t, 6-CH₂C*H*_{*3*}), 1.18-1.31 (m, CH₂C*H*_{*2*}C*H*_{*2*}CH₃), 1.44 and 1.64 (two m, C*H*_{*2*}CH₂CH₂CH₃), 2.06 and 2.32 (two ddd, 9-C*H*_{*2*}), 2.51 and 2.62 (two ddd, 8-C*H*_{*2*}), 2.59 and 2.73 (two dq, 6-C*H*_{*2*}CH₃), 2.96 and 3.28 (two d, 10-C*H*_{*2*}), 7.51 (d, *H*-4), 7.79 (d, *H*-5) and 8.16 (s, *H*-1); mass spectrum m/z 309.4 (M+1). | | |

| | | |
|---|---|---|
| 65 | R³ = CH₃ | 9a-(3,3-dimethylbutyl)-6-methyl-8,9,9a,10-tetrahydroindeno[2,1-*e*]indazol-7(3*H*)-one |
| | R9 = CH₂CH₂C(CH₃)₃ | |
| ¹H NMR (CDCl₃, 500 MHz) δ 0.79 (s, C(C*H*_{*3*})₃), 1.19, 1.27, 1.35, and 1.61 (four dt, C*H*_{*2*}C*H*_{*2*}C(CH₃)₃), 2.04 and 2.32 (two ddd, 9-C*H*_{*2*}), 2.16 (s, 6-C*H*_{*3*}), 2.51 and 2.59 (two ddd, 8-C*H*_{*2*}), 2.92 and 3.25 (two d, 10-C*H*_{*2*}), 7.47 (d, *H*-4), 7.83 (d, *H*-5), and 8.12 (s, *H*-1). | | |

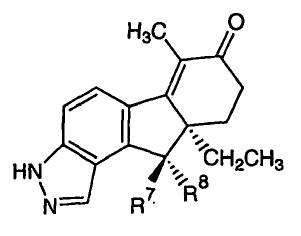

| | | |
|---|---|---|
| 66 | R⁷ = OCH₃ | (*rac*)-(9a*R*,10*R*)-9a-ethyl-10-methoxy-6-methyl-8,9,9a,10-tetrahydroindeno[2,1-*e*]indazol-7(3*H*)-one |
| | R⁸ = H | |
| ¹H NMR (CDCl₃, 500 MHz) δ 0.92 (t, CH₂C*H*_{*3*}), 1.35 and 1.75 (two m, C*H*_{*2*}CH₃), 2.08 and 2.50 (two ddd, 9-C*H*_{*2*}), 2.17 (s, 6-C*H*_{*3*}), 2.59 and 2.64 (two ddd, 8-C*H*_{*2*}), 3.54 (s, OC*H*_{*3*}), 4.66 (s, *H*-10), 7.58 (d, *H*-4), 7.84 (d, *H*-5), 8.21 (s, *H*-1), and 10.37 (br s, N*H*). | | |

| | | |
|---|---|---|
| 67 | R⁷ = NH₂ | (*rac*)-(9a*R*,10*R*)-10-amino-9a-ethyl-6-methyl-8,9,9a,10-tetrahydroindeno[2,1-*e*]indazol-7(3*H*)-one |
| | R⁸ = H | |
| ¹H NMR (CDCl₃, 500 MHz) δ 0.91 (t, CH₂C*H*_{*3*}), 1.42 and 1.75 (two m, C*H*_{*2*}CH₃), 2.04 and 2.32 (two ddd, 9-C*H*_{*2*}), 2.17 (s, 6-C*H*_{*3*}), 2.61 and 2.67 (two ddd, 8-C*H*_{*2*}), 4.44 (s, *H*-10), 7.52 (dd, *H*-4), 7.81 (d, *H*-5), and 8.27 (d, *H*-1); mass spectrum m/z 265.1 (M+1-NH₃). | | |

| | | |
|---|---|---|
| 68 | R⁷ = H | (*rac*)-(9a*R*,10*S*)-10-amino-9a-ethyl-6-methyl-8,9,9a,10-tetrahydroindeno[2,1-*e*]indazol-7(3*H*)-one |
| | R⁸ = NH₂ | |
| ¹H NMR (CDCl₃, 500 MHz) δ 0.53 (t, CH₂C*H*_{*3*}), 1.71 and 1.86 (two m, C*H*_{*2*}CH₃), 2.12 and 2.29 (two ddd, 9-C*H*_{*2*}), 2.16 (s, 6-C*H*_{*3*}), 2.56 and 2.71 (two ddd, 8-C*H*_{*2*}), 4.45 (s, *H*-10), 7.48 (dd, *H*-4), 7.75 (d, *H*-5), and 8.55 (d, *H*-1). | | |

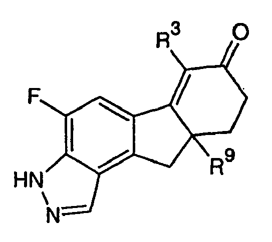

| | | |
|---|---|---|
| 69 | R³ = CH₃ | 9a-ethyl-4-fluoro-6-methyl-8,9,9a,10-tetrahydroindeno[2,1-*e*]indazol-7(3*H*)-one |
| | R⁹ = CH₂CH₃ | |
| ¹H NMR (CDCl₃, 500 MHz) δ 0.87 (t, CH₂C*H*_{*3*}), 1.53 and 1.68 (two dq, C*H*_{*2*}CH₃), 2.07 and 2.32 (two ddd, 9-C*H*_{*2*}), 2.14 (s, 6-C*H*_{*3*}), 2.53 and 2.62 (two ddd, 8-C*H*_{*2*}), 2.90 and 3.23 (two d, 10-C*H*_{*2*}), 7.52 (d, *H*-5), and 8.19 (d, *H*-1); mass spectrum m/z 285.2 (M+1), 348.2 (M+Na+MeCN), and 591.2 (2M+1). | | |

| | | |
|---|---|---|
| 70 | R³ = CH₂CH₃ | 6,9a-diethyl-4-fluoro-8,9,9a,10-tetrahydroindeno[2,1-*e*]indazol-7(3*H*)-one |
| | R⁹ = CH₂CH₃ | |
| ¹H NMR (CDCl₃, 500 MHz) δ 0.86 (t, 9a-CH₂C*H*_{*3*}), 1.15 (t, 6-CH₂C*H*_{*3*}), 1.55 and 1.68 (two dq, 9a-C*H*_{*2*}CH₃), 2.07 and 2.31 (two ddd, 9-C*H*_{*2*}), 2.52 and 2.60 (two ddd, 8-C*H*_{*2*}), 2.55 and 2.70 (two dq, 6-C*H*_{*2*}CH₃), 2.89 and 3.32 (two d, 10-C*H*_{*2*}), 7.46 (d, *H*-5), and 8.19 (d, *H-*1); mass spectrum m/z 299.2 (M+1). | | |

| | | |
|---|---|---|
| 71 | R³ = Br | 6-bromo-9a-ethyl-4-fluoro-8,9,9a,10-tetrahydroindeno[2,1-*e*]indazol-7(3*H*)-one |
| | R⁹ = CH₂CH₃ | |
| ¹H NMR (CDCl₃, 500 MHz) δ 0.89 (t, CH₂C*H*_{*3*}), 1.53 and 1.75 (two dq, C*H*_{*2*}CH₃), 2.17 and 2.35 (two ddd, 9-C*H*_{*2*}), 2.72-2.83 (m, 8-C*H*_{*2*}), 2.98 and 3.29 (two d, 10-C*H*_{*2*}), 8.19 (d, *H*-1), and 8.38 (d, *H*-5). | | |

| | | |
|---|---|---|
| 72 | R³ = CF₃ | 9a-ethyl-4-fluoro-6-trifluoromethyl-8,9,9a,10-tetrahydroindeno[2,1-*e*]indazol-7(3*H*)-one |
| | R⁹ = CH₂CH₃ | |
| ¹H NMR (CDCl₃, 500 MHz) δ 0.86 (t, CH₂C*H*_{*3*}), 1.47 and 1.64 (two dq, C*H*_{*2*}CH₃), 2.16 and 2.32 (two ddd, 9-C*H*_{*2*}), 2.53-2.67 (m, 8-C*H*_{*2*}), 3.02 and 3.27 (two d, 10-C*H*_{*2*}), 7.54 (qd, *H*-5), and 8.18 (d, *H*-1); mass spectrum m/z 339.1 (M+1) and 402.0 (M+Na+MeCN). | | |

| | | |
|---|---|---|
| 73 | R³ = CH₃ | 9a-butyl-4-fluoro-6-methyl-8,9,9a,10-tetrahydroindeno[2,1-*e*]indazol-7(3*H*)-one |
| | R⁹ = CH₂CH₂CH₂CH₃ | |
| ¹H NMR (CDCl₃, 500 MHz) δ 0.82 (t, CH₂CH₂CH₂C*H*_{*3*}), 1.16-1.28 (m, CH₂C*H*_{*2*}C*H*_{*2*}CH₃), 1.44 and 1.65 (two m, C*H*_{*2*}CH₂CH₂CH₃), 2.07 and 2.32 (two ddd, 9-C*H*_{*2*}), 2.14 (s, 6-C*H*_{*3*}), 2.53 and 2.63 (two ddd, 8-C*H*_{*2*}), 2.92 and 3.24 (two d, 10-C*H*_{*2*}), 7.54 (d, *H*-5), and 8.19 (s, *H*-1); mass spectrum m/z 313.2 (M+1), 376.2 (M+Na+MeCN), and 647.3 (2M+Na). | | |

| | | |
|---|---|---|
| 74 | R³ = Br | 6-bromo-9a-butyl-4-fluoro-8,9,9a,10-tetrahydroindeno[2,1-*e*]indazol-7(3*H*)-one |
| | R⁹ = CH₂CH₂CH₂CH₃ | |
| ¹H NMR (CDCl₃, 500 MHz) δ 0.83 (t, CH₂CH₂CH₂C*H*_{*3*}), 1.15-1.32 (m, CH₂C*H*_{*2*}C*H*_{*2*}CH₃), 1.54 and 1.71 (two m, C*H*_{*2*}CH₂CH₂CH₃), 2.16 and 2.34 (two ddd, 9-C*H*_{*2*}), 2.72-2.85 (m, 8-C*H*_{*2*}), 3.00 and 3.30 (two d, 10-C*H*_{*2*}), 8.22 (br s, *H-*1*),* and 8.40 (d, *H*-5); mass spectrum m/z 377.0 (M+1) and 379.0 (M+3). | | |

| | | |
|---|---|---|
| 75 | R³ = CN | 9a-butyl-6-cyano-4-fluoro-8,9,9a,10-tetrahydroindeno[2,1-*e*]indazol-7(3*H*)-one |
| | R⁹ = CH₂CH₂CH₂CH₃ | |
| ¹H NMR (CDCl₃, 500 MHz) δ 0.85 (t, CH₂CH₂CH₂C*H*_{*3*}), 1.13-1.33 (m, CH₂C*H*_{*2*}C*H*_{*2*}CH₃)*,* 1.50 and 1.72 (two m, C*H*_{*2*}CH₂CH₂CH₃), 2.10 and 2.41 (two ddd, 9-C*H*_{*2*}), 2.67 (dd, 8-C*H*_{*2*}), 2.99 and 3.37 (two d, 10-C*H*_{*2*}), 8.11 (d, *H*-5), and 8.24 (br s, *H*-1); mass spectrum m/z 324.2 (M+1). | | |

| | | |
|---|---|---|
| 76 | R³ = CF₃ | 9a-butyl-4-fluoro-6-trifluoromethyl-8,9,9a,10-tetrahydroindeno[2,1-*e*]indazol-7(3*H*)-one |
| | R⁹ = CH₂CH₂CH₂CH₃ | |
| ¹H NMR (CDCl₃, 500 MHz) δ 0.81 (t, CH₂CH₂CH₂C*H*_{*3*}), 1.13-1.30 (m, CH₂C*H*_{*2*}C*H*_{*2*}CH₃), 1.35 and 1.59 (two m, C*H*_{*2*}CH₂CH₂CH₃), 2.16 and 2.31 (two ddd, 9-C*H*_{*2*}), 2.54-2.67 (m, 8-C*H*_{*2*}), 3.03 and 3.28 (two d, 10-C*H*_{*2*}), 7.54 (qd, *H*-5), and 8.19 (d, *H*-1); mass spectrum m/z 367.2 (M+1) and 430.2 (M+Na+MeCN). | | |

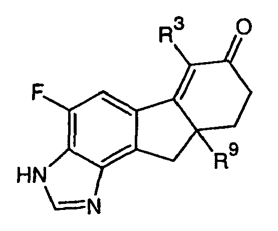

| | | |
|---|---|---|
| 77 | R³ = CH₂CH₃ | 6,9a-diethyl-4-fluoro-8,9,9a,10-tetrahydrofluoreno[1,2-*d*]imidazol-7(3*H*)-one |
| | R⁹ = CH₂CH₃ | |
| ¹H NMR (CDCl₃, 500 MHz) δ 0.85 (t, 9a-CH₂C*H*_{*3*}), 1.15 (t, 6-CH₂C*H*_{*3*}), 1.53 and 1.66 (two dq, 9a-CH₂C*H*_{*3*}), 2.06 and 2.31 (two ddd, 9-C*H*_{*2*}), 2.50 and 2.60 (two ddd, 8-C*H*_{*2*}), 2.54 and 2.71 (two dq, 6-C*H*_{*2*}CH₃), 2.90 and 3.35 (two d, 10-C*H*_{*2*}), 7.43 (d, *H*-5), and 8.63 (br s, *H*-2); mass spectrum m/z 299.2 (M+1), 362.2 (M+Na+MeCN), and 619.3 (2M+1). | | |

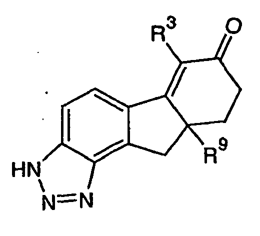

| | | |
|---|---|---|
| 78 | R³=H | 9a-ethyl-8,9,9a,10-tetrahydrofluoreno[1,2-*d*][1,2,3]triazol-7(3*H*)-one |
| | R⁹ = CH₂CH₃ | |
| ¹H NMR (CDCl₃, 500 MHz) δ 0.92 (t, CH₂C*H*_{*3*}), 1.63 and 1.77 (two m, C*H*_{*2*}CH₃), 2.11 and 2.44 (two ddd, 9-C*H*_{*2*}), 2.58 and 2.67 (two ddd, 8-C*H*_{*2*}), 3.09 and 3.61 (two d, 10-C*H*_{*2*}), 6.37 (s, *H*-6), 7.65 (d, *H*-5 or *H*-4), and 7.79 (br s, *H*-4 or *H*-5); mass spectrum m/z 254.2 (M+1). | | |

| | | |
|---|---|---|
| 79 | R³ = CH₂CH₂CH₂CH₃ | 6-butyl-9a-ethyl-8,9,9a,10-tetrahydrofluoreno[1,2-*d*][1,2,3]triazol-7(3*H*)-one |
| | R⁹ = CH₂CH₃ | |
| ¹H NMR (CD₃OD, 500 MHz) δ 0.86 and 0.97 (two t, CH₂C*H*_{*3*} and CH₂CH₂CH₂C*H*_{*3*}), 1.36-1.56 (m, CH₂C*H*_{*2*}C*H*_{*2*}CH₃), 1.55 and 1.70 (two m, C*H*_{*2*}CH₃), 2.11 and 2.38 (two ddd, 9-C*H*_{*2*}), 2.46 and 2.65 (two ddd, 8-C*H*_{*2*}), 2.61 and 2.70 (two m, C*H*_{*2*}CH₂CH₂CH₃), 3.04 and 3.46 (two d, 10-C*H*_{*2*}), and 7.82 (s, *H*-4 and *H*-5); mass spectrum m/z 310.3 (M+1).. | | |

| | | |
|---|---|---|
| 80 | R3 = 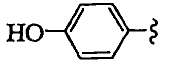 | 9a-ethyl-6-(4-hydroxyphenyl)-8,9,9a,10-tetrahydrofluoreno[1,2-*d*] [1,2,3]triazol-7(3*H*)-one |
| | R⁹ = CH₂CH₃ | |
| ¹H NMR (CD₃OD, 500 MHz) δ 0.94 (t, CH₂C*H*_{*3*}), 1.68 and 1.86 (two m, C*H*_{*2*}CH₃), 2.27 and 2.48 (two ddd, 9-C*H*_{*2*}), 2.57 and 2.77 (two ddd, 8-C*H*_{*2*}), 3.12 and 3.52 (two d, 10-C*H*_{*2*}), 6.56 (d, *H*-4), 6.87 (br s, C₆*H*_{*4*}), and 7.38 (d, *H*-5); mass spectrum m/z 346.4 (M+1). | | |

| | | |
|---|---|---|
| 81 | R³ = Br | 6-bromo-9a-propyl-8,9,9a,10-tetrahydrofluoreno[1,2-*d*][1,2,3]triazol-7(3*H*)-one |
| | R⁹ = CH₂CH₂CH₃ | |
| ¹H NMR (CDCl₃, 500 MHz) δ 0.83 (t, CH₂CH₂C*H*_{*3*}), 1.17-1.37 (m, CH₂C*H*_{*2*}CH₃), 1.56 and 1.71 (two m, C*H*_{*2*}CH₂CH₃), 2.23 and 2.41 (two m, 9-C*H*_{*2*}), 2.68-2.93 (m, 8-C*H*_{*2*}), 3.21 and 3.67 (two d, 10-C*H*_{*2*}), 7.84 (d, *H*-4), and 8.75 (d, *H*-5); mass spectrum m/z 346.1 (M+1) and 348.1 (M+3). | | |

| | | |
|---|---|---|
| 82 | R³ = CH₃ | 6-methyl-9a-propyl-8,9,9a,10-tetrahydrofluoreno[1,2-*d*][1,2,3]triazol-7(3*H*)-one |
| | R⁹ = CH₂CH₂CH₃ | |
| ¹H NMR (CDCl₃, 500 MHz) δ 0.81 (t, CH₂CH₂C*H*_{*3*}), 1.15-1.34 (m, CH₂C*H*_{*2*}CH₃), 1.46 and 1.63 (two m, C*H*_{*2*}CH₂CH₃), 2.12 and 2.35 (two m, 9-C*H*_{*2*}), 2.20 (s, 6-C*H*_{*3*}), 2.59 and 2.70 (two m, 8-C*H*_{*2*}), 3.10 and 3.56 (two d, 10-C*H*_{*2*}), 7.82 (br d, *H*-4 or *H*-5), and 7.90 (d, *H*-5 or *H*-4); mass spectrum m/z 282.3 (M+1). | | |

| | | |
|---|---|---|
| 83 | R³ = CH=CH₂ | 9a-propyl-6-vinyl-8,9,9a,10-tetrahydrofluoreno[1,2-*d*][1,2,3]triazol-7(3*H*)-one |
| | R⁹ = CH₂CH₂CH₃ | |
| ¹H NMR (CDCl₃, 500 MHz) δ 0.83 (t, CH₂CH₂C*H*_{*3*}), 1.18-1.37 (m, CH₂C*H*_{*2*}CH₃), 1.47 and 1.66 (two m, C*H*_{*2*}CH₂CH₃), 2.15 and 2.36 (two m, 9-C*H*_{*2*}), 2.59 and 2.69 (two m, 8-C*H*_{*2*}), 3.12 and 3.56 (two d, 10-C*H*_{*2*}), 5.61 and 5.81 (two m, CH=C*H*_{*2*}), 6.60 (dd, C*H*=CH₂), 7.67 (br s, *H*-4), and 8.03 (d, *H*-5). | | |

| | | |
|---|---|---|
| 84 | R³ = CH₂CH₃ | 6-ethyl-9a-propyl-8,9,9a,10-tetrahydrofluoreno[1,2-*d*][1,2,3]triazol-7(3*H*)-one |
| | R⁹ = CH₂CH₂CH₃ | |
| ¹H NMR (CDCl₃, 500 MHz) δ 0.82 (t, CH₂CH₂C*H*_{*3*}), 1.17 (t, 6-CH₂C*H*_{*3*}), 1.23 and 1.30 (two m, CH₂C*H*_{*2*}CH₃), 1.46 and 1.62 (two dt, C*H*_{*2*}CH₂CH₃), 2.11 and 2.36 (two ddd, 9-C*H*_{*2*}), 2.54 and 2.65 (two ddd, 8-C*H*_{*2*}), 2.60 and 2.75 (two m, 6-C*H*_{*2*}CH₃), 3.10 and 3.54 (two d, 10-C*H*_{*2*}), 7.78 (d, *H*-4 or *H*-5), and 7.86 (d, *H*-5 or *H*-4); mass spectrum m/z 296.2 (M+1). | | |

| | | |
|---|---|---|
| 85 | R³ = CH₂CH=CH₂ | 6-allyl-9a-propyl-8,9,9a,10-tetrahydrofluoreno[1,2-*d*][1,2,3]triazol-7(3*H*)-one |
| | R⁹ = CH₂CH₂CH₃ | |
| ¹H NMR (CDCl₃, 500 MHz) δ 0.83 (t, CH₂CH₂C*H*_{*3*}), 1.18-1.37 (m, CH₂C*H*_{*2*}CH₃), 1.51 and 1.69 (two m, C*H*_{*2*}CH₂CH₃), 2.15 and 2.39 (two m, 9-C*H*_{*2*}), 2.60 and 2.72 (two m, 8-C*H*_{*2*}), 3.12 and 3.59 (two d, 10-C*H*_{*2*}), 3.24 and 3.59 (two m, C*H*_{*2*}CH=CH₂), 5.07 (m, CH=C*H*_{*2*}), 6.04 (m, C*H*=CH₂), 7.76 (d, *H*-4 or *H*-5), and 7.82 (d, *H*-5 or *H*-4); mass spectrum m/z 308.3 (M+1). | | |

| | | |
|---|---|---|
| 86 | R³ = CH₂CH₂CH₃ | 6,9a-dipropyl-8,9,9a,10-tetrahydrofluoreno[1,2-*d*[1,2,3]triazol-7(3*H*)-one |
| | R⁹ = CH₂CH₂CH₃ | |
| ¹H NMR (CDCl₃, 500 MHz) δ 0.82 (t, CH₂CH₂C*H*_{*3*}), 1.04 (t, 6-CH₂CH₂C*H*_{*3*}), 1.16-1.35, 1.40-1.52, and 1.58-1.69 (three m, 9a-C*H*_{*2*}C*H*_{*2*}CH₃ and 6-CH₂C*H*_{*2*}CH₃), 2.11 and 2.35 (two ddd, 9-C*H*_{*2*}), 2.51-2.59 and 2.61-2.74 (two m, 6-C*H*_{*2*}CH₂CH₃ and 8-C*H*_{*2*}), 3.09 and 3.53 (two d, 10-C*H*_{*2*}), 7.77 (d, *H*-4 or *H*-5), and 7.80 (d, *H*-5 or *H*-4); mass spectrum m/z 310.3 (M+1). | | |

| | | |
|---|---|---|
| 87 | R³ = Br | 6-bromo-9a-butyl-8,9,9a,10-tetrahydrofluoreno[1,2-*d*][1,2,3]triazol-7(3*H*)-one |
| | R⁹ = CH₂CH₂CH₂CH₃ | |
| ¹H NMR (CDCl₃, 500 MHz) δ 0.81 (t, CH₂CH₂CH₂C*H*_{*3*}), 1.17-1.33 (m, CH₂C*H*_{*2*}C*H*_{*2*}CH₃), 1.54 and 1.74 (two m, C*H*_{*2*}CH₂CH₂CH₃), 2.22 and 2.41 (two ddd, 9-C*H*_{*2*}), 2.79-2.91 (m, 8-C*H*_{*2*}), 3.20 and 3.67 (two d, 10-C*H*_{*2*}), 7.84 (d, *H*-4), and 8.75 (d, *H*-5); mass spectrum m/z 360.2 (M+1) and 362.2 (M+3). | | |

| | | |
|---|---|---|
| 88 | R³ = CH₃ | 9a-butyl-6-methyl-8,9,9a,10-tetrahydrofluoreno[1,2-*d*][1,2,3]triazol-7(3*H*)-one |
| | R⁹ = CH₂CH₂CH₂CH₃ | |
| ¹H NMR (CDCl₃, 500 MHz) δ 0.80 (t, CH₂CH₂CH₂C*H*_{*3*}), 1.15-1.32 (m, CH₂C*H*_{*2*}C*H*_{*2*}CH₃), 1.44 and 1.66 (two m, C*H*_{*2*}CH₂CH₂CH₃), 2.12 and 2.37 (two m, 9-C*H*_{*2*}), 2.20 (s, 6-C*H*_{*3*}), 2.59 and 2.69 (two m, 8-C*H*_{*2*}), 3.10 and 3.57 (two d, 10-C*H*_{*2*}), 7.82 (d, *H*-4 or *H*-5), and 7.91 (d, *H*-5 or *H*-4). | | |

| | | |
|---|---|---|
| 89 | R³ = CH₂CH₃ | 9a-butyl-6-ethyl-8,9,9a,10-tetrahydrofluoreno[1,2-*d*][1,2,3]triazol-7(3*H*)-one |
| | R⁹ = CH₂CH₂CH₂CH₃ | |
| ¹H NMR (CDCl₃, 500 MHz) δ 0.81 (t, CH₂CH₂CH₂C*H*_{*3*}), 1.18 (t, 6-CH₂C*H*_{*3*}), 1.23 (m, CH₂C*H*_{*2*}C*H*_{*2*}CH₃), 1.44 and 1.65 (two dt, C*H*_{*2*}CH₂CH₂CH₃), 2.10 and 2.37 (two ddd, 9-C*H*_{*2*}), 2.57 and 2.67 (two ddd, 8-C*H*_{*2*}), 2.62 and 2.77 (two m, 6-C*H*_{*2*}CH₃), 3.09 and 3.56 (two d, 10-C*H*_{*2*}), 7.81 (d, *H*-4 or *H*-5), and 7.85 (d, *H*-5 or *H*-4); mass spectrum m/z 310.3 (M+1). | | |

| | | |
|---|---|---|
| 90 | R³ = CH₂CH=CH₂ | 6-allyl-9a-butyl-8,9,9a,10-tetrahydrofluoreno[1,2-*d*][1,2,3]triazol-7(3*H*)-one |
| | R⁹ = CH₂CH₂CH₂CH₃ | |
| ¹H NMR (CDCl₃, 500 MHz) δ 0.81 (t, CH₂CH₂CH_{Z}C*H*_{*3*}), 1.15-1.35 (m, CH₂C*H*_{*2*}C*H*_{*2*}CH₃), 1.50 and 1.72 (two m, C*H*_{*2*}CH₂CH₂CH₃), 2.15 and 2.40 (two ddd, 9-C*H*_{*2*}), 2.61 and 2.72 (two ddd, 8-C*H*_{*2*}), 3.12 and 3.59 (two d, 10-C*H*_{*2*}), 3.26 and 3.59 (two m, C*H*_{*2*}CH=CH₂), 5.07 (m, CH=C*H*_{*2*}), 6.04 (m, C*H*+CH₂), 7.78 (d, *H*-4 or *H*-5), and 7.82 (d, *H*-5 or *H*-4); mass spectrum m/z 322.3 (M+1). | | |

| | | |
|---|---|---|
| 91 | R³ = CH₂CH₂CH₃ | 9a-butyl-6-propyl-8,9,9a,10-tetrahydrofluoreno[1,2-*d*] [1,2,3]triazol-7(3*H*)-one |
| | R⁹ = CH₂CH₂CH₂CH₃ | |
| ¹H NMR (CDCl₃, 500 MHz) δ 0.80 (t, CH₂CH₂CH₂C*H*_{*3*}), 1.04 (t, 6-CH₂CH₂C*H*_{*3*}), 1.14-1.31, 1.40-1.51, and 1.58-1.69 (three m, C*H*_{*2*}C*H*_{*2*}C*H*_{*2*}CH₃ and 6-CH₂C*H*_{*2*}CH₃), 2.10 and 2.35 (two ddd, 9-C*H*_{*2*}), 2.51-2.59 and 2.60-2.74 (two m, 6-C*H*_{*2*}CH₂CH₃ and 8-C*H*_{*2*}), 3.08 and 3.54 (two d, 10-C*H*_{*2*}), and 7.79 (s, *H*-4 and *H*-5); mass spectrum m/z 324.3 (M+1). | | |

| | | |
|---|---|---|
| 92 | R³ = CF₃ | 9a-butyl-6-trifluoromethyl-8,9,9a,10-tetrahydrofluoreno[1,2-*d*][1,2,3]triazol-7(3*H*)-one |
| | R⁹ = CH₂CH₂CH₂CH₃ | |
| ¹H NMR (CDCl₃, 500 MHz) δ 0.79 (t, CH₂CH₂CH₂C*H*_{*3*}), 1.13-1.27 (m, CH₂C*H*_{*2*}C*H*_{*2*}CH₃), 1.36 and 1.62 (two m, C*H*_{*2*}CH₂CH₂CH₃), 2.22 and 2.39 (two ddd, 9-C*H*_{*2*}), 2.60-2.73 (m, 8-C*H*_{*2*}), 3.26 and 3.68 (two d, 10-C*H*_{*2*}), 7.77 (d, *H*-4), and 7.96 (qd, *H*-5); mass spectrum m/z 350.1 (M+1) and 413.1 (M+Na+MeCN). | | |

| | | |
|---|---|---|
| 93 | R3= 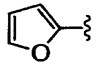 | 9a-butyl-6-(2-furyl)-8,9,9a,10-tetrahydrofluoreno[1,2-*d*][1,2,3]triazol-7(3*H*)-one |
| | R⁹ = CH₂CH₂CH₂CH₃ | |
| ¹H NMR (CDCl₃, 500 MHz) δ 0.84 (t, CH₂CH₂CH₂C*H*_{*3*}), 1.15-1.36 (m, CH₂C*H*_{*2*}C*H*_{*2*}CH₃), 1.54 and 1.78 (two m, C*H*_{*2*}CH₂CH₂CH₃), 2.25 and 2.47 (two ddd, 9-C*H*_{*2*}), 2.68 and 2.75 (two ddd, 8-C*H*_{*2*}), 3.27 and 3.72 (two d, 10-C*H*_{*2*}), 4.0 (br s, N*H*), 6.51 (d, furyl *H*-3), 6.57 (dd, furyl *H*-4), 6.74 (d, *H*-5), 7.50 (s, furyl *H*-5), and 7.71 (d, *H*-4); mass spectrum m/z 348.2 (M+1). | | |

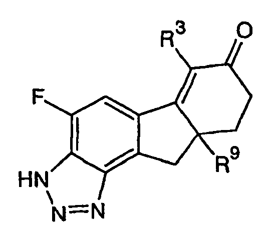

| | | |
|---|---|---|
| 94 | R³ = CH₂CH₃ | 6,9a-diethyl-4-fluoro-8,9,9a,10-tetrahydrofluoreno[1,2-*d*][1,2,3]triazol-7(3*H*)-one |
| | R⁹ = CH₂CH₃ | |
| ¹H NMR (CDCl₃, 500 MHz) δ 0.88 (t, 9a-CH₂C*H*_{*3*}), 1.17 (t, 6-CH₂C*H*_{*3*}), 1.56 and 1.70 (two dq, 9a-C*H*_{*2*}CH₃), 2.10 and 2.36 (two ddd, 9-C*H*_{*2*}), 2.56 and 2.65 (two ddd, 8-C*H*_{*2*}), 2.57 and 2.73 (two dq, 6-C*H*_{*2*}CH₃), 3.02 and 3.47 (two d, 10-C*H*_{*2*}), and 7.52 (d, *H*-5); mass spectrum m/z 300.2 (M+1), 363.2 (M+Na+MeCN), and 621.3 (2M+1) | | |

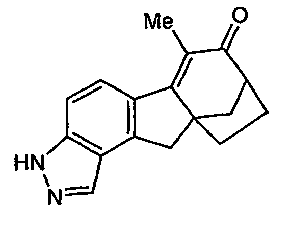

| | |
|---|---|
| 95 | 6-methyl-3,9,10,11-tetnahydro-8,10a-methanoqzuleno[2,1-*e*]indazol-7(8*H*-one |
| ¹H NMR (CDCl₃, 500 MHz) δ 1.69 and 1.92 (two ddd, 10-C*H*_{*2*}), 1.84. and 2.30 (two m, 9-C*H*_{*2*}), 1.99 and 2.04 (two dd, 12-C*H*_{*2*}), 2.15 (s, 6-C*H*_{*3*}), 3.12 (dd, *H*-8), 3.33 and 3.49 (two d, 11-C*H*_{*2*}), 7.53 (d, *H*-4), 7.88 (d, *H*-5), and 8.19 (s, *H*-1); mass spectrum m/z 265.2 (M+1) and 328.2 (M+Na+MeCN) | |

### EXAMPLE 96

The following compounds are prepared using methods analogous to those described in the preceding examples:

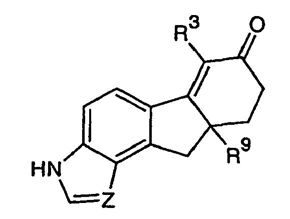

| Z | R³ | R⁹ |
|---|---|---|
| CH | Br | CH₂CH₃ |
| CH | CH₂CH₃ | CH₂CH₃ |
| CH | CH₂CH₃ | CH₂CH₂CH₃ |
| CH | Br | CH₂CH₂CH₂CH₃ |
| CH | CH₃ | CH₂CH₂CH₂CH₃ |
| CH | CH₂CH₃ | CH₂CH₂CH₂CH₃ |
| CCl | CH₃ | CH₂CH₂CH₂CH₃ |
| N | CH₃ | CH₂CH₂CH₃ |
| N | CH₂CH₃ | CH₂CH₂CH₃ |
| N | Br | CH₂CH₂CH₂CH₃ |
| N | CH₃ | CH₂CH₂CH₂CH₃ |
| N | CH₂CH₃ | CH₂CH₂CH₂CH₃ |

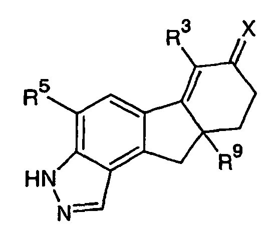

| X | R³ | R⁵ | R⁹ |
|---|---|---|---|
| O | CH₂CH₃ | H | CH₂CH₂CH₃ |
| O | CH₃ | H | CH₂CH(CH₃)CH₂CH₃ |
| O | CH₃ | H | CH₂CH(CH₃)₂ |
| O | CH₃ | H | CH=CHCH₃ |
| O | CH₃ | H | CH=CHCH₂CH₃ |
| O | CH₂CH₃ | H | CH₂CH=CHCH₃ |
| O | Cl | H | CH₂CH₃ |
| O | I | H | CH₂CH₃ |
| O | 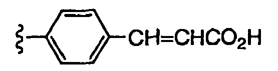 | H | CH₂CH₃ |
| O | 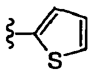 | H | CH₂CH₃ |
| O | 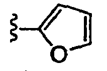 | H | CH₂CH₃ |
| O | 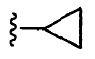 | H | CH₂CH₃ |
| O | 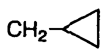 | H | CH₂CH₃ |
| O | 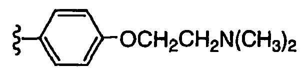 | H | CH₂CH₃ |
| O | 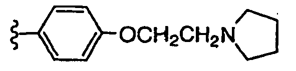 | H | CH₂CH₃ |
| O | Cl | H | CH₂CH₂CH₃ |
| O | Cl | H | CH₂CH₂CH₂CH₃ |
| O | I | H | CH₂CH₂CH₂CH₃ |
| O | 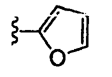 | H | CH₂CH₂CH₂CH₃ |
| O | Br | H | CH₂CH₂F |
| O | Br | H | CH₂HF₂ |
| O | Br | H | CH₂CF₃ |
| O | Br | H | CH₂CH₂CH₂F |
| O | Br | H | CH₂CH₂CF₃ |
| O | Br | H | CH₂CF₂CF₃ |
| O | Br | H | CH₂CH₂CH₂CF₃ |
| O | Br | H | CH₂CH₂CF₂CF₃ |
| O | CH₂CH₃ | H | CH₂CH₂F |
| O | CH₂CH₃ | H | CH₂CF₃ |
| O | CH₂CH₃ | H | 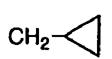 |
| O | CF₃ | H | 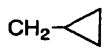 |
| O | CF₃ | H | 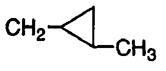 |
| O | CF₃ | H | 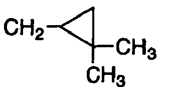 |
| O | CF₃ | H | 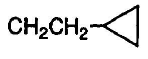 |
| O | CF₃ | H | 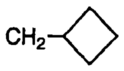 |
| O | CF₃ | H | 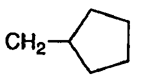 |
| O | CF₃ | H | 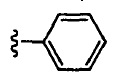 |
| O | CF₃ | H | 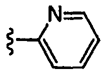 |
| O | CF₃ | H | 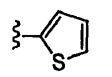 |
| O | CH₃ | H | CH₂CH=CHCl |
| O | CH₃ | H | CH₂CH=CHBr |
| O | Cl | F | CH₂CH₃ |
| O | 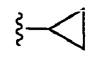 | F | CH₂CH₃ |
| O | 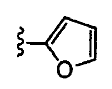 | F | CH₂CH₃ |
| O | Cl | F | CH₂CH₂CH₂CH₃ |
| O | 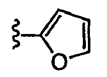 | F | CH₂CH₂CH₂CH₃ |
| O | Br | F | CH₂CF₃ |
| O | Br | F | CH₂CH₂CF₃ |
| O | CH₂CH₃ | F | 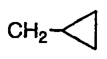 |
| O | CF₃ | F | 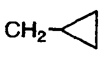 |
| O | CF₃ | F | 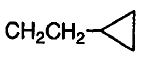 |
| O | CH₃ | F | CH₂CH=CHCl |
| NOH | CH₃ | H | CH₂CH₃ |
| NOCH₃ | CH₃ | H | CH₂CH₃ |
| NNH₂ | CH₃ | H | CH₂CH₃ |
| CH₂ | CH₃ | H | CH₂CH₃ |
| NOH | CH₂CH₃ | H | CH₂CH₂CH₂CH₃ |

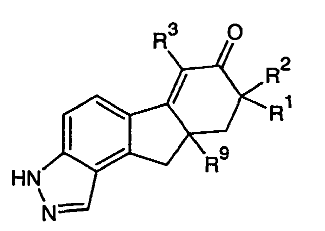

| R¹ | R² | R³ | R⁹ |
|---|---|---|---|
| CH₂CH₃ | H | CH₃ | CH₂CH₃ |
| CH₂CH=CH₂ | H | CH₃ | CH₂CH₃ |
| CH₂CH₂CH₃ | H | CH₃ | CH₂CH₃ |
| OH | CH₂CH₂CH₃ | CH₃ | CH₂CH₃ |
| CH₃ | CH₃ | CH₃ | CH₂CH₃ |
| OH | H | CH₂CH₃ | CH₂CH₂CH₂CH₃ |

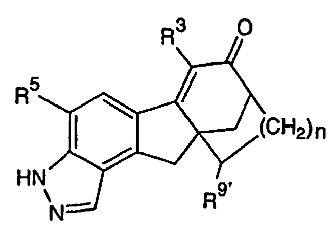

| n | R³ | R⁵ | R^{9'} |
|---|---|---|---|
| 1 | Br | H | CH₃ |
| 1 | Br | H | CH₂CH₃ |
| 1 | Br | H | CH₂CH=CH₂ |
| 1 | Br | H | CH₂CH₂CH₃ |
| 1 | CH₃ | H | CH₂CH₂CH₃ |
| 1 | CF₃ | H | CH₂CH₂CH₃ |
| 1 | 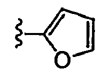 | H | CH₂CH₂CH₃ |
| 1 | Cl | H | CH₂CH₂CH₃ |
| 1 | CH₂CH₃ | H | CH=CH₂ |
| 1 | CH₃ | H | CH=CHCl |
| 1 | CH₂CH₃ | H | CF₃ |
| 1 | CH₂CH₃ | H | CH₂CH₂F |
| 1 | CH₂CH₃ | H | CH₂CF₃ |
| 1 | CF₃ | H | 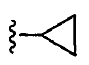 |
| 1 | CF₃ | H | 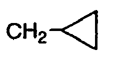 |
| 1 | Br | F | CH₂CH₂CH₃ |
| 1 | CF₃ | F | CH₂CH₂CH₃ |
| 1 | 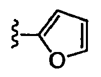 | F | CH₂CH₂CH₃ |
| 1 | Cl | F | CH₂CH₂CH₃ |
| 1 | CH₂CH₃ | F | CH₂CF₃ |
| 1 | CF₃ | F | 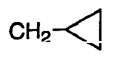 |
| 2 | Br | H | CH₂CH₂CH₃ |
| 2 | CF₃ | H | CH₂CH₂CH₃ |
| 2 | 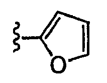 | H | CH₂CH₂CH₃ |
| 2 | Cl | H | CH₂CH₂CH₃ |
| 2 | CH₂CH₃ | H | CH₂CF₃ |
| 2 | CF₃ | H | 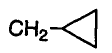 |
| 2 | Br | F | CH₂CH₂CH₃ |
| 2 | CF₃ | F | CH₂CH₂CH₃ |
| 2 | 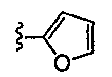 | F | CH₂CH₂CH₃ |
| 2 | Cl | F | CH₂CH₂CH₃ |
| 2 | CH₂CH₃ | F | CH₂CF₃ |
| 2 | CF₃ | F | 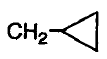 |

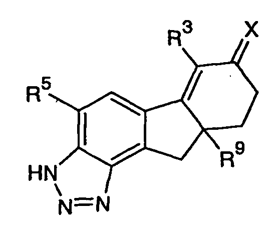

| X | R³ | R⁵ | R⁹ |
|---|---|---|---|
| O | CH₃ | H | CH₂CH₂(CH₃)₃ |
| O | CH₃ | H | CH₂CH(CH₃)CH₂CH₃ |
| O | CH₃ | H | CH₂CH(CH₃)₂ |
| O | CH₂CH₃ | H | CH=CHCH₂CH₃ |
| O | CH₂CH₃ | H | CH₂CH=CHCH₃ |
| O | CH₂CH₃ | H | CH₂CH₂CH=CH₂ |
| O | Cl | H | CH₂CH₃ |
| O | I | H | CH₂CH₃ |
| O | 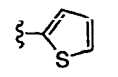 | H | CH₂CH₂CH₂CH₃ |
| O | 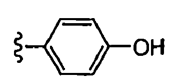 | H | CH₂CH₂CH₂CH₃ |
| O | 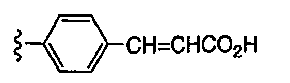 | | ₂CH₃ |
| O | 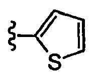 | H | CH₂CH₃ |
| O | 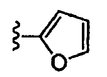 | H | CH₂CH₃ |
| O | 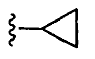 | H | CH₂CH₃ |
| O | 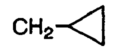 | H | CH₂CH₃ |
| O | 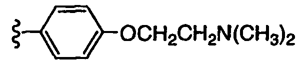 | H | CH₂CH₃ |
| O | 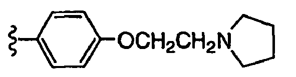 | H | CH₂CH₃ |
| O | Cl | H | CH₂CH₂CH₃ |
| O | Cl | H | CH₂CH₂CH₂CH₃ |
| O | I | H | CH₂CH₂CH₂CH₃ |
| O | 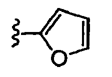 | H | CH₂CH₂CH₂CH₃ |
| O | Br | H | CH₂CH₂F |
| O | Br | H | CH₂CHF₂ |
| O | Br | H | CH₂CF₃ |
| O | Br | H | CH₂CH₂CH₂F |
| O | Br | H | CH₂CH₂CF₃ |
| O | Br | H | CH₂CF₂CF₃ |
| O | Br | H | CH₂CH₂CH₂CF₃ |
| O | Br | H | CH₂CH₂CF₂CF₃ |
| O | CH₂CH₃ | H | CH₂CH₂F |
| O | CH₂CH₃ | H | CH₂CF₃ |
| O | CH₂CH₃ | H | 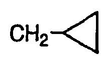 |
| O | CF₃ | H | 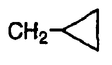 |
| O | 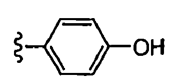 | H | CH₂CH₂CH₂CH₃ |
| O | 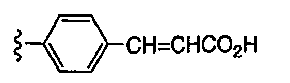 | | ₂CH₃ |
| O | 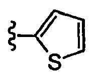 | H | CH₂CH₃ |
| O | 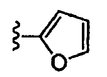 | H | CH₂CH₃ |
| O | 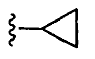 | H | CH₂CH₃ |
| O | 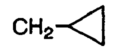 | H | CH₂CH₃ |
| O | 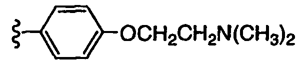 | H | CH₂CH₃ |
| O | 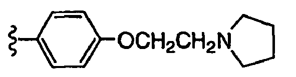 | H | CH₂CH₃ |
| O | Cl | H | CH₂CH₂CH₃ |
| O | Cl | H | CH₂CH₂CH₂CH₃ |
| O | I | H | CH₂CH₂CH₂CH₃ |
| O | 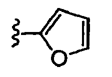 | H | CH₂CH₂CH₂CH₃ |
| O | Br | H | CH₂CH₂F |
| O | Br | H | CH₂CHF₂ |
| O | Br | H | CH₂CF₃ |
| O | Br | H | CH₂CH₂CH₂F |
| O | Br | H | CH₂CH₂CF₃ |
| O | Br | H | CH₂CF₂CF₃ |
| O | Br | H | CH₂CH₂CH₂CF₃ |
| O | Br | H | CH₂CH₂CF₂CF₃ |
| O | CH₂CH₃ | H | CH₂CH₂F |
| O | CH₂CH₃ | H | CH₂CF₃ |
| O | CH₂CH₃ | H | 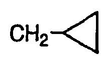 |
| O | CF₃ | H | 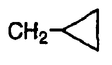 |
| O | CF₃ | H | 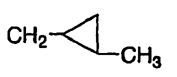 |
| O | CF₃ | H | 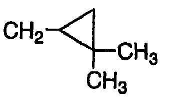 |
| O | CF₃ | H | 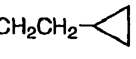 |
| O | CF₃ | H | 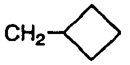 |
| O | CF₃ | H | 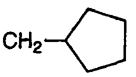 |
| O | CF₃ | H | 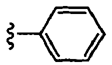 |
| O | CF₃ | H | 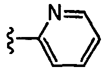 |
| O | CF₃ | H | 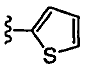 |
| O | CH₃ | H | CH₂CH=CHCl |
| O | CH₃ | H | CH₂CH=CHBr |
| NOH | CH₂CH₃ | H | CH₂CH₂CH₂CH₃ |
| NOCH₃ | CH₃ | H | CH₂CH₃ |

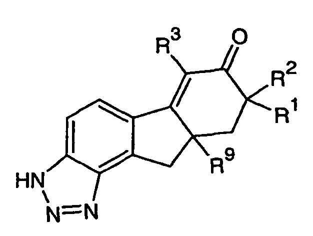

| R¹ | R² | R³ | R⁹ |
|---|---|---|---|
| OH | H | CH₃ | CH₂CH₃ |
| CH₃ | H | CH₃ | CH₂CH₃ |
| CH₂CH₃ | H | CH₃ | CH₂CH₃ |
| CH₂CH=CH₂ | H | CH₃ | CH₂CH₃ |
| CH₂CH₂CH₃ | H | CH₃ | CH₂CH₃ |
| OH | CH₂CH₂CH₃ | CH₃ | CH₂CH₃ |
| CH₂CH₂CH₃ | CH₃ | CH₃ | CH₂CH₃ |
| OH | H | CH₂CH₃ | CH₂CH₂CH₂CH₃ |

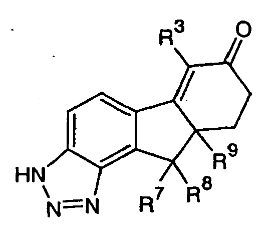

| R³ | R⁷ | R⁸ | R⁹ |
|---|---|---|---|
| CH₃ | CH₃ | H | CH₂CH₃ |
| CH₃ | CH₃ | CH₃ | CH₂CH₃ |
| CH₃ | =O | | CH₂CH₃ |
| CH₂CH₃ | =O | | CH₂CH₂CH₂CH₃ |
| CH₂CH₃ | CH₃ | H | CH₂CH₂CH₂CH₃ |

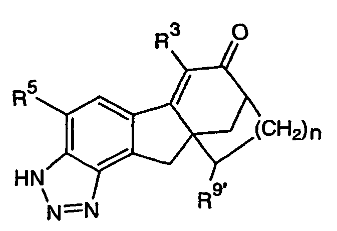

| n | R³ | R⁵ | R^{9'} |
|---|---|---|---|
| 1 | Br | H | CH₃ |
| 1 | Br | H | CH₂CH₃ |
| 1 | Br | H | CH₂CH=CH₂ |
| 1 | Br | H | CH₂CH₂CH₃ |
| 1 | CH₃ | H | CH₂CH₂CH₃ |
| 1 | CF₃ | H | CH₂CH₂CH₃ |
| 1 | 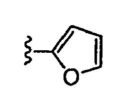 | H | CH₂CH₂CH₃ |
| 1 | Cl | H | CH₂CH₂CH₃ |
| 1 | CH₂CH₃ | H | CH=CH₂ |
| 1 | CH₃ | H | CH=CHCl |
| 1 | CH₂CH₃ | H | CF₃ |
| 1 | CH₂CH₃ | H | CH₂CH₂F |
| 1 | CH₂CH₃ | H | CH₂CF₃ |
| 1 | CF₃ | H | 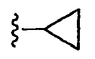 |
| 1 | CF₃ | H | 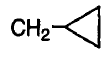 |
| 2 | Br | H | CH₂CH₂CH₃ |
| 2 | CF₃ | H | CH₂CH₂CH₃ |
| 2 | 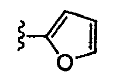 | H | CH₂CH₂CH₃ |
| 2 | Cl | H | CH₂CH₂CH₃ |
| 2 | CH₂CH₃ | H | CH₂CF₃ |
| 2 | CF₃ | H | 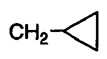 |

### Estrogen Receptor Binding Assay

The estrogen receptor ligand binding assays are designed as scintillation proximity assays employing the use of tritiated estradiol and recombinant expressed estrogen receptors. The full length recombinant human ER-α and ER-β proteins are produced in a bacculoviral expression system. ER-α or ER-β extracts are diluted 1:400 in phosphate buffered saline containing 6 mM α-monothiolglycerol. 200 µL aliquots of the diluted receptor preparation are added to each well of a 96-well Flashplate. Plates are covered with Saran Wrap and incubated at 4 ° C overnight.

The following morning, a 20 ul aliquot of phosphate buffered saline containing 10% bovine serum albumin is added to each well of the 96 well plate and allowed to incubate at 4° C for 2 hours. Then the plates are washed with 200 ul of buffer containing 20 mM Tris (pH 7.2), 1 mM EDTA, 10% Glycerol, 50 mM KCI, and 6 mM α-monothiolglycerol. To set up the assay in these receptor coated plates, add 178 ul of the same buffer to each well of the 96 well plate. Then add 20 ul of a 10 nM solution of ³H-estradiol to each well of the plate.

Test compounds are evaluated over a range of concentrations from 0.01 nM to 1000 nM. The test compound stock solutions should be made in 100% DMSO at 100X the final concentration desired for testing in the assay. The amount of DMSO in the test wells of the 96 well plate should not exceed 1%. The final addition to the assay plate is a 2 ul aliquot of the test compound which has been made up in 100% DMSO. Seal the plates and allow them to equilibrate at room temperature for 3 hours. Count the plates in a scintillation counter equipped for counting 96 well plates.

The compounds of Examples 1-40 exhibit binding affinities to the estrogen receptor α-subtype in the range of IC₅₀ = 36 - >10000 nm, and to the estrogen receptor β-subtype in the range of IC₅₀ = 1.4 - 283 nm.

### Pharmaceutical Composition

As a specific embodiment of this invention, 25 mg of tetrahydrofluorenone from Example 6, is formulated with sufficient finely divided lactose to provide a total amount of 580 to 590 mg to fill a size 0, hard-gelatin capsule.

## Claims

1. A compound of the formula: 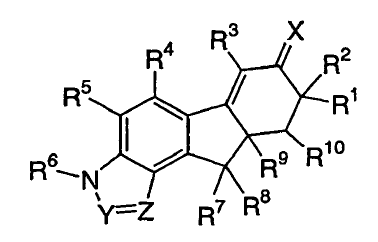 wherein
X is selected from the group consisting of: O, N-OR^{a}, N-NR^{a}R^{b} and C₁₋₆ alkylidene, wherein said alkylidene group is unsubstituted or substituted with a group selected from hydroxy, amino, O(C₁₋₄alkyl), NH(C₁₋₄alkyl), or N(C₁₋₄alkyl)₂;
Y is selected from the group consisting of N and CR^{e};
Z is selected from the group consisting of N and CR^{f};
R¹ is selected from the group consisting of hydrogen, C₁₋₆alkyl, C₂₋₆alkenyl, and C₂₋₆alkynyl, wherein said alkyl, alkenyl and alkynyl groups are either unsubstituted or substituted with a group selected from OR^{c}, SR^{c}, NR^{b}R^{c}, C(=O)R^{c}, C(=O)CH₂OH, bromo, 1-3 chloro, 1-5 fluoro or phenyl, wherein said phenyl group can either be unsubstituted or substituted with a substituent selected from the group consisting of C₁₋₄alkyl, OH and O(C₁₋₄alkyl);
R² is selected from the group consisting of hydrogen, hydroxy, iodo, O(C=O)R^{c}, C(=O)R^{c}, CO₂R^{c}, C₁₋₆alkyl, C₂₋₆alkenyl, and C₂₋₆alkynyl, wherein said alkyl, alkenyl and alkynyl groups are either unsubstituted or substituted with a group selected from OR^{c}, SR^{c}, NR^{b}R^{c} ,C(=O)R^{c}, C(=O)CH₂OH, or phenyl, wherein said phenyl group can either be unsubstituted or substituted with a substituent selected from the group consisting of C₁₋₄alkyl, OH and O(C₁₋₄alkyl);
or R¹ and R², when taken together with the carbon atom to which they are attached, form a carbonyl group;
or R¹ and R², when taken together with the carbon atom to which they are attached, form a C₃₋₇ cycloalkyl or ₃₋₇ heterocycloalkyl ring,
wherein said ring is either unsubstituted or substituted with a group selected from C₁₋₄ alkyl, OH, O(C₁₋₄ alkyl) and oxo;
or R¹ and R², when taken together, form a C₁₋₆ alkylidene group,
wherein said alkylidene group is either unsubstituted or substituted with a group selected from the group consisting of hydroxy, O(C₁₋₄alkyl), N(C₁₋₄alkyl)₂, and phenyl; wherein said phenyl group can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄alkyl);
R³ is selected from the group consisting of hydrogen, fluoro, chloro, bromo, iodo, cyano, nitro, NR^{a}R^{c}, OR^{a}, S(O)R^{a}, SO₂R^{a}, SR^{a}, C(=O)R^{a}, CO₂R^{c}, CONR^{a}R^{c}, C₁₋₁₀alkyl, C₂₋₁₀alkenyl, C₂₋₁₀alkynyl, C₃₋₇cycloalkyl, 4-7 membered heterocycloalkyl, cycloalkylalkyl, aryl, heteroaryl, arylalkyl, and heteroarylalkyl, wherein said alkyl, alkenyl, alkynyl, cycloalkyl, aryl and heteroaryl groups are either unsubstituted or independently substituted with 1, 2 or 3 groups selected from fluoro, chloro, bromo, iodo, cyano, OR^{a}, NR^{a}R^{c}, O(C=O)R^{a}, O(C=O)NR^{a}R^{c}, NR^{a}(C=O)R^{c}, NR^{a}(C=O)OR^{c}, C(=O)R^{a}, CO₂R^{a}, CONR^{a}R^{c}, CSNR^{a}R^{c}, SR^{a}, S(O)R^{a}, SO₂R^{a}, SO₂NR^{a}R^{c}, LR^{d}, and MLR^{d} ;
R⁴ and R⁵ are each independently selected from the group consisting of hydrogen, hydroxy, amino, methyl, CF₃, fluoro, chloro, and bromo;
R⁶ is selected from the group consisting of hydrogen; (C=O)R^{a}, (C=O)OR^{a}, and SO₂R^{a};
R⁷ and R⁸ are each independently selected from the group consisting of hydrogen, C₁₋₆alkyl, C₂₋₆alkenyl, C₂₋₆alkynyl, fluoro, chloro, bromo, cyano, hydroxy, O(C₁₋₆ alkyl), azido, amino, NH(C₁₋₄alkyl); and N(C₁₋₄alkyl)₂;
or R⁷ and R⁸, when taken together with the carbon atom to which they are attached, form a 3-5 membered cycloalkyl ring;
or R⁷ and R⁸; when taken together with the carbon atom to which they are attached, form a carbonyl group;
or R⁷ and R⁸, when taken together, form a C₁₋₆alkylidene group,
wherein said alkylidene group is either unsubstituted or substituted with a group selected from cyano,C(=O)H, C(=O)(C₁₋₄alkyl), or C(=O)OC₁₋₄alkyl;
R⁹ is selected from the group consisting of hydrogen, C₁₋₁₀alkyl; C₂₋₁₀alkenyl, C₂₋₁₀alkynyl, C₃₋₆cycloalkyl, cycloalkylalkyl, aryl, heteroaryl, arylalkyl and heteroarylalkyl, wherein said alkyl, alkenyl, alkynyl, cycloalkyl, cycloalkylalkyl, aryl, heteroaryl, arylalkyl and heteroarylalkyl groups can be optionally substituted with a group selected from bromo, iodo, OR^{b}, SR^{b}, C(=O)R^{b}, 1-3 chloro, or 1-5 fluoro;
or R⁹ and R¹, when taken together with the three intervening carbon atoms to which they are attached, form a 5-6 membered cycloalkyl or cycloalkenyl ring which can be optionally substituted with 1-3 groups independently selected from oxo, hydroxy, fluoro, C₁₋₆alkyl, C₂₋₆alkenyl, C₂₋₆alkynyl, C₁₋₆alkylidenyl, C₃₋₆cycloalkyl, cycloalkylalkyl, phenyl, or phenylalkyl, wherein said alkyl, alkenyl, alkynyl, alkylidenyl, cycloalkyl, cycloalkylalkyl, phenyl, and phenylalkyl groups can be optionally substituted with a group selected from chloro, bromo, iodo, R^{b}, SR^{b}, C₁₋₃alkyl, C(=O)R^{b}, or 1-5 fluoro;
or R⁹ and R⁸, when taken together with the two intervening carbon atoms to which they are attached, form a cyclopropyl ring which can be optionally substituted with 1-2 groups independently selected from C₁₋₆alkyl, C₂₋₆alkenyl, C₂₋₆alkynyl, C₃₋₆cycloalkyl, cycloalkylalkyl, phenyl, or phenylalkyl, wherein said alkyl, alkenyl, alkynyl, cycloalkyl, cycloalkylalkyl, phenyl, and phenylalkyl groups can be optionally substituted with a group selected from chloro, bromo, iodo, OR^{b}, SR^{b}, C₁₋₃alkyl, C(=O)R^{b}, or 1-5 fluoro;
R¹⁰ is selected from the group consisting of hydrogen, C₁₋₁₀alkyl, and C₂₋₁₀alkenyl;
R^{a} is selected from the group consisting of hydrogen, C₁₋₁₀alkyl, and phenyl,
wherein said alkyl group can be optionally substituted with a group selected from hydroxy, amino, O(C₁₋₄alkyl), NH(C₁₋₄alkyl), N(C₁₋₄ alkyl)₂, phenyl, or 1-5 fluoro, and
wherein said phenyl groups can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄alkyl);
R^{b} is selected from the group consisting of hydrogen, C₁₋₁₀alkyl, benzyl and phenyl,
wherein said phenyl group can either be unsubstituted or substituted with 1-3 substituents independently selected from the group consisting of C₁₋₄alkyl, OH, O(C₁₋₄alkyl), NH₂, NH(C₁₋₄alkyl), N(C₁₋₄alkyl)₂, halo, CN, NO₂, CO₂H, CO₂(C₁₋₄alkyl), C(O)H, and C(O)(C₁₋₄alkyl);
R^{c} is selected from the group consisting of hydrogen, C₁₋₁₀alkyl and phenyl, wherein said phenyl group can either be unsubstituted or substituted with 1-3 substituents independently selected from the group consisting of C₁₋₄alkyl, OH, O(C₁₋₄alkyl), NH₂, N(C₁₋₄alkyl)₂ halo, CN, NO₂, CO₂H, CO₂(C₁₋₄alkyl), C(O)H, and C(O)(C₁₋₄ alkyl);
or R^{a} and R^{c}, whether or not on the same atom, can be taken together with any attached and intervening atoms to form a 4-7 membered ring;
R^{d} is selected from the group consisting of NR^{b}R^{c}, OR^{a}, CO₂R^{a}, O(C=O)R^{a}, CN, NR^{c}(C=O)R^{b}, CONR^{a}R^{c}, SO₂NR^{a}R^{c}, and a 4-7 membered N-heterocycloalkyl ring that can be optionally interrupted by O, S, NR^{c}, or C=O;
R^{e} is selected from the group consisting of hydrogen, C₁₋₆alkyl, C₂₋₆alkenyl, CF₃, halo, O(C₁₋₄alkyl), NH₂, NH(C₁₋₄alkyl), and N(C₁₋₄alkyl)₂;
R^{f} is selected from the group consisting of hydrogen, C₁₋₆alkyl, C₂₋₆alkenyl, CF₃, halo, O(C₁₋₄alkyl), NO₂, NH₂, NH(C₁₋₄alkyl), and N(C₁₋₄alkyl)₂;
L is selected from the group consisting of CR^{b}R^{c}, C₂₋₆ alkylene and C₂₋₆alkenylene, wherein said alkylene and alkenylene groups can be optionally interrupted by O, S, or NR^{c};
M is selected from the group consisting of O, S, NR^{c} C=O, O(C=O), (C=O)O, NR^{c}(C=O) or (C=O)NR^{c};
and the pharmaceutically acceptable salts thereof.

2. A compound according to Claim 1 wherein
X is selected from the group consisting of O and N-OR^{a};
Y is selected from the group consisting of N and CH;
Z is selected from the group consisting of N and CR^{f};
R¹ is selected from the group consisting of hydrogen, C₁₋₆alkyl, C₂₋₆alkenyl, and C₂₋₆alkynyl, wherein said alkyl, alkenyl and alkynyl groups are either unsubstituted or substituted with a group selected from OR^{c} or C(=O)R^{c};
R² is selected from the group consisting of hydrogen, hydroxy, iodo, C₁₋₆alkyl, C₂₋₆alkenyl, and C₂₋₆alkynyl, wherein said alkyl, alkenyl and alkynyl groups are either unsubstituted or substituted with a group selected from OR^{c} or C(=O)R^{c};
R³ is selected from the group consisting of hydrogen, chloro, bromo, iodo, C_{1-10 0}alkyl, C₂₋₁₀alkenyl, C₃₋₇cycloalkyl, aryl and heteroaryl, wherein said alkyl, alkenyl, cycloalkyl, aryl and heteroaryl groups are either unsubstituted or independently substituted with 1, 2 or 3 groups selected from fluoro, chloro, bromo, cyano, OR^{a}, NR^{a}R^{c}, C(=O)R^{a}, CO₂R^{a}, CONR^{a}R^{c}, SR^{a}, NR^{a}(C=O)R^{c}, LR^{d}, and MLR^{d} ;
R⁴ is selected from the group consisting of hydrogen, hydroxy, methyl, fluoro and chloro;
R⁵ is selected from the group consisting of hydrogen, hydroxy, fluoro and chloro;
R⁶ is selected from the group consisting of hydrogen, (C=O)R^{a} and C(=O)OR^{a};
R⁷ and R⁸ are each independently selected from the group consisting of hydrogen and C₁₋₆alkyl;
or R⁷ and R⁸, when taken together with the carbon atom to which they are attached, form a carbonyl group;
R⁹ is selected from the group consisting of hydrogen, C₁₋₁₀alkyl, C₂₋₁₀alkenyl, C₃₋₆cycloalkyl and cycloalkylalkyl, wherein said alkyl, alkenyl, cycloalkyl and cycloalkylalkyl groups can be optionally substituted with a group selected from chloro, OR^{b}, SR^{b} or 1-5 fluoro;
or R⁹ and R¹, when taken together with the three intervening carbon atoms to which they are attached, form a 5-6 membered cycloalkyl ring which can be optionally substituted with a group selected from C₁₋₆alkyl, C₂₋₆alkenyl, and C₃₋₆cycloalkylalkyl, wherein said alkyl, alkenyl, and cycloalkylalkyl groups can be optionally substituted with a group selected from chloro, OR^{b}. SR^{b} or 1-5 fluoro;
R¹⁰ is selected from the group consisting of hydrogen and C₁₋₁₀alkyl;
and the pharmaceutically acceptable salts thereof.

3. A compound according to Claim 2 wherein
X is selected from the group consisting of O and N-OH, N-OCH₃;
Y is selected from the group consisting of N and CH;
Z is selected from the group consisting of N, CH, CF and CCl;
R¹ is selected from the group consisting of hydrogen and C₁₋₃alkyl;
R² is selected from the group consisting of hydrogen, hydroxy, iodo and C₁₋₃alkyl;
R³ is selected from the group consisting of hydrogen, chloro, bromo, iodo, C₁₋₁₀alkyl, C₂₋₁₀alkenyl, C₃₋₇cycloalkyl and aryl, wherein said alkyl, alkenyl, cycloalkyl and aryl groups are either unsubstituted or independently substituted with 1, 2 or 3 groups selected from fluoro, OR^{a}, NR^{a}R^{c}, LR^{d} and MLR^{d} ;
R⁴ is selected from the group consisting of hydrogen, methyl and fluoro;
R⁵ is selected from the group consisting of hydrogen and fluoro;
R⁶ is selected from the group consisting of hydrogen and C(=O)OR^{a};
R⁷ and R⁸ are each independently selected from the group consisting of hydrogen and C₁₋₆alkyl;
R⁹ is selected from the group consisting of C₁₋₁₀alkyl, C₂₋₁₀alkenyl, C₃₋₆cycloalkyl and cycloalkylalkyl;
R¹⁰ is hydrogen;
and the pharmaceutically acceptable salts thereof.

4. A compound according to Claim 3 wherein
X is O and Z is selected from the group consisting of N and CH;
and the pharmaceutically acceptable salts thereof.

5. A compound according to Claim 1 selected from the group consisting of:
9a-ethyl-1,6-dimethyl-8,9,9a,10-tetrahydroindeno[2,1-*e*]indol-7(3*H*)-one;
9a-ethyl-6-methyl-8,9,9a,10-tetrahydroindeno[2,1-*e*]indol-7(3*H*)-one;
1-chloro-9a-ethyl-6-methyl-8,9,9a,10-tetrahydroindeno[2,1-*e*]indol-7(3*H*)-one;
9a-ethyl-6-methyl-1-nitro-8,9,9a,10-tetrahydroindeno[2,1-*e*]indol-7(3*H*)-one;
6-acetyl-9a-butyl-4-fluoro-8,9,9a,10-tetrahydroindeno[2,1-*e*]indol-7(3*H*)-one;
6-methyl-9a-propyl-8,9,9a,10-tetrahydroindeno[2,1-*e*]indol-7(3*H*)-one;
9a-ethyl-4-fluoro-6-methyl-8,9,9a,10-tetrahydroindeno[2,1-*e*]indol-7(3*H*)-one;
6,9a-diethyl-4-fluoro-8,9,9a,10-tetrahydroindeno[2,1-*e*]indol-7(3*H*)-one;
9a-butyl-4-fluoro-6-methyl-8,9,9a,10-tetrahydroindeno[2,1-*e*]indol-7(3*H*)-one;
9a-butyl-6-ethyl-4-fluoro-8,9,9a,10-tetrahydroindeno[2,1-*e*]indol-7(3*H*)-one;
6,9a-dimethyl-8,9,9a,10-tetrahydroindeno[2,1-*e*]indazol-7(3*H*)-one;
6-bromo-9a-methyl-8,9,9a,10-tetrahydroindeno[2,1-*e*]indazol-7(3*H*)-one;
9a-ethyl-8,9,9a,10-tetrahydroindeno[2,1-*e*]indazol-7(3*H*)-one;
9a-ethyl-6-methyl-8,9,9a,10-tetrahydroindeno[2,1-*e*]indazol-7(3*H*)-one;
6-bromo-9a-ethyl-8,9,9a,10-tetrahydroindeno[2,1-*e*]indazol-7(3*H*)-one;
9a-ethyl-6-trifluoromethyl-8,9,9a,10-tetrahydroindeno[2,1-*e*]indazol-7(3*H*)-one;
9a-ethyl-6-{4-[2-(1-piperidinyl)ethoxy]phenyl}-8,9,9a,10-tetrahydroindeno[2,1-*e*]indazol-7(3*H*)-one hydrochloride salt;
9a-ethyl-6-(4-hydroxyphenyl)-8,9,9a,10-tetrahydroindeno[2,1-*e*]indazol-7(3*H*)-one;
9a-ethyl-6-vinyl-8,9,9a,10-tetrahydroindeno[2,1-*e*]indazol-7(3*H*)-one;
6,9a-diethyl-8,9,9a,10-tetrahydroindeno[2,1-*e*]indazol-7(3*H*)-one;
6-allyl-9a-ethyl-8,9,9a,10-tetrahydroindeno[2,1-*e*]indazol-7(3*H*)-one;
9a-ethyl-6-isopropyl-8,9,9a,10-tetrahydroindeno[2,1-*e*]indazol-7(3*H*)-one;
6-butyl-9a-ethyl-8,9,9a,10-tetrahydroindeno[2,1-*e*]indazol-7(3*H*)-one;
6-cyclopentyl-9a-ethyl-8,9,9a,10-tetrahydroindeno[2,1-*e*]indazol-7(3*H*)-one;
6-cyano-9a-ethyl-8,9,9a,10-tetrahydroindeno[2,1-*e*]indazol-7(3*H*)-one;
9a-ethyl-6-methoxy-8,9,9a,10-tetrahydroindeno[2,1-*e*]indazol-7(3*H*)-one;
1-chloro-9a-ethyl-6-methyl-8,9,9a,10-tetrahydroindeno[2,1-*e*]indazol-7(3*H*)-one;
1-bromo-9a-ethyl-6-methyl-8,9,9a,10-tetrahydroindeno[2,1-*e*]indazol-7(3*H*)-one;
9a-ethyl-6-methyl-9,9a-dihydroindeno[2,1-*e*]indazole-7,10(3*H*,8*H*)-dione;
10-chloro-9a-ethyl-6-methyl-8,9,9a,10-tetrahydroindeno[2,1-*e*]indazol-7(3*H*)-one;
10-azido-9a-ethyl-6-methyl-8,9,9a,10-tetrahydroindeno[2,1-*e*]indazol-7(3*H*)-one;
6-bromo-9a-ethyl-9,9a-dihydroindeno[2,1-*e*]indazole-7,10(3*H*,8*H*)-dione;
10-amino-9a-ethyl-6-methyl-8,9,9a,10-tetrahydroindeno[2,1-*e*]indazol-7(3*H*)-one;
9a-ethyl-10-methoxy-6-methyl-8,9,9a,10-tetrahydroindeno[2,1-*e*]indazol-7(3*H*)-one;
9a-ethyl-6,10-dimethyl-8,9,9a,10-tetrahydroindeno[2,1-*e*]indazol-7(3*H*)-one;
9a-ethyl-4-fluoro-6-methyl-8,9,9a,10-tetrahydroindeno[2,1-*e*]indazol-7(3*H*)-one;
6,9a-diethyl-4-fluoro-8,9,9a,10-tetrahydroindeno[2,1-*e*]indazol-7(3*H*)-one;
6-bromo-9a-ethyl-4-fluoro-8,9,9a,10-tetrahydroindeno[2,1-*e*]indazol-7(3*H*)-one;
9a-ethyl-4-fluoro-6-trifluoromethyl-8,9,9a,10-tetrahydroindeno[2,1-*e*]indazol-7(3*H*)-one;
6-methyl-9a-propyl-8,9,9a,10-tetrahydroindeno[2,1-*e*]indazol-7(3*H*)-one;
6-bromo-9a-propyl-8,9,9a,10-tetrahydroindeno[2,1-*e*]indazol-7(3*H*)-one;
6-cyano-9a-propyl-8,9,9a,10-tetrahydroindeno[2,1-*e*]indazol-7(3*H*)-one;
6-methyl-9a-propyl-8,9,9a,10-tetrahydroindeno[2,1-*e*]indazol-7(3*H*)-one oxime;
9a-butyl-8,9,9a,10-tetrahydroindeno[2,1-*e*]indazol-7(3*H*)-one;
6-bromo-9a-butyl-8,9,9a,10-tetrahydroindeno [2,1-*e*]indazol-7(3*H*)-one;
9a-butyl-6-trifluoromethyl-8,9,9a,10-tetrahydroindeno[2,1-*e*]indazol-7(3*H*)-one;
9a-butyl-6-methyl-8,9,9a,10-tetrahydroindeno[2,1-*e*]indazol-7(3*H*)-one;
9a-butyl-6-ethyl-8,9,9a,10-tetrahydroindeno[2,1-*e*]indazol-7(3*H*)-one;
9a-(3,3-dimethylbutyl)-6-methyl-8,9,9a,10-tetrahydroindeno[2,1-*e*]indazol-7(3*H*)-one;
9a-butyl-6-ethyl-4-fluoro-8,9,9a,10-tetrahydroindeno[2,1-*e*]indazol-7(3*H*)-one;
6-acetyl-9a-butyl-4-fluoro-8,9,9a,10-tetrahydroindeno[2,1-*e*]indazol-7(3*H*)-one;
9a-butyl-4-fluoro-6-methyl-8,9,9a,10-tetrahydroindeno[2,1-*e*]indazol-7(3*H*)-one;
6-bromo-9a-butyl-4-fluoro-8,9,9a,10-tetrahydroindeno[2,1-*e*]indazol-7(3*H*)-one;
9a-butyl-6-cyano-4-fluoro-8,9,9a,10-tetrahydroindeno[2,1-*e*]indazol-7(3*H*)-one;
9a-butyl-4-fluoro-6-trifluoromethyl-8,9,9a,10-tetrahydroindeno[2,1-*e*]indazol-7(3*H*)-one;
6-methyl-3,9,10,11-tetrahydro-8,10a-methanoazuleno[2,1-*e*]indazol-7(8*H*)-one;
6-ethyl-3,9,10,11-tetrahydro-8,10a-methanoazuleno[2,1-*e*]indazol-7(8*H*)-one;
9a-ethyl-6-methyl-8,9,9a,10-tetrahydrofluoreno[1,2-*d*]imidazol-7(3*H*)-one;
6-bromo-9a-ethyl-8,9,9a,10-tetrahydrofluoreno[1,2-*d*]imidazol-7(3*H*)-one;
6,9a-diethyl-4-fluoro-8,9,9a,10-tetrahydrofluoreno[1,2-*d*]imidazol-7(3*H*)-one;
9a-butyl-6-ethyl-4-fluoro-8,9,9a,10-tetrahydrofluoreno[1,2-*d*]imidazol-7(3*H*)-one;
9a-ethyl-8,9,9a,10-tetrahydrofluoreno[1,2-*d*][1,2,3]triazol-7(3*H*)-one;
9a-ethyl-6-methyl-8,9,9a,10-tetrahydrofluoreno[1,2-*d*][1,2,3]triazol-7(3*H*)-one;
6-allyl-9a-ethyl-8,9,9a,10-tetrahydrofluoreno[1,2-*d*] [1,2,3]triazol-7(3*H*)-one;
9a-ethyl-6-propyl-8,9,9a,10-tetrahydrofluoreno[1,2-*d*][1,2,3]triazol-7(3*H*)-one;
9a-ethyl-6-trifluoromethyl-8,9,9a,10-tetrahydrofluoreno[1,2-*d*][1,2,3]triazol-7(3*H*)-one;
6-bromo-9a-ethyl-8,9,9a,10-tetrahydrofluoreno[1,2-*d*][1,2,3]triazol-7(3*H*)-one;
6,9a-diethyl-8,9,9a,10-tetrahydrofluoreno[1,2-*d*][1,2,3]triazol-7(3*H*)-one;
6-butyl-9a-ethyl-8,9,9a,10-tetrahydrofluoreno[1,2-*d*] [1,2,3]triazol-7(3*H*)-one;
9a-ethyl-6-(4-hydroxyphenyl)-8,9,9a,10-tetrahydrofluoreno[1,2-*d*] [1,2,3]triazol-7(3*H*)-one;
6-bromo-9a-propyl-8,9,9a,10-tetrahydrofluoreno[1,2-*d*][1,2,3]triazol-7(3*H*)-one;
6-methyl-9a-propyl-8,9,9a,10-tetrahydrofluoreno[1,2-*d*][1,2,3]triazol-7(3*H*)-one;
9a-propyl-6-vinyl-8,9,9a,10-tetrahydrofluoreno[1,2-*d*][1,2,3]triazol-7(3*H*)-one;
6-ethyl-9a-propyl-8,9,9a, 1 0-tetrahydrofluoreno[1,2-*d*][1,2,3]triazol-7(3*H*)-one;
6-allyl-9a-propyl-8,9,9a,10-tetrahydrofluoreno[1,2-*d*][1,2,3]triazol-7(3*H*)-one;
6,9a-dipropyl-8,9,9a,10-tetrahydrofluoreno[1,2-*d*][1,2,3]triazol-7(3*H*)-one;
6-bromo-9a-butyl-8,9,9a,10-tetrahydrofluoreno[1,2-*d*][1,2,3]triazol-7(3*H*)-one;
9a-butyl-6-methyl-8,9,9a,10-tetrahydrofluoreno[1,2-*d*][1,2,3]triazol-7(3*H*)-one;
9a-butyl-6-ethyl-8,9,9a,10-tetrahydrofluoreno[1,2-*d*][1,2,3]triazol-7(3*H*)-one;
6-allyl-9a-butyl-8,9,9a,10-tetrahydrofluoreno[1,2-*d*][1,2,3]triazol-7(3*H*)-one;
9a-butyl-6-propyl-8,9,9a,10-tetrahydrofluoreno[1,2-*d*][1,2,3]triazol-7(3*H*)-one;
9a-butyl-6-trifluoromethyl-8,9,9a,10-tetrahydrofluoreno[1,2-*d*][1,2,3]triazol-7(3*H*)-one;
9a-butyl-6-(2-furyl)-8,9,9a,10-tetrahydrofluoreno[1,2-*d*][1,2,3]triazol-7(3*H*)-one;
6,9a-diethyl-4-fluoro-8,9,9a,10-tetrahydrofluoreno[1,2-*d*][,2,3]triazol-7(3*H*)-one;
9a-butyl-6-ethyl-4-fluoro-8,9,9a,10-tetrahydrofluoreno[1,2-*d*][1,2,3]triazol-7(3*H*one;
and the pharmaceutically acceptable salts thereof.

6. A pharmaceutical composition comprising a compound according to Claim 1 and a pharmaceutically acceptable carrier.

7. A pharmaceutical composition made by combining a compound according to Claim 1 and a pharmaceutically acceptable carrier.

8. A process for making a pharmaceutical composition comprising combining a compound according to Claim 1 and a pharmaceutically acceptable carrier.

9. Use of a compound according to Claim 1 for the preparation of a medicament for eliciting an estrogen receptor modulating effect in a mammal in need thereof.

10. Use of a compound according to Claim 1 for the preparation of a medicament for treating or preventing hot flashes in a mammal in need thereof.

## Patentansprüche

1. Eine Verbindung der Formel: 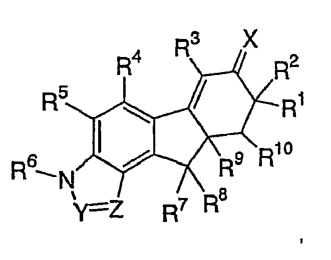 wobei
X ausgewählt ist aus der Gruppe, bestehend aus: O, N-OR^{a}, N-NR^{a}R^{b} und C₁₋₆-Alkyliden, wobei die Alkylidengruppe unsubstituiert oder substituiert ist mit einer Gruppe, ausgewählt aus Hydroxy, Amino, O(C₁₋₄-Alkyl), NH(C₁₋₄-Alkyl) oder N(C₁₋₄-Alkyl)₂,
Y ausgewählt ist aus der Gruppe, bestehend aus N und CR^{e},
Z ausgewählt ist aus der Gruppe, bestehend aus N und CR^{f},
R¹ ausgewählt ist aus der Gruppe, bestehend aus Wasserstoff, C₁₋₆-Alkyl, C₂₋₆-Alkenyl und C₂₋₆₋Alkinyl, wobei die Alkyl-, Alkenyl- und Alkinylgruppen entweder unsubstituiert oder substituiert sind mit einer Gruppe, ausgewählt aus OR^{c}, SR^{c}, NR^{b}R^{c}, C(=O)R^{c}, C(=O)CH₂OH, Brom, 1-3 Chlor, 1-5 Fluor oder Phenyl, wobei die Phenylgruppe entweder unsubstituiert oder substituiert sein kann mit einem Substituenten, ausgewählt aus der Gruppe, bestehend aus C₁₋₄-Alkyl, OH und O(C₁₋₄-Alkyl),
R² ausgewählt ist aus der Gruppe, bestehend aus Wasserstoff, Hydroxy, lod, O(C=O)R`, C(=O)R^{c}, CO₂R^{c}, C₁₋₆-Alkyl, C₂₋₆ Alkenyl und C₂₋₆-Alkinyl, wobei die Alkyl-, Alkenyl- und Alkinylgruppen entweder unsubstituiert oder substituiert sind mit einer Gruppe, ausgewählt aus OR^{c}, SR^{c}, NR^{b}R^{c}, C(=O)R^{c}, C(=O)CH₂OH oder Phenyl, wobei die Phenylgruppe entweder unsubstituiert oder substituiert sein kann mit einem Substituenten, ausgewählt aus der Gruppe, bestehend aus C₁₋₄-Alkyl, OH und O(C₁₋₄-Alkyl),
oder R¹ und R², wenn sie mit dem Kohlenstoffatom, an das sie gebunden sind, zusammengenommen werden, eine Carbonylgruppe bilden,
oder R¹ und R², wenn sie mit dem Kohlenstoffatom, an das sie gebunden sind, zusammengenommen werden, einen C₃₋₇-Cycloalkyl- oder ₃₋₇-Heterocycloalkylring bilden, wobei der Ring entweder unsubstituiert oder substituiert ist mit einer Gruppe, ausgewählt aus C₁₋₄₋Alkyl, OH, O(C₁₋₄-Alkyl) und Oxo,
oder R¹ und R², wenn zusammengenommen, eine C₁₋₆-Alkylidengruppe bilden, wobei die Alkylidengruppe entweder unsubstituiert oder substituiert ist mit einer Gruppe, ausgewählt aus der Gruppe, bestehend aus Hydroxy, O(C₁₋₄-Alkyl), N(C₁₋₄-Alkyl)₂ und Phenyl, wobei die Phenylgruppe entweder unsubstituiert oder substituiert sein kann mit 1-3 Substituenten, unabhängig ausgewählt aus der Gruppe, bestehend aus C₁₋₄-Alkyl, OH, O(C₁₋₄-Alkyl), NH₂, NH(C₁₋₄-Alkyl), N(C₁₋₄-Alkyl)₂, Halogen, CN, NO₂, CO₂H, CO₂(C₁₋₄-Alkyl), C(O)H und C(O)(C₁₋₄-Alkyl),
R³ ausgewählt ist aus der Gruppe, bestehend aus Wasserstoff, Fluor, Chlor, Brom, lod, Cyano, Nitro, NR^{a}R^{c}, OR^{a}, S(O)R^{a}, SO₂R^{a}, SR^{a}, C(=O)R^{a}, CO₂R^{c}, CONR^{a}R^{c}, C₁₋₁₀-Alkyl, C₂₋₁₀₋Alkenyl, C₂₋₁₀-Alkinyl, C₃₋₇-Cycloalkyl, 4-7gliedrigem Heterocycloalkyl, Cycloalkylalkyl, Aryl, Heteroaryl, Arylalkyl und Heteroarylalkyl, wobei die Alkyl-, Alkenyl-, Alkinyl-, Cycloalkyl-, Aryl- und Heteroarylgruppen entweder unsubstituiert oder unabhängig substituiert sind mit 1, 2 oder 3 Gruppen, ausgewählt aus Fluor, Chlor, Brom, lod, Cyano, OR^{a}, NR^{a}R^{c}, O(C=O)R^{a}, O(C=O)NR^{a}R^{c}, NR^{a}(C=O)R^{c}, NR^{a}(C=O)OR^{c}, C(=O)R^{a}, CO₂R^{a}, CONR^{a}R^{c}, CSNR^{a}R^{c}, SR^{a}, S(O)R^{a}, SO₂R^{a}, SO₂NR^{a}R^{c}, LR^{d} und MLR^{d},
R⁴ und R⁵ jeweils unabhängig ausgewählt sind aus der Gruppe, bestehend aus Wasserstoff, Hydroxy, Amino, Methyl, CF₃, Fluor, Chlor und Brom,
R⁶ ausgewählt ist aus der Gruppe, bestehend aus Wasserstoff, (C=O)R^{a}, (C=O)OR^{a} und SO₂R^{a},
R⁷ und R⁸ jeweils unabhängig ausgewählt sind aus der Gruppe, bestehend aus Wasserstoff, C₁₋₆-Alkyl, C₂₋₆-Alkenyl, C₂₋₆-Alkinyl, Fluor, Chlor, Brom, Cyano, Hydroxy, O(C₁₋₆-Alkyl), Azido, Amino, NH(C₁₋₄-Alkyl) und N(C₁₋₄-Alkyl)₂,
oder R⁷ und R⁸, wenn sie mit dem Kohlenstoffatom, an das sie gebunden sind, zusammengenommen werden, einen 3-5gliedrigen Cycloalkylring bilden,
oder R⁷ und R⁸, wenn sie mit dem Kohlenstoffatom, an das sie gebunden sind, zusammengenommen werden, eine Carbonylgruppe bilden,
oder R⁷ und R⁸, wenn zusammengenommen, eine C₁₋₆-Alkylidengruppe bilden, wobei die Alkylidengruppe entweder unsubstituiert oder substituiert ist mit einer Gruppe, ausgewählt aus Cyano, C(=O)H, C(=O)(C₁₋₄-Alkyl) oder C(=O)OC₁₋₄-Alkyl,
R⁹ ausgewählt ist aus der Gruppe, bestehend aus Wasserstoff, C₁₋₁₀-Alkyl, C₂₋₁₀-Alkenyl, C₂₋₁₀₋Alkinyl, C₃₋₆Cycloalkyl, Cycloalkylalkyl, Aryl, Heteroaryl, Arylalkyl und Heteroarylalkyl, wobei die Alkyl-, Alkenyl-, Alkinyl-, Cycloalkyl-, Cycloalkylalkyl-, Aryl-, Heteroaryl-, Arylalkyl- und Heteroarylalkylgruppen gegebenenfalls substituiert sein können mit einer Gruppe, ausgewählt aus Brom, lod, OR^{b}, SR^{b}, C(=O)R^{b}, 1-3 Chlor oder 1-5 Fluor,
oder R⁹ und R¹, wenn sie mit den drei dazwischenliegenden Kohlenstoffatomen, an die sie gebunden sind, zusammengenommen werden, einen 5-6gliedrigen Cycloalkyl- oder Cycloalkenylring bilden, der gegebenenfalls substituiert sein kann mit 1-3 Gruppen, unabhängig ausgewählt aus Oxo, Hydroxy, Fluor, C₁₋₆-Alkyl, C₂₋₆-Alkenyl, C₂₋₁-Alkinyl, C₁₋₆₋Alkylidenyl, C₃₋₆-Cycoalkyl, Cycloalkylalkyl, Phenyl oder Phenylalkyl, wobei die Alkyl-, Alkenyl-, Alkinyl-, Alkylidenyl-, Cycloalkyl-, Cycloalkylalkyl-, Phenyl- und Phenylalkylgruppen gegebenenfalls substituiert sein können mit einer Gruppe, ausgewählt aus Chlor, Brom, lod, OR^{b}, SR^{b}, C₁₋₃ Alkyl, C(=O)R^{b} oder 1-5 Fluor,
oder R⁹ und R⁸, wenn sie mit den zwei dazwischenliegenden Kohlenstoffatomen, an die sie gebunden sind, zusammengenommen werden, einen Cyclopropylring bilden, der gegebenenfalls substituiert sein kann mit 1-2 Gruppen, unabhängig ausgewählt aus C₁₋₆-Alkyl, C₂₋₆₋Alkenyl, C₂₋₆-Alkinyl, C₃₋₆-Cycloalkyl, Cycloalkylalkyl, Phenyl oder Phenylalkyl, wobei die Alkyl-, Alkenyl-, Alkinyl-, Cycloalkyl-, Cycloalkylalkyl-, Phenyl- und Phenylalkylgruppen gegebenenfalls substituiert sein können mit einer Gruppe, ausgewählt aus Chlor, Brom, lod, OR^{b}, SR^{b}, C₁₋₃-Alkyl, C(=O)R^{b} oder 1-5 Fluor,
R¹⁰ ausgewählt ist aus der Gruppe, bestehend aus Wasserstoff, C₁₋₁₀-Alkyl und C₂₋₁₀-Alkenyl,
R^{a} ausgewählt ist aus der Gruppe, bestehend aus Wasserstoff, C₁₋₁₀-Alkyl und Phenyl, wobei die Alkylgruppe gegebenenfalls substituiert sein kann mit einer Gruppe, ausgewählt aus Hydroxy, Amino, O(C₁₋₄-Alkyl), NH(C₁₋₄-Alkyl), N(C₁₋₄-Alkyl)₂, Phenyl oder 1-5 Fluor, und wobei die Phenylgruppen entweder unsubstituiert oder substituiert sein können mit 1-3 Substituenten, unabhängig ausgewählt aus der Gruppe, bestehend aus C₁₋₄-Alkyl, OH, O(C₁₋₄-Alkyl), NH₂, NH(C₁₋₄-Alkyl), N(C₁₋₄-Alkyl)₂, Halogen, CN, NO₂, CO₂H, CO₂(C₁₋₄-Alkyl), C(O)H und C(O)(C₁₋₄-Alkyl),
R^{b} ausgewählt ist aus der Gruppe, bestehend aus Wasserstoff, C₁₋₁₀-Alkyl, Benzyl und Phenyl, wobei die Phenylgruppe entweder unsubstituiert oder substituiert sein kann mit 1-3 Substituenten, unabhängig ausgewählt aus der Gruppe, bestehend aus C₁₋₄-Alkyl, OH, O(C₁₋₄-Alkyl), NH₂, NH(C₁₋₄-Alkyl), N(C₁₋₄-Alkyl)₂, Halogen, CN, NO₂, CO₂H, CO₂(C₁₋₄-Alkyl), C(O)H und C(O)(C₁₋₄-Alkyl),
R^{c} ausgewählt ist aus der Gruppe, bestehend aus Wasserstoff, C₁₋₁₀-Alkyl und Phenyl, wobei die Phenylgruppe entweder unsubstituiert oder substituiert sein kann mit 1-3 Substituenten, unabhängig ausgewählt aus der Gruppe, bestehend aus C₁₋₄Alkyl, OH, O(C_{1,4}-Alkyl), NH₂, NH(C₁₋₄-Alkyl), N(C₁₋₄-Alkyl)₂, Halogen, CN, NO₂, CO₂H, CO₂(C₁₋₄-Alkyl), C(O)H und C(O)(C₁₋₄-Alkyl),
oder R^{a} und R^{c}, egal ob am selben Atom oder nicht, mit beliebigen gebundenen und dazwischenliegenden Atomen zusammengenommen werden kann, um einen 4-7gliedrigen Ring zu bilden,
R^{d} ausgewählt ist aus der Gruppe, bestehend aus NR^{b}R^{c}, OR^{a}, CO₂R^{a}, O(C=O)R^{a}, CN, NR^{c}(C=O)R^{b}, CONR^{a}R^{c}, SO₂NR^{a}R^{c} und einem 4-7gliedrigen N-Heterocycloalkylring, der gegebenenfalls unterbrochen sein kann durch O, S, NR^{c} oder C=O,
R^{e} ausgewählt ist aus der Gruppe, bestehend aus Wasserstoff, C₁₋₆-Alkyl, C₂₋₆-Alkenyl, CF₃, Halogen, O(C₁₋₄-Alkyl), NH₂, NH(C₁₋₄-Alkyl) und N(C₁₋₄-Alkyl)₂,
R^{f} ausgewählt ist aus der Gruppe, bestehend aus Wasserstoff, C₁₋₆-Alkyl, C₂₋₆-Alkenyl, CF₃, Halogen, O(C₁₋₄-Alkyl), NO₂, NH₂, NH(C₁₋₄-Alkyl) und N(C₁₋₄-Alkyl)₂,
L ausgewählt ist aus der Gruppe, bestehend aus CR^{b}R^{c}, C₂₋₆-Alkylen und C₂₋₆-Alkenylen, wobei die Alkylen- und Alkenylengruppen gegebenenfalls unterbrochen sein können durch O, S oder NR^{c},
M ausgewählt ist aus der Gruppe, bestehend aus O, S, NR^{c}, C=O, O(C=O), (C=O)O, ^{Nrc}(C=O) oder (C=O)NR^{c},
und die pharmazeutisch annehmbaren Salze davon.

2. Eine Verbindung gemäß Anspruch 1, wobei
X ausgewählt ist aus der Gruppe, bestehend aus O und N-OR^{a},
Y ausgewählt ist aus der Gruppe, bestehend aus N und CH,
Z ausgewählt ist aus der Gruppe, bestehend aus N und CR^{f},
R¹ ausgewählt ist aus der Gruppe, bestehend aus Wasserstoff, C₁₋₆-Alkyl, C₂₋₆-Alkenyl und C₂₋₆₋Alkinyl, wobei die Alkyl-, Alkenyl- und Alkinylgruppen entweder unsubstituiert oder substituiert sind mit einer Gruppe, ausgewählt aus OR^{c} oder C(=O)R^{c},
R² ausgewählt ist aus der Gruppe, bestehend aus Wasserstoff, Hydroxy, lod, C₁₋₆-Alkyl, C₂₋₆-Alkenyl und C₂₋₆-Alkinyl, wobei die Alkyl-, Alkenyl- und Alkinylgruppen entweder unsubstituiert oder substituiert sind mit einer Gruppe, ausgewählt aus OR^{c} oder C(=O)R^{c},
R³ ausgewählt ist aus der Gruppe, bestehend aus Wasserstoff, Chlor, Brom, lod, C₁₋₁₀-Alkyl, C₂₋₁₀₋Alkenyl, C₃₋₇-Cycloalkyl, Aryl und Heteroaryl, wobei die Alkyl-, Alkenyl-, Cycloalkyl-, Aryl- und Heteroarylgruppen entweder unsubstituiert oder unabhängig substituiert sind mit 1, 2 oder 3 Gruppen, ausgewählt aus Fluor, Chlor, Brom, Cyano, OR^{a}, NR^{a}R^{c}, C(=O)R^{a}, CO₂R^{a}, CONR^{a}R^{c}, SR^{a}, NR^{a}(C=O)R^{c}, LR^{d} und MLR^{d},
R⁴ ausgewählt ist aus der Gruppe, bestehend aus Wasserstoff, Hydroxy, Methyl, Fluor und Chlor,
R⁵ ausgewählt ist aus der Gruppe, bestehend aus Wasserstoff, Hydroxy, Fluor und Chlor,
R⁶ ausgewählt ist aus der Gruppe, bestehend aus Wasserstoff, (C=O)R^{a} und C(=O)OR^{a},
R⁷ und R⁸ jeweils unabhängig ausgewählt sind aus der Gruppe, bestehend aus Wasserstoff und C₁₋₆-Alkyl,
oder R⁷ und R³, wenn sie mit dem Kohlenstoffatom, an das sie gebunden sind, zusammengenommen werden, eine Carbonylgruppe bilden,
R⁹ ausgewählt ist aus der Gruppe, bestehend aus Wasserstoff, C₁₋₁₀-Alkyl, C₂₋₁₀-Alkenyl, C₃₋₆₋Cycloalkyl und Cycloalkylalkyl, wobei die Alkyl-, Alkenyl-, Cycloalkyl- und Cycloalkylalkylgruppen gegebenenfalls substituiert sein können mit einer Gruppe, ausgewählt aus Chlor, OR^{b}, SR^{b} oder 1-5 Fluor,
oder R⁹ und R¹, wenn sie mit den drei dazwischenliegenden Kohlenstoffatomen, an die sie gebunden sind, zusammengenommen werden, einen 5-6gliedrigen Cycloalkylring bilden, der gegebenenfalls substituiert sein kann mit einer Gruppe, ausgewählt aus C₁₋₆-Alkyl, C₂₋₆₋Alkenyl und C₃₋₆-Cycloalkylalkyl, wobei die Alkyl-, Alkenyl- und Cycloalkylalkylgruppen gegebenenfalls substituiert sein können mit einer Gruppe, ausgewählt aus Chlor, OR^{b}, SR^{b} oder 1-5 Fluor,
R¹⁰ ausgewählt ist aus der Gruppe, bestehend aus Wasserstoff und C₁₋₁₀-Alkyl,
und die pharmazeutisch annehmbaren Salze davon.

3. Eine Verbindung gemäß Anspruch 2, wobei
X ausgewählt ist aus der Gruppe, bestehend aus O und N-OH, N-OCH₃,
Y ausgewählt ist aus der Gruppe, bestehend aus N und CH,
Z ausgewählt ist aus der Gruppe, bestehend aus N, CH, CF und CCI,
R¹ ausgewählt ist aus der Gruppe, bestehend aus Wasserstoff und C₁₋₃-Alkyl,
R² ausgewählt ist aus der Gruppe, bestehend aus Wasserstoff, Hydroxy, lod und C₁₋₃-Alkyl,
R³ ausgewählt ist aus der Gruppe, bestehend aus Wasserstoff, Chlor, Brom, lod, C₁₋₁₀-Alkyl, C₂₋₁₀₋Alkenyl, C₃₋₇-Cycloalkyl und Aryl, wobei die Alkyl-, Alkenyl-, Cycloalkyl- und Arylgruppen entweder unsubstituiert oder unabhängig substituiert sind mit 1, 2 oder 3 Gruppen, ausgewählt aus Fluor, OR^{a}, NR^{a}R^{c}, LR^{d} und MLR^{d} ,
R⁴ ausgewählt ist aus der Gruppe, bestehend aus Wasserstoff, Methyl und Fluor,
R⁵ ausgewählt ist aus der Gruppe, bestehend aus Wasserstoff und Fluor,
R⁶ ausgewählt ist aus der Gruppe, bestehend aus Wasserstoff und C(=O)OR^{a},
R⁷ und R⁸ jeweils unabhängig ausgewählt sind aus der Gruppe, bestehend aus Wasserstoff und C₁₋₆-Alkyl,
R⁹ ausgewählt ist aus der Gruppe, bestehend aus C₁₋₁₀-Alkyl, C₂₋₁₀-Alkenyl, C₃₋₆-Cycloalkyl und Cycloalkylalkyl,
R¹⁰ Wasserstoff ist,
und die pharmazeutisch annehmbaren Salze davon.

4. Eine Verbindung gemäß Anspruch 3, wobei
X O ist und Z ausgewählt ist aus der Gruppe, bestehend aus N und CH,
und die pharmazeutisch annehmbaren Salze davon.

5. Eine Verbindung gemäß Anspruch 1, ausgewählt aus der Gruppe, bestehend aus:
9a-Ethyl-1,6-dimethyl-8,9,9a,10-tetrahydroindeno[2,1-e]indol-7(3H)-on,
9a-Ethyl-6-m ethyl-8,9,9a,10-tetrahydroindeno[2,1-e]indol-7(3H)-on,
1-Chlor-9a-ethyl-6-methyl-8,9,9a,10-tetrahydroindeno[2,1-e] indol-7(3H)-on,
9a-Ethyl-6-methyl-1-nitro-8,9,9a,10-tetrahydroindeno[2,1-e]indol-7(3H)-on,
6-Acetyl-9a-butyl-4-fluor-8,9,9a,10-tetrahydroindeno[2,1-e]indol-7(3H)-on,
6-Methyl-9a-propyl-8,9,9a,10-tetrahydroindeno[2,1-e]indol-7(3H)-on,
9a-Ethyl-4-fluor-6-methyl-8,9,9a,10-tetrahydroindeno[2,1-e]indol-7(3H)-on,
6,9a-Diethyl-4-fluor-8,9,9a,10-tetrahydroindeno[2,1-e]indol-7(3H)-on,
9a-Butyl-4-fluor-6-methyl-8,9,9a,10-tetrahydroindeno[2,1-e]indol-7(3H)-on,
9a-Butyl-6-ethyl-4-fluor-8,9,9a,10-tetrahydroindeno[2,1-e]indol-7(3H)-on,
6,9a-Dimethyl-8,9,9a,10-tetrahydroindeno[2,1-e]indazol-7(3H)-on,
6-Brom-9-a-methyl-8,9,9a,10-tetrahydroindeno[2,1-e]indazol-7(3H)-on,
9a-Ethyl-8,9,9a,10-tetrahydroindeno[2,1-e]indazol-7(3H)-on,
9a-Ethyl-6-methyl-8,9,9a,10-tetrahydroindeno[2,1-e]indazol-7(3H)-on,
6-Brom-9a-ethyl-8,9,9a,10-tetrahydroindeno[2,1-e] indazol-7(3H)-on,
9a-Ethyl-6-trifluormethyl-8,9,9a,10-tetrahydroindeno[2,1-e]indazol-7(3H)-on,
9a-Ethyl-6-{4-[2-(1-piperidinyl)ethoxy]phenyl)-8,9,9a,10-tetrahydroindeno[2,1-e]indazol-7(3H)-on-Hydrochloridsalz,
9a-Ethyl-6-(4-hydroxyphenyl)-8,9,9a,10-tetrahydroindeno[2,1-e]indazol-7(3H)-on,
9a-Ethyl-6-vinyl-8,9,9a,10-tetrahydroindeno[2,1-*e*]indazol-7(3H)-on,
6,9a-Diethyl-8,9,9a,10-tetrahydroindeno[2,1-e]indazol-7(3H)-on,
6-Allyl-9a-ethyl-8,9,9a,10-tetrahydroindeno[2,1-e]indazol-7(3H)-on,
9a-Ethyl-6-isopropyl-8,9,9a,10-tetrahydroindeno[2,1-e]indazol-7(3H)-on,
6-Butyl-9a-ethyl-8,9,9a,10-tetrahydroindeno[2,1-e]indazol-7(3H)-on,
6-Cyclopentyl-9a-ethyl-8,9,9a,10-tetrahydroindeno[2,1-e]indazol-7(3H)-on,
6-Cyano-9a-ethyl-8,9,9a,10-tetrahydroindeno[2,1-e]indazol-7(3H)-on,
9a-Ethyl-6-methoxy-8,9,9a,10-tetrahydroindeno[2,1-e]indazol-7(3H)-on,
1-Chlor-9a-ethyl-6-methyl-8,9,9a,10-tetrahydroindeno[2,1-e]indazol-7(3H)-on,
1-Brom-9a-ethyl-6-methyl-8,9,9a,10-tetrahydroindeno[2,1-e]indazol-7(3H)-on,
9a-Ethyl-6-methyl-9,9a-dihydroindeno[2,1-e]indazol-7,10(3H,8H)-dion,
10-Chlor-9a-ethyl-6-methyl-8,9,9a,10-tetrahydroindeno[2,1-e] indazol-7(3H)-on,
10-Azido-9a-ethyl-6-methyl-8,9,9a,10-tetrahydroindeno[2,1-e]indazol-7(3H)-on,
6-Brom-9a-ethyl-9,9a-dihydroindeno[2,1-e]indazol-7,10(3H,8H)dion,
10-Amino-9a-ethyl-6-methyl-8,9,9a,10-tetrahydroindeno[2,1-e]indazol-7(3H)-on,
9a-Ethyl-10-methoxy-6-methyl-8,9,9a,10-tetrahydroindeno[2,1-e]indazol-7(3H)-on,
9a-Ethyl-6,10-dimethyl-8,9,9a,10-tetrahydroindeno[2,1-e]indazol-7(3H)-on,
9a-Ethyl-4-fluor-6-methyl-8,9,9a,10-tetrahydroindeno[2,1-e] indazol-7(3H)-on,
6,9a-Diethyl-4-fluor-8,9,9a,10-tetrahydroindeno[2,1-e]indazol-7(3H)-on,
6-Brom-9a-ethyl-4-fluor-8,9,9a,10-tetrahydroindeno[2,1-e]indazol-7(3H)-on,
9a-Ethyl-4-fluor-6-trifluormethyl-8,9,9a,10-tetrahydroindeno[2,1-e]indazol-7(3H)-on,
6-Methyl-9a-propyl-8,9,9a,10-tetrahydroindeno[2,1-e]indazol-7(3H)-on,
6-Brom-9a-propyl-8,9,9a,10-tetrahydroindeno[2,1-e] indazol-7(3H)-on,
6-Cyano-9a-propyl-8,9,9a,10-tetrahydroindeno[2,1-e]indazol-7(3H)-on,
6-Methyl-9a-propyl-8,9,9a,10-tetrahydroindeno[2,1-e]indazol-7(3H)-onoxim,
9a-Butyl-8,9,9a,10-tetrahydroindeno[2,1-e]indazol-7(3H)-on,
6-Brom-9a-butyl-8,9,9a,10-tetrahydroindeno[2,1-e]indazol-7(3H)-on,
9a-Butyl-6-trifluormethyl-8,9,9a,10-tetrahydroindeno[2,1-e]indazol-7(3H)-on,
9a-Butyl-6-methyl-8,9,9a,10-tetrahydroindeno[2,1-e]indazol-7(3H)-on,
9a-Butyl-6-ethyl-8,9,9a,10-tetrahydroindeno[2,1-e]indazol-7(3H)-on,
9a-(3,3-Dimethylbutyl)-6-methyl-8,9,9a,10-tetrahydroindeno[2,1-e]indazol-7(3H)-on,
9a-Butyl-6-ethyl-4-fluor-8,9,9a,10-tetrahydroindeno[2,1-e]indazol-7(3H)-on,
6-Acetyl-9a-butyl-4-fluor-8,9,9a,10-tetrahydroindeno[2,1-e]indazol-7(3H)-on,
9a-Butyl-4-fluor-6-methyl-8,9,9a,10-tetrahydroindeno[2,1-e]indazol-7(3H)-on,
6-Brom-9a-butyl-4-fluor-8,9,9a,10-tetrahydroindeno[2,1-e]indazol-7(3H)-on,
9a-Butyi-6-cyano-4-fluor-8,9,9a,10-tetrahydroindeno[2,1-e]indazol-7(3H)-on,
9a-Butyl-4-fluor-6-trifluormethyl-8,9,9a,10-tetrahydroindeno[2,1-e]indazol-7(3H)-on,
6-Methyl-3,9,10,11-tetrahydro-8,10a-methanoazuleno[2,1-e]indazol-7(8H)-on,
6-Ethyl-3,9,10,11-tetrahydro-8,10a-methanoazuleno[2,1-e]indazol-7(8H)-on,
9a-Ethyl-6-methyl-8,9,9a,10-tetrahydrofluoreno[1,2-d]imidazol-7(3H)-on,
6-Brom-9a-ethyl-8,9,9a,10-tetrahydrofluoreno[1,2-d]imidazol-7(3H)-on,
6,9a-Diethyl-4-fluor-8,9,9a,10-tetrahydrofluoreno[1,2-d]imidazol-7(3H)-on,
9a-Butyl-6-ethyl-4-fluor-8,9,9a,10-tetrahydrofluoreno[1,2-d]imidazol-7(3H)-on,
9a-Ethyl-8,9,9a,10-tetrahydrofluoreno[1,2-d][1,2,3]triazol-7(3H)-on,
9a-Ethyl-6-methyl-8,9,9a,10-tetrahydrofluoreno[1,2-d][1,2,3]triazol-7(3H)-on,
6-Allyl-9a-ethyl-8,9,9a,10-tetrahydrofluoreno[1,2-d][1,2,3]triazol-7(3H)-on,
9a-Ethyl-6-propyl-8,9,9a,10-tetrahydrofluoreno[1,2-d][1,2,3]triazol-7(3H)-on,
9a-Ethyl-6-trifluormethyl-8,9,9a,10-tetrahydrofluoreno[1,2-d][1,2,3]triazol-7(3H)-on,
6-Brom-9a-ethyl-8,9,9a,10-tetrahydrofluoreno[1,2-d][1,2,3]triazol-7(3H)-on,
6,9a-Diethyl-8,9,9a,10-tetrahydrofluoreno[1,2-d][1,2,3]triazol-7(3H)-on,
6-Butyl-9a-ethyl-8,9,9a,10-tetrahydrofluoreno[1,2-d][1,2,3]triazol-7(3H)-on,
9a-Ethyl-6-(4-hydroxyphenyl)-8,9,9a,10-tetrahydrofluoreno[1,2-d][1,2,3]triazol-7(3H)-on,
6-Brom-9a-propyl-8,9,9a,10-tetrahydrofluoreno[1,2-d][1,2,3]triazol-7(3H)-on,
6-Methyl-9a-propyl-8,9,9a,10-tetrahydrofluoreno[1,2-d][1,2,3]triazol-7(3H)-on,
9a-Propyl-6-vinyl-8,9,9a,10-tetrahydrofluoreno[1,2-d][1,2,3]triazol-7(3H)-on,
6-Ethyl-9a-propyl-8,9,9a,10-tetrahydrofluoreno[1,2-d][1,2,3]triazol-7(3H)-on,
6-Allyl-9a-propyl-8,9,9a,10-tetrahydrofluoreno[1,2-d][1,2,3]triazol-7(3H)-on,
6,9a-Dipropyl-8,9,9a,10-tetrahydrofluoreno[1,2-d][1,2,3]triazol-7(3H)-on,
6-Brom-9a-butyl-8,9,9a,10-tetrahydrofluoreno[1,2-d][1,2,3]triazol-7(3H)-on,
9a-Butyl-6-methyl-8,9,9a,10-tetrahydrofluoreno[1,2-d][1,2,3]triazol-7(3H)-on,
9a-Butyl-6-ethyl-8,9,9a,10-tetrahydrofluoreno[1,2-d][1,2,3]triazol-7(3H)-on,
6-Allyl-9a-butyl-8,9,9a,10-tetrahydrofluoreno[1,2-d][1,2,3]triazol-7(3H)-on,
9a-Butyl-6-propyl-8,9,9a,10-tetrahydrofluoreno[1,2-d][1,2,3]triazol-7(3H)-on,
9a-Butyl-6-trifluormethyl-8,9,9a,10-tetrahydrofluoreno[1,2-d][1,2,3]triazol-7(3H)-on,
9a-Butyl-6-(2-furyl)-8,9,9a,10-tetrahydrofluoreno[1,2-d][1,2,3]triazol-7(3H)-on,
6,9a-diethyl-4-fluor-8,9,9a,10-tetrahydrofluoreno[1,2-d][1,2,3]triazol-7(3H)-on,
9a-Butyl-6-ethyl-4-fluor-8,9,9a,10-tetrahydrofluoreno[1,2-d][1,2,3]triazol-7(3H)-on,
und die pharmazeutisch annehmbaren Salze davon.

6. Eine pharmazeutische Zusammensetzung, die eine Verbindung gemäß Anspruch 1 und einen pharmazeutisch annehmbaren Träger enthält.

7. Eine pharmazeutische Zusammensetzung, die durch Kombination einer Verbindung gemäß Anspruch 1 und eines pharmazeutisch annehmbaren Trägers erzeugt wird.

8. Ein Verfahren zur Herstellung einer pharmazeutischen Zusammensetzung, umfassend die Kombination einer Verbindung gemäß Anspruch 1 und eines pharmazeutisch annehmbaren Trägers.

9. Verwendung einer Verbindung gemäß Anspruch 1 zur Herstellung eines Medikaments zur Auslösung einer Östrogenrezeptormodulierungswirkung bei einem Säuger, der diese benötigt.

10. Verwendung einer Verbindung gemäß Anspruch 1 zur Herstellung eines Medikaments zur Behandlung oder Prävention von Hitzewallungen bei einem Säuger, der diese benötigt.

## Revendications

1. Composé de formule : 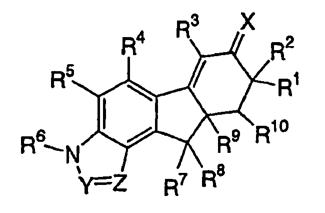 où
X est choisi dans le groupe constitué par : O, N-OR^{a}, N-NR^{a}R^{b} et alkylidène en C_{1-6,} où ledit groupe alkylidène est non-substitué ou substitué par un groupe choisi parmi hydroxy, amino, O(alkyle en C₁₋₄), NH(alkyle en C₁₋₄), ou N(alkyle en C₁₋₄)₂ ;
Y est choisi dans le groupe constitué par N et CR^{e} ;
Z est choisi dans le groupe constitué par N et CR^{f} ;
R¹ est choisi dans le groupe constitué par l'hydrogène, les groupes alkyle en C₁₋₆, alcényle en C₂₋₆ et alcynyle en C₂₋₆, où lesdits groupes alkyle, alcényle, et alcynyle sont non-substitués ou substitués par un groupe choisi parmi OR^{c}, SR^{c}, NR^{b}R^{c}, C(=O)R^{c}, C(=O)CH₂OH, bromo, 1 - 3 chloro, 1 - 5 fluoro ou phényle, où ledit groupe phényle peut être non-substitué ou substitué par un substituant choisi dans le groupe constitué par alkyle en C₁₋₄, OH et O(alkyle en C₁₋₄) ;
R² est choisi dans le groupe constitué par l'hydrogène, les groupes hydroxy, iodo, O(C=O)R^{c}, C(=O)R^{c}, CO₂R^{c}, alkyle en C₁₋₆, alcényle en C₂₋₆, et alcynyle en C₂₋₆, où lesdits groupes alkyle, alcényle et alcynyle peuvent être non-substitués ou substitués par un groupe choisi parmi OR^{c}, SR^{c}, NR^{b}R^{c}, C(=O)R^{c}, C(=O)CH₂OH, ou phényle, où ledit groupe phényle peut être non-substitué ou substitué par un substituant choisi dans le groupe constitué par alkyle en C₁₋₄, OH et O(alkyle en C₁₋₄) ;
ou bien R¹ et R², considérés ensemble avec l'atome de carbone auquel ils sont attachés, forment un groupe carbonyle ;
ou bien R¹ et R², considérés ensemble avec l'atome de carbone auquel ils sont attachés, forment un cycle cycloalkyle en C₃₋₇ ou hétérocycloalkyle de 3 à 7 côtés, où ledit cycle est non-substitué ou substitué par un groupe choisi parmi alkyle en C₁₋₄, OH, O(alkyle en C₁₋₄) et oxo ;
ou bien R¹ et R², considérés ensemble, forment un cycle alkylidène en C₁₋₆, où ledit groupe alkylidène est non-substitué ou substitué par un groupe choisi dans le groupe constitué par les groupes hydroxy, O(alkyle en C₁₋₄), N(alkyle en C₁₋₄)₂, et phényle, où ledit groupe phényle peut être non-substitué ou substitué par 1 à 3 substituants choisis de façon indépendante dans le groupe constitué par alkyle en C₁₋₄, OH, O(alkyle en C₁₋₄), NH₂, NH(alkyle en C₁₋₄), N(alkyle en C₁₋₄)₂, halogéno, CN, NO₂, CO₂H, CO₂(alkyle en C₁₋₄), C(O)H, et C(O)(alkyle en C₁₋₄) ;
R³ est choisi dans le groupe constitué par l'hydrogène, les groupes fluoro, chloro, bromo, iodo, cyano, nitro, NR^{a}R^{c}, OR^{a}, S(O)R^{a}, SO₂R^{a}, SR^{a}, C(=O)R^{a}, CO₂R^{c}, CONR^{a}R^{c}, alkyle en C₁₋₁₀, alcényle en C₂₋₁₀, alcynyle en C₂₋₁₀, cycloalkyle en C₃₋₇, hétérocycloalkyle de 4 à 7 côtés, cycloalkylalkyle, aryle, hétéroaryle, arylalkyle, et hétéroarylalkyle, où lesdits groupes alkyle, alcényle, alcynyle, cycloalkyle, aryle et hétéroaryle peuvent être non-substitués ou substitués, de façon indépendante, par 1, 2 ou 3 groupes choisis parmi fluoro, chloro, bromo, iodo, cyano, OR^{a}, NR^{a}R^{c}, O(C=O)R^{a}, O(C=O)NR^{a}R^{c}, NR^{a}(C=O)R^{c}, NR^{a}(C=O)OR^{c}, C(=O)R^{a}, CO₂R^{a}, CONR^{a}R^{c}, CSNR^{a}R^{c}, SR^{a}, S(O)R^{a}, SO₂R^{a}, SO₂NR^{a}R^{c}, LR^{d}, et MLR^{d} ;
R⁴ et R⁵ sont, chacun de façon indépendante, choisis dans le groupe constitué par l'hydrogène et les groupes hydroxy, amino, méthyle, CF₃, fluoro, chloro, et bromo ;
R⁶ est choisi dans le groupe constitué par l'hydrogène et les groupes (C=O)R^{a}, (C=O)OR^{a}, et SO₂R^{a} ;
R⁷ et R⁸ sont chacun de façon indépendante, choisis dans le groupe constitué par l'hydrogène et les groupes alkyle en C₁₋₆, alcényle en C₂₋₆, alcynyle en C₂₋₆, fluoro, chloro, bromo, cyano, hydroxy, O(alkyle en C₁₋₆), azido, amino, NH(alkyle en C₁₋₄), et N(alkyle en C₁₋₄)₂;
ou bien R⁷ et R⁸, considérés ensemble avec l'atome de carbone auquel ils sont attachés, forment un cycle cycloalkyle de 3 à 5 côtés ;
ou bien R⁷ et R⁸, considérés ensemble avec l'atome de carbone auquel ils sont attachés, forment un groupe carbonyle ;
ou bien R⁷ et R⁸, considérés ensemble, forment un groupe alkylidène en C₁₋₆, où ledit groupe alkylidène est non-substitué ou substitué par un groupe choisi parmi cyano, C(=O)H, C(=O)(alkyle en C₁₋₄), ou C(=O)O(alkyle en C₁₋₄) ;
R⁹ est choisi dans le groupe constitué par l'hydrogène et les groupes alkyle en C₁₋₁₀, alcényle en C₂₋₁₀, alcynyle en C₂₋₁₀, cycloalkyle en C₃₋₆, cycloalkylalkyle, aryle, hétéroaryle, arylalkyle et hétéroarylalkyle, où lesdits groupes alkyle, alcényle, alcynyle, cycloalkyle, cycloalkylalkyle, aryle, hétéroaryle, arylalkyle et hétéroarylalkyle peuvent être substitués de façon optionnelle par un groupe choisi parmi bromo, iodo, OR^{b}, SR^{b}, C(=O)R^{b}, 1-3 chloro, ou 1-5 fluoro ;
ou bien R⁹ et R¹, considérés ensemble avec les trois atomes de carbone intervenants auxquels ils sont attachés, forment un cycle cycloalkyle ou cycloalcényle penta- ou hexagonal qui peut être optionnellement substitué par 1 à 3 groupes choisis de façon indépendante parmi oxo, hydroxy, fluoro, alkyle en C₁₋₆, alcényle en C₂₋₆, alcynyle en C₂₋₆, alkylidényle en C₁₋₆, cycloalkyle en C₃₋₆, cycloalkylalkyle, phényle, ou phénylalkyle, où lesdits groupes alkyle, alcényle, alcynyle, alkylidényle, cycloalkyle, cycloalkylalkyle, phényle, et phénylalkyle peuvent être substitués optionnellement par un groupe choisi parmi chloro, bromo, iodo, OR^{b}, SR^{b}, alkyle en C₁₋₃, C(=O)R^{b}, ou 1 - 5 fluoro ;
ou bien R⁹ et R⁸, considérés ensemble avec les deux atomes de carbone intervenants auxquels ils sont attachés, forment un cycle cyclopropyle, qui peut être substitué de façon optionnelle par 1 ou 2 groupes choisis de façon indépendante parmi alkyle en C₁₋₆, alcényle en C₂₋₆, alcynyle en C₂₋₆, cycloalkyle en C₃₋₆, cycloalkylalkyle, phényle, ou phénylalkyle, où lesdits groupes alkyle, alcényle, alcynyle, cycloalkyle, cycloalkylalkyle, phényle, et phénylalkyle peuvent être optionnellement substitués par un groupe choisi parmi chloro, bromo, iodo, OR^{b}, SR^{b}, alkyle en C₁₋₃, C(=O)R^{b}, ou 1 - 5 fluoro ;
R¹⁰ est choisi dans le groupe constitué par l'hydrogène et les groupes alkyle en C₁₋₁₀ et alcényle en C₂₋₁₀ ;
R^{a} est choisi dans le groupe constitué par l'hydrogène et les groupes alkyle en C₁₋₁₀, et phényle, où ledit groupe alkyle peut être optionnellement substitué par un groupe choisi parmi hydroxy, amino, O(alkyle en C₁₋₄), NH(alkyle en C₁₋₄), N(alkyle en C₁₋₄)₂, phényle, ou 1 - 5 fluoro, et où lesdits groupes phényle peuvent être non-substitués ou substitués par 1 à 3 substituants choisis de manière indépendante dans le groupe constitué par alkyle en C₁₋₄, OH, O(alkyle en C₁₋₄), NH₂, NH(alkyle en C₁₋₄), N(alkyle en C₁₋₄)₂, halogéno, CN, NO₂, CO₂H, CO₂(alkyle en C₁₋₄), C(O)H, et C(O)(alkyle en C₁₋₄) ;
R^{b} est choisi dans le groupe constitué par l'hydrogène et les groupes alkyle en C₁₋₁₀, benzyle et phényle, où ledit groupe phényle peut être non-substitué ou substitué par 1 à 3 substituants choisis de manière indépendante dans le groupe constitué par alkyle en C₁₋₄, OH, O(alkyle en C₁₋₄), NH₂, NH(alkyle en C₁₋₄), N(alkyle en C₁₋₄)₂, halogéno, CN, NO₂, CO₂H, CO₂(alkyle en C₁₋₄), C(O)H, et C(O) (alkyle en C₁₋₄) ;
R^{c} est choisi dans le groupe constitué par l'hydrogène et les groupes alkyle en C₁₋₁₀, et phényle, où ledit groupe phényle peut être non-substitué ou substitué par 1 à 3 substituants choisis de manière indépendante dans le groupe constitué par alkyle en C₁₋₄, OH, O(alkyle en C₁₋₄), NH₂, NH(alkyle en C₁₋₄), N(alkyle en C₁₋₄)₂, halogéno, CN, NO₂, CO₂H, CO₂(alkyle en C₁₋₄), C(O)H, et C(O) (alkyle en C₁₋₄) ;
ou bien R^{a} et R^{c}, qu'ils soient ou non sur le même atome, peuvent former, considérés ensemble, avec des atomes intervenants auxquels ils sont attachés, un cycle de 4 à 7 côtés ;
R^{d} est choisi dans le groupe constitué par NR^{b}R^{c}, OR^{a}, CO₂R^{a}, O(C=O)R^{a}, CN, NR^{c}(C=O)R^{b}, CONR^{a}R^{c}, SO₂NR^{a}R^{c}, et un cycle N-hétérocycloalkyle de 4 à 7 côtés, qui peut être optionnellement interrompu par O, S, NR^{c}, ou C=O ;
R^{e} est choisi dans le groupe constitué par l'hydrogène et les groupes alkyle en C₁₋₆, alcényle en C₂₋₆, CF₃, halogéno, O(alkyle en C₁₋₄), NH₂, NH(alkyle en C₁₋₄), et N(alkyle en C₁₋₄)₂ ;
R^{f} est choisi dans le groupe constitué par l'hydrogène et les groupes alkyle en C₁₋₆, alcényle en C₂₋₆, CF₃, halogéno, O(alkyle en C₁₋₄), NO₂, NH₂, NH(alkyle en C₁₋₄), et N(alkyle en C₁₋₄)₂ ;
L est choisi dans le groupe constitué par CR^{b}R^{c}, alkylène en C₂_ 6 et alcénylène en C₂₋₆, où lesdits groupes alkylène et alcénylène peuvent être optionnellement interrompus par O, S, ou NR^{c} ;
M est choisi dans le groupe constitué par O, S, NR^{c}, C=O, O(C=O), (C=O)O, NR^{c}(C=O) ou (C=O)NR^{c};
et les sels pharmaceutiquement acceptables de celui-ci.

2. Composé selon la revendication 1, dans lequel
X est choisi dans le groupe constitué par O et N-OR^{a} ;
Y est choisi dans le groupe constitué par N et CH ;
Z est choisi dans le groupe constitué par N et CR^{f} ;
R¹ est choisi dans le groupe constitué par l'hydrogène, les groupes alkyle en C₁₋₆, alcényle en C₂₋₆ et alcynyle en C_{2-6,} où lesdits groupes alkyle, alcynyle, et alcynyle sont non-substitués ou substitués par un groupe choisi parmi OR^{c} ou C(=O)R^{c} ;
R² est choisi dans le groupe constitué par l'hydrogène, les groupes hydroxy, iodo, alkyle en C₁₋₆, alcényle en C₂₋₆, et alcynyle en C₂₋₆, où lesdits groupes alkyle, alcényle et alcynyle sont non-substitués ou substitués par un groupe choisi parmi OR^{c} ou C(=O)R^{c} ;
R³ est choisi dans le groupe constitué par l'hydrogène, les groupes chloro, bromo, iodo, alkyle en C₁₋₁₀, alcényle en C₂₋₁₀, cycloalkyle en C₃₋₇, aryle et hétéroaryle, où lesdits groupes alkyle, alcényle, cycloalkyle, aryle et hétéroaryle sont non-substitués ou substitués, de façon indépendante, par 1, 2 ou 3 groupes choisis parmi fluoro, chloro, bromo, cyano, OR^{a}, NR^{a}R^{c}, C(=O)R^{a}, CO₂R^{a}, CONR^{a}R^{c}, SR^{a}, NR^{a}(C=O)R^{c}, LR^{d}, et MLR^{d};
R⁴ est choisi dans le groupe constitué par l'hydrogène et les groupes hydroxy, méthyle, fluoro et chloro ;
R⁵ est choisi dans le groupe constitué par l'hydrogène et les groupes hydroxy, fluoro et chloro ;
R⁶ est choisi dans le groupe constitué par l'hydrogène et les groupes (C=O)R^{a} et (C=O)OR^{a} ;
R⁷ et R⁸ sont chacun de façon indépendante, choisis dans le groupe constitué par l'hydrogène et les groupes alkyle en C₁₋₆ ;
ou bien R⁷ et R⁸, considérés ensemble avec l'atome de carbone auquel ils sont attachés, forment un groupe carbonyle ;
R⁹ est choisi dans le groupe constitué par l'hydrogène et les groupes alkyle en C₁₋₁₀, alcényle en C₂₋₁₀, cycloalkyle en C₃₋₆ et cycloalkylalkyle, où lesdits groupes alkyle, alcényle, cycloalkyle et cycloalkylalkyle peuvent être substitués de façon optionnelle par un groupe choisi parmi chloro, OR^{b}, SR^{b} ou 1 - 5 fluoro ;
ou bien R⁹ et R¹, considérés ensemble avec les trois atomes de carbone intervenants auxquels ils sont attachés, forment un cycle cycloalkyle penta- ou hexagonal qui peut être optionnellement substitué par un groupe choisi parmi alkyle en C₁₋₆, alcényle en C₂₋₆, et cycloalkylalkyle en C₃₋₆, où lesdits groupes alkyle, alcényle et cycloalkylalkyle peuvent être substitués optionnellement par un groupe choisi parmi chloro, OR^{b}, SR^{b}, ou 1 - 5 fluoro ;
R¹⁰ est choisi dans le groupe constitué par l'hydrogène et les groupes alkyle en C₁₋₁₀ ;
et les sels pharmaceutiquement acceptables de celui-ci.

3. Composé selon la revendication 2, dans lequel
X est choisi dans le groupe constitué par O et N-OH, N-OCH₃;
Y est choisi dans le groupe constitué par N et CH ;
Z est choisi dans le groupe constitué par N, CH, CF et CCl ;
R¹ est choisi dans le groupe constitué par l'hydrogène et les groupes alkyle en C₁₋₃ ;
R² est choisi dans le groupe constitué par l'hydrogène et les groupes hydroxy, iodo et alkyle en C₁₋₃ ;
R³ est choisi dans le groupe constitué par l'hydrogène et les groupes chloro, bromo, iodo, alkyle en C₁₋₁₀, alcényle en C₂₋₁₀, cycloalkyle en C₃₋₇ et aryle, où lesdits groupes alkyle, alcényle, cycloalkyle et aryle sont non-substitués ou substitués, de façon indépendante, par 1, 2 ou 3 groupes choisis parmi fluoro, OR^{a}, NR^{a}R^{c}, LR^{d}, et MLR^{d} ;
R⁴ est choisi dans le groupe constitué par l'hydrogène, le méthyle et le fluoro ;
R⁵ est choisi dans le groupe constitué par l'hydrogène et le fluoro ;
R⁶ est choisi dans le groupe constitué par l'hydrogène et (C=O)OR^{a} ;
R⁷ et R⁸ sont chacun de façon indépendante, choisis dans le groupe constitué par l'hydrogène et les groupes alkyle en C₁₋₆;
R⁹ est choisi dans le groupe constitué par les groupes alkyle en C₁₋₁₀, alcényle en C₂₋₁₀, cycloalkyle en C₃₋₆ et cycloalkylalkyle ;
R¹⁰ est un hydrogène ;
et les sels pharmaceutiquement acceptables de celui-ci.

4. Composé selon la revendication 3, dans lequel
X est O et Z est choisi dans le groupe constitué par N et CH ;
et les sels pharmaceutiquement acceptables de celui-ci.

5. Composé selon la revendication 1, choisi dans le groupe constitué par :
la 9a-éthyl-1,6-diméthyl-8,9,9a,10-tétrahydroindéno[2,1-*e*]indol-7(3H)-one ;
la 9a-éthyl-6-méthyl-8,9,9a,10-tétrahydroindéno[2,1-*e*]indol-7(3H)-one ;
la 1-chloro-9a-éthyl-6-méthyl-8,9,9a,10-tétrahydroindéno[2,1-*e*]indol-7(3H)-one ;
la 9a-éthyl-6-méthyl-1-nitro-8,9,9a,10-tétrahydroindéno[2,1-*e*]indol-7(3H)-one ;
la 6-acétyl-9a-butyl-4-fluoro-8,9,9a,10-tétrahydroindéno[2,1-*e*]indol-7(3H)-one ;
la 6-méthyl-9a-propyl-8,9,9a,10-tétrahydroindéno[2,1-*e*]indol-7(3H)-one ;
la 9a-éthyl-4-fluoro-6-méthyl-8,9,9a,10-tétrahydroindéno[2,1-*e*]indol-7(3H)-one ;
la 6,9a-diéthyl-4-fluoro-8,9,9a,10-tétrahydroindéno[2,1-*e*]indol-7(3H)-one ;
la 9a-butyl-4-fluoro-6-méthyl-8,9,9a,10-tétrahydroindéno[2,1-*e*]indol-7(3H)-one ;
la 9a-butyl-6-éthyl-4-fluoro-8,9,9a,10-tétrahydroindéno[2,1-*e*]indol-7(3H)-one ;
la 6,9a-diméthyl-8,9,9a,10-tétrahydroindéno[2,1-*e*]indazol-7(3H)-one ;
la 6-bromo-9a-méthyl-8,9,9a,10-tétrahydroindéno[2,1-*e*]indazol-7(3H)-one ;
la 9a-éthyl-8,9,9a,10-tétrahydroindéno[2,1-*e*]indazol-7(3H)-one ;
la 9a-éthyl-6-méthyl-8,9,9a,10-tétrahydroindéno[2,1-*e*]indazol-7(3H)-one ;
la 6-bromo-9a-éthyl-8,9,9a,10-tétrahydroindéno[2,1-*e*]indazol-7(3H)-one ;
la 9a-éthyl-6-trifluorométhyl-8,9,9a,10-tétrahydroindéno[2,1-*e*]indazol-7(3H)-one ;
le chlorhydrate (sel) de la 9a-éthyl-6-{4-[2-(1-pipéridinyl)éthoxy]phényl}-8,9,9a,10-tétrahydroindéno[2,1*e*]indazol-7(3H)-one ;
la 9a-éthyl-6-(4-hydroxyphényl)-8,9,9a,10-tétrahydroindéno[2,1-*e*]indazol-7(3H)-one ;
la 9a-éthyl-6-vinyl-8,9,9a,10-tétrahydroindéno[2,1-*e*]indazol-7(3H)-one ;
la 6,9a-diéthyl-8,9,9a,10-tétrahydroindéno[2,1-*e*]indazol-7(3H)-one ;
la 6-allyl-9a-éthyl-8,9,9a,10-tétrahydroindéno[2,1-*e*]indazol-7(3H)-one ;
la 9a-éthyl-6-isopropyl-8,9,9a,10-tétrahydroindéno [2,1-*e*]indazol-7(3H)-one ;
la 6-butyl-9a-éthyl-8,9,9a,10-tétrahydroindéno[2,1-*e*]indazol-7(3H)-one ;
la 6-cyclopentyl-9a-éthyl-8,9,9a,10-tétrahydroindéno[2,1-*e*]indazol-7(3H)-one ;
la 6-cyano-9a-éthyl-8,9,9a,10-tétrahydroindéno[2,1-*e*]indazol-7(3H)-one ;
la 9a-éthyl-6-méthoxy-8,9,9a,10-tétrahydroindéno[2,1-*e*]indazol-7(3H)-one ;
la 1-chloro-9a-éthyl-6-méthyl-8,9,9a,10-tétrahydroindéno[2,1-*e*]indazol-7(3H)-one ;
la 1-bromo-9a-éthyl-6-méthyl-8,9,9a,10-tétrahydroindéno[2,1-*e*]indazol-7(3H)-one ;
la 9a-éthyl-6-méthyl-9,9a-dihydroindéno [2,1-*e*] indazole-7,10(3H,8H)-dione ;
la 10-chloro-9a-éthyl-6-méthyl-8,9,9a,10-tétrahydroindéno[2,1-*e*]indazol-7(3H)-one ;
la 10-azido-9a-éthyl-6-méthyl-8,9,9a,10-tétrahydroindéno[2,1-*e*]indazol-7(3H)-one ;
la 6-bromo-9a-éthyl-9,9a-dihydroindéno [2,1-*e*] indazole-7,10(3H,8H)-dione ;
la 10-amino-9a-éthyl-6-méthyl-8,9,9a,10-tétrahydroindéno[2,1-*e*]indazol-7(3H)-one ;
la 9a-éthyl-10-méthoxy-6-méthyl-8,9,9a,10-tétrahydroindéno[2,1-*e*]indazol-7(3H)-one ;
la 9a-éthyl-6,10-diméthyl-8,9,9a,10-tétrahydroindéno[2,1-*e*]indazol-7(3H)-one ;
la 9a-éthyl-4-fluoro-6-méthyl-8,9,9a,10-tétrahydroindéno[2,1-*e*]indazol-7(3H)-one ;
la 6,9a-diéthyl-4-fluoro-8,9,9a,10-tétrahydroindéno[2,1-*e*]indazol-7(3H)-one ;
la 6-bromo-9a-éthyl-4-fluoro-8,9,9a,10-tétrahydroindéno[2,1-*e*]indazol-7(3H)-one ;
la 9a-éthyl-4-fluoro-6-trifluorométhyl-8,9,9a,10-tétrahydroindéno[2,1-*e*]indazol-7(3H)-one ;
la 6-méthyl-9a-propyl-8,9,9a,10-tétrahydroindéno[2,1-*e*]indazol-7(3H)-one ;
la 6-bromo-9a-propyl-8,9,9a,10-tétrahydroindéno[2,1-*e*]indazol-7(3H)-one ;
la 6-cyano-9a-propyl-8,9,9a,10-tétrahydroindéno[2,1-*e*]indazol-7(3H)-one ;
l'oxime de la 6-méthyl-9a-propyl-8,9,9a,10-tétrahydroindéno[2,1-*e*]indazol-7(3H)-one ;
la 9a-butyl-8,9,9a,10-tétrahydroindéno[2,1-*e*]indazol-7(3H)-one
la 6-bromo-9a-butyl-8,9,9a,10-tétrahydroindéno[2,1-*e*]indazol-7(3H)-one ;
la 9a-butyl-6-trifluorométhyl-8,9,9a,10-tétrahydroindéno[2,1-*e*]indazol-7(3H)-one ;
la 9a-butyl-6-méthyl-8,9,9a,10-tétrahydroindéno[2,1-*e*]indazol-7(3H)-one ;
la 9a-butyl-6-éthyl-8,9,9a,10-tétrahydroindéno[2,1-*e*]indazol-7(3H)-one ;
la 9a-(3,3-diméthylbutyl)-6-méthyl-8,9,9a,10-tétrahydroindéno[2,1-*e*]indazol-7(3H)-one ;
la 9a-butyl-6-éthyl-4-fluoro-8,9,9a,10-tétrahydroindéno[2,1-*e*]indazol-7(3H)-one ;
la 6-acétyl-9a-butyl-4-fluoro-8,9,9a,10-tétrahydroindéno[2,1-*e*]indazol-7(3H)-one ;
la 9a-butyl-4-fluoro-6-méthyl-8,9,9a,10-tétrahydroindéno[2,1-*e*]indazol-7(3H)-one ;
la 6-bromo-9a-butyl-4-fluoro-8,9,9a,10-tétrahydroindéno[2,1-*e*]indazol-7(3H)-one ;
la 9a-butyl-6-cyano-4-fluoro-8,9,9a,10-tétrahydroindéno[2,1-*e*]indazol-7(3H)-one ;
la 9a-butyl-4-fluoro-6-trifluorométhyl-8,9,9a,10-tétrahydroindéno[2,1-*e*]indazol-7(3H)one ;
la 6-méthyl-3,9,10,11-tétrahydro-8,10a-méthanoazuléno[2,1-*e*]indazol-7(8H)-one ;
la 6-éthyl-3,9,10,11-tétrahydro-8,10a-méthanoazuléno[2,1-*e*]indazol-7(8H)-one ;
la 9a-éthyl-6-méthyl-8,9,9a,10-tétrahydrofluoréno[1,2-*d*]imidazol-7(3H)-one ;
la 6-bromo-9a-éthyl-8,9,9a,10-tétrahydrofluoréno[1,2-*d*]imidazol-7(3H)-one ;
la 6,9a-diéthyl-4-fluoro-8,9,9a,10-tétrahydrofluoréno[1,2-*d*]imidazol-7(3H)-one ;
la 9a-butyl-6-éthyl-4-fluoro-8,9,9a,10-tétrahydrofluoréno[1,2-*d*]imidazol-7(3H)-one ;
la 9a-éthyl-8,9,9a,10-tétrahydrofluoréno[1,2-*d*][1,2,3]triazol-7(3H)-one ;
la 9a-éthyl-6-méthyl-8,9,9a,10-tétrahydrofluoréno[1,2-*d*][1,2,3]triazol-7(3H)-one ;
la 6-allyl-9a-éthyl-8,9,9a,10-tétrahydrofluoréno[1,2-*d*][1,2,3]triazol-7(3H)-one ;
la 9a-éthyl-6-propyl-8,9,9a,10-tétrahydrofluoréno[1,2-*d*][1,2,3]triazol-7(3H)-one ;
la 9a-éthyl-6-trifluorométhyl-8,9,9a,10-tétrahydrofluoréno[1,2-*d*][1,2,3]triazol-7(3H)one ;
la 6-bromo-9a-éthyl-8,9,9a,10-tétrahydrofluoréno[1,2-*d*][1,2,3]triazol-7(3H)-one ;
la 6,9a-diéthyl-8,9,9a,10-tétrahydrofluoréno[1,2-*d*][1,2,3]triazol-7(3H)-one ;
la 6-butyl-9a-éthyl-8,9,9a,10-tétrahydrofluoréno[1,2-*d*][1,2,3]triazol-7(3H)-one ;
la 9a-éthyl-6-(4-hydroxyphényl)-8,9,9a,10-tétrahydrofluoréno[1,2-*d*][1,2,3]triazol-7(3H)-one ;
la 6-bromo-9a-propyl-8,9,9a,10-tétrahydrofluoréno[1,2-*d*][1,2,3]triazol-7(3H)-one ;
la 6-méthyl-9a-propyl-8,9,9a,10-tétrahydrofluoréno[1,2-*d*][1,2,3]triazol-7(3-one ;
la 9a-propyl-6-vinyl-8,9,9a,10-tétrahydrofluoréno[1,2-*d*][1,2,3]triazol-7(3H)-one ;
la 6-éthyl-9a-propyl-8,9,9a,10-tétrahydrofluoréno[1,2-*d*][1,2,3]triazol-7(3H)-one ;
la 6-allyl-9a-propyl-8,9,9a,10-tétrahydrofluoréno[1,2-*d*][1,2,3]triazol-7(3H)-one ;
la 6,9a-dipropyl-8,9,9a,10-tétrahydrofluoréno[1,2-*d*][1,2,3]triazol-7(3H)-one ;
la 6-bromo-9a-butyl-8,9,9a,10-tétrahydrofluoréno[1,2-*d*][1,2,3]triazol-7(3H)-one ;
la 9a-butyl-6-méthyl-8,9,9a,10-tétrahydrofluoréno[1,2-*d*][1,2,3]triazol-7(3H)-one ;
la 9a-butyl-6-éthyl-8,9,9a,10-tétrahydrofluoréno[1,2-*d*][1,2,3]triazol-7(3H)-one ;
la 6-allyl-9a-butyl-8,9,9a,10-tétrahydrofluoréno[1,2-*d*][1,2,3]triazol-7(3H)-one ;
la 9a-butyl-6-propyl-8,9,9a,10-tétrahydrofluoréno[1,2-*d*][1,2,3]triazol-7(3H)-one ;
la 9a-butyl-6-trifluorométhyl-8,9,9a,10-tétrahydrofluoréno[1,2-*d*][1,2,3]triazol-7(3H)-one ;
la 9a-butyl-6-(2-furyl)-8,9,9a,10-tétrahydrofluoréno[1,2-*d*][1,2,3]triazol-7(3H)-one ;
la 6,9a-diéthyl-4-fluoro-8,9,9a,10-tétrahydrofluoréno[1,2-*d*][1,2,3]triazol-7(3H)-one ;
la 9a-butyl-6-éthyl-4-fluoro-8,9,9a,10-tétrahydrofluoréno[1,2-*d*][1,2,3]triazol-7(3H)one ;
et les sels pharmaceutiquement acceptables de celui-ci.

6. Composition pharmaceutique comprenant un composé selon la revendication 1 et un support pharmaceutiquement acceptable.

7. Composition pharmaceutique fabriquée en combinant un composé selon la revendication 1 et un support pharmaceutiquement acceptable.

8. Procédé pour la fabrication d'une composition pharmaceutique comprenant la combinaison d'un composé selon la revendication 1 et d'un support pharmaceutiquement acceptable.

9. Utilisation d'un composé selon la revendication 1 pour la préparation d'un médicament destiné à provoquer un effet modulateur sur des récepteurs oestrogéniques chez un mammifère en ayant besoin.

10. Utilisation d'un composé selon la revendication 1 pour la préparation d'un médicament pour le traitement ou la prévention des bouffées de chaleur chez un mammifère en ayant besoin.
